# EUROPÄISCHE PATENTANMELDUNG

(11) **EP 2 332 983 A2**
(43) Veröffentlichungstag der Anmeldung: **15.06.2011**
(21) Anmeldenummer: 10009762.5
(22) Anmeldetag: 23.09.2004
(51) Int. Cl.: C07K 14/705, A61K 39/395, C12N 5/10, C07K 14/47, C07K 16/30, C07K 16/32, A61K 48/00

(54) **Identifizierung von tumorassoziierten Zelloberflächen-Antigenen für die Diagnose und Therapie**

(30) Priorität: 26.09.2003 DE 10344799
(62) Teilanmeldung aus: 04786996.1
(71) Anmelder: Ganymed Pharmaceuticals AG, 55131 Mainz (DE)
(72) Erfinder: Türeci, Özlem, 55116 Mainz (DE); Sahin, Ugur, 55116 Mainz (DE); Helftenbein, Gerd, 35329 Gemünden (Felda) (DE); Schlüter, Volker, 82061 Neuried (DE)
(74) Vertreter: Schnappauf, Georg

(57) **Zusammenfassung**

Erfindungsgemäß wurden Tumor-assoziierte Genprodukte und die dafür kodierenden Nukleinsäuren identifiziert. Die vorliegende Erfindung betrifft die Therapie und Diagnose von Erkrankungen, bei denen diese Tumor-assoziierten Genprodukte aberrant exprimiert werden. Des weiteren betrifft die Erfindung Proteine, Polypeptide und Peptide, die Tumor-assoziiert exprimiert werden und die dafür kodierenden Nukleinsäuren.

SEQUENZPROTOKOLL

<110>  Ganymed Pharmaceuticals AG

<120>  Identifizierung von tumorassoziierten Zelloberflächen-Antigenen für die
Diagnose und Therapie

<130>  342-10 EPT15

<150>  DE 103 44 799.7
<151>  2003-09-26

<160>  312

<170>  PatentIn version 3.1

<210>  1
<211>  920
<212>  DNA
<213>  Homo sapiens

<400>  1
tctgtagagg ggaatggctg ctgtgtcatg ggggtgcatg agcagcccag tggagaggtg      60

cacttggtga gaaaccgatg cctctgccaa ccacctgcac taacctgctg ggtctgagac     120

tgagccactt tggaagctga tcttggagca ccagtcaagc ccttagctgg ctgcagccac     180

agccaacaac aagactgcaa cctcctgggg gatcctgagc cagaatcccc tggctaaatt     240

gctccttgat tcttaaccca cagaaattgt gtaagacctc catcaggtgt cgacaaggaa     300

gatcccagta gggcaggaga caggagcacc tctgctgtgg ccaatgcagg aatgctggcc     360

atcattgctt ctgctgggcg actgagaagc atcacccact tccccagaac cttttttacg     420

tggagtgaaa actttaaggg gctgtccagc taaacctcca acctccagat cccatgccaa     480

tttctctgct tctgcaaaag gacttcaagt gaaagacatc tgcagctgtg aacgggggta     540

aaaccctccc tgccccaggc cccaagcaag gatttcccta gcggggagga aggtagaatc     600

gagagacctc taaccctggg agaggaggga gggaaatctc cgaggaccag ggttatgcaa     660

caacacaagg gaagtacctg ctgggttctg ggggttgggg aaggaaaatc cctactgccc     720

caagagccag ccccgaaccc aaggcacagc ttatactggc cccggggcct gggggggcac     780

gaaaaccttg aaaaaggggc gccttcccag cttccccggg ggtaagggct ttacccccca     840

gagggggggg gaaaaatccg agtgggatct ttcccaaccg ccgaagacta aaacctttaa     900

acccccaaag aaaccttcta                                                 920

<210>  2
<211>  88
<212>  PRT
<213>  Homo sapiens

<400>  2

Arg Arg Phe Leu Trp Gly Phe Lys Gly Phe Ser Leu Arg Arg Leu Gly
1               5                   10                  15

Lys Ile Pro Leu Gly Phe Phe Pro Pro Pro Leu Gly Gly Lys Ala Leu
            20                  25                  30

Thr Pro Gly Glu Ala Gly Lys Ala Pro Leu Phe Gln Gly Phe Arg Ala
        35                  40                  45

Pro Pro Gly Pro Gly Ala Ser Ile Ser Cys Ala Leu Gly Ser Gly Leu
    50                  55                  60

Ala Leu Gly Ala Val Gly Ile Phe Leu Pro Gln Pro Pro Glu Pro Ser
65                  70                  75                  80

Arg Tyr Phe Pro Cys Val Val Ala
                85

<210>  3
<211>  22
<212>  DNA
<213>  künstliche Sequenz

<220>
<223>  Oligonukleotid

<400>  3
gcagccacag ccaacaacaa ga                                                22

<210>  4
<211>  25
<212>  DNA
<213>  künstliche Sequenz

<220>
<223>  Oligonukleotid

<400>  4
acagcagagg tgctcctgtc tcctg                                             25

<210>  5
<211>  2856
<212>  DNA
<213>  Homo sapiens

<400>  5
atggccaaaa gaaatctcag cactgtgaca gagttcattc ttgtagtctt cacagatcac      60

cctgaactgg cagttccact cttcctagtg tttctcagtt tctatcttgt cacttttctg     120

gggaatgggg ggatgatcat tctaatccaa gtggatgccc aactccacac ccccgtgtac     180

ttcttcctga gccaccttgc tttcctggat gcctgctgtg cctcagtaat cacccctcag     240

attctggcca cactggccac agacaagaca gttatctcct atggctgccg tgctgtgcag     300

ttctctttct tcaccatatg tgcaggcaca gagtgttacc tgctgtcagt gatggcctat     360

gaccgctttg ttgccattag caatccactg cactgtaaca tgaccatgac tccaggtacc     420

tgcagggtct ttttggccag tgccttcatc tgtggggtgt caggggccat tctgcatacc     480

acgtgcacct tcaccctctc cttctgttgt gacaatcaga tcaacttctt cttctgtgac     540

ctcccacccc tgctgaagct cgcctgcagc agcatgacac aaactgagat tgtcattctc     600

ctttgtgcaa aatgcatgtt cctagccaat gtcatggtta tcctgatctg ctacatgctc 660
attatcagag ccattttgag ggtgaagtcg gcaggaggcc tcctgatagc atctgctcat 720
ttcgatgcat atgtatatga gacaggcatc aactacaaca cagtttatgg ctcaggaaag 780
gcagtagggt ggtcctggag gagcctgcgg gaaaccaacc acatgagacc aggaaatact 840
tcaaaacact cagcagccca gctgcatcaa tgcctcatcc agcaagttgg caggtggccc 900
ttgcagagca tgcccttccc cgtttctgca gggccacctt ataagtcagt gcagcctctc 960
cctggagacc cccggcctct cctgtgcatc accggattat ttctgacttt gaagatgatg 1020
gggtgtgggc ccaggaggcc cagggacagg aagtctgact tcttcataaa cacagaccct 1080
ggtgcagggt caccagaaga acagaggtgt ggatgggaag ggcatccttc ccactcctat 1140
accctggggc tgtctctgcc agtcaacttc ggcctgaaat gtccatggtg gacactatct 1200
ggacccccag ctacctgcca acgtccagac ctgcagacac cttctccacc aaaggagata 1260
tgttcatccg ggctgcgacc ccttacacac agcgctggac cagacagaag tcaagttcca 1320
gcagcctccg gagcagccac tatgctgaca aaggggctgc ccgacatcac tgtgggactg 1380
cagatttatg actcctgcat ctcagggatc caggctctgg ggagcaccct ggccctgctg 1440
tccaatcagc ttccacccac aaccaactat gcttgtggct cccagcaaca tctcctgggc 1500
gtggttggag ggatgacctt cctggagtca gagcccatgt ctgagctgct ctccatctac 1560
agagtccctc agggccaaag actcaccaaa aactttgaag taaaagaact tgtctgcaca 1620
tatctggtag gacagcttcc ttatggcctg gtcagttatg acaacagcaa ctttgagtgg 1680
ctggatcagc agctgcagaa gcagatcggg ggcgagggac ttcctgttgg cgctgcgccc 1740
agccgtgtag ccaggcaaca gtctgatgag gaagctgtgg gaggagtgca gggatacagg 1800
tggtctggat taggggcttc catccaaagt gccagagaag gggcttggca tcgcacaggg 1860
ctggagaaca tgaccactgc ccacctgtct gccttcaaac ttcctgatct aactgccact 1920
taccaagcct acctggcagc caaagccctg tgggttgcct atcagaactt gatgtcctgc 1980
tctgagagag agggaccatt cctgggaggc acgtatgcca atgcatggga agccaggctt 2040
tctcaggtta acttcaccac caaagcccaa gaagaggttt tcttcgccaa agatggggaa 2100
gtgctgacaa cgtttgacat taaaaacatc tatgttctcc cagacctgtc aggacagaca 2160
gccattgttg gacactttga cttcagagca ccttctggaa aagagcttct gttggatgac 2220
agcgcaattg tctgggcaga aggaccctta aagattagag ctgagagaac cctaagaacc 2280
aagaccacac agcacctctc acatcccaag ctccaggagt cccttcctct gtctgcaacg 2340
aaaaacgtcc tgtggaaacc aggaagtcaa ccctatttga gaagtcaaaa tgctgctaca 2400
aaagccttcc ctgacccaga agagaaatcg caatgtcacc agtttctctt tctcccttca 2460
gatagtgttg catgtcagaa gtgctctgac aaccagtggc ccaatgtgca gaagggcgag 2520
tgcatcccca aaacccttga cttcttgttc tatcacaagc cccttgacac agcgttggct 2580
gtctgcacag ccctgctctt tctccttgcc ctggccatct taggcatctt ccatgttgtc 2640

tgctcctgtg tctgggtgtc cttcatacct gcccacatgc atgcccacag caaagacacc 2700

atggccatgg aggtctttgt catcttggca tcagcaggag gcctcatgtc ctccctcttc 2760

ttttccaaat gctacatcat ccttctccat cctgaaaaga acacaaaaga ccaaatgttt 2820

ggccggcatc atcgcaagtg ggaaaaactg aagtga 2856

<210> 6
<211> 951
<212> PRT
<213> Homo sapiens

<400> 6

Met Ala Lys Arg Asn Leu Ser Thr Val Thr Glu Phe Ile Leu Val Val
1               5                   10                  15

Phe Thr Asp His Pro Glu Leu Ala Val Pro Leu Phe Leu Val Phe Leu
            20                  25                  30

Ser Phe Tyr Leu Val Thr Phe Leu Gly Asn Gly Gly Met Ile Ile Leu
        35                  40                  45

Ile Gln Val Asp Ala Gln Leu His Thr Pro Val Tyr Phe Phe Leu Ser
    50                  55                  60

His Leu Ala Phe Leu Asp Ala Cys Cys Ala Ser Val Ile Thr Pro Gln
65                  70                  75                  80

Ile Leu Ala Thr Leu Ala Thr Asp Lys Thr Val Ile Ser Tyr Gly Cys
                85                  90                  95

Arg Ala Val Gln Phe Ser Phe Phe Thr Ile Cys Ala Gly Thr Glu Cys
            100                 105                 110

Tyr Leu Leu Ser Val Met Ala Tyr Asp Arg Phe Val Ala Ile Ser Asn
        115                 120                 125

Pro Leu His Cys Asn Met Thr Met Thr Pro Gly Thr Cys Arg Val Phe
    130                 135                 140

Leu Ala Ser Ala Phe Ile Cys Gly Val Ser Gly Ala Ile Leu His Thr
145                 150                 155                 160

Thr Cys Thr Phe Thr Leu Ser Phe Cys Cys Asp Asn Gln Ile Asn Phe
                165                 170                 175

Phe Phe Cys Asp Leu Pro Pro Leu Leu Lys Leu Ala Cys Ser Ser Met
            180                 185                 190

Thr Gln Thr Glu Ile Val Ile Leu Leu Cys Ala Lys Cys Met Phe Leu
        195                 200                 205

Ala Asn Val Met Val Ile Leu Ile Cys Tyr Met Leu Ile Ile Arg Ala
    210                 215                 220

Ile Leu Arg Val Lys Ser Ala Gly Gly Leu Leu Ile Ala Ser Ala His
225                 230                 235                 240

Phe Asp Ala Tyr Val Tyr Glu Thr Gly Ile Asn Tyr Asn Thr Val Tyr
                245                 250                 255

Gly Ser Gly Lys Ala Val Gly Trp Ser Trp Arg Ser Leu Arg Glu Thr
            260                 265                 270

Asn His Met Arg Pro Gly Asn Thr Ser Lys His Ser Ala Ala Gln Leu
        275                 280                 285

His Gln Cys Leu Ile Gln Gln Val Gly Arg Trp Pro Leu Gln Ser Met
    290                 295                 300

Pro Phe Pro Val Ser Ala Gly Pro Pro Tyr Lys Ser Val Gln Pro Leu
305                 310                 315                 320

Pro Gly Asp Pro Arg Pro Leu Leu Cys Ile Thr Gly Leu Phe Leu Thr
                325                 330                 335

Leu Lys Met Met Gly Cys Gly Pro Arg Arg Pro Arg Asp Arg Lys Ser
            340                 345                 350

Asp Phe Phe Ile Asn Thr Asp Pro Gly Ala Gly Ser Pro Glu Glu Gln
        355                 360                 365

Arg Cys Gly Trp Glu Gly His Pro Ser His Ser Tyr Thr Leu Gly Leu
    370                 375                 380

Ser Leu Pro Val Asn Phe Gly Leu Lys Cys Pro Trp Trp Thr Leu Ser
385                 390                 395                 400

Gly Pro Pro Ala Thr Cys Gln Arg Pro Asp Leu Gln Thr Pro Ser Pro
                405                 410                 415

Pro Lys Glu Ile Cys Ser Ser Gly Leu Arg Pro Leu Thr His Ser Ala
            420                 425                 430

Gly Pro Asp Arg Ser Gln Val Pro Ala Ala Ser Gly Ala Ala Thr Met
        435                 440                 445

Leu Thr Lys Gly Leu Pro Asp Ile Thr Val Gly Leu Gln Ile Tyr Asp
    450                 455                 460

Ser Cys Ile Ser Gly Ile Gln Ala Leu Gly Ser Thr Leu Ala Leu Leu
465                 470                 475                 480

Ser Asn Gln Leu Pro Pro Thr Thr Asn Tyr Ala Cys Gly Ser Gln Gln
                485                 490                 495

His Leu Leu Gly Val Val Gly Gly Met Thr Phe Leu Glu Ser Glu Pro
            500                 505                 510

Met Ser Glu Leu Leu Ser Ile Tyr Arg Val Pro Gln Gly Gln Arg Leu
        515                 520                 525

Thr Lys Asn Phe Glu Val Lys Glu Leu Val Cys Thr Tyr Leu Val Gly
    530                 535                 540

Gln Leu Pro Tyr Gly Leu Val Ser Tyr Asp Asn Ser Asn Phe Glu Trp
545                 550                 555                 560

Leu Asp Gln Gln Leu Gln Lys Gln Ile Gly Gly Glu Gly Leu Pro Val
                565                 570                 575

Gly Ala Ala Pro Ser Arg Val Ala Arg Gln Gln Ser Asp Glu Glu Ala
            580                 585                 590

Val Gly Gly Val Gln Gly Tyr Arg Trp Ser Gly Leu Gly Ala Ser Ile
        595                 600                 605

Gln Ser Ala Arg Glu Gly Ala Trp His Arg Thr Gly Leu Glu Asn Met
    610                 615                 620

Thr Thr Ala His Leu Ser Ala Phe Lys Leu Pro Asp Leu Thr Ala Thr
625                 630                 635                 640

Tyr Gln Ala Tyr Leu Ala Ala Lys Ala Leu Trp Val Ala Tyr Gln Asn
                645                 650                 655

Leu Met Ser Cys Ser Glu Arg Glu Gly Pro Phe Leu Gly Gly Thr Tyr
            660                 665                 670

Ala Asn Ala Trp Glu Ala Arg Leu Ser Gln Val Asn Phe Thr Thr Lys
        675                 680                 685

Ala Gln Glu Glu Val Phe Phe Ala Lys Asp Gly Glu Val Leu Thr Thr
    690                 695                 700

Phe Asp Ile Lys Asn Ile Tyr Val Leu Pro Asp Leu Ser Gly Gln Thr
705                 710                 715                 720

Ala Ile Val Gly His Phe Asp Phe Arg Ala Pro Ser Gly Lys Glu Leu
                725                 730                 735

Leu Leu Asp Asp Ser Ala Ile Val Trp Ala Glu Gly Pro Leu Lys Ile
            740                 745                 750

Arg Ala Glu Arg Thr Leu Arg Thr Lys Thr Thr Gln His Leu Ser His
        755                 760                 765

Pro Lys Leu Gln Glu Ser Leu Pro Leu Ser Ala Thr Lys Asn Val Leu
    770                 775                 780

Trp Lys Pro Gly Ser Gln Pro Tyr Leu Arg Ser Gln Asn Ala Ala Thr
785                 790                 795                 800

Lys Ala Phe Pro Asp Pro Glu Glu Lys Ser Gln Cys His Gln Phe Leu
                805                 810                 815

Phe Leu Pro Ser Asp Ser Val Ala Cys Gln Lys Cys Ser Asp Asn Gln
            820                 825                 830

Trp Pro Asn Val Gln Lys Gly Glu Cys Ile Pro Lys Thr Leu Asp Phe
        835                 840                 845

Leu Phe Tyr His Lys Pro Leu Asp Thr Ala Leu Ala Val Cys Thr Ala
    850                 855                 860

Leu Leu Phe Leu Leu Ala Leu Ala Ile Leu Gly Ile Phe His Val Val
865                 870                 875                 880

Cys Ser Cys Val Trp Val Ser Phe Ile Pro Ala His Met His Ala His
                885                 890                 895

Ser Lys Asp Thr Met Ala Met Glu Val Phe Val Ile Leu Ala Ser Ala
            900                 905                 910

Gly Gly Leu Met Ser Ser Leu Phe Phe Ser Lys Cys Tyr Ile Ile Leu
        915                 920                 925

Leu His Pro Glu Lys Asn Thr Lys Asp Gln Met Phe Gly Arg His His
    930                 935                 940

Arg Lys Trp Glu Lys Leu Lys
945                 950

<210>  7
<211>  22
<212>  DNA
<213>  künstliche Sequenz

<220>
<223>  Oligonukleotid

<400>  7
aggtggtgtg acgctgctgc ta                                                22

<210>  8
<211>  22
<212>  DNA
<213>  künstliche Sequenz

<220>
<223>  Oligonukleotid

<400>  8
tcttcttggg ctttggtggt ga                                                22

<210>  9
<211>  543
<212>  DNA
<213>  Homo sapiens

<400>  9
ataaagcggg acaacacaga acttcccagt tacaccaggc atcctggccc aaagtttccc      60

aaatccaggc ggctagaggc ccactgcttc ccaactacca gctgaggggg tccgtcccga     120

gaagggagaa gaggccgaag aggaaacatg aacttctatt tactcctagc gagcagcatt     180

ctgtgtgcct tgattgtctt ctggaaatat cgccgctttc agagaaacac tggcgaaatg     240

tcatcaaatt caactgctct tgcactagtg agaccctctt cttctgggtt aattaacagc     300

aatacagaca acaatcttgc agtctacgac ctctctcggg atattttaaa taatttccca     360

cactcaatag ccaggcagaa gcgaatattg gtaaacctca gtatggtgga aaacaagctg     420

gttgaactgg aacatactct acttagcaag ggtttcagag gtgcatcacc tcaccggaaa     480

tccacctaaa agcgtacagg atgtaatgcc agtggtggaa atcattaaag acactttgag     540

tag                                                                   543

<210>  10
<211>  113
<212>  PRT
<213>  Homo sapiens

<400>  10

Met Asn Phe Tyr Leu Leu Leu Ala Ser Ser Ile Leu Cys Ala Leu Ile
1               5                   10                  15

Val Phe Trp Lys Tyr Arg Arg Phe Gln Arg Asn Thr Gly Glu Met Ser
            20                  25                  30

Ser Asn Ser Thr Ala Leu Ala Leu Val Arg Pro Ser Ser Ser Gly Leu
        35                  40                  45

Ile Asn Ser Asn Thr Asp Asn Asn Leu Ala Val Tyr Asp Leu Ser Arg
    50                  55                  60

Asp Ile Leu Asn Asn Phe Pro His Ser Ile Ala Arg Gln Lys Arg Ile
65                  70                  75                  80

Leu Val Asn Leu Ser Met Val Glu Asn Lys Leu Val Glu Leu Glu His
                85                  90                  95

Thr Leu Leu Ser Lys Gly Phe Arg Gly Ala Ser Pro His Arg Lys Ser
            100                 105                 110

Thr

<210> 11
<211> 22
<212> DNA
<213> künstliche Sequenz

<220>
<223> Oligonukleotid

<400> 11
gtgtgccttg attgtcttct gg 22

<210> 12
<211> 19
<212> DNA
<213> künstliche Sequenz

<220>
<223> Oligonukleotid

<400> 12
cctggctatt gagtgtggg 19

<210> 13
<211> 2761
<212> DNA
<213> Homo sapiens

<400> 13
ctaggcctca gtctgtctgc atccaggtgc ttattaaaac agtgtgttgc tccacaccgc 60

ctcgtgttgt ctgttggcgc gctctccggg ttccaaccaa tgcaagagcc ttggggctgg 120

ccctgaaacc tgcgaggggc ttccgtccac gtccccagtg gacctaccac ccctccatct 180

gggaaagcag gccacagcag ccggacaaag gaagctcctc agcctctagt cgcctctctg 240

tgcatgcaca tcggtcactg atctcgccta ctggcacaga cgtgtttatc ggccaaactg 300

accctcacaa aaagctacca ccgaagtgga caggccccta cactgtgata ctcagcacac 360

caactgcagt gagagtccga ggactcccca actggatcca tcgcaccagg gtcaagctca 420

cccccaaggc agcttcttcc tccaaaacat taacagctaa gtgtttgtct gggccaattt 480

ctcctaccaa gtttaaatta accaacattt ttttcttaaa accaaaacac aaggaagact 540

aaccacgtgc ttccaggaat ggcctgtatc tacccaacca ctttctatac ctctcttcca 600

accaaaagtc ttaatatggg aatatccctc accacgatcc taatactgtc agtagctgtc 660

ctgctgtcca cagcagcccc tccgagctgc cgtgagtgtt atcagtcttt gcactacaga 720

ggggagatgc aacaatactt tacttaccat actcatatag aaagatcctg ttatggaaac 780

ttaatcgagg aatgtgttga atcaggaaag agttattata aagtaaagaa tctaggagta 840

tgtggcagtc gtaatggggc tatttgcccc agagggaagc agtggctttg cttcaccaaa 900

attggacaat ggggagtaaa cactcaggtg cttgaggaca taaagagaga acagattata 960

gccaaagcca aagcctcaaa accaacaact ccccctgaaa atcgcccgcg gcatttccat 1020
tcctttatac aaaaactata agcagatgca tcccttccta agccaggaaa aaatctgttt 1080
gtagatctag gagaaccatt gtgcttacca tgaatgtgtc caattgttgg gtatgcgggg 1140
gagctttatg agtgaacagt ggctgtggga cgggatagac attccccctt acttacaggc 1200
atcccaaaac cccagactca ctttcactcc tcaggaatgc ccgcagtcct ggacacttac 1260
caacccagta tgagggacgg tgtgcatatc ccgcaagtgg actgataaaa cccatcgcgc 1320
cgtaggtgaa aacccgtcac caaaccctaa cagtcaatgc ctccatagct gagtggtggc 1380
caaggttacc ccctggagcc tggtctcctt ctaacttaag ctacctcaat tgtgtcttgt 1440
caaaaaaggc ctggtactgt acgaacacca ctaaccctta tgccgcatac ctccgcctaa 1500
gtgtactatg cgacaatcct aggaacacca gctgacaatg gactgccact gacggattcc 1560
tgtggatatg gggaacccag gcttactcac agctacctta tcactggcaa ggtacttgct 1620
tcctaggcac aattcaacct ggattctttt tacttccgaa gcaggcgggc aacaccctca 1680
gagtccctgt gtatgataac cagagaaaaa tgatccttgg aggtaggagg gagccaaaga 1740
ttgtgagagg acgagtggcc tctgcaacgg atcattgaat actatggtcc tgccacttgg 1800
gcagaggatg gttcatgggg ttatcgcact cccatatata tgccaaatag agcgattaga 1860
ctacaagctg ttctagagat aatcactaac caaactgcct cagccctaga aatgctcgcg 1920
caacaacaaa accaaatgcg cgcggcaatt tatcaaaaca ggctggccct agactactta 1980
ttagcagaag agggtgcggg ctgtggtaag tttaacatct ccaattgctg tcttaacata 2040
ggcaataatg gagaagaggt tctggaaatc gcttcaaaca tcagaaaagt agcccgtgta 2100
ccagtccaaa cctgggaggg atgggaccca gcaaaccttc taggagggtg gttctctaat 2160
ttaggaggat ttaaaatgct ggtggggaca gtcattttca tcactggggt cctcctgttt 2220
ctcccctgtg gtatcccatt aaaactcttg ttgaaactac agttaacctc ctgacaatcc 2280
agatgatgct cctgctacag cggcacgatg gataccaacc cgtctctcaa gaatacccca 2340
aaaattaagt ttttcttttt ccaaggtgcc cacgccaccc ctatgtcacg cctgaagtag 2400
ttattgagaa agtcgtccct ttcccctttt ctataaccaa atagacagga atggaagatt 2460
ctcctcgggg cctgaaagct tgcgggatga ataactcctc ctcctcaggc ccagtcccaa 2520
ggtacaaact tgcaccagca gcaagatagc agaggcagga agagagctgg ctggaagaca 2580
cgtaccccct gaagatcaag agggaggtcg ccctggtact acatagcagt cacgttaggc 2640
tgggacaatt cctgtttaca gaggactata aaccccctgc cccatcctca cttggggctg 2700
atgccatttt aggcctcagc ctgtctgcat gcaggcgctc attaaaacag catgttgctc 2760
c 2761

<210> 14
<211> 160
<212> PRT
<213> Homo sapiens

<400> 14

Met Ala Cys Ile Tyr Pro Thr Thr Phe Tyr Thr Ser Leu Pro Thr Lys
1               5                   10                  15

Ser Leu Asn Met Gly Ile Ser Leu Thr Thr Ile Leu Ile Leu Ser Val
            20                  25                  30

Ala Val Leu Leu Ser Thr Ala Ala Pro Pro Ser Cys Arg Glu Cys Tyr
        35                  40                  45

Gln Ser Leu His Tyr Arg Gly Glu Met Gln Gln Tyr Phe Thr Tyr His
    50                  55                  60

Thr His Ile Glu Arg Ser Cys Tyr Gly Asn Leu Ile Glu Glu Cys Val
65                  70                  75                  80

Glu Ser Gly Lys Ser Tyr Tyr Lys Val Lys Asn Leu Gly Val Cys Gly
                85                  90                  95

Ser Arg Asn Gly Ala Ile Cys Pro Arg Gly Lys Gln Trp Leu Cys Phe
            100                 105                 110

Thr Lys Ile Gly Gln Trp Gly Val Asn Thr Gln Val Leu Glu Asp Ile
        115                 120                 125

Lys Arg Glu Gln Ile Ile Ala Lys Ala Lys Ala Ser Lys Pro Thr Thr
    130                 135                 140

Pro Pro Glu Asn Arg Pro Arg His Phe His Ser Phe Ile Gln Lys Leu
145                 150                 155                 160

<210> 15
<211> 21
<212> DNA
<213> künstliche Sequenz

<220>
<223> Oligonukleotid

<400> 15
cctctagtcg cctctctgtg c                                                21

<210> 16
<211> 17
<212> DNA
<213> künstliche Sequenz

<220>
<223> Oligonukleotid

<400> 16
accctggtgc gatggat                                                     17

<210> 17
<211> 1635

<212>  DNA
<213>  Homo sapiens

<400>  17
   gcctgtccct gccttaagtg cctactggat cccgggagcc tgggctgggg cctgggcact      60
   gcttcctcct tggcccctca ggcccttgga agcagagaga gaacctcttg cagatcccag     120
   gctcgtcccc agcacagcag acaccaggaa ggtggccaga gcctcactga gccgaaccga     180
   cggccgccca cccacccagg ctggagccat ggataaattc cgcatgctct tccagcactt     240
   ccagtcaagc tcggagtcgg tgatgaatgg catctgcctg ctgctggctg cggtcaccgt     300
   caagctgtac tcctcctttg acttcaactg tccctgcctg gtgcactaca atgcactcta     360
   cggcctgggc ctgctgctga cgcccccgct cgccctgttt ctctgcggcc tcctcgccaa     420
   ccggcagtct gtggtgatgg tcgaggagtg gcgccggccc gcagggcacc ggaggaagga     480
   cccaggcatc atcaggtaca tgtgctcctc tgtgctgcag agggcgctgg ccgcccccct     540
   ggtctggatc ctgctggccc tccttgacgg gaagtgcttc gtgtgtgcct tcagcagctc     600
   tgtggaccct gagaagtttc tggactttgc caacatgacc cccagccagg tacagctctt     660
   cctggccaag gttccctgca aggaggatga gctggtcagg gatagccctg ctcggaaggc     720
   agtgtctcgc tacctgcggt gcctgtcaca ggccatcggc tggagcgtca ccctgctgct     780
   gatcatcgcg gccttcctgg cccgctgcct gaggccctgc ttcgaccaga cagtcttcct     840
   gcagcgcaga tactggagca actacgtgga cctggagcag aagctcttcg acgagacctg     900
   ctgtgagcat gcgcgggact tcgcgcaccg ctgcgtgctg cacttctttg ccagcatgcg     960
   gagtgagctg caggcgcggg ggctgcgccg gggcaatgca ggcaggagac tcgagctccc    1020
   cgcagtgcct gagcccccag aaggcctgga tagtggaagt gggaaggccc acctgcgcgc    1080
   aatctccagc cgggagcagg tggaccgcct cctaagcacg tggtactcca gcaagccgcc    1140
   gctggacctg gctgcatccc ccgggctctg cgggggtggc cttagccacc gcgcccctac    1200
   cttggcactg ggcacgaggc tgtcacaaca caccgacgtg tagggtcctg gccaggcttg    1260
   aagcggcagt gttcgcaggt gaaatgccgc gctgacaaag ttctggagtc tttccaggcc    1320
   gtggggaccc cacggcaggc accctaagtc ttgttagcct cctttttaaa gtagcccaat    1380
   ctctgcctag tttctgggtg tggcctccag cgcgcttcac aaactttaat gtggactcgg    1440
   ttcaccgagg gccttgttaa atacaggttc agacagtgta gccaggaccg agtctgagat    1500
   tctgcatttt aaacaagctc ctggaggctg atgtgctttt ggtcagtgaa ccaaactttg    1560
   agtagcaaga atctaagtaa atctgccatg ggttctgggt tctagatgtc aattctaaat    1620
   aataataatg acctt                                                     1635

<210>  18
<211>  344
<212>  PRT
<213>  Homo sapiens

<400>  18

Met Asp Lys Phe Arg Met Leu Phe Gln His Phe Gln Ser Ser Ser Glu
1               5                   10                  15

Ser Val Met Asn Gly Ile Cys Leu Leu Leu Ala Ala Val Thr Val Lys
            20                  25                  30

Leu Tyr Ser Ser Phe Asp Phe Asn Cys Pro Cys Leu Val His Tyr Asn
        35                  40                  45

Ala Leu Tyr Gly Leu Gly Leu Leu Leu Thr Pro Pro Leu Ala Leu Phe
    50                  55                  60

Leu Cys Gly Leu Leu Ala Asn Arg Gln Ser Val Val Met Val Glu Glu
65                  70                  75                  80

Trp Arg Arg Pro Ala Gly His Arg Arg Lys Asp Pro Gly Ile Ile Arg
                85                  90                  95

Tyr Met Cys Ser Ser Val Leu Gln Arg Ala Leu Ala Ala Pro Leu Val
            100                 105                 110

Trp Ile Leu Leu Ala Leu Leu Asp Gly Lys Cys Phe Val Cys Ala Phe
        115                 120                 125

Ser Ser Ser Val Asp Pro Glu Lys Phe Leu Asp Phe Ala Asn Met Thr
    130                 135                 140

Pro Ser Gln Val Gln Leu Phe Leu Ala Lys Val Pro Cys Lys Glu Asp
145                 150                 155                 160

Glu Leu Val Arg Asp Ser Pro Ala Arg Lys Ala Val Ser Arg Tyr Leu
                165                 170                 175

Arg Cys Leu Ser Gln Ala Ile Gly Trp Ser Val Thr Leu Leu Leu Ile
            180                 185                 190

Ile Ala Ala Phe Leu Ala Arg Cys Leu Arg Pro Cys Phe Asp Gln Thr
        195                 200                 205

Val Phe Leu Gln Arg Arg Tyr Trp Ser Asn Tyr Val Asp Leu Glu Gln
    210                 215                 220

Lys Leu Phe Asp Glu Thr Cys Cys Glu His Ala Arg Asp Phe Ala His
225                 230                 235                 240

Arg Cys Val Leu His Phe Phe Ala Ser Met Arg Ser Glu Leu Gln Ala
                245                 250                 255

Arg Gly Leu Arg Arg Gly Asn Ala Gly Arg Arg Leu Glu Leu Pro Ala
            260                 265                 270

Val Pro Glu Pro Pro Glu Gly Leu Asp Ser Gly Ser Gly Lys Ala His
        275                 280                 285

Leu Arg Ala Ile Ser Ser Arg Glu Gln Val Asp Arg Leu Leu Ser Thr
    290                 295                 300

Trp Tyr Ser Ser Lys Pro Pro Leu Asp Leu Ala Ala Ser Pro Gly Leu
305                 310                 315                 320

Cys Gly Gly Gly Leu Ser His Arg Ala Pro Thr Leu Ala Leu Gly Thr
                325                 330                 335

Arg Leu Ser Gln His Thr Asp Val
            340

<210>  19
<211>  20
<212>  DNA
<213>  künstliche Sequenz

<220>
<223>  Oligonukleotid

<400>  19
gaggaaggac ccaggcatca                                                20

<210>  20
<211>  20
<212>  DNA
<213>  künstliche Sequenz

<220>
<223>  Oligonukleotid

<400>  20
gaaggcacac acgaagcact                                                20

<210>  21
<211>  4556
<212>  DNA
<213>  Homo sapiens

<400>  21
gcggccgccc cattcccaga ccggccgcca gcccatctgg ttagctcccg ccgctccgcg      60

ccgcccggga gtcgggagcc gcggggaacc gggcacctgc acccgcctct gggagtgagt     120

ggttccagct ggtgcctggc ctgtgtctct tggatgccct gtggcttcag tccgtctcct     180

gttgcccacc acctcgtccc tgggccgcct gataccccag cccaacagct aaggtgtgga     240

tggacagtag ggggctggct tctctcactg gtcaggggtc ttctcccctg tctgcctccc     300

ggagctagga ctgcagaggg gcctatcatg gtgcttgcag gccccctggc tgtctcgctg     360

ttgctgccca gcctcacact gctggtgtcc cacctctcca gctcccagga tgtctccagt     420

gagcccagca gtgagcagca gctgtgcgcc cttagcaagc accccaccgt ggcctttgaa     480

gacctgcagc cgtgggtctc taacttcacc taccctggag cccgggattt ctcccagctg     540

gctttggacc cctccgggaa ccagctcatc gtgggagcca ggaactacct cttcagactc 600
agccttgcca atgtctctct tcttcaggcc acagagtggg cctccagtga ggacacgcgc 660
cgctcctgcc aaagcaaagg gaagactgag gaggagtgtc agaactacgt gcgagtcctg 720
atcgtcgccg gccggaaggt gttcatgtgt ggaaccaatg ccttttcccc catgtgcacc 780
agcagacagg tggggaacct cagccggact attgagaaga tcaatggtgt ggcccgctgc 840
ccctatgacc cacgccacaa ctccacagct gtcatctcct cccaggggga gctctatgca 900
gccacggtca tcgacttctc aggtcgggac cctgccatct accgcagcct gggcagtggg 960
ccaccgcttc gcactgccca atataactcc aagtggctta atgagccaaa cttcgtggca 1020
gcctatgata ttgggctgtt tgcatacttc ttcctgcggg agaacgcagt ggagcacgac 1080
tgtggacgca ccgtgtactc tcgcgtggcc cgcgtgtgca agaatgacgt ggggggccga 1140
ttcctgctgg aggacacatg gaccacattc atgaaggccc ggctcaactg ctcccgcccg 1200
ggcgaggtcc ccttctacta taacgagctg cagagtgcct tccacttgcc ggagcaggac 1260
ctcatctatg gagttttcac aaccaacgta aacagcatcg cggcttctgc tgtctgcgcc 1320
ttcaacctca gtgctatctc ccaggctttc aatggcccat ttcgctacca ggagaacccc 1380
agggctgcct ggctccccat agccaacccc atccccaatt tccagtgtgg caccctgcct 1440
gagaccggtc ccaacgagaa cctgacggag cgcagcctgc aggacgcgca gcgcctcttc 1500
ctgatgagcg aggccgtgca gccggtgaca cccgagccct gtgtcaccca ggacagcgtg 1560
cgcttctcac acctcgtggt ggacctggtg caggctaaag acacgctcta ccatgtactc 1620
tacattggca ccgagtcggg caccatcctg aaggcgctgt ccacggcgag ccgcagcctc 1680
cacggctgct acctggagga gctgcacgtg ctgccccccg ggcgccgcga gcccctgcgc 1740
agcctgcgca tcctgcacag cgcccgcgcg ctcttcgtgg ggctgagaga cggcgtcctg 1800
cgggtcccac tggagaggtg cgccgcctac cgcagccagg gggcatgcct gggggcccgg 1860
gacccgtact gtggctggga cgggaagcag caacgttgca gcacactcga ggacagctcc 1920
aacatgagcc tctggaccca gaacatcacc gcctgtcctg tgcggaatgt gacacgggat 1980
gggggcttcg gcccatggtc accatggcaa ccatgtgagc acttggatgg ggacaactca 2040
ggctcttgcc tgtgtcgagc tcgatcctgt gattcccctc gaccccgctg tgggggcctt 2100
gactgcctgg ggccagccat ccacatcgcc aactgctcca ggaatggggc gtggaccccg 2160
tggtcatcgt gggcgctgtg cagcacgtcc tgtggcatcg gcttccaggt ccgccagcga 2220
agttgcagca accctgctcc ccgccacggg ggccgcatct gcgtgggcaa gagccgggag 2280
gaacggttct gtaatgagaa cacgccttgc ccggtgccca tcttctgggc ttcctggggc 2340
tcctggagca agtgcagcag caactgtgga gggggcatgc agtcgcggcg tcgggcctgc 2400
gagaacggca actcctgcct gggctgcggc gtggagttca agacgtgcaa ccccgagggc 2460
tgccccgaag tgcggcgcaa caccccctgg acgccgtggc tgcccgtgaa cgtgacgcag 2520
ggcggggcac ggcaggagca gcggttccgc ttcacctgcc gcgcgcccct tgcagacccg 2580

cacggcctgc agttcggcag gagaaggacc gagacgagga cctgtcccgc ggacggctcc 2640
ggctcctgcg acaccgacgc cctggtggag gtcctcctgc gcagcgggag cacctccccg 2700
cacacggtga gcgggggctg ggccgcctgg ggcccgtggt cgtcctgctc ccgggactgc 2760
gagctgggct tccgcgtccg caagagaacg tgcactaacc cggagccccg caacgggggc 2820
ctgccctgcg tgggcgatgc tgccgagtac caggactgca acccccaggc ttgcccagtt 2880
cggggtgctt ggtcctgctg gacctcatgg tctccatgct cagcttcctg tggtgggggt 2940
cactatcaac gcacccgttc ctgcaccagc cccgcaccct ccccaggtga ggacatctgt 3000
ctcgggctgc acacggagga ggcactatgt gccacacagg cctgcccaga aggctggtcg 3060
ccctggtctg agtggagtaa gtgcactgac gacggagccc agagccgaag ccggcactgt 3120
gaggagctcc tcccagggtc cagcgcctgt gctggaaaca gcagccagag ccgcccctgc 3180
ccctacagcg agattcccgt catcctgcca gcctccagca tggaggaggc caccgactgt 3240
gcagggttca atctcatcca cttggtggcc acgggcatct cctgcttctt gggctctggg 3300
ctcctgaccc tagcagtgta cctgtcttgc cagcactgcc agcgtcagtc ccaggagtcc 3360
acactggtcc atcctgccac ccccaaccat ttgcactaca agggcggagg caccccgaag 3420
aatgaaaagt acacacccat ggaattcaag accctgaaca agaataactt gatccctgat 3480
gacagagcca acttctaccc attgcagcag accaatgtgt acacgactac ttactaccca 3540
agccccctga acaaacacag cttccggccc gaggcctcac ctggacaacg gtgcttcccc 3600
aacagctgat accgccgtcc tggggacttg ggcttcttgc cttcataagg cacagagcag 3660
atggagatgg gacagtggag ccagtttggt tttctccctc tgcactaggc caagaacttg 3720
ctgccttgcc tgtggggggt cccatccggc ttcagagagc tctggctggc attgaccatg 3780
ggggaaaggg ctggtttcag gctgacatat ggccgcaggt ccagttcagc ccaggtctct 3840
catggttatc ttccaaccca ctgtcacgct gacactatgc tgccatgcct gggctgtgga 3900
cctactgggc atttgaggaa ttggagaatg gagatggcaa gagggcaggc ttttaagttt 3960
gggttggaga caacttcctg tggcccccac aagctgagtc tggccttctc cagctggccc 4020
caaaaaaggc ctttgctaca tcctgattat ctctgaaagt aatcaatcaa gtggctccag 4080
tagctctgga ttttctgcca gggctgggcc attgtggtgc tgccccagta tgacatggga 4140
ccaaggccag cgcaggttat ccacctctgc ctggaagtct atactctacc cagggcatcc 4200
ctctggtcag aggcagtgag tactgggaac tggaggctga cctgtgctta gaagtccttt 4260
aatctgggct ggtacaggcc tcagccttgc cctcaatgca cgaaaggtgg cccaggagag 4320
aggatcaatg ccataggagg cagaagtctg gcctctgtgc ctctatggag actatcttcc 4380
agttgctgct caacagagtt gttggctgag acctgcttgg gagtctctgc tggcccttca 4440
tctgttcagg aacacacaca cacacacact cacacacgca cacacaatca caatttgcta 4500
cagcaacaaa aaagacattg ggctgtggca ttattaatta aagatgatat ccagtc 4556

<210> 22

<211>  1151
<212>  PRT
<213>  Homo sapiens

<400>  22

Met Pro Cys Gly Phe Ser Pro Ser Pro Val Ala His His Leu Val Pro
1               5                   10                  15

Gly Pro Pro Asp Thr Pro Ala Gln Gln Leu Arg Cys Gly Trp Thr Val
            20                  25                  30

Gly Gly Trp Leu Leu Ser Leu Val Arg Gly Leu Leu Pro Cys Leu Pro
        35                  40                  45

Pro Gly Ala Arg Thr Ala Glu Gly Pro Ile Met Val Leu Ala Gly Pro
    50                  55                  60

Leu Ala Val Ser Leu Leu Leu Pro Ser Leu Thr Leu Leu Val Ser His
65                  70                  75                  80

Leu Ser Ser Ser Gln Asp Val Ser Ser Glu Pro Ser Ser Glu Gln Gln
                85                  90                  95

Leu Cys Ala Leu Ser Lys His Pro Thr Val Ala Phe Glu Asp Leu Gln
            100                 105                 110

Pro Trp Val Ser Asn Phe Thr Tyr Pro Gly Ala Arg Asp Phe Ser Gln
        115                 120                 125

Leu Ala Leu Asp Pro Ser Gly Asn Gln Leu Ile Val Gly Ala Arg Asn
    130                 135                 140

Tyr Leu Phe Arg Leu Ser Leu Ala Asn Val Ser Leu Leu Gln Ala Thr
145                 150                 155                 160

Glu Trp Ala Ser Ser Glu Asp Thr Arg Arg Ser Cys Gln Ser Lys Gly
                165                 170                 175

Lys Thr Glu Glu Glu Cys Gln Asn Tyr Val Arg Val Leu Ile Val Ala
            180                 185                 190

Gly Arg Lys Val Phe Met Cys Gly Thr Asn Ala Phe Ser Pro Met Cys
        195                 200                 205

Thr Ser Arg Gln Val Gly Asn Leu Ser Arg Thr Ile Glu Lys Ile Asn
    210                 215                 220

Gly Val Ala Arg Cys Pro Tyr Asp Pro Arg His Asn Ser Thr Ala Val
225                 230                 235                 240

Ile Ser Ser Gln Gly Glu Leu Tyr Ala Ala Thr Val Ile Asp Phe Ser
                245                 250                 255

Gly Arg Asp Pro Ala Ile Tyr Arg Ser Leu Gly Ser Gly Pro Pro Leu
            260                 265                 270

Arg Thr Ala Gln Tyr Asn Ser Lys Trp Leu Asn Glu Pro Asn Phe Val
        275                 280                 285

Ala Ala Tyr Asp Ile Gly Leu Phe Ala Tyr Phe Phe Leu Arg Glu Asn
    290                 295                 300

Ala Val Glu His Asp Cys Gly Arg Thr Val Tyr Ser Arg Val Ala Arg
305                 310                 315                 320

Val Cys Lys Asn Asp Val Gly Gly Arg Phe Leu Leu Glu Asp Thr Trp
                325                 330                 335

Thr Thr Phe Met Lys Ala Arg Leu Asn Cys Ser Arg Pro Gly Glu Val
            340                 345                 350

Pro Phe Tyr Tyr Asn Glu Leu Gln Ser Ala Phe His Leu Pro Glu Gln
        355                 360                 365

Asp Leu Ile Tyr Gly Val Phe Thr Thr Asn Val Asn Ser Ile Ala Ala
    370                 375                 380

Ser Ala Val Cys Ala Phe Asn Leu Ser Ala Ile Ser Gln Ala Phe Asn
385                 390                 395                 400

Gly Pro Phe Arg Tyr Gln Glu Asn Pro Arg Ala Ala Trp Leu Pro Ile
                405                 410                 415

Ala Asn Pro Ile Pro Asn Phe Gln Cys Gly Thr Leu Pro Glu Thr Gly
            420                 425                 430

Pro Asn Glu Asn Leu Thr Glu Arg Ser Leu Gln Asp Ala Gln Arg Leu
        435                 440                 445

Phe Leu Met Ser Glu Ala Val Gln Pro Val Thr Pro Glu Pro Cys Val
    450                 455                 460

Thr Gln Asp Ser Val Arg Phe Ser His Leu Val Val Asp Leu Val Gln
465                 470                 475                 480

Ala Lys Asp Thr Leu Tyr His Val Leu Tyr Ile Gly Thr Glu Ser Gly
                485                 490                 495

Thr Ile Leu Lys Ala Leu Ser Thr Ala Ser Arg Ser Leu His Gly Cys
            500                 505                 510

Tyr Leu Glu Glu Leu His Val Leu Pro Pro Gly Arg Arg Glu Pro Leu
        515                 520                 525

Arg Ser Leu Arg Ile Leu His Ser Ala Arg Ala Leu Phe Val Gly Leu
    530                 535                 540

Arg Asp Gly Val Leu Arg Val Pro Leu Glu Arg Cys Ala Ala Tyr Arg
545                 550                 555                 560

Ser Gln Gly Ala Cys Leu Gly Ala Arg Asp Pro Tyr Cys Gly Trp Asp
                565                 570                 575

Gly Lys Gln Gln Arg Cys Ser Thr Leu Glu Asp Ser Ser Asn Met Ser
            580                 585                 590

Leu Trp Thr Gln Asn Ile Thr Ala Cys Pro Val Arg Asn Val Thr Arg
        595                 600                 605

Asp Gly Gly Phe Gly Pro Trp Ser Pro Trp Gln Pro Cys Glu His Leu
    610                 615                 620

Asp Gly Asp Asn Ser Gly Ser Cys Leu Cys Arg Ala Arg Ser Cys Asp
625                 630                 635                 640

Ser Pro Arg Pro Arg Cys Gly Gly Leu Asp Cys Leu Gly Pro Ala Ile
                645                 650                 655

His Ile Ala Asn Cys Ser Arg Asn Gly Ala Trp Thr Pro Trp Ser Ser
            660                 665                 670

Trp Ala Leu Cys Ser Thr Ser Cys Gly Ile Gly Phe Gln Val Arg Gln
        675                 680                 685

Arg Ser Cys Ser Asn Pro Ala Pro Arg His Gly Gly Arg Ile Cys Val
    690                 695                 700

Gly Lys Ser Arg Glu Glu Arg Phe Cys Asn Glu Asn Thr Pro Cys Pro
705                 710                 715                 720

Val Pro Ile Phe Trp Ala Ser Trp Gly Ser Trp Ser Lys Cys Ser Ser
                725                 730                 735

Asn Cys Gly Gly Gly Met Gln Ser Arg Arg Arg Ala Cys Glu Asn Gly
            740                 745                 750

Asn Ser Cys Leu Gly Cys Gly Val Glu Phe Lys Thr Cys Asn Pro Glu
        755                 760                 765

Gly Cys Pro Glu Val Arg Arg Asn Thr Pro Trp Thr Pro Trp Leu Pro
    770                 775                 780

Val Asn Val Thr Gln Gly Gly Ala Arg Gln Glu Gln Arg Phe Arg Phe
785                 790                 795                 800

Thr Cys Arg Ala Pro Leu Ala Asp Pro His Gly Leu Gln Phe Gly Arg
                805                 810                 815

Arg Arg Thr Glu Thr Arg Thr Cys Pro Ala Asp Gly Ser Gly Ser Cys
            820                 825                 830

Asp Thr Asp Ala Leu Val Glu Val Leu Leu Arg Ser Gly Ser Thr Ser
        835                 840                 845

Pro His Thr Val Ser Gly Gly Trp Ala Ala Trp Gly Pro Trp Ser Ser
    850                 855                 860

Cys Ser Arg Asp Cys Glu Leu Gly Phe Arg Val Arg Lys Arg Thr Cys
865                 870                 875                 880

Thr Asn Pro Glu Pro Arg Asn Gly Gly Leu Pro Cys Val Gly Asp Ala
                885                 890                 895

Ala Glu Tyr Gln Asp Cys Asn Pro Gln Ala Cys Pro Val Arg Gly Ala
            900                 905                 910

Trp Ser Cys Trp Thr Ser Trp Ser Pro Cys Ser Ala Ser Cys Gly Gly
        915                 920                 925

Gly His Tyr Gln Arg Thr Arg Ser Cys Thr Ser Pro Ala Pro Ser Pro
    930                 935                 940

Gly Glu Asp Ile Cys Leu Gly Leu His Thr Glu Glu Ala Leu Cys Ala
945                 950                 955                 960

Thr Gln Ala Cys Pro Glu Gly Trp Ser Pro Trp Ser Glu Trp Ser Lys
                965                 970                 975

Cys Thr Asp Asp Gly Ala Gln Ser Arg Ser Arg His Cys Glu Glu Leu
            980                 985                 990

Leu Pro Gly Ser Ser Ala Cys Ala  Gly Asn Ser Ser Gln  Ser Arg Pro
        995                 1000                 1005

Cys Pro  Tyr Ser Glu Ile Pro  Val Ile Leu Pro Ala  Ser Ser Met
    1010                 1015                 1020

Glu Glu  Ala Thr Asp Cys Ala  Gly Phe Asn Leu Ile  His Leu Val
    1025                 1030                 1035

Ala Thr  Gly Ile Ser Cys Phe  Leu Gly Ser Gly Leu  Leu Thr Leu
    1040                 1045                 1050

Ala Val  Tyr Leu Ser Cys Gln  His Cys Gln Arg Gln  Ser Gln Glu
    1055                 1060                 1065

Ser Thr Leu Val His Pro Ala Thr Pro Asn His Leu His Tyr Lys
    1070                1075                1080

Gly Gly Gly Thr Pro Lys Asn Glu Lys Tyr Thr Pro Met Glu Phe
    1085                1090                1095

Lys Thr Leu Asn Lys Asn Asn Leu Ile Pro Asp Asp Arg Ala Asn
    1100                1105                1110

Phe Tyr Pro Leu Gln Gln Thr Asn Val Tyr Thr Thr Thr Tyr Tyr
    1115                1120                1125

Pro Ser Pro Leu Asn Lys His Ser Phe Arg Pro Glu Ala Ser Pro
    1130                1135                1140

Gly Gln Arg Cys Phe Pro Asn Ser
    1145                1150

<210>  23
<211>  21
<212>  DNA
<213>  künstliche Sequenz

<220>
<223>  Oligonukleotid

<400>  23
tgcagcacgt cctgtggcat c                                                21

<210>  24
<211>  21
<212>  DNA
<213>  künstliche Sequenz

<220>
<223>  Oligonukleotid

<400>  24
gttgcacgtc ttgaactcca c                                                21

<210>  25
<211>  1299
<212>  DNA
<213>  Homo sapiens

<400>  25
atgaaattca agctgcttgc tgagtcctat tgccggctgc tgggagccag gagagccctg      60

aggagtagtc actcagtagc agctgacgcg tgggtccacc atgaactgga gtatctttga     120

gggactcctg agtggggtca acaagtactc cacagccttt gggcgcatct ggctgtctct     180

ggtcttcatc ttccgcgtgc tggtgtacct ggtgacggcc gagcgtgtgt ggagtgatga     240

ccacaaggac ttcgactgca atactcgcca gcccggctgc tccaacgtct gctttgatga     300

gttcttccct gtgtcccatg tgcgcctctg ggccctgcag cttatcctgg tgacatgccc     360

ctcactgctc gtggtcatgc acgtggccta ccgggaggtt caggagaaga ggcaccgaga 420
agcccatggg gagaacagtg ggcgcctcta cctgaacccc ggcaagaagc ggggtgggct 480
ctggtggaca tatgtctgca gcctagtgtt caaggcgagc gtggacatcg cctttctcta 540
tgtgttccac tcattctacc ccaaatatat cctccctcct gtggtcaagt gccacgcaga 600
tccatgtccc aatatagtgg actgcttcat ctccaagccc tcagagaaga acattttcac 660
cctcttcatg gtggccacag ctgccatctg catcctgctc aacctcgtgg agctcatcta 720
cctggtgagc aagagatgcc acgagtgcct ggcagcaagg aaagctcaag ccatgtgcac 780
aggtcatcac ccccacggta ccacctcttc ctgcaaacaa gacgacctcc tttcgggtga 840
cctcatcttt ctgggctcag acagtcatcc tcctctctta ccagaccgcc cccgagacca 900
tgtgaagaaa accatcttgt gaggggctgc ctggactggt ctggcaggtt gggcctggat 960
ggggaggctc tagcatctct cataggtgca acctgagagt gggggagcta agccatgagg 1020
taggggcagg caagagagag gattcagacg ctctgggagc cagttcctag tcctcaactc 1080
cagccacctg ccccagctcg acggcactgg gccagttccc cctctgctct gcagctcggt 1140
ttccttttct agaatggaaa tagtgagggc caatgcccag ggttggaggg aggagggcgt 1200
tcatagaaga acacacatgc gggcaccttc atcgtgtgtg gcccactgtc agaacttaat 1260
aaaagtcaac tcatttgctg gaaaaaaaaa aaaaaaaaa 1299

<210> 26
<211> 273
<212> PRT
<213> Homo sapiens

<400> 26

Met Asn Trp Ser Ile Phe Glu Gly Leu Leu Ser Gly Val Asn Lys Tyr
1               5                   10                  15

Ser Thr Ala Phe Gly Arg Ile Trp Leu Ser Leu Val Phe Ile Phe Arg
            20                  25                  30

Val Leu Val Tyr Leu Val Thr Ala Glu Arg Val Trp Ser Asp Asp His
        35                  40                  45

Lys Asp Phe Asp Cys Asn Thr Arg Gln Pro Gly Cys Ser Asn Val Cys
    50                  55                  60

Phe Asp Glu Phe Phe Pro Val Ser His Val Arg Leu Trp Ala Leu Gln
65                  70                  75                  80

Leu Ile Leu Val Thr Cys Pro Ser Leu Leu Val Val Met His Val Ala
                85                  90                  95

Tyr Arg Glu Val Gln Glu Lys Arg His Arg Glu Ala His Gly Glu Asn
            100                 105                 110

Ser Gly Arg Leu Tyr Leu Asn Pro Gly Lys Lys Arg Gly Gly Leu Trp
        115                 120                 125

Trp Thr Tyr Val Cys Ser Leu Val Phe Lys Ala Ser Val Asp Ile Ala
    130                 135                 140

Phe Leu Tyr Val Phe His Ser Phe Tyr Pro Lys Tyr Ile Leu Pro Pro
145                 150                 155                 160

Val Val Lys Cys His Ala Asp Pro Cys Pro Asn Ile Val Asp Cys Phe
                165                 170                 175

Ile Ser Lys Pro Ser Glu Lys Asn Ile Phe Thr Leu Phe Met Val Ala
            180                 185                 190

Thr Ala Ala Ile Cys Ile Leu Leu Asn Leu Val Glu Leu Ile Tyr Leu
        195                 200                 205

Val Ser Lys Arg Cys His Glu Cys Leu Ala Ala Arg Lys Ala Gln Ala
    210                 215                 220

Met Cys Thr Gly His His Pro His Gly Thr Thr Ser Ser Cys Lys Gln
225                 230                 235                 240

Asp Asp Leu Leu Ser Gly Asp Leu Ile Phe Leu Gly Ser Asp Ser His
                245                 250                 255

Pro Pro Leu Leu Pro Asp Arg Pro Arg Asp His Val Lys Lys Thr Ile
            260                 265                 270

Leu

<210>  27
<211>  22
<212>  DNA
<213>  künstliche Sequenz

<220>
<223>  Oligonukleotid

<400>  27
ggagtagtca ctcagtagca gc                                              22

<210>  28
<211>  19
<212>  DNA
<213>  künstliche Sequenz

<220>
<223>  Oligonukleotid

<400>  28
gaactcatca aagcagacg                                                  19

<210> 29
<211> 1528
<212> DNA
<213> Homo sapiens

<400> 29
ggaaggcaca ggcctgagaa gtctgcggct gagctgggag caaatccccc accccctacc 60

tgggggacag ggcaagtgag acctggtgag ggtggctcag caggaaggga aggagaggtg 120

tctgtgcgtc ctgcacccac atctttctct gtcccctcct tgccctgtct ggaggctgct 180

agactcctat cttctgaatt ctatagtgcc tgggtctcag cgcagtgccg atggtggccc 240

gtccttgtgg ttcctctcta cctggggaaa taaggtgcag cggccatggc tacagcaaga 300

cccccctgga tgtgggtgct ctgtgctctg atcacagcct tgcttctggg ggtcacagag 360

catgttctcg ccaacaatga tgtttcctgt gaccacccct ctaacaccgt gccctctggg 420

agcaaccagg acctgggagc tggggccggg gaagacgccc ggtcggatga cagcagcagc 480

cgcatcatca atggatccga ctgcgatatg cacacccagc cgtggcaggc cgcgctgttg 540

ctaaggccca accagctcta ctgcggggcg gtgttggtgc atccacagtg gctgctcacg 600

gccgcccact gcaggaagaa agttttcaga gtccgtctcg gccactactc cctgtcacca 660

gtttatgaat ctgggcagca gatgttccag ggggtcaaat ccatccccca ccctggctac 720

tcccaccctg gccactctaa cgacctcatg ctcatcaaac tgaacagaag aattcgtccc 780

actaaagatg tcagacccat caacgtctcc tctcattgtc cctctgctgg gacaaagtgc 840

ttggtgtctg gctgggggac aaccaagagc ccccaagtgc acttccctaa ggtcctccag 900

tgcttgaata tcagcgtgct aagtcagaaa aggtgcgagg atgcttaccc gagacagata 960

gatgacacca tgttctgcgc cggtgacaaa gcaggtagag actcctgcca gggtgattct 1020

ggggggcctg tggtctgcaa tggctccctg cagggactcg tgtcctgggg agattaccct 1080

tgtgcccggc ccaacagacc gggtgtctac acgaacctct gcaagttcac caagtggatc 1140

caggaaacca tccaggccaa ctcctgagtc atcccaggac tcagcacacc ggcatcccca 1200

cctgctgcag ggacagccct gacactcctt tcagaccctc attccttccc agagatgttg 1260

agaatgttca tctctccagc ccctgacccc atgtctcctg gactcagggt ctgcttcccc 1320

cacattgggc tgaccgtgtc tctctagttg aaccctggga acaatttcca aaactgtcca 1380

gggcgggggt tgcgtctcaa tctccctggg gcactttcat cctcaagctc agggcccatc 1440

ccttctctgc agctctgacc caaatttagt cccagaaata aactgagaag tggaaaaaaa 1500

aaaaaaaaaa aaaaaaaaaa aaaaaaaa 1528

<210> 30
<211> 293
<212> PRT
<213> Homo sapiens

<400> 30

Met Ala Thr Ala Arg Pro Pro Trp Met Trp Val Leu Cys Ala Leu Ile
1               5                   10                  15

Thr Ala Leu Leu Leu Gly Val Thr Glu His Val Leu Ala Asn Asn Asp
            20                      25                      30

Val Ser Cys Asp His Pro Ser Asn Thr Val Pro Ser Gly Ser Asn Gln
        35                      40                      45

Asp Leu Gly Ala Gly Ala Gly Glu Asp Ala Arg Ser Asp Asp Ser Ser
    50                      55                      60

Ser Arg Ile Ile Asn Gly Ser Asp Cys Asp Met His Thr Gln Pro Trp
65                      70                      75                      80

Gln Ala Ala Leu Leu Leu Arg Pro Asn Gln Leu Tyr Cys Gly Ala Val
                85                      90                      95

Leu Val His Pro Gln Trp Leu Leu Thr Ala Ala His Cys Arg Lys Lys
            100                     105                     110

Val Phe Arg Val Arg Leu Gly His Tyr Ser Leu Ser Pro Val Tyr Glu
        115                     120                     125

Ser Gly Gln Gln Met Phe Gln Gly Val Lys Ser Ile Pro His Pro Gly
    130                     135                     140

Tyr Ser His Pro Gly His Ser Asn Asp Leu Met Leu Ile Lys Leu Asn
145                     150                     155                     160

Arg Arg Ile Arg Pro Thr Lys Asp Val Arg Pro Ile Asn Val Ser Ser
                165                     170                     175

His Cys Pro Ser Ala Gly Thr Lys Cys Leu Val Ser Gly Trp Gly Thr
            180                     185                     190

Thr Lys Ser Pro Gln Val His Phe Pro Lys Val Leu Gln Cys Leu Asn
        195                     200                     205

Ile Ser Val Leu Ser Gln Lys Arg Cys Glu Asp Ala Tyr Pro Arg Gln
    210                     215                     220

Ile Asp Asp Thr Met Phe Cys Ala Gly Asp Lys Ala Gly Arg Asp Ser
225                     230                     235                     240

Cys Gln Gly Asp Ser Gly Gly Pro Val Val Cys Asn Gly Ser Leu Gln
                245                     250                     255

Gly Leu Val Ser Trp Gly Asp Tyr Pro Cys Ala Arg Pro Asn Arg Pro
            260                     265                     270

Gly Val Tyr Thr Asn Leu Cys Lys Phe Thr Lys Trp Ile Gln Glu Thr
        275                     280                     285

Ile Gln Ala Asn Ser
    290


<210>  31
<211>  19
<212>  DNA
<213>  künstliche Sequenz

<220>
<223>  Oligonukleotid

<400>  31
cagaaaaggt gcgaggatg                                                    19


<210>  32
<211>  21
<212>  DNA
<213>  künstliche Sequenz

<220>
<223>  Oligonukleotid

<400>  32
ctgggatgac tcaggagttg g                                                 21


<210>  33
<211>  636
<212>  DNA
<213>  Homo sapiens

<400>  33
atgacagaag cagcatcgct tgtccctaag aggccaagga ggctcagagg cagccacaag      60

ctgcgagttc tggcatggcc agtggtcgtg gtggtgaact ttgtttggca gtgcaacggc     120

agcattgctc acaccttcct ggagctaagc ttcgcctgcc ctggaggaag gtacgcaggc     180

agtcgcccag ccccggttgc agggatggac cgcgaccagc agagggcaga aagtgcctgt     240

gtcccccatt ctcgatcccg gggccccaac ctcccatcgg ctcagtcccc cgcccaatct     300

ctgccaggcc cggagctttc ccagacccct cacccacact ccaggctcac tccccgttcc     360

tgggcctggg ccccccttgc acgagtccag ggccagccgt cctcgccttc tgcccgcccc     420

cgtccttcgt tcctgggagc cggccctctc cgcggaccaa gcggccccga gcaggcgccg     480

ccgcccgggg gactccgact cagcccccgc gacctacctc ggccgacagt cgggggttcc     540

caagcggcca ctcccggccg gcgccgtccc ctggcggagc cgccgcgctc cctgccgtcc     600

gcgcagtctg gcctcgctcg gggccactcc tcgtag                               636


<210>  34
<211>  211
<212>  PRT
<213>  Homo sapiens

<400>  34

<210>  35
<211>  21
<212>  DNA
<213>  künstliche Sequenz

<220>
<223>  Oligonukleotid

<400>  35
tgctctcact gtggtcctca g                                                21

<210>  36
<211>  21

<212>  DNA
<213>  künstliche Sequenz

<220>
<223>  Oligonukleotid

<400>  36
tttgtaaagc tccagcgcta c                                                21


<210>  37
<211>  969
<212>  DNA
<213>  Homo sapiens

<400>  37
atgaaggact gtaggaacaa tggcaaggat tgtcaaagtg cccctgcaac acgtaggcac      60

ctcttctctg aagctgccct gcccccttat cgtctttccc aagggcactt cctcacagcc      120

ctggggggcc tcatggcggt gccattcatc ctggccaagg acctgtgcct gcagcaggac      180

cccctgacac agagctacct catcagcacc attttctttg ctccagcatc tgcatgctcc      240

tgcaagctgc ccattcccca gggaggtacg tttgcttttg tggtaatttc tctggccatg      300

ctctcccttc cctcctggaa ttgccctgag tggacactca gtgccagcca ggtgaacacc      360

aactttccag aattcactca gaaatggcag aagaggatcc aagagggtgc tatcatggtc      420

acttcctgtg tccggatgct ggtgggcttc tcaggcctga ctggctttct catgggtttc      480

atctgctcct tggccgttgc tccaactaac tgcctagtgg ccctgcccct cttggattct      540

gcaggcaata atgccgggat ccagtggggg atttctgcca tgtattgctt cgtgttgcgt      600

cttcgcaagg atgagctctg gccatttggt tctccacggc tgcgtttgcc accatcccca      660

ccccgtgatc ggaggcatgt ccccaccccc gtgatcggag gcatgaccct gtttggggtc      720

atcactgccg tggggatctc caatctgcag tacgtggaca tgaacttgtc caggagcctc      780

ttcgcctttg gcttctccat ctactgtggg ctcaccattc ccaaccgggt gagcaaaaac      840

cccgagatgc tccagacagg gattctccag ccggaccagg ttgttcagat gctgctgacc      900

atgggcatgt tcatcagtgg atttctgggt tttcttctag acaacaccat ccccgagctc      960

cttcaataa                                                              969


<210>  38
<211>  322
<212>  PRT
<213>  Homo sapiens

<400>  38

<210> 39
<211> 20
<212> DNA
<213> künstliche Sequenz

<220>
<223> Oligonukleotid

<400> 39
atggcggtgc cattcatcct 20

<210> 40
<211> 20
<212> DNA
<213> künstliche Sequenz

<220>
<223> Oligonukleotid

<400> 40
caggagggaa gggagagcat 20

<210> 41
<211> 1679
<212> DNA
<213> Homo sapiens

<400> 41
gaggaggcgc gcgtcgccgc cccgcgtccc gcctgcggcc cgcgcccccg gcgtcaccgc 60

ctcctgcccg cctgcccgcc tgcccgcctg cccgcctacc cgcctacccg cctacccgcc 120

tacccccctg ccggcctgcc gtccttccac gcggagagcc atggagggag tgagcgcgct 180

gctggcccgc tgccccacgg ccggcctggc cggcggcctg ggggtcacgg cgtgcgccgc 240

ggccggcgtg ttgctctacc ggatcgcgcg gaggatgaag ccaacgcaca cgatggtcaa 300

ctgctggttc tgcaaccagg atacgctggt gccctatggg aaccgcaact gctgggactg 360

tccccactgc gagcagtaca acggcttcca ggagaacggc gactacaaca agccgatccc 420

cgcccagtac ttggagcacc tgaaccacgt ggtgagcagc gcgcccagcc tgcgcgaccc 480

ttcgcagccg cagcagtggg tgagcagcca agtcctgctg tgcaagaggt gcaaccacca 540

ccagaccacc aagatcaagc agctggccgc cttcgctccc cgcgaggagg gcaggtatga 600

cgaggaggtc gaggtgtacc ggcatcacct ggagcagatg tacaagctgt gccggccgtg 660

ccaagcggct gtggagtact acatcaagca ccagaaccgc cagctgcgcg ccctgttgct 720

cagccaccag ttcaagcgcc gggaggccga ccagacccac gcacagaact tctcctccgc 780

cgtgaagtcc ccggtccagg tcatcctgct ccgtgccctc gccttcctgg cctgcgcctt 840

cctactgacc accgcgctgt atggggccag cggacacttc gccccaggca ccactgtgcc 900

cctggccctg ccacctggtg gcaatggctc agccacacct gacaatggca ccacccctgg 960

ggccgagggc tggcggcagt tgctgggcct actccccgag cacatggcgg agaagctgtg 1020
tgaggcctgg gcctttgggc agagccacca gacgggcgtc gtggcactgg gcctactcac 1080
ctgcctgctg gcaatgctgc tggctggccg catcaggctc cggaggatcg atgccttctg 1140
cacctgcctg tgggccctgc tgctggggct gcacctggct gagcagcacc tgcaggccgc 1200
ctcgcctagc tggctagaca cgctcaagtt cagcaccaca tctttgtgct gcctggttgg 1260
cttcacggcg gctgtggcca caaggaaggc aacgggccca cggaggttcc ggccccgaag 1320
gtcagagaag cagccatgac tgcgggggga ggacacacgg atgctcaggc ccaggctttg 1380
ccaggtccga agcgggcccc tctctgtcct gcctcttttc acctgctcac gccctcccac 1440
ccccacccta cagccccagg tcctggccca gtccctccac tgcctcgaag agtcagtctg 1500
ccctgccttt tcctttcggg caccaccagc catccccgag tgccctgtag ccactcacca 1560
ctgctgccac ctctctggcc aatggccctt tcactggcct ggtgactgga atgtgggcag 1620
cgcccacaca ggctctggcc catggcttcc tactggcagc tccaggcacc cccctctca 1679

<210> 42
<211> 392
<212> PRT
<213> Homo sapiens

<400> 42

Met Glu Gly Val Ser Ala Leu Leu Ala Arg Cys Pro Thr Ala Gly Leu
1               5                   10                  15

Ala Gly Gly Leu Gly Val Thr Ala Cys Ala Ala Ala Gly Val Leu Leu
            20                  25                  30

Tyr Arg Ile Ala Arg Arg Met Lys Pro Thr His Thr Met Val Asn Cys
        35                  40                  45

Trp Phe Cys Asn Gln Asp Thr Leu Val Pro Tyr Gly Asn Arg Asn Cys
    50                  55                  60

Trp Asp Cys Pro His Cys Glu Gln Tyr Asn Gly Phe Gln Glu Asn Gly
65                  70                  75                  80

Asp Tyr Asn Lys Pro Ile Pro Ala Gln Tyr Leu Glu His Leu Asn His
                85                  90                  95

Val Val Ser Ser Ala Pro Ser Leu Arg Asp Pro Ser Gln Pro Gln Gln
            100                 105                 110

Trp Val Ser Ser Gln Val Leu Leu Cys Lys Arg Cys Asn His His Gln
        115                 120                 125

Thr Thr Lys Ile Lys Gln Leu Ala Ala Phe Ala Pro Arg Glu Glu Gly
    130                 135                 140

Arg Tyr Asp Glu Glu Val Glu Val Tyr Arg His His Leu Glu Gln Met
145                 150                 155                 160

Tyr Lys Leu Cys Arg Pro Cys Gln Ala Ala Val Glu Tyr Tyr Ile Lys
                165                 170                 175

His Gln Asn Arg Gln Leu Arg Ala Leu Leu Leu Ser His Gln Phe Lys
            180                 185                 190

Arg Arg Glu Ala Asp Gln Thr His Ala Gln Asn Phe Ser Ser Ala Val
        195                 200                 205

Lys Ser Pro Val Gln Val Ile Leu Leu Arg Ala Leu Ala Phe Leu Ala
    210                 215                 220

Cys Ala Phe Leu Leu Thr Thr Ala Leu Tyr Gly Ala Ser Gly His Phe
225                 230                 235                 240

Ala Pro Gly Thr Thr Val Pro Leu Ala Leu Pro Pro Gly Gly Asn Gly
                245                 250                 255

Ser Ala Thr Pro Asp Asn Gly Thr Thr Pro Gly Ala Glu Gly Trp Arg
            260                 265                 270

Gln Leu Leu Gly Leu Leu Pro Glu His Met Ala Glu Lys Leu Cys Glu
        275                 280                 285

Ala Trp Ala Phe Gly Gln Ser His Gln Thr Gly Val Val Ala Leu Gly
    290                 295                 300

Leu Leu Thr Cys Leu Leu Ala Met Leu Leu Ala Gly Arg Ile Arg Leu
305                 310                 315                 320

Arg Arg Ile Asp Ala Phe Cys Thr Cys Leu Trp Ala Leu Leu Leu Gly
                325                 330                 335

Leu His Leu Ala Glu Gln His Leu Gln Ala Ala Ser Pro Ser Trp Leu
            340                 345                 350

Asp Thr Leu Lys Phe Ser Thr Thr Ser Leu Cys Cys Leu Val Gly Phe
        355                 360                 365

Thr Ala Ala Val Ala Thr Arg Lys Ala Thr Gly Pro Arg Arg Phe Arg
    370                 375                 380

Pro Arg Arg Ser Glu Lys Gln Pro
385                 390

<210>  43
<211>  23
<212>  DNA
<213>  künstliche Sequenz

<220>
<223>  Oligonukleotid

<400>  43
ctacatcaag caccagaacc gcc                                              23


<210>  44
<211>  19
<212>  DNA
<213>  künstliche Sequenz

<220>
<223>  Oligonukleotid

<400>  44
ggacttcacg gcggaggag                                                   19


<210>  45
<211>  727
<212>  DNA
<213>  Homo sapiens

<400>  45
aggcagttgc gggttgcagg agttcaggaa aggaggtggg actagagtca acctggaata      60

gctctacagt aacaatggca gcctttttgt tgctgggaca tccatacagg caacttagct     120

ggtgaaagga ctctggattg gttggcagtc tgcttttttt tttccaaggt gatcacttta     180

ctgtagaaga aatgaggtta acagaaaaga gtgagggaga acaacaactc aagcccaaca     240

actctaatgc acccaatgaa gatcaagaag aagaaatcca acagtcagaa cagcatactc     300

cagcaaggca gcgaacacaa agagcagaca cacagccatc cagatgtcga ttgccttcac     360

gtaggacacc tacaacatcc agcgacagaa cgatcaacct tcttgaagtc cttccgtggc     420

ctactgagtg gattttcaac ccctatcgat tgcctgctct ttttgagctt tatcctgaat      480

ttcttctggt gtttaaagaa gccttccatg acatatccca ttgtctgaaa gcccagatgg     540

aaaagatcgg actgcccatc atactccacc tcttcgcact ctccaccctc tacttctaca     600

agtttttcct tcctacaatt ctttcccttt ctttctttat tcttcttgta cttctgcttc     660

tgctttttat tattgtcttc attctgatct tcttctgatt cttttgtttc aataaacagc     720

aatgagc                                                               727


<210>  46
<211>  168
<212>  PRT
<213>  Homo sapiens

<400>  46

Met Arg Leu Thr Glu Lys Ser Glu Gly Glu Gln Gln Leu Lys Pro Asn
1               5                   10                  15


Asn Ser Asn Ala Pro Asn Glu Asp Gln Glu Glu Glu Ile Gln Gln Ser
            20                  25                  30

Glu Gln His Thr Pro Ala Arg Gln Arg Thr Gln Arg Ala Asp Thr Gln
        35                  40                  45

Pro Ser Arg Cys Arg Leu Pro Ser Arg Arg Thr Pro Thr Thr Ser Ser
    50                  55                  60

Asp Arg Thr Ile Asn Leu Leu Glu Val Leu Pro Trp Pro Thr Glu Trp
65                  70                  75                  80

Ile Phe Asn Pro Tyr Arg Leu Pro Ala Leu Phe Glu Leu Tyr Pro Glu
                85                  90                  95

Phe Leu Leu Val Phe Lys Glu Ala Phe His Asp Ile Ser His Cys Leu
            100                 105                 110

Lys Ala Gln Met Glu Lys Ile Gly Leu Pro Ile Ile Leu His Leu Phe
        115                 120                 125

Ala Leu Ser Thr Leu Tyr Phe Tyr Lys Phe Phe Leu Pro Thr Ile Leu
    130                 135                 140

Ser Leu Ser Phe Phe Ile Leu Leu Val Leu Leu Leu Leu Leu Phe Ile
145                 150                 155                 160

Ile Val Phe Ile Leu Ile Phe Phe
                165

<210> 47
<211> 20
<212> DNA
<213> künstliche Sequenz

<220>
<223> Oligonukleotid

<400> 47
gctggtgaaa ggactctgga 20

<210> 48
<211> 20
<212> DNA
<213> künstliche Sequenz

<220>
<223> Oligonukleotid

<400> 48
tcgctggatg ttgtaggtgt 20

<210> 49
<211> 950
<212> DNA
<213> Homo sapiens

<400> 49
gcgagcccga gcaggcagac gcgcggccgg cggtctgggg gcgcgccgcc tcccggtccc 60

caaaatgtga agcggggagg gcggagacgc agagacggcc cggccgggcg ccctcgccgc 120

cctccggcag ccgcgccgct ccctccgctg cacgcccagg cctgagcagc gaggccaccg 180

ggccgcgcgc tcccagcttc gctcggacgc ggcttcggcc cgcagagggt tcgtggcccg 240

gacgcggcga gagctgggcc caggacggtg cgtccggcct cgcccgcggc tgctcgcacc 300

aacaagtttg aacaatgatc accgtcaacc ccgatgggaa gataatggtc agaagatgcc 360

tggtcaccct gagacccttt cggctttttg tcctgggcat cggcttcttc actctctgct 420

tcctgatgac gtctctggga ggccagttct cggcccggcg cctgggggac tcgccattca 480

ccatccgcac agaagtgatg gggggccccg agtcccgcgg cgtcctgcgc aagatgagcg 540

acctgctgga gctgatggtg aagcgcatgg acgcactggc caggctggag aacagcagtg 600

agctgcaccg ggccggcggc gacctgcact ttcccgcaga caggatgccc cctggggccg 660

gcctcatgga gcggatccag gctattgccc agaacgtctc cgacatcgct gtgaaggtgg 720

accagatcct gcgccacagt ctgctcctgc acagcaaggt gtcagaaggc cggcgggacc 780

agtgtgaggc acccagtgac cccaagttcc ctgactgctc agggaaggtg gcagtggatg 840

cgtgcccgct ggacctctga cccctgctac gccttctttg gggtggacgg caccgagtgc 900

tccttcctca tctacctcag tgaggtcgag tggttctgcc ccccgctgcc 950

<210> 50
<211> 181
<212> PRT
<213> Homo sapiens

<400> 50

Met Ile Thr Val Asn Pro Asp Gly Lys Ile Met Val Arg Arg Cys Leu
1               5                   10                  15

Val Thr Leu Arg Pro Phe Arg Leu Phe Val Leu Gly Ile Gly Phe Phe
            20                  25                  30

Thr Leu Cys Phe Leu Met Thr Ser Leu Gly Gly Gln Phe Ser Ala Arg
        35                  40                  45

Arg Leu Gly Asp Ser Pro Phe Thr Ile Arg Thr Glu Val Met Gly Gly
    50                  55                  60

Pro Glu Ser Arg Gly Val Leu Arg Lys Met Ser Asp Leu Leu Glu Leu
65                  70                  75                  80

Met Val Lys Arg Met Asp Ala Leu Ala Arg Leu Glu Asn Ser Ser Glu
                85                  90                  95

Leu His Arg Ala Gly Gly Asp Leu His Phe Pro Ala Asp Arg Met Pro
            100                 105                 110

Pro Gly Ala Gly Leu Met Glu Arg Ile Gln Ala Ile Ala Gln Asn Val
        115                 120                 125

Ser Asp Ile Ala Val Lys Val Asp Gln Ile Leu Arg His Ser Leu Leu
    130                 135                 140

Leu His Ser Lys Val Ser Glu Gly Arg Arg Asp Gln Cys Glu Ala Pro
145                 150                 155                 160

Ser Asp Pro Lys Phe Pro Asp Cys Ser Gly Lys Val Ala Val Asp Ala
                165                 170                 175

Cys Pro Leu Asp Leu
            180

<210>   51
<211>   21
<212>   DNA
<213>   künstliche Sequenz

<220>
<223>   Oligonukleotid

<400>   51
agatgcctgg tcaccctgag a                                                21

<210>   52
<211>   20
<212>   DNA
<213>   künstliche Sequenz

<220>
<223>   Oligonukleotid

<400>   52
ggccccccat cacttctgtg                                                  20

<210>   53
<211>   396
<212>   DNA
<213>   Homo sapiens

<400>   53
ctgcaagacc gcatcgccac gttcttcttc ccaaaaggca tgatgctcac cacggctgcg      60

ctgatgctct tcttcttaca cctgggcatc ttcatcagag acgtgcacaa cttctgcatc     120

acctaccact atgaccacat gagctttcac tacacggtcg tcctgatgtt ctcccaggtg     180

atcagcatct gctgggctgc catggggtca ctctatgctg agatgacaga aaacaatgct     240

caacggagcc atgttcttca accgcctgtc cttggagttt ctggccatcg agtaccggga     300

ggagcaccac tgaggcctgg ggagtcggaa cagggctaag gagggggaag caaaaggctg     360

cctcgggtgt tttaataaag ttgttgttta tttcca                               396

<210>   54
<211>   99
<212>   PRT
<213>   Homo sapiens

<400> 54

Met Met Leu Thr Thr Ala Ala Leu Met Leu Phe Phe Leu His Leu Gly
1               5                   10                  15

Ile Phe Ile Arg Asp Val His Asn Phe Cys Ile Thr Tyr His Tyr Asp
            20                  25                  30

His Met Ser Phe His Tyr Thr Val Val Leu Met Phe Ser Gln Val Ile
        35                  40                  45

Ser Ile Cys Trp Ala Ala Met Gly Ser Leu Tyr Ala Glu Met Thr Glu
    50                  55                  60

Asn Asn Ala Gln Arg Ser His Val Leu Gln Pro Pro Val Leu Gly Val
65                  70                  75                  80

Ser Gly His Arg Val Pro Gly Gly Ala Pro Leu Arg Pro Gly Glu Ser
                85                  90                  95

Glu Gln Gly

<210> 55
<211> 22
<212> DNA
<213> künstliche Sequenz

<220>
<223> Oligonukleotid

<400> 55
ccgttgagca ttgttttctg tc                                              22

<210> 56
<211> 22
<212> DNA
<213> künstliche Sequenz

<220>
<223> Oligonukleotid

<400> 56
tgctcttctt cttacacctg gg                                              22

<210> 57
<211> 539
<212> DNA
<213> Homo sapiens

<400> 57
ggtgccttaa tgtttgtggc atggatgact actgttagca taggtgtact ggttgcccgg      60

ttcttcaagc cagtttggtc aaaagctttc ttgcttggtg aagcagcttg gtttcaggtg     120

catcggatgc tcatgttcac cacaactgtc ctcacctgca ttgcttttgt tatgccgttt     180

atatacaggg gaggctggag taggcatgca ggttaccacc catacctcgg ctgtatagtg     240

atgactttgg cagttcttca gcctcttctg gcagtcttca ggccaccttt acatgaccca     300

agaaggcaaa tgtttaactg gactcattgg agtatgggaa cagctgctag aataatagca     360

gacttaaaac aatctggaaa atgtgggtgc atctctttta aggattggta gattacgcag     420

ccataaaaaa gaatgaagtc atgtcttttg tagcaacatg gatgctgctg gaagtgatta     480

tcctacatga attaatgcag aaacagaaaa tcacatacca catgttctca cttataaat      539

<210>  58
<211>  133
<212>  PRT
<213>  Homo sapiens

<400>  58

Met Phe Val Ala Trp Met Thr Thr Val Ser Ile Gly Val Leu Val Ala
1               5                   10                  15

Arg Phe Phe Lys Pro Val Trp Ser Lys Ala Phe Leu Leu Gly Glu Ala
            20                  25                  30

Ala Trp Phe Gln Val His Arg Met Leu Met Phe Thr Thr Thr Val Leu
        35                  40                  45

Thr Cys Ile Ala Phe Val Met Pro Phe Ile Tyr Arg Gly Gly Trp Ser
    50                  55                  60

Arg His Ala Gly Tyr His Pro Tyr Leu Gly Cys Ile Val Met Thr Leu
65                  70                  75                  80

Ala Val Leu Gln Pro Leu Leu Ala Val Phe Arg Pro Pro Leu His Asp
                85                  90                  95

Pro Arg Arg Gln Met Phe Asn Trp Thr His Trp Ser Met Gly Thr Ala
            100                 105                 110

Ala Arg Ile Ile Ala Asp Leu Lys Gln Ser Gly Lys Cys Gly Cys Ile
        115                 120                 125

Ser Phe Lys Asp Trp
    130

<210>  59
<211>  20
<212>  DNA
<213>  künstliche Sequenz

<220>
<223>  Oligonukleotid

<400>  59
ttgtggcatg gatgactact                                                  20

<210>  60
<211>  20

<212>  DNA
<213>  künstliche Sequenz

<220>
<223>  Oligonukleotid

<400>  60
catcactata cagccgaggt                                                    20


<210>  61
<211>  3317
<212>  DNA
<213>  Homo sapiens

<400>  61
acactgcgtc cccatcagct caaagaatac gcatggggac aagcctgggg ggccgtctga      60

gagtccccca accctggatc cccacggcag cccccactgt tgggtttttc agtggctggt     120

gtgccctggg ctggtcacct ctgcattttg ctctgctggg agtttgctcc tggccctcca     180

acagcgcctc ctctgtgagg aggaactcct gttcccgtgg ctctgctggc tctggaggct     240

ggagttcccg tgctgggccc tcctgggcgg gttctctctt gctgccgcca gtaccctgcc     300

cctctcgtcc tcctgggtag cctgggagga atggcagaag aaagcagtga agccaggtag     360

cagtagcccg gccaccccac caggctctgc tgtaggctgg gctctcaagg cagctgctcc     420

aggaggggcc ccctaggaag ggactgccac actcctggga gcgttcctgg ccccctccag     480

tgcaaatgac cctgggcccc aaggctccga acacccgccc ctctgctcca ggctagcttg     540

gctgagcccg atgcttctca aggtgaggag ggcgtccttg aagcctccgg ccaccccaca     600

ccaaggagct ttcagggcag gaaatgtgat cgggcagctg atttatctcc ttacctggtc     660

tttgttcaca gcctggctcc ggccccccac cctgctgcag ggcccgagga cgtctcccca     720

ggggtcccca cctcggtctc cttgggggga ctgtgctgag cccagctgcc tctgtgagat     780

gaagataaga aggcgaagac atgaagggcc tgcctggggg cagtctggct ttcttgcagg     840

ggggctgcac ctggttccct cctccctctc gctggcagcc tgcggggtgg tgaggatgaa     900

ggggctgtgg ggccggggtg cagggattag agggaggtga ctgccatctc ttcctcctca     960

tcgtgttttt cacctcttaa gtcaacttta gattctcgga ctcagagttc tctcctgacg    1020

gtggcagggt cctcagatca ccggtgcaga cagggccaga cagggccaat gtggggaccc    1080

actcagcctg tggcctctgc aggagggagg tcggaggcct cagcagccac cccggccacc    1140

tcctgaaaca gtgaatgtcc ttcattttca gctggcaagc tctgatctta caacgaggta    1200

tggaactgtt cagaaaactt tcagcagacg ttcgagggaa aacagctcag cttcccatgc    1260

cccccacctc tgccaggagc gaccccatat cccccaaaca gaattctggt agcccgggac    1320

cacagggtct tcctgtgcct cccctgccag ctctgcatga ctttgtcacg tacttgagtg    1380

ctggctgaga tgatgctacc gctaccaaac aggtgggagg ccagccccag ccccagcccc    1440

agccccaccg gggccggagc tcccggtgaa gaagcgtctg cctggttcgc aggtgtccag    1500

gacacaccag tcgcctgact cccggtcagg caaacgcaca catcaagttc ttgcaagcca    1560

gggctctgct ggcatcttca agaggaggga gggtcctggc cctgaccaca gggctccctt 1620
aacaggagga gttacaaact cggcttcctg gggggcatcg tggggtgtgc tgcctgccag 1680
gagaccccac tcctggtcac ggggttccgt cccacacagt ggcaggagcc atgcatgatt 1740
cttggctgaa gaagaacccg cacagctatg tggtctgccg cccagcaggg aagcccccac 1800
atcagcccta agggaacttc ccaaagctca gcaggtgcct cttcctgcca tccgctaggt 1860
cttctcttgg cccctctccc aagccttgac ccatagctga cacttctaga aaagtcttta 1920
ccgagaaacg gaccggctgc atgggtggtg aggagggcag ttgcccaggg cctggcatca 1980
gaggggcctg tggctaaggc tgtcctgaaa ttcttaatca ttttacctct gaacttgcgg 2040
gtttttgttg ttgttttttg aggcagagtc ttgctctgtc acccaggctg gagtgcagtg 2100
gtgcgatctt ggcttactgc aacttccgac tcccaggttc aagcgattct cctgcctcag 2160
cctcccgagt agctgggact acagaagtgc accaccacac ccggttaatt tttgtatttt 2220
tagtagagac ggggtttcac catgttggcc aggctgatct caaactcctg atacacccgc 2280
ctcggcctct caaagcactg ggattacagg tgtgagccac cgcgcccggc ccttttcctg 2340
cctcctaaac aagtggccag gaattctcct cctgcaccgg gtccccagat tgtgtggcaa 2400
gccctgcaga tggcacaggg gactggttct tcctcgtgga aagccaggcc cggacacctc 2460
tcgggcatcg cctgttgggg tgaccctccc acacccagcc tggaacccta gccagctcag 2520
cctccgtccg ctgagaaatc aaggtgacct tgtggctcag ccctcagggg gcactcacca 2580
cacaagagtt ccctttcaag accccctgtt cggggctggg gcccccagga acggttgggg 2640
caccttcctg gggccctgtt tttccccagg agcggggcct gggagctgag ggcgtctcat 2700
ctccccacag gcatctgctg ctgctcctgg ctgccactca cccctgtgag atgctgaggg 2760
caggatacct gtctgtgcgg ggcgtgggaa aaagggagaa agcctggcag agggttgggg 2820
gctaagaagc aaagggcgtg gaagggccac cgtgcacttt tgaagtctct acttgccagt 2880
ggccacccca cctctccctg ccctcatcca aggacggaca ggcctggcag gtggaccgga 2940
gctgtggggc agaagcatcc caggcctggc ctcagaggag ggaggccatg gtgaaagtgg 3000
aggctgtctg catccacctc cccagccttt gtcaccggga cctcagcctg accccaggcc 3060
caccccaggc tgctcaccga ggtgggtacc ctgcccaccg ccagctcaga tgcggtgtgt 3120
ggactccctt ctctctgggg gtgagcggga gttccctccc ctccacatca ggagctgggg 3180
gagagctgga gggccctggg atccccttga ccctggtcat cagccccagc cctgacaggc 3240
cctgcgtgtg ccatgtgtgg cctgggtttg gagctcagca ccctgcggga attctattaa 3300
atctccgatt ttatctg 3317

<210> 62
<211> 129
<212> PRT
<213> Homo sapiens

<400> 62

Met Leu Leu Lys Val Arg Arg Ala Ser Leu Lys Pro Pro Ala Thr Pro
1               5                   10                  15

His Gln Gly Ala Phe Arg Ala Gly Asn Val Ile Gly Gln Leu Ile Tyr
            20                  25                  30

Leu Leu Thr Trp Ser Leu Phe Thr Ala Trp Leu Arg Pro Pro Thr Leu
        35                  40                  45

Leu Gln Gly Pro Arg Thr Ser Pro Gln Gly Ser Pro Pro Arg Ser Pro
    50                  55                  60

Trp Gly Asp Cys Ala Glu Pro Ser Cys Leu Cys Glu Met Lys Ile Arg
65                  70                  75                  80

Arg Arg Arg His Glu Gly Pro Ala Trp Gly Gln Ser Gly Phe Leu Ala
                85                  90                  95

Gly Gly Leu His Leu Val Pro Ser Ser Leu Ser Leu Ala Ala Cys Gly
            100                 105                 110

Val Val Arg Met Lys Gly Leu Trp Gly Arg Gly Ala Gly Ile Arg Gly
        115                 120                 125

Arg

<210>  63
<211>  20
<212>  DNA
<213>  künstliche Sequenz

<220>
<223>  Oligonukleotid

<400>  63
ccccaaggct ccgaacaccc                                                20

<210>  64
<211>  20
<212>  DNA
<213>  künstliche Sequenz

<220>
<223>  Oligonukleotid

<400>  64
cccgatcaca tttcctgccc                                                20

<210>  65
<211>  3338
<212>  DNA
<213>  Homo sapiens

<400>  65
gtaggaagta tatgggtagg gtcagataat atttctgaaa ggaaacaccc aggagtatcc      60

caagttaatg acattttaga ccctccaaca accacacaag tcagctcctt ggaaagactc 120
tggttacttt tacaaagcaa accaggagaa ttttcataat acctgataac tatgtaagac 180
ttggaatatt tgaatttcta ggacatggga ttgtgcaacc attcatttta tcccataata 240
ttgaaatctc cctcagataa gcctctcggc acctaataga gttttcttag tgaagggcta 300
cctttctgtg ggtaacaggg aagggcaaaa taaacaacca aataatatca taatcacgag 360
tgtcaatgat tgctggaaca ggtgggggtt ggtcattaaa ttctagttgt ttccactatt 420
ccagtaggag ttgtgtgaat gttagcaaaa gaccagggtg ttacgatctg actgtgtttc 480
atcaattgcc ttgacttttg gatgaaatgc gatttgagga catatcatta ttagatttgc 540
cacagattcc aatttttttc tctaatatga ggctaaccat gatgtccttt cccaggaagg 600
acaatctctc ctttatcagg gaaaaatcag taggggcttc ctcaattttc tccttcatcc 660
ccaccacaga gtcatagagg tcaagtcctt ttcttgtgaa acctaaaaaa tgcaaattcc 720
aaggttgctg ctatggtgta ctaattttgt cacagtgaca tgccctgtca cagggcgtat 780
gtgttctgtt atacagttga aatattggtt atactattga aatgtttttg tactattgaa 840
atcccaaata aacttaattc taaaagaagc atgacctcaa cagcctcaca cctacttata 900
tcttgtagtt ctttctgtct aatgctggca atctaagcat gttccaggca agcaacattc 960
aatagcgttt tactgctcca ataagttggt tcaattagca atgtcaaagg cagtcactaa 1020
atagatagtg tataaccttc atacaatctc gtattatttt ccactaatta ctatagaaaa 1080
atcgatgaag tttcattaca atggaataac ttcaatcaca cttcaaaaac tacatacgga 1140
agatagccac aacttgctgc tctcaaaaaa cacagagatg gcatctttac tttgtttcaa 1200
atccccaacc ctggtggcgg tccaaagtta tggcagttat aaccccttat gtcattataa 1260
ggaggaaggg taaatattaa gtcaacatcc tttaaagcta agagtatgac tacagtgggg 1320
tggaatttgg gacttcatgc ccactccctg tttctgttct attttacctt tcctgacctc 1380
taagccaaca ggagaggggg aagggccaca cttttgtgac ccttgttaaa gaattgtgag 1440
tttaggaaac aaagatggac ttctgagggg gtagttgagg atgggctgaa ggcacagaag 1500
aaaccagctg gtgtgcccct ctccccacta gcagaccctt cttcctcatt ggttcagggc 1560
aaacaatccc ccaaaaattc aagaaaacta acttagagtt attttctgtt atttctcttt 1620
tccttgatct ggagccaatg cagaaagaaa tctaaaggtg aaggaaaggc agcgttcagc 1680
actgagcaag tccatgttgg agaaagttca cagggaattg gaaatccttg tcttcgtggt 1740
tcctggctca gcaggacccc tgtggggcct ctccctctct tgggaaagag attgctctag 1800
aaggtttact acaccagtga ggagaagatg agcgcaaggg ggattggccg gctgagggcg 1860
aaatcaagac tggagccaag tgcgctgagc tctcacatga ggtcctttgc tcctgttccc 1920
tggaggcata agtggctggg gtagagagaa gcaggggtat ttcttctgtc ctttcttgct 1980
tagggattgg gggtggaaat ctccccgcat ctaaggaaat ttgaaaagac aaactatggc 2040
tgcttcttca agcaaaccac ctcaccacac tatccagggg ataaaacccg cttgctgctg 2100

ctaaattatg ccaagagaga acattctgat atttctcctc aattctaggc atgacagcgt 2160

gacttggtgc ttaaaggcat ggagttttga gttgcagacc taggtttgag tgctgaatct 2220

actagcttca gggtgttaaa aaagtttctt aatctctcta aaccttattt ttctcaaaga 2280

taaaaaactg ggtgtagttg tgagtatagt gaatgcacat agtatgtgcc tttggcatgt 2340

taattcacta ttattctgga cataatttct cctaagaaaa aggatgaact aattgcaggg 2400

cctagcctaa gctctgagaa gtcattcgtt atagcatttc agtccatagt aaacaagaag 2460

aaatgaggta aagagtttaa accagggaag gcatagctgt ggtcaccaaa caacctgtta 2520

aaggcgagct gtaggcacca aaaaacctat tatggactga attgtgttcc tcaaattcat 2580

atgttgaagt gctaacccca agtaccaaat gtgactgtat ttggggatag ggtccctgaa 2640

gaagtcactc agctggaagg agtcatattg gattaggtgt tgggaattgg ctggccaagg 2700

gagaaatcaa ggctggaacc aagtgctgaa ctctcacatc aggtcctttg ctcctgttcc 2760

ctggacccta atccaatatg actggcatct ttatatgaag aggaagaggc accagagggt 2820

acacacgcag agaaaaggcc atgtgtggac acagtaagat gacggacatc tgtaagccaa 2880

ggagggaaac ctcagaagaa accagccttg cctgcacctt gatcttggag gtccagtctc 2940

cagaactgtg aaaaaaatga actggtgttg tttaaatccc ccagtcgtgg tattttgtca 3000

tggtggccct agaagacaat atacaaccca aaggaatatt ctttccactt tctccctctt 3060

ccactttata gttttttctc cttcgtttct ttctttttct cttttacttt ccttttcttc 3120

tcttctcttt cctctggttt ttaattttaa ttttaatttt tggccttcct atacctccat 3180

ttgcctctcc aggaagctga attccagaca attaatcatt catctcatca gttcagcaaa 3240

gcaaatgccc tcaatggttt cttttgtgat tcgattatta tgggatcaga atgtatctta 3300

ttcctctggg aaaaatgaaa cataaaaatt tcagaaat 3338

<210> 66
<211> 122
<212> PRT
<213> Homo sapiens

<400> 66

Met Asn Trp Cys Cys Leu Asn Pro Pro Val Val Val Phe Cys His Gly
1               5                   10                  15

Gly Pro Arg Arg Gln Tyr Thr Thr Gln Arg Asn Ile Leu Ser Thr Phe
            20                  25                  30

Ser Leu Phe His Phe Ile Val Phe Ser Pro Ser Phe Leu Ser Phe Ser
        35                  40                  45

Leu Leu Leu Ser Phe Ser Ser Leu Leu Phe Pro Leu Val Phe Asn Phe
    50                  55                  60

Asn Phe Asn Phe Trp Pro Ser Tyr Thr Ser Ile Cys Leu Ser Arg Lys
65                  70                  75                  80

Leu Asn Ser Arg Gln Leu Ile Ile His Leu Ile Ser Ser Ala Lys Gln
                85                  90                  95

Met Pro Ser Met Val Ser Phe Val Ile Arg Leu Leu Trp Asp Gln Asn
            100                 105                 110

Val Ser Tyr Ser Ser Gly Lys Asn Glu Thr
        115                 120

<210>  67
<211>  19
<212>  DNA
<213>  künstliche Sequenz

<220>
<223>  Oligonukleotid

<400>  67
ctaaaggtga aggaaaggc                                                    19

<210>  68
<211>  17
<212>  DNA
<213>  künstliche Sequenz

<220>
<223>  Oligonukleotid

<400>  68
cgctcatctt ctcctca                                                      17

<210>  69
<211>  1119
<212>  DNA
<213>  Homo sapiens

<400>  69
tcaccctcct ggccaattgt gttgcacctt gggcactgaa tcacatgagc cgtcgactaa      60

gccagatgct tctcatgttc ctactggcaa cctgccttct ggccatcata tttgtgcctc     120

aagaaatgca gaccctgcgt gtggttttgg caaccctggg tgtgggagct gcttctcttg     180

gcattacctg ttctactgcc caagaaaatg aactaattcc ttccataatc aggtacaaaa     240

gtttatgtgt gctctgtcat tctcaaaatg gacctgtctc aaccaattga cacttaacaa     300

gggaaaaaaa tccaagacaa gttagttaaa aaacaatcaa atgtaatagt cataaaaaca     360

acaaattaca gcccaagttt atatcaagct gactttgttc cagacgctgc attaagtctt     420

ttaatgcagt atcccatgta ccttctgaac cacctgaaag gttgatgtta aggaaaatag     480

cattttgtaa atgataaaaa tgtgtctaat tcacttgtga atctaaaata aattgctagc     540

aaataagaga aaatttcaaa agcaagagta tgttatcacc tccatgtgtt taagtgctca     600

tccataatca cagcaaaatg ataaatcaca aattatatgt atgattttta acaacttttc     660

ctctgttgct gtttttactc caaggggaag agctactgga atcactggaa actttgctaa     720

tattggggga gccctggctt ccctcatgat gatcctaagc atatattctc gacccctgcc 780
ctggatcatc tatggagtct ttgccatcct ctctggcctt gttgtcctcc tccttcctga 840
aaccaggaac cagcctcttc ttgacagcat ccaggatgtg gaaaatgagg gagtaaatag 900
cctagctgcc cctcagagga gctctgtgct ataggtctgt gctgaggaaa gcaaaacacc 960
atttagggct accatccccc aaaaaggctt agatctgggc tattcccatg tagtcagtgc 1020
ctttgccttt ggtgtatcct catcccttcc acagtgacct catacatccc ctgagcctca 1080
ctagatcaca cagaccatct ctgcccagcc tgtccagga 1119

<210> 70
<211> 97
<212> PRT
<213> Homo sapiens

<400> 70

<210> 71
<211> 21
<212> DNA
<213> künstliche Sequenz

<220>
<223> Oligonukleotid

<400> 71
ttctggccat catatttgtg c 21

<210> 72
<211> 21
<212> DNA
<213> künstliche Sequenz

<220>

<223>  Oligonukleotid

<400>  72
agtgattccc agtagctctt c                                                21


<210>  73
<211>  2837
<212>  DNA
<213>  Homo sapiens

<400>  73
atatcacctc ctaggaaata tgcagtaaga tggattgtgt gtctaaaggt taaactcttt      60

ttccaacaga tggatctagg ccgtatggag gattcactgc ttctcatacc tccagtgaag     120

atgcagataa agtgggatgt tgtaaatgta cttcatttta atcaggaaga agctgctatg     180

gtgaatttaa aacttgtaat gccattagat gagcttctag cacagtttca gtcatgttac     240

catgaggatt ggtgtgacct gttccatatt ccgtggtcca ttatttggtg ctgaaagaga     300

ccatctacct cctagaagtg tgtggtgggt ctcttccaaa tactcctgaa ggaaacttta     360

cttctcctgg ctatgatgga gtcaggaatt actcgagaaa cctaaactgt gaatggactc     420

tcagcaatcc aaatcaggga aattcatcta tttatattca ttttgaagat ttttacctag     480

aaagtcacca agactgtcaa tttgatgtcc ttgagtttcg agtgggtttg ttaagagcct     540

ggtaagaagt gcaagattga caaaggtaag gttagtagcg gaggtaagtg aaagcttgaa     600

tataggaaac cttggaccac ttgccattgc agtggataaa attttcaaga tttcgttgaa     660

tttgaaagtc aaagattcca ttttaaagcc attgactacc attgtccagt cgctattggg     720

gccaggccat gttacaaagg atattgaacg tttgggctta atgtgagggc ttgtgaccta     780

gagtctggag gttgcaaggg agacagccaa gtgatgtgtc atggggaaac cttcttcagg     840

tggattttga ggcttcactg caatactagc ttcctgttgc tgctgcaaca aattattatt     900

attattatta ttattattat tattattatt ttcagatgga gtctcgctct gtcactcagg     960

ctggagtgca gtggtgcgat ctcagctcgt tgcaagctcc gccttgtggg ttcatgccat    1020

tctcgtgcct cagcctccca agtagctggg actacaggaa cccgccacca cacctggcta    1080

atgtttcgta tttttagtag aggtggggtt tcatcgtgtt agccaggatg gtctcggtct    1140

cctgacctag tgattcacct gtcttggcct cccaaagttc tgggattaca ggcgtgagcc    1200

acacacttag tgtctttaaa caacatatat gtattctctc acagttctgg aggccagaat    1260

tctaaattcc ctcccactga gtcaaggtgg gagcagggca agtgccttcg gaggctctgt    1320

gggagaatcc atttcctggc tctggaggca gcctgcactc ctcgactttt gatgccctcc    1380

ttgaatgact ccaatttctc gcttccatca ctacacctcc caccactctc ccatcacctg    1440

ctctgctctt acaaggatca gtgagtacat caacttgcca cctaaagaag ccgggataat    1500

cttccctgcc aaaggtcctt aacttcatta catctgcaaa gcttctttta ccatataagg    1560

tgcaccgggt acttcttgag cattgggatg atctgcttca cctccagtca cacagcttcc    1620

aggcactggg agtggtcctc ctgcaggatg ttcagcttcg acttggccag agaaatggaa    1680

tggttgcatc acttatctac gtaaacaatt gaagaattgt ctgaaagaaa agcagaagga 1740
acatctgaag gaacacctga tgaggctgca cccttggcgg aaagaacacc tgacatggct 1800
gaaagcttgg tggaaaaacc acctgatgag gctgcaccct tggtggaggg aacagctgac 1860
aaaattcaat gtttggggaa agcaacatct ggaaagtttg aacagtcagc agaagaaaca 1920
cctaagaaaa ttatgaggac tgcaaaagaa acatctaaga aatttgcatg gccagcaaaa 1980
gaaagaccta ggaagatcac atgggaggaa aaataaacat ctgtaaagac tgaatgcgtg 2040
gcaggagtaa tacctaataa aactgaagtt ttggaaaaag gaacatctaa gatgctcacg 2100
tgtcctacaa aagaaacatc tacaaaagca agtacaaatg tggatgtgag ttctgtagag 2160
tctatattca gagtctcacc ctgtcaccca ggctggaatg caatggcacg atctcggctc 2220
actgcaacct ccacctccca gaaggaagca acaaagacag caactgaaca acaagaaaat 2280
gatattggaa ttattgaatg agcgccataa gatctaacaa ataagatgcc cacatcagag 2340
tcaggacaaa aagaagatac gaaatcacct tcagtttctg aggtcacagc tatggatgtg 2400
gaagagatag gaaaggcctc accacttaag atagaagcag cagctgcata gtggtaacag 2460
caatgagtgg atgtcaaaag acagattcaa ctagcctatc aatattcttg ggtgcagttc 2520
cttctcatga aagagcaagg gaacttaaaa aatatcactg tgaacaactt acagcaaaaa 2580
taaaacaaat gaaaaataag ttttgggtac tacaaaagga actatcagaa gcaaaaataa 2640
aattgcagta agtgaatcaa aaggttaaat gggaacaaga gctctgcagt gtgagcttgg 2700
aatgaagttg ataatagtga gaccttgttg gtacaagact atgtaacaca acctgcactt 2760
ctcaacaaaa aattgctttt ctgacttctg cactcagtag gtatctttgg aaaataatct 2820
cctattggta ctgaggc 2837

<210> 74
<211> 102
<212> PRT
<213> Homo sapiens

<400> 74

Met Cys His Gly Glu Thr Phe Phe Arg Trp Ile Leu Arg Leu His Cys
1               5                   10                  15

Asn Thr Ser Phe Leu Leu Leu Leu Gln Gln Ile Ile Ile Ile Ile Ile
            20                  25                  30

Ile Ile Ile Ile Ile Ile Ile Phe Arg Trp Ser Leu Ala Leu Ser Leu
        35                  40                  45

Arg Leu Glu Cys Ser Gly Ala Ile Ser Ala Arg Cys Lys Leu Arg Leu
    50                  55                  60

Val Gly Ser Cys His Ser Arg Ala Ser Ala Ser Gln Val Ala Gly Thr
65                  70                  75                  80

Thr Gly Thr Arg His His Thr Trp Leu Met Phe Arg Ile Phe Ser Arg
            85                  90                  95

Gly Gly Val Ser Ser Cys
            100

<210> 75
<211> 21
<212> DNA
<213> künstliche Sequenz

<220>
<223> Oligonukleotid

<400> 75
catctacctc ctagaagtgt g                                              21

<210> 76
<211> 21
<212> DNA
<213> künstliche Sequenz

<220>
<223> Oligonukleotid

<400> 76
cactcgaaac tcaaggacat c                                              21

<210> 77
<211> 5868
<212> DNA
<213> Homo sapiens

<400> 77
gatctctccc atgaagtgac caggatagag aagcaccaga accgccaaaa gtatgggctg      60

tgcgtcatct tcctttcctg taccatgatg cccaacttta aagagctgat ccatttcgag     120

gtcagcatcg gtcactatgg gaacaagatg gacctgaatt acaagcctct agtctcaagc     180

acaccgtaca gcccagtgat atatgatggg aacatctacc attatgtgcc ctggtacaac     240

accaagcctg tcgtggccgt gacctccaac tgggaggacg tcagcttccg catgaactgc     300

ctcaacctcc tccacttcac tcgggaccgc ctgaaagcca acctggacac cctgaaatcc     360

acgcggaatc cgaaggatcc agctctcctc taccagtggg agaaactgct gagggagctg     420

gcagaggact gcaagcgccc tctgccctgc atgacctatc agcccaaagc caccagcctg     480

gacaggaaga ggtggcagct ccgcagcctc ctcctgcagg aactggccca aaaggccaag     540

caagccaagc ccaaggacat ggtggccaca gcggaggact ggctgtaccg cctcaacacc     600

gtgctccctg agccccagat gggcctccct gacgtgatga tttggctggt ggccaaggag     660

cagcgagtgg cctatgcaca gtacccagag ggtgaaggac agaaggatgt gctcccagct     720

cacctccggg tctgcatgtg gcttggcaat gtcacagaca gcaaggacct gcagctgctc     780

cgccagggtg acacagcggt gtacgccgag atggtgagtg tatgagaatc aggccaagta     840

taaagaccag tgggggcagc aggggctgta tcactgcccc aacttctcgg atgtcatggg     900

gaacaagacc ctccccatga cggatttcca accacccctg ggatggcact ggcaggacag 960
ctggacagtg gaacctcaga gaaggctcct cctggacata gacatcaaca agagccaggt 1020
gctggaggag gtatatgaga accagggccg tgacaccaga ggggcctggg ggcctgccgc 1080
catcccaaac acagacgtga atggacagcc catggaggcc cgggagaacg tgaagtgccc 1140
ccaaggctgg cactttaaga aggactgggt ggtggagctg aaccacgcag tggacagtaa 1200
gggctgggag tatggagtgg ggatcccacc gtcgggcctg ccccaggtct ggagcccggt 1260
ggagaagacc taccactcgt gccgccgccg gcgctgggcg cgtgtgcgct tcaggaacca 1320
tggggagctg agccacgagc aggagaccct ctccttcctg cagctgggcc tggccaaggg 1380
cgaggaggag ggctgggagt atgacacctt cggctccaag ttccacctca accctcagcc 1440
ccagagccgg ttccgccgcc gctgctggcg ccgcaggctg gcccccaaca aggacaaggg 1500
catcgcgccc atattcctcc tggaggggtc cttggctatg gatctgaaat accacgctgg 1560
gaaggaagag gacagcaaga catggccatg gggtctggac agacagttca gggaccccca 1620
gaggcaggac acccggcccc ccaacttgcc cttcatctac tgcaccttca ataagcccca 1680
ctactaccag ctcttctgct acatctacca ggcccggaac ctggtgtcca atcagatcct 1740
gacattccaa gggcccttca ttcgggtggt cttcctgaac cacagccagt gcacccaaac 1800
cctgaggagc tctgcaggcc ccacatgggc ccagacactc atcttccagc acctccttct 1860
gtacgagaac ccacaggaca ccaaagagag cccaccgctt gtggtgctgg agctgtggca 1920
gcgtgacttc tggggcaagg agagcttgtg gggacggagc gtgtggcccc caatggtctg 1980
gctggatctc caggaccgga tcctgccccc catgaggtgg catccccttg taaaggagtt 2040
ggggaaggaa gagggcgaga tcttggcatc ctgtgagctg atcctccaga ctgagaagct 2100
tggagagaag cagctgccta tcttaagcgt tccctggaag aatggggcat acacactccc 2160
caagagcatc cagcccacga taaagaggat ggccattgag gtgctggcga tgtgggatgg 2220
ggacggtggg caggacaggc gggggtggtc tggagtgcgc tgcagccttc tgctggtcct 2280
ccctgactac tggatccaaa gctcacaccc cgaaaaagac tacctgggag gtggagggag 2340
acaggagaga aacgaagagg ttctggtgta acactggaaa tcattttacc acaaacctct 2400
gcagtgagga gtaggcaaag ggctgtagca tgcatgatca cttgtgggac tcacgctgcc 2460
cctgcgcagt agcaactact ttgcagagaa ggaaatagag gctccaagag ataacacatt 2520
ccacgcacag tgatgcaggg actaactgac agggccattt aggcccagcc ctgtctgact 2580
gcagatgcca ggatgttgct cacctctctt ctgagagtag catgagggtc ctcattcaga 2640
agctgtgtgc cctgccgcaa atgtggcaaa gagcacaaga cggtcaggcc tctgggactg 2700
aaggcttccc caagatcagg caacttggct ggttcccgct ttaggccccg aggaggccca 2760
aagtcagggt gcagctattt cctggcagga tgccaggtca ctgaatggcc atggggtcct 2820
caatgagcta gacggcacag gggccctgag aaatccaggc acttcctgct tcttcaggcc 2880
tcagaggcag tcggcttcag gaactcctac ctgagaactg atgaggccag acaaggcagc 2940

gggtgaggag gggcaatgcc tgcgggctat ggaggtcagt ggaggatgca gccagtggcc 3000
agaggtcacc tccctcatgg gttgggggac agcgtcccag ccccgagggc aagcactgat 3060
ccctcacagg acggggaagc ctgtccttgt gcgccttcag acactggctc ctctgcagcc 3120
ccattccctg gccctgcagg ctcctgctgc accgctattg cccctcagcc cccttctctg 3180
gccaggaccc cattacagag gcgctgcctg ccccttgtcc tgccctcctt ctttgttctg 3240
gtagatcctg gcctggggcc ttcggaacat gaagaaggcg agctcccccc agctcctggt 3300
ggaattcggg gaagagtccc tgaggacaga acccatcagg gactttcaga ccaaccccaa 3360
cttccccgag tctgagtctg tcctagtcct cacagtgctc atgccgacgg aggaggccta 3420
tgcactgccc ctcgtggtga aggtggtaga caactgggcc ttcggccagc agaccgtgac 3480
gggccaggcc aacatcgact tcctccagcc ctacttctgt gacccctggg ctcaagacta 3540
tatgcaccca aagcttccaa cgctgtctga gaagaagcac caagacttcc taggctacct 3600
ctacagaaag ttctggttca agtccagtaa agcagaggat gagtatgagc atgaggtgga 3660
ctggtggagc aagctgttct gggccacaga tgagcacaag tccctgaagt acaagtacaa 3720
agactaccac accctcaagg tgtatgagtg tgagctggag gccgtgccag ccttccaggg 3780
cctgcaggac ttctgccaga ccttcaaact ctaccaggag cagcccaagt tggacagccc 3840
cgtggtaggg gagttcaagg gcctttttcg catctacccc tttcctgaga atccagaagc 3900
cccaaagccc ccgctgcagt tcttggtttg gccagagaga gaggacttcc cccagccgtg 3960
cttggtgcgg gtgtacatgg tacgagccat caacctgcag ccccaggact acaatggcct 4020
gtgtgaccct tatgtgatcc tgaaactggg caagacagag cttggcaacc gggacatgta 4080
ccagcccaac actctggatc ccatctttgg catgatgttt gaactcacct gcaacatacc 4140
cctggagaag gacctagaga tccagctcta tgacttcgac ctattttcac ctgatgataa 4200
gataggaacc acagtcatcg accttgaaaa ccgactccta tctggctttg gagctcattg 4260
tgggctctcc aaatcctact gccagtcagg gccctttaga tggcgggatc agatgccccc 4320
aagctacctc ctagaacgct atgccaagcg gaaagggcta cctccgcctc tgttcagtcc 4380
tgaggaagat gctgttttct ataatgggaa aaagttcaag ctgcaaagct ttgagcccaa 4440
aacccctact gttcatggtt tgggacccaa gaaggaacgc cttgcactgt acctcctgca 4500
cacccagggg ctggtacctg agcacgtgga gacccgcaca ctgtacagcc acagccagcc 4560
aggcatcgac cagggaaagg tgcaaatgtg ggtggacatc ttccccaaga agctggggcc 4620
tcctggcccc caagtcaaca tcaaccccag aaagcctaaa cggtatgagc tgcgatgcat 4680
catctggaag actgccaatg tggacctggt ggatgacaat ttaagtagag agaagacgag 4740
cgacatctac atcaaagggt ggttatacgg gctggagaag gacatgcaga agacagacat 4800
ccactaccac tcgctgactg gggaggccga cttcaactgg cggttcatct ttaccatgga 4860
ctacctggcg gcggagcgca cgtgtgtcca gagccagaag gattacatat ggagcctgga 4920
tgccacgtcc atgaagttcc cagcccgact tatcatccag gtctgggaca atgacatctt 4980

ctcccccgac gacttcctag gggtcctgga gctggatttg tctgacatgc ccctcccggc 5040

tcggcacgcc aagcagtgct ccatcaggat gatggacgcc gaccccaagt ggccctattt 5100

catccaatac aagcacttct ccctctttaa gaagaagact gtgactggct ggtggccttg 5160

ccaggtcctc gatggtggca aatggcgctt gtcgggcaag gtgaagatga gcctggagat 5220

tctgtcagag aaggaagcct taatcaagcc agccgggcga ggccagtcgg aacccaacca 5280

gtaccccaca cttcatcctc ccctacgcac caacacctct ttcacgtggc tgcggtcacc 5340

agttcaaaac ttctgctata ttttctggaa acgctatcgc ttcaaactca tagcctttat 5400

ggtcatatcg attatagcac ttatgctgtt taacttcatc tattcagctc cgcactattt 5460

ggccatgagc tggatcaaac ctcaacttca gctgtatcct cccattaaaa tattcaatat 5520

catcaattca ctaaacacca gcaacgccag ctcttccatc cttcccaccc aggatccaaa 5580

cctaaagcct acaatagacc atgagtggaa actccaccca ggacccacaa atcacctgag 5640

tgatattttc ccagaacttc cagccccagg agactaatta gtccatgctg cctggctttc 5700

ctcctgctac caacagccct ccccttgggc tggctaccag ttctttgttt ctatcttcta 5760

gaatatatgc aagatgctag gaatattctg gctattgtgt tcagaaatca ctttcaacaa 5820

gacgagcaga gctgtaattt tccactgaaa taaacaagtt ctataaca 5868

<210> 78
<211> 802
<212> PRT
<213> Homo sapiens

<400> 78

Met Lys Lys Ala Ser Ser Pro Gln Leu Leu Val Glu Phe Gly Glu Glu
1               5                   10                  15

Ser Leu Arg Thr Glu Pro Ile Arg Asp Phe Gln Thr Asn Pro Asn Phe
            20                  25                  30

Pro Glu Ser Glu Ser Val Leu Val Leu Thr Val Leu Met Pro Thr Glu
        35                  40                  45

Glu Ala Tyr Ala Leu Pro Leu Val Val Lys Val Val Asp Asn Trp Ala
    50                  55                  60

Phe Gly Gln Gln Thr Val Thr Gly Gln Ala Asn Ile Asp Phe Leu Gln
65                  70                  75                  80

Pro Tyr Phe Cys Asp Pro Trp Ala Gln Asp Tyr Met His Pro Lys Leu
                85                  90                  95

Pro Thr Leu Ser Glu Lys Lys His Gln Asp Phe Leu Gly Tyr Leu Tyr
            100                 105                 110

Arg Lys Phe Trp Phe Lys Ser Ser Lys Ala Glu Asp Glu Tyr Glu His
        115                 120                 125

Glu Val Asp Trp Trp Ser Lys Leu Phe Trp Ala Thr Asp Glu His Lys
    130                 135                 140

Ser Leu Lys Tyr Lys Tyr Lys Asp Tyr His Thr Leu Lys Val Tyr Glu
145                 150                 155                 160

Cys Glu Leu Glu Ala Val Pro Ala Phe Gln Gly Leu Gln Asp Phe Cys
                165                 170                 175

Gln Thr Phe Lys Leu Tyr Gln Glu Gln Pro Lys Leu Asp Ser Pro Val
            180                 185                 190

Val Gly Glu Phe Lys Gly Leu Phe Arg Ile Tyr Pro Phe Pro Glu Asn
        195                 200                 205

Pro Glu Ala Pro Lys Pro Pro Leu Gln Phe Leu Val Trp Pro Glu Arg
    210                 215                 220

Glu Asp Phe Pro Gln Pro Cys Leu Val Arg Val Tyr Met Val Arg Ala
225                 230                 235                 240

Ile Asn Leu Gln Pro Gln Asp Tyr Asn Gly Leu Cys Asp Pro Tyr Val
                245                 250                 255

Ile Leu Lys Leu Gly Lys Thr Glu Leu Gly Asn Arg Asp Met Tyr Gln
            260                 265                 270

Pro Asn Thr Leu Asp Pro Ile Phe Gly Met Met Phe Glu Leu Thr Cys
        275                 280                 285

Asn Ile Pro Leu Glu Lys Asp Leu Glu Ile Gln Leu Tyr Asp Phe Asp
    290                 295                 300

Leu Phe Ser Pro Asp Asp Lys Ile Gly Thr Thr Val Ile Asp Leu Glu
305                 310                 315                 320

Asn Arg Leu Leu Ser Gly Phe Gly Ala His Cys Gly Leu Ser Lys Ser
                325                 330                 335

Tyr Cys Gln Ser Gly Pro Phe Arg Trp Arg Asp Gln Met Pro Pro Ser
            340                 345                 350

Tyr Leu Leu Glu Arg Tyr Ala Lys Arg Lys Gly Leu Pro Pro Pro Leu
        355                 360                 365

Phe Ser Pro Glu Glu Asp Ala Val Phe Tyr Asn Gly Lys Lys Phe Lys
    370                 375                 380

Leu Gln Ser Phe Glu Pro Lys Thr Pro Thr Val His Gly Leu Gly Pro
385                 390                 395                 400

Lys Lys Glu Arg Leu Ala Leu Tyr Leu Leu His Thr Gln Gly Leu Val
                405                 410                 415

Pro Glu His Val Glu Thr Arg Thr Leu Tyr Ser His Ser Gln Pro Gly
            420                 425                 430

Ile Asp Gln Gly Lys Val Gln Met Trp Val Asp Ile Phe Pro Lys Lys
        435                 440                 445

Leu Gly Pro Pro Gly Pro Gln Val Asn Ile Asn Pro Arg Lys Pro Lys
    450                 455                 460

Arg Tyr Glu Leu Arg Cys Ile Ile Trp Lys Thr Ala Asn Val Asp Leu
465                 470                 475                 480

Val Asp Asp Asn Leu Ser Arg Glu Lys Thr Ser Asp Ile Tyr Ile Lys
                485                 490                 495

Gly Trp Leu Tyr Gly Leu Glu Lys Asp Met Gln Lys Thr Asp Ile His
            500                 505                 510

Tyr His Ser Leu Thr Gly Glu Ala Asp Phe Asn Trp Arg Phe Ile Phe
        515                 520                 525

Thr Met Asp Tyr Leu Ala Ala Glu Arg Thr Cys Val Gln Ser Gln Lys
    530                 535                 540

Asp Tyr Ile Trp Ser Leu Asp Ala Thr Ser Met Lys Phe Pro Ala Arg
545                 550                 555                 560

Leu Ile Ile Gln Val Trp Asp Asn Asp Ile Phe Ser Pro Asp Asp Phe
                565                 570                 575

Leu Gly Val Leu Glu Leu Asp Leu Ser Asp Met Pro Leu Pro Ala Arg
            580                 585                 590

His Ala Lys Gln Cys Ser Ile Arg Met Met Asp Ala Asp Pro Lys Trp
        595                 600                 605

Pro Tyr Phe Ile Gln Tyr Lys His Phe Ser Leu Phe Lys Lys Lys Thr
    610                 615                 620

Val Thr Gly Trp Trp Pro Cys Gln Val Leu Asp Gly Gly Lys Trp Arg
625                 630                 635                 640

Leu Ser Gly Lys Val Lys Met Ser Leu Glu Ile Leu Ser Glu Lys Glu
                645                 650                 655

Ala Leu Ile Lys Pro Ala Gly Arg Gly Gln Ser Glu Pro Asn Gln Tyr
            660                 665                 670

Pro Thr Leu His Pro Pro Leu Arg Thr Asn Thr Ser Phe Thr Trp Leu
        675                 680                 685

Arg Ser Pro Val Gln Asn Phe Cys Tyr Ile Phe Trp Lys Arg Tyr Arg
    690                 695                 700

Phe Lys Leu Ile Ala Phe Met Val Ile Ser Ile Ile Ala Leu Met Leu
705                 710                 715                 720

Phe Asn Phe Ile Tyr Ser Ala Pro His Tyr Leu Ala Met Ser Trp Ile
                725                 730                 735

Lys Pro Gln Leu Gln Leu Tyr Pro Pro Ile Lys Ile Phe Asn Ile Ile
            740                 745                 750

Asn Ser Leu Asn Thr Ser Asn Ala Ser Ser Ser Ile Leu Pro Thr Gln
        755                 760                 765

Asp Pro Asn Leu Lys Pro Thr Ile Asp His Glu Trp Lys Leu His Pro
    770                 775                 780

Gly Pro Thr Asn His Leu Ser Asp Ile Phe Pro Glu Leu Pro Ala Pro
785                 790                 795                 800

Gly Asp

<210>  79
<211>  21
<212>  DNA
<213>  künstliche Sequenz

<220>
<223>  Oligonukleotid

<400>  79
tgttcagtcc tgaggaagat g                                                21

<210>  80
<211>  21
<212>  DNA
<213>  künstliche Sequenz

<220>
<223>  Oligonukleotid

<400>  80
atgatgcatc gcagctcata c                                                21

<210>  81
<211>  3170
<212>  DNA
<213>  Homo sapiens

<400>  81

ggctcaccga caacttcatc gccgccgtgc gccgccgaga cttcgccaac atgaccagcc 60
tggtgcacct cactctctcc cggaacacca tcggccaggt ggcagctggc gccttcgccg 120
acctgcgtgc cctccgggcc ctgcacctgg acagcaaccg cctggcggag gtgcgcggcg 180
accagctccg cggcctgggc aacctccgcc acctgatcct tggaaacaac cagatccgcc 240
gggtggagtc ggcggccttt gacgccttcc tgtccaccgt ggaggacctg gatctgtcct 300
acaacaacct ggaggccctg ccgtgggagg cggtgggcca gatggtgaac ctaaacaccc 360
tcacgctgga ccacaacctc atcgaccaca tcgcggaggg gaccttcgtg cagcttcaca 420
agctggtccg tctggacatg acctccaacc gcctgcataa actcccgccc gacgggctct 480
tcctgaggtc gcagggcacc gggcccaagc cgcccacccc gctgaccgtc agcttcggcg 540
gcaaccccct gcactgcaac tgcgagctgc tctggctgcg gcggctgacc cgcgaggacg 600
acttagagac ctgcgccacg cccgaacacc tcaccgaccg ctacttctgg tccatccccg 660
aggaggagtt cctgtgtgag cccccgctga tcacacggca ggcggggggc cgggccctgg 720
tggtggaagg ccaggcggtg agcctgcgct gccgagcggt gggtgacccc gagccggtgg 780
tgcactgggt ggcacctgat gggcggctgc tggggaactc cagccggacc cgggtccggg 840
gggacgggac gctggatgtg accatcacca ccttgaggga cagtggcacc ttcacttgta 900
tcgcctccaa tgctgctggg gaagcgacgg cgcccgtgga ggtgtgcgtg gtacctctgc 960
ctctgatggc acccccgccg gctgccccgc cgcctctcac cgagcccggc tcctctgaca 1020
tcgccacgcc gggcagacca ggtgccaacg attctgcggc tgagcgtcgg ctcgtggcag 1080
ccgagctcac ctcgaactcc gtgctcatcc gctggccagc ccagaggcct gtgcccggaa 1140
tacgcatgta ccaggttcag tacaacagtt ccgttgatga ctccctcgtc tacaggatga 1200
tcccgtccac cagtcagacc ttcctggtga atgacctggc ggcgggccgt gcctacgact 1260
tgtgcgtgct ggcggtctac gacgacgggg ccacagcgct gccggcaacg cgagtggtgg 1320
gctgtgtaca gttcaccacc gctggggatc cggcgccctg ccgcccgctg agggcccatt 1380
tcttgggcgg caccatgatc atcgccatcg ggggcgtcat cgtcgcctcg gtcctcgtct 1440
tcatcgttct gctcatgatc cgctataagg tgtatggcga cggggacagc cgccgcgtca 1500
agggctccag gtcgctcccg cgggtcagcc acgtgtgctc gcagaccaac ggcgcaggca 1560
caggcgcggc acaggccccg gccctgccgg cccaggacca ctacgaggcg ctgcgcgagg 1620
tggagtccca ggctgccccc gccgtcgccg tcgaggccaa ggccatggag gccgagacgg 1680
catccgcgga gccggaggtg gtccttggac gttctctggg cggctcggcc acctcgctgt 1740
gcctgctgcc atccgaggaa acttccgggg aggagtctcg ggccgcggtg ggccctcgaa 1800
ggagccgatc cggcgccctg gagccaccaa cctcggcgcc ccctactcta gctctagttc 1860
ctgggggagc cgcggcccgg ccgaggccgc agcagcgcta ttcgttcgac ggggactacg 1920
gggcactatt ccagagccac agttacccgc gccgcgcccg gcggacaaag cgccaccggt 1980
ccacgccgca cctggacggg gctggagggg gcgcggccgg ggaggatgga gacctggggc 2040

tgggctccgc cagggcgtgc ctggctttca ccagcaccga gtggatgctg gagagtaccg 2100
tgtgagcggc gggcgggcgc cgggacgcct gggtgccgca gaccaaacgc ccagccgcac 2160
ggacgctggg gcgggactgg gagaaagcgc agcgccaaga cattggacca gagtggagac 2220
gcgcccttgt ccccgggagg gggcggggca gcctcgggct gcggctcgag gccacgcccc 2280
cgtgcccagg gcggggttcg gggaccggct gccggcctcc cttcccctat ggactcctcg 2340
acccccctcc tacccctccc ctcgcgcgct cgcggacctc gctggagccg gtgccttaca 2400
cagcgaagcg cggggagggg cagggccccc tgacactgca gcactgagac acgagccccc 2460
tcccccagcc cgtcacccgg ggccggggcg aggggcccat ttcttgtatc tggctggact 2520
agatcctatt ctgtcccgcg gcggcctcca aagcctccca ccccacccca cgcacattcc 2580
tggtccggtc gggtctggct tggggtcccc ctttctctgt ttccctcgtt tgtctctatc 2640
ccgccctctt gtcgtctctc tgtagtgcct gtctttccct atttgcctct cctttctctc 2700
tgtcctgtcg tctcttgtcc ctcggccctc cctggttttg tctagtctcc ctgtctctcc 2760
tgatttcttc tctttactca ttctcccggg caggtcccac tggaaggacc agactctccc 2820
aaataaatcc ccacacgaac aaaatccaaa accaaatccc cctccctacc ggagccggga 2880
ccctccgccg cagcagaatt aaactttttt ctgtgtctga ggccctgctg acctgtgtgt 2940
gtgtctgtat gtgtgtccgc gtgtagtgtg tgtgtgtgtg tgtgtgtgtg tgtgtgtgtg 3000
tgtgtgttgg gggagggtga cctagattgc agcataagga ctctaagtga gactgaagga 3060
agatgggaag atgactaact ggggccggag gagactggca gacaggcttt tatcctctga 3120
gagacttaga ggtggggaat aatcacaaaa ataaaatgat cataatagct 3170

<210> 82
<211> 684
<212> PRT
<213> Homo sapiens

<400> 82

Met Thr Ser Leu Val His Leu Thr Leu Ser Arg Asn Thr Ile Gly Gln
1               5                   10                  15

Val Ala Ala Gly Ala Phe Ala Asp Leu Arg Ala Leu Arg Ala Leu His
            20                  25                  30

Leu Asp Ser Asn Arg Leu Ala Glu Val Arg Gly Asp Gln Leu Arg Gly
        35                  40                  45

Leu Gly Asn Leu Arg His Leu Ile Leu Gly Asn Asn Gln Ile Arg Arg
    50                  55                  60

Val Glu Ser Ala Ala Phe Asp Ala Phe Leu Ser Thr Val Glu Asp Leu
65                  70                  75                  80

Asp Leu Ser Tyr Asn Asn Leu Glu Ala Leu Pro Trp Glu Ala Val Gly
                85                  90                  95

Gln Met Val Asn Leu Asn Thr Leu Thr Leu Asp His Asn Leu Ile Asp
            100                 105                 110

His Ile Ala Glu Gly Thr Phe Val Gln Leu His Lys Leu Val Arg Leu
        115                 120                 125

Asp Met Thr Ser Asn Arg Leu His Lys Leu Pro Pro Asp Gly Leu Phe
    130                 135                 140

Leu Arg Ser Gln Gly Thr Gly Pro Lys Pro Pro Thr Pro Leu Thr Val
145                 150                 155                 160

Ser Phe Gly Gly Asn Pro Leu His Cys Asn Cys Glu Leu Leu Trp Leu
                165                 170                 175

Arg Arg Leu Thr Arg Glu Asp Asp Leu Glu Thr Cys Ala Thr Pro Glu
            180                 185                 190

His Leu Thr Asp Arg Tyr Phe Trp Ser Ile Pro Glu Glu Glu Phe Leu
        195                 200                 205

Cys Glu Pro Pro Leu Ile Thr Arg Gln Ala Gly Gly Arg Ala Leu Val
    210                 215                 220

Val Glu Gly Gln Ala Val Ser Leu Arg Cys Arg Ala Val Gly Asp Pro
225                 230                 235                 240

Glu Pro Val Val His Trp Val Ala Pro Asp Gly Arg Leu Leu Gly Asn
                245                 250                 255

Ser Ser Arg Thr Arg Val Arg Gly Asp Gly Thr Leu Asp Val Thr Ile
            260                 265                 270

Thr Thr Leu Arg Asp Ser Gly Thr Phe Thr Cys Ile Ala Ser Asn Ala
        275                 280                 285

Ala Gly Glu Ala Thr Ala Pro Val Glu Val Cys Val Val Pro Leu Pro
    290                 295                 300

Leu Met Ala Pro Pro Pro Ala Ala Pro Pro Pro Leu Thr Glu Pro Gly
305                 310                 315                 320

Ser Ser Asp Ile Ala Thr Pro Gly Arg Pro Gly Ala Asn Asp Ser Ala
                325                 330                 335

Ala Glu Arg Arg Leu Val Ala Ala Glu Leu Thr Ser Asn Ser Val Leu
            340                 345                 350

Ile Arg Trp Pro Ala Gln Arg Pro Val Pro Gly Ile Arg Met Tyr Gln
        355                 360                 365

Val Gln Tyr Asn Ser Ser Val Asp Asp Ser Leu Val Tyr Arg Met Ile
    370                 375                 380

Pro Ser Thr Ser Gln Thr Phe Leu Val Asn Asp Leu Ala Ala Gly Arg
385                 390                 395                 400

Ala Tyr Asp Leu Cys Val Leu Ala Val Tyr Asp Asp Gly Ala Thr Ala
                405                 410                 415

Leu Pro Ala Thr Arg Val Val Gly Cys Val Gln Phe Thr Thr Ala Gly
            420                 425                 430

Asp Pro Ala Pro Cys Arg Pro Leu Arg Ala His Phe Leu Gly Gly Thr
        435                 440                 445

Met Ile Ile Ala Ile Gly Gly Val Ile Val Ala Ser Val Leu Val Phe
    450                 455                 460

Ile Val Leu Leu Met Ile Arg Tyr Lys Val Tyr Gly Asp Gly Asp Ser
465                 470                 475                 480

Arg Arg Val Lys Gly Ser Arg Ser Leu Pro Arg Val Ser His Val Cys
                485                 490                 495

Ser Gln Thr Asn Gly Ala Gly Thr Gly Ala Ala Gln Ala Pro Ala Leu
            500                 505                 510

Pro Ala Gln Asp His Tyr Glu Ala Leu Arg Glu Val Glu Ser Gln Ala
        515                 520                 525

Ala Pro Ala Val Ala Val Glu Ala Lys Ala Met Glu Ala Glu Thr Ala
    530                 535                 540

Ser Ala Glu Pro Glu Val Val Leu Gly Arg Ser Leu Gly Gly Ser Ala
545                 550                 555                 560

Thr Ser Leu Cys Leu Leu Pro Ser Glu Glu Thr Ser Gly Glu Glu Ser
                565                 570                 575

Arg Ala Ala Val Gly Pro Arg Arg Ser Arg Ser Gly Ala Leu Glu Pro
            580                 585                 590

Pro Thr Ser Ala Pro Pro Thr Leu Ala Leu Val Pro Gly Gly Ala Ala
        595                 600                 605

Ala Arg Pro Arg Pro Gln Gln Arg Tyr Ser Phe Asp Gly Asp Tyr Gly
    610                 615                 620

Ala Leu Phe Gln Ser His Ser Tyr Pro Arg Arg Ala Arg Arg Thr Lys
625                 630                 635                 640

Arg His Arg Ser Thr Pro His Leu Asp Gly Ala Gly Gly Gly Ala Ala
                645                 650                 655

Gly Glu Asp Gly Asp Leu Gly Leu Gly Ser Ala Arg Ala Cys Leu Ala
            660                 665                 670

Phe Thr Ser Thr Glu Trp Met Leu Glu Ser Thr Val
        675                 680

<210> 83
<211> 17
<212> DNA
<213> künstliche Sequenz

<220>
<223> Oligonukleotid

<400> 83
cgaactccgt gctcatc 17

<210> 84
<211> 17
<212> DNA
<213> künstliche Sequenz

<220>
<223> Oligonukleotid

<400> 84
cgcacaagtc gtaggca 17

<210> 85
<211> 2206
<212> DNA
<213> Homo sapiens

<400> 85
cgacaacgtc acccgcagac cggccaatcc cgccaggccg cggcccagtg gcgccggcgc 60
acaccgaaga cgacaccagc catccggcca atcccgcccc gccgcgcccc gcaggcccgc 120
ccactcctcg cttctccact tcccttctcg aagtgtccgg tcgcttctcg caggcggcgc 180
gcttgctggg tcacagtgag gcggctccgc gcaggcgcag ccgggcgggc gaggagcggg 240
gaagctgact cagggctgcg gccggggtcc tgcggggtag gagcgcgagg ccggcctgag 300
ggaggaggcc tagcgaccca tccggcgcct cccgccccgg gcacccgccc gcggccgcgc 360
atcctgcggg ccccaggagg cctccatctc aaaacaacgt gtttttagga tctcatccac 420
tatcacagtt tcagctttcc ccaaactgga atgtgtcttt gcagacgccc atccttatta 480
aagggcaaag acttctcata cacctaggat ggatcttata ttcttggcgg gactgcagag 540
aaggtgccgt gtcctgagtc ctcatgtcag ggcacaggct tccagccagt tctacctggg 600
ttatgtttat ctcaattccc tggtggtatt ggtgtctgct gggttttgcc agaatgaaga 660
caccgtgttt tcatttgtca gttgattcgt attttccagg aagacattct gagattacag 720

cattgtctta gtcaaggtgc tgcagaagga cagaactaat aggatatatg tacatatgaa 780
agaaagttta tgaagaactg gctcacacca tcacaaggca aagtcccatg acaggccatc 840
tgcaagctga ggagcgagga agccagcagt ggctcagccg gagtccaaca gcctcaaacg 900
gaatccaaca gttcaggctt cagtctgtgg ccaaatgccc agagaccccg gaaagctact 960
ggtgttagtc ccagagccgg aaggccaaag aacctggagt gtgatgtcca agggcaggag 1020
gaatggacag aagcatccag catggggtaa agacgaaagc cagaagactc agcaagctag 1080
cttacctact ttcttctgcc tgccttgttc tagccgcgct ggcagccggt tggagggtgc 1140
ccacccccac tgagggtgga tcttcctctc ctagtccact gactcaaatt tcagtctctc 1200
tgggagcacc atcacaccag aaacaatacc agccatctag ccacccttca gttcaccatc 1260
acaaccattg tcttattcat gaaacttctg cagacccacc ttaacctcca tcggtgactt 1320
ctacctgaag ccctctgatt gttgcccagt ggtgcttttt aaaataattt ccatagtttc 1380
ttctacacct ttagttggca ttctactgta aaggagagat tttattttct tactcattta 1440
tttgttagtt tatagtcacc accatatgga tgcagagttc tgtctcattc actgggaagt 1500
attctattgc agtcatgatt tattttgatg ttcacatccc agagttggtg agtgagcgcc 1560
ccttcacgct ggctcccgag tgctgacgtg tccccgtcct tctctgcact tttccttacc 1620
tcctggcctc agatattcca gggtcatttg ttctctccct gctccaaccc tgcagtcagc 1680
catctcccta gggacgttgg ttcctttatg gaaggtggca tttagaagcc aggatttggg 1740
ctgagcactg tggctcatgc ttgtaatccc agcacttggg gaggccgaag tgggcggatc 1800
gctggaggcc aagagtctga gaccagcctg gctaacatgg tgaaaccctt ccccgtctct 1860
actacaaata aaaaattagc tgggtgtgtt ggcacgtgcc tgtaatccca gttactcagg 1920
aggctgaagc accagaatct cttgaaccca ggaggccgag gttgcagtga gccaagattg 1980
caccactgca ctacagcttg ggtgacagcg cgagacaccg tctcaaaaag gataataatt 2040
taaaaaacag caggatttgg gtgagcagtg cgctcattgc ttctgggctc tctcggtgga 2100
cataggctag gaatgtaaga tgtatgtgcc tgtgtatata cacacgtctg tagctatgtc 2160
tatgttgcat acatgtgttt ttccaaaaac caaatccata accatg 2206

<210> 86
<211> 93
<212> PRT
<213> Homo sapiens

<400> 86

Met Asp Arg Ser Ile Gln His Gly Val Lys Thr Lys Ala Arg Arg Leu
1               5                   10                  15

Ser Lys Leu Ala Tyr Leu Leu Ser Ser Ala Cys Leu Val Leu Ala Ala
            20                  25                  30

Leu Ala Ala Gly Trp Arg Val Pro Thr Pro Thr Glu Gly Gly Ser Ser
        35                  40                  45

Ser Pro Ser Pro Leu Thr Gln Ile Ser Val Ser Leu Gly Ala Pro Ser
    50                  55                  60

His Gln Lys Gln Tyr Gln Pro Ser Ser His Pro Ser Val His His His
65                  70                  75                  80

Asn His Cys Leu Ile His Glu Thr Ser Ala Asp Pro Pro
                85                  90

<210> 87
<211> 21
<212> DNA
<213> künstliche Sequenz

<220>
<223> Oligonukleotid

<400> 87
aaactacgtg tggccaggat c                                                21

<210> 88
<211> 21
<212> DNA
<213> künstliche Sequenz

<220>
<223> Oligonukleotid

<400> 88
cgacatgagg actcaggaca c                                                21

<210> 89
<211> 455
<212> DNA
<213> Homo sapiens

<400> 89
gtgaagacag ggagctcaag tgacctcctc cagggtatat agctgtggtg tgggaagcat      60

catgagaaca cggtctttga tggggataat tactctgaat ctaccaggct gattaagcca     120

cagcagatca gcaggtgaga attcaactgt ccagatagaa aggtggacat ggaaaaattg     180

ggctttgcaa atggtcaccc aattcttgcc ttcctggtct ccagatcacc cttcctatac     240

cgccactctg gagaaagaag tacagaacgc taacaaggat ggcttggagt tgcagtggtc     300

acctcagatc ttaaggtcac tttggagatg gaacccctgt gactaggaat ggcagaagag     360

aaaggtagaa agagattgag tcctggggat gtggcagagc accatcctag ccccgtactg     420

cgtacttctg gacttccttt aaattgagag aaaca                                455

<210> 90
<211> 61
<212> PRT
<213> Homo sapiens

<400> 90

<210>  91
<211>  20
<212>  DNA
<213>  künstliche Sequenz

<220>
<223>  Oligonukleotid

<400>  91
gaacacggtc tttgatgggg                                                  20

<210>  92
<211>  21
<212>  DNA
<213>  künstliche Sequenz

<220>
<223>  Oligonukleotid

<400>  92
gccatccttg ttagcgttct g                                                21

<210>  93
<211>  1230
<212>  DNA
<213>  Homo sapiens

<400>  93
   aggggcagag gggtcttccc aaccctaccc ctattttcgg tgatttttgt gtgagaatat      60
   taatattaaa aataaacgga gaaaaaaaat cctgtttcgc taacggctgg tggtagcagg     120
   ttgagtaccg ggagggctgc aagaccgtga ttgatgggga ggactgcgca gaccctggcg     180
   agggtgagcc cctccccgga ggcgcctgtg gaatgtccag ggctctggtc cgctcctcgg     240
   gatggggggt gcctaatcct agagccgcat tccaggataa ggggggtggg gagaggctgg     300
   gccgggggag gggcaggaaa gagggctata agggcagcgg cccaggcggg cgggatccag     360
   gcgggccatg gcggatgtcc ccggggcaca gcgagcggtt cctggtgacg gcccagagcc     420
   ccgggacccc ctggactgtt gggcctgcgc tgttcttgta acagcccaga atctgctggt     480
   ggctgccttc aatcttctcc tgctggtgct ggtgctaggg accatcttgc tacccgctgt     540
   caccatgctg ggcttcggct tcctctgcca ctctcagttc ctgcgctccc aggcaccccc     600
   ttgcaccgcg cacctgcggg accccggttt cacggcccta ctggtcaccg gattcctgct     660
   cctcgtgccg ctgctcgtgc ttgctctggc cagctaccgc cgcctctgcc tgcgcctccg 720
   cctagccgat tgcctcgtgc cctacagccg agccctttat cggcgtcggc gcgccccgca 780
   gccgcggcaa atccgggcct caccagggtc ccaggccgtt cccacatcag gaaaggtctg 840
   ggtctaatga ccctcgagtc aagaacaacc ctgacggctg ccctccctct tattcggccc 900
   aaggacttga agcccggcat cttccgacct gccctgcccc cacccctgcc tgagcggagt 960
   cctagcatcc ccttgggagc agcagcgtca gtggacccag tgctgagaaa agcccccaca 1020
   tcccggaaaa cccactttcc tttcacgacc cacatctcaa tcctgaacat ctaggctgga 1080
   acctgcacac ctccccctca gctccgtcgt gaatgggaca acaatctcgt gccctcgttt 1140
   tatggtgcag cttctctagt atttctgggg ctggggggcg gggctggagg ggaaggagtg 1200
   tccacgcatc aataaagatt taacgaactg 1230

<210> 94
<211> 159
<212> PRT
<213> Homo sapiens

<400> 94

Met Ala Asp Val Pro Gly Ala Gln Arg Ala Val Pro Gly Asp Gly Pro
1               5                   10                  15

Glu Pro Arg Asp Pro Leu Asp Cys Trp Ala Cys Ala Val Leu Val Thr
            20                  25                  30

Ala Gln Asn Leu Leu Val Ala Ala Phe Asn Leu Leu Leu Leu Val Leu
        35                  40                  45

Val Leu Gly Thr Ile Leu Leu Pro Ala Val Thr Met Leu Gly Phe Gly
    50                  55                  60

Phe Leu Cys His Ser Gln Phe Leu Arg Ser Gln Ala Pro Pro Cys Thr
65                  70                  75                  80

Ala His Leu Arg Asp Pro Gly Phe Thr Ala Leu Leu Val Thr Gly Phe
                85                  90                  95

Leu Leu Leu Val Pro Leu Leu Val Leu Ala Leu Ala Ser Tyr Arg Arg
            100                 105                 110

Leu Cys Leu Arg Leu Arg Leu Ala Asp Cys Leu Val Pro Tyr Ser Arg
        115                 120                 125

Ala Leu Tyr Arg Arg Arg Arg Ala Pro Gln Pro Arg Gln Ile Arg Ala
    130                 135                 140

Ser Pro Gly Ser Gln Ala Val Pro Thr Ser Gly Lys Val Trp Val
145                 150                 155

<210> 95
<211> 21
<212> DNA
<213> künstliche Sequenz

<220>
<223> Oligonukleotid

<400> 95
ttcctctgcc actctcagtt c 21


<210> 96
<211> 21
<212> DNA
<213> künstliche Sequenz

<220>
<223> Oligonukleotid

<400> 96
cgataaaggg ctcggctgta g 21


<210> 97
<211> 1020
<212> DNA
<213> Homo sapiens

<400> 97
atggaggagg aggaggagga tgatgactat gagaactcaa cacctcccta caaggacctt 60

cctcccaagc cagggaccat ggaggaggag gaggaggatg atgactatga gaactcaaca 120

cctccctaca aggaccttcc tcccaagcca gggaccatgg aggaggagga ggaggatgat 180

gactatgaga actcaacacc tccctacaag gaccttcctc ccaagccagg ttcaagtgct 240

ccaccaagac ctccaagggc agcaaaggaa acagagaaac ccccacttcc ttgcaagccc 300

cggaacatga caggcctgga cctcgccgct gtcacctgtc cacctcctca actggctgtg 360

aatcttgagc cttctccatt gcagccatcc ctggccgcaa ctccagtccc ctggctcaat 420

cagaggtctg gaggtcctgg ctgctgccag aagaggtgga tggtgtacct gtgtctgctg 480

gtggtgactt ccctgttcct gggctgcctt ggtctcactg tgaccctgat taagttgact 540

ggcatggcag ggctagctgg cctgaagcat gacattgccc gtgtaagagc tgacaccaac 600

cagtccctgg tggaactttg gggcttatta gactgccgcc gaattacctg tcctgaaggc 660

tggctgccct ttgagggcaa gtgttactac ttctccccaa gcaccaagtc atgggatgag 720

gcccggatgt tctgccagga gaattactct cacttggtca tcatcaatag ctttgctgag 780

cacaattttg tggccaaggc ccatggctct ccacgggtgt actggctggg gctgaatgac 840

agggcccagg aaggggactg gaggtggctg gatgggtctc ctgtgacatt aaggcaacca 900

gaggaaccca ataacatcca cgatgaggac tgtgctacca tgaacaaagg tggcacctgg 960

aatgatctct cttgctacaa aactacgtat tggatttgtg agcggaaatg ttcctgttga 1020


<210> 98
<211> 339
<212> PRT

<213>  Homo sapiens

<400>  98

Met Glu Glu Glu Glu Glu Asp Asp Asp Tyr Glu Asn Ser Thr Pro Pro
1               5                   10                  15

Tyr Lys Asp Leu Pro Pro Lys Pro Gly Thr Met Glu Glu Glu Glu Glu
            20                  25                  30

Asp Asp Asp Tyr Glu Asn Ser Thr Pro Pro Tyr Lys Asp Leu Pro Pro
        35                  40                  45

Lys Pro Gly Thr Met Glu Glu Glu Glu Glu Asp Asp Asp Tyr Glu Asn
    50                  55                  60

Ser Thr Pro Pro Tyr Lys Asp Leu Pro Pro Lys Pro Gly Ser Ser Ala
65                  70                  75                  80

Pro Pro Arg Pro Pro Arg Ala Ala Lys Glu Thr Glu Lys Pro Pro Leu
                85                  90                  95

Pro Cys Lys Pro Arg Asn Met Thr Gly Leu Asp Leu Ala Ala Val Thr
            100                 105                 110

Cys Pro Pro Pro Gln Leu Ala Val Asn Leu Glu Pro Ser Pro Leu Gln
        115                 120                 125

Pro Ser Leu Ala Ala Thr Pro Val Pro Trp Leu Asn Gln Arg Ser Gly
    130                 135                 140

Gly Pro Gly Cys Cys Gln Lys Arg Trp Met Val Tyr Leu Cys Leu Leu
145                 150                 155                 160

Val Val Thr Ser Leu Phe Leu Gly Cys Leu Gly Leu Thr Val Thr Leu
                165                 170                 175

Ile Lys Leu Thr Gly Met Ala Gly Leu Ala Gly Leu Lys His Asp Ile
            180                 185                 190

Ala Arg Val Arg Ala Asp Thr Asn Gln Ser Leu Val Glu Leu Trp Gly
        195                 200                 205

Leu Leu Asp Cys Arg Arg Ile Thr Cys Pro Glu Gly Trp Leu Pro Phe
    210                 215                 220

Glu Gly Lys Cys Tyr Tyr Phe Ser Pro Ser Thr Lys Ser Trp Asp Glu
225                 230                 235                 240

Ala Arg Met Phe Cys Gln Glu Asn Tyr Ser His Leu Val Ile Ile Asn
                245                 250                 255

Ser Phe Ala Glu His Asn Phe Val Ala Lys Ala His Gly Ser Pro Arg
            260                 265                 270

Val Tyr Trp Leu Gly Leu Asn Asp Arg Ala Gln Glu Gly Asp Trp Arg
        275                 280                 285

Trp Leu Asp Gly Ser Pro Val Thr Leu Arg Gln Pro Glu Glu Pro Asn
    290                 295                 300

Asn Ile His Asp Glu Asp Cys Ala Thr Met Asn Lys Gly Gly Thr Trp
305                 310                 315                 320

Asn Asp Leu Ser Cys Tyr Lys Thr Thr Tyr Trp Ile Cys Glu Arg Lys
                325                 330                 335

Cys Ser Cys

<210> 99
<211> 21
<212> DNA
<213> künstliche Sequenz

<220>
<223> Oligonukleotid

<400> 99
atagctttgc tgagcacctt c                                                21

<210> 100
<211> 21
<212> DNA
<213> künstliche Sequenz

<220>
<223> Oligonukleotid

<400> 100
aagagacact cagatatgga c                                                21

<210> 101
<211> 1680
<212> DNA
<213> Homo sapiens

<400> 101
atggccaatg tcaccttggt gacaggattt cttcttatgg ggttttctaa tatccagaag      60

ctgcggattt tatatggtgt gctcttccta ctgatttacc tggcagccct aatgagtaac     120

cttctcatca ttactctcat taccctggac gtaaagctcc aaacacccat gtacttcttc     180

ctgaagaact tatccttttt ggatgtcttc ctggtgtctg ttccaatccc aaaattcatt     240

gtcaacaacc taacccacaa caattccatt tccattctag gatgtgcctt ccagctactt     300

ttaatgactt ccttctcagc aggagagata tttatcctca ctgccatgtc ctatgaccgc     360

tatgtagcca tctgctgtcc cctgaactac gaggtaatca tgaatactgg agtctgtgtg     420

ttaatggcaa gtgtttcctg ggccattgga gggctctttg gtactgcgta cacagctggc 480
acattttcca tgcctttctg tggctccagt gtgattccac agtttttctg tgatgttcct 540
tcattactaa ggatttcctg ttctgaaaca ctaatggtaa tttatgcagg tattggagtt 600
ggtgcatgtt taagcatttc ttgtttcatc tgtattgtga tctcttacat ttatatcttc 660
tccactgtac tgaagatccc taccactaaa ggactgtgtg attgggttaa agggctcagt 720
gcggggactc tgttttctgg tttcagtacc acaatggaca caggcaacaa aactctgccc 780
caggactttc tcttactggg ctttcctggt tctcaaactc ttcagctctc tctctttatg 840
ctttttctgg tgatgtacat cctcacagtt agtggtaatg tggctatctt gatgttggtg 900
agcacctccc atcagttgca tacccccatg tacttctttc tgagcaacct ctccttcctg 960
gagatttggt ataccacagc agcagtgccc aaagcactgg ccatcctact ggggagaagt 1020
cagaccatat catttacaag ctgtcttttg cagatgtact ttgttttctc attaggctgc 1080
acagagtact tcctcctggc agccatggct tatgaccgct gtcttgccat ctgctatcct 1140
ttacactacg gagccatcat gagtagcctg ctctcagcgc agctggccct gggctcctgg 1200
gtgtgtggtt tcgtggccat tgcagtgccc acagccctca tcagtggcct gtccttctgt 1260
ggcccccgtg ccatcaacca cttcttctgt gacattgcac cctggattgc cctggcctgc 1320
accaacacac aggcagtaga gcttgtggcc tttgtgattg ctgttgtggt tatcctgagt 1380
tcatgcctca tcacctttgt ctcctatgtg tacatcatca gcaccatcct caggatcccc 1440
tctgccagtg gccggagcaa agccttctcc acgtgctcct cgcatctcac cgtggtgctc 1500
atttggtatg ggtccacagt tttccttcac gtccgcacct ctatcaaaga tgccttggat 1560
ctgatcaaag ctgtccacgt cctgaacact gtggtgactc cagttttaaa ccccttcatc 1620
tatacgcttc gtaataagga agtaagagag actctgctga agaaatggaa gggaaaataa 1680

<210> 102
<211> 559
<212> PRT
<213> Homo sapiens

<400> 102

Met Ala Asn Val Thr Leu Val Thr Gly Phe Leu Leu Met Gly Phe Ser
1               5                   10                  15

Asn Ile Gln Lys Leu Arg Ile Leu Tyr Gly Val Leu Phe Leu Leu Ile
            20                  25                  30

Tyr Leu Ala Ala Leu Met Ser Asn Leu Leu Ile Ile Thr Leu Ile Thr
        35                  40                  45

Leu Asp Val Lys Leu Gln Thr Pro Met Tyr Phe Phe Leu Lys Asn Leu
    50                  55                  60

Ser Phe Leu Asp Val Phe Leu Val Ser Val Pro Ile Pro Lys Phe Ile
65                  70                  75                  80

Val Asn Asn Leu Thr His Asn Asn Ser Ile Ser Ile Leu Gly Cys Ala
                85                  90                  95

Phe Gln Leu Leu Leu Met Thr Ser Phe Ser Ala Gly Glu Ile Phe Ile
            100                 105                 110

Leu Thr Ala Met Ser Tyr Asp Arg Tyr Val Ala Ile Cys Cys Pro Leu
        115                 120                 125

Asn Tyr Glu Val Ile Met Asn Thr Gly Val Cys Val Leu Met Ala Ser
    130                 135                 140

Val Ser Trp Ala Ile Gly Gly Leu Phe Gly Thr Ala Tyr Thr Ala Gly
145                 150                 155                 160

Thr Phe Ser Met Pro Phe Cys Gly Ser Ser Val Ile Pro Gln Phe Phe
                165                 170                 175

Cys Asp Val Pro Ser Leu Leu Arg Ile Ser Cys Ser Glu Thr Leu Met
            180                 185                 190

Val Ile Tyr Ala Gly Ile Gly Val Gly Ala Cys Leu Ser Ile Ser Cys
        195                 200                 205

Phe Ile Cys Ile Val Ile Ser Tyr Ile Tyr Ile Phe Ser Thr Val Leu
    210                 215                 220

Lys Ile Pro Thr Thr Lys Gly Leu Cys Asp Trp Val Lys Gly Leu Ser
225                 230                 235                 240

Ala Gly Thr Leu Phe Ser Gly Phe Ser Thr Thr Met Asp Thr Gly Asn
                245                 250                 255

Lys Thr Leu Pro Gln Asp Phe Leu Leu Leu Gly Phe Pro Gly Ser Gln
            260                 265                 270

Thr Leu Gln Leu Ser Leu Phe Met Leu Phe Leu Val Met Tyr Ile Leu
        275                 280                 285

Thr Val Ser Gly Asn Val Ala Ile Leu Met Leu Val Ser Thr Ser His
    290                 295                 300

Gln Leu His Thr Pro Met Tyr Phe Phe Leu Ser Asn Leu Ser Phe Leu
305                 310                 315                 320

Glu Ile Trp Tyr Thr Thr Ala Ala Val Pro Lys Ala Leu Ala Ile Leu
                325                 330                 335

Leu Gly Arg Ser Gln Thr Ile Ser Phe Thr Ser Cys Leu Leu Gln Met
            340                 345                 350

Tyr Phe Val Phe Ser Leu Gly Cys Thr Glu Tyr Phe Leu Leu Ala Ala
        355                 360                 365

Met Ala Tyr Asp Arg Cys Leu Ala Ile Cys Tyr Pro Leu His Tyr Gly
    370                 375                 380

Ala Ile Met Ser Ser Leu Leu Ser Ala Gln Leu Ala Leu Gly Ser Trp
385                 390                 395                 400

Val Cys Gly Phe Val Ala Ile Ala Val Pro Thr Ala Leu Ile Ser Gly
                405                 410                 415

Leu Ser Phe Cys Gly Pro Arg Ala Ile Asn His Phe Phe Cys Asp Ile
            420                 425                 430

Ala Pro Trp Ile Ala Leu Ala Cys Thr Asn Thr Gln Ala Val Glu Leu
        435                 440                 445

Val Ala Phe Val Ile Ala Val Val Val Ile Leu Ser Ser Cys Leu Ile
    450                 455                 460

Thr Phe Val Ser Tyr Val Tyr Ile Ile Ser Thr Ile Leu Arg Ile Pro
465                 470                 475                 480

Ser Ala Ser Gly Arg Ser Lys Ala Phe Ser Thr Cys Ser Ser His Leu
                485                 490                 495

Thr Val Val Leu Ile Trp Tyr Gly Ser Thr Val Phe Leu His Val Arg
            500                 505                 510

Thr Ser Ile Lys Asp Ala Leu Asp Leu Ile Lys Ala Val His Val Leu
        515                 520                 525

Asn Thr Val Val Thr Pro Val Leu Asn Pro Phe Ile Tyr Thr Leu Arg
    530                 535                 540

Asn Lys Glu Val Arg Glu Thr Leu Leu Lys Lys Trp Lys Gly Lys
545                 550                 555

<210>  103
<211>  25
<212>  DNA
<213>  künstliche Sequenz

<220>
<223>  Oligonukleotid

<400>  103
catttcttgt ttcatctgta ttgtg                                          25

<210>  104
<211>  20

<212>  DNA
<213>  künstliche Sequenz

<220>
<223>  Oligonukleotid

<400>  104
tgttgcctgt gtccattgtg                                                    20


<210>  105
<211>  499
<212>  DNA
<213>  Homo sapiens

<400>  105
acacccacat ggtcggcgtg caggatattt cgctggaccc tagaaaagcc accacgacct      60

gtgggccatg atgctacccc aatggctgct gctgctgttc cttctcttct tctttctctt     120

cctcctcacc aggggctcac tttctccaac aaaatacaac cttttggagc tcaaggagtc     180

ttgcatccgg aaccaggact gcgagactgg ctgctgccaa cgtgctccag acaattgcga     240

gtcgcactgc gcggagaagg ggtccgaggg cagtctgtgt caaacgcagg tgttctttgg     300

ccaatataga gcgtgtccct gcctgcggaa cctgacttgt atatattcaa agaatgagaa     360

atggcttagc atcgcctatg gccgttgtca gaaaattgga aggcagaagt tggctaagaa     420

aatgttcttc tagtgctccc tccttcttgc tgcctcctcc tcctccacct gctcctcctc     480

ctacccagag ctctgtgtt                                                  499


<210>  106
<211>  121
<212>  PRT
<213>  Homo sapiens

<400>  106

Met Met Leu Pro Gln Trp Leu Leu Leu Leu Phe Leu Leu Phe Phe Phe
1               5                   10                  15


Leu Phe Leu Leu Thr Arg Gly Ser Leu Ser Pro Thr Lys Tyr Asn Leu
            20                  25                  30


Leu Glu Leu Lys Glu Ser Cys Ile Arg Asn Gln Asp Cys Glu Thr Gly
        35                  40                  45


Cys Cys Gln Arg Ala Pro Asp Asn Cys Glu Ser His Cys Ala Glu Lys
    50                  55                  60


Gly Ser Glu Gly Ser Leu Cys Gln Thr Gln Val Phe Phe Gly Gln Tyr
65                  70                  75                  80


Arg Ala Cys Pro Cys Leu Arg Asn Leu Thr Cys Ile Tyr Ser Lys Asn
                85                  90                  95


Glu Lys Trp Leu Ser Ile Ala Tyr Gly Arg Cys Gln Lys Ile Gly Arg
            100                 105                 110

Gln Lys Leu Ala Lys Lys Met Phe Phe
        115                 120

<210>  107
<211>  17
<212>  DNA
<213>  künstliche Sequenz

<220>
<223>  Oligonukleotid

<400>  107
tgtgtcaaac gcaggtg                                                    17

<210>  108
<211>  20
<212>  DNA
<213>  künstliche Sequenz

<220>
<223>  Oligonukleotid

<400>  108
ggagggagca ctagaagaac                                                 20

<210>  109
<211>  659
<212>  DNA
<213>  Homo sapiens

<400>  109
agcaaattac accattaatg tcatcctggc gaatgaaaca agagaatagt atttatcaga      60

gaaagtctgg tgagttgaag tccaagaccc caggaaacaa ctagccctgc tgggctgccc     120

ctccttcgga gtgggactat atgatcctca tcaggccaat ccacgtcaca gaatggtcta     180

ggcattggat gagtgcctca atctgagcca atgaaggtca ttgctgagac attttactgg     240

ttgccaggct gcaggcatcc caggcttcct gctgccctca tgtctacaac ctgtcgtctg     300

gaacattcca ggagccactt ttatcacttg cagcaatctt cttcagtgag ttccccagga     360

cttgatttca tcttacaatc tgattccatg tgtctcccat attttaagga ttctttatta     420

tttctggctt acagagaaca aacattattt tttgctttcc tggtctgttc tagattttca     480

aaaataactc tgtcacttct gttatatggt atcattgctt gtaattatct atttacttat     540

ctgtctctgg actggactct ttacagacag gcaataacta attatctgtc tgtctggcat     600

ttggtagtca ctcataaatc gtttattgca ttactaacta aataaaaaag ttgaccttg      659

<210>  110
<211>  144
<212>  PRT
<213>  Homo sapiens

<400>  110

<210>  111
<211>  25
<212>  DNA
<213>  künstliche Sequenz

<220>
<223>  Oligonukleotid

<400>  111
atcctggcga atgaaacaag agaat                                          25

<210>  112
<211>  26
<212>  DNA
<213>  künstliche Sequenz

<220>
<223>  Oligonukleotid

<400>  112
gcaaccagta aaatgtctca gcaatg                                         26

<210>  113
<211>  831
<212>  DNA
<213>  Homo sapiens

<400>  113
atgcgaagaa agaacctcac agaggtaaca gagtttgttt tcctgggatt ctccagattc      60

cacaaacatc acatcactct ctttgtggtt tttctcatcc tgtacacatt aactgtggct     120

ggcaatgcca tcatcatgac catcatctgc attgaccgtc acctccacac tcccatgtac 180

ttcttcctga gcatgctggc tagctcaaag acagtgtaca cactgttcat cattccacag 240

atgctctcca gcttcgtaac ccagacccag ccaatctccc tagcaggttg taccacccaa 300

acgttcttct ttgttacctt ggccatcaac aattgcttct tgctcacagt gatgggctat 360

gaccactata tggccatctg caatcccttg agatacaggg tcattacgag caagaaggtg 420

tgtgtccagc tggtgtgtgg agcctttagc attggcctgg ccatggcagc tgtccaggta 480

acatccatat ttaccttacc tttttgtcac acggtggttg gtcatttctt ctgtgacatc 540

ctccctgtca tgaaactctc ctgtattaat accactatca atgagataat caattttgtt 600

gtcaggttat ttgtcatcct ggtccccatg ggtctggtct tcatctccta tgtcctcatc 660

atctccactg tcctcaagat tgcctcagct gagggttgga agaagacctt tgccacctgt 720

gccttccacc tcactgtggt cattgtccat tatggctgtg cttccattgc ctacctcatg 780

cccaagtcag aaaactctat agaacaagac ctccttctct cagtgaccta a 831

<210> 114
<211> 276
<212> PRT
<213> Homo sapiens

<400> 114

Met Arg Arg Lys Asn Leu Thr Glu Val Thr Glu Phe Val Phe Leu Gly
1               5                   10                  15

Phe Ser Arg Phe His Lys His His Ile Thr Leu Phe Val Val Phe Leu
            20                  25                  30

Ile Leu Tyr Thr Leu Thr Val Ala Gly Asn Ala Ile Ile Met Thr Ile
        35                  40                  45

Ile Cys Ile Asp Arg His Leu His Thr Pro Met Tyr Phe Phe Leu Ser
    50                  55                  60

Met Leu Ala Ser Ser Lys Thr Val Tyr Thr Leu Phe Ile Ile Pro Gln
65                  70                  75                  80

Met Leu Ser Ser Phe Val Thr Gln Thr Gln Pro Ile Ser Leu Ala Gly
                85                  90                  95

Cys Thr Thr Gln Thr Phe Phe Phe Val Thr Leu Ala Ile Asn Asn Cys
            100                 105                 110

Phe Leu Leu Thr Val Met Gly Tyr Asp His Tyr Met Ala Ile Cys Asn
        115                 120                 125

Pro Leu Arg Tyr Arg Val Ile Thr Ser Lys Lys Val Cys Val Gln Leu
    130                 135                 140

Val Cys Gly Ala Phe Ser Ile Gly Leu Ala Met Ala Ala Val Gln Val
145                 150                 155                 160

Thr Ser Ile Phe Thr Leu Pro Phe Cys His Thr Val Val Gly His Phe
                165                 170                 175

Phe Cys Asp Ile Leu Pro Val Met Lys Leu Ser Cys Ile Asn Thr Thr
            180                 185                 190

Ile Asn Glu Ile Ile Asn Phe Val Val Arg Leu Phe Val Ile Leu Val
        195                 200                 205

Pro Met Gly Leu Val Phe Ile Ser Tyr Val Leu Ile Ile Ser Thr Val
    210                 215                 220

Leu Lys Ile Ala Ser Ala Glu Gly Trp Lys Lys Thr Phe Ala Thr Cys
225                 230                 235                 240

Ala Phe His Leu Thr Val Val Ile Val His Tyr Gly Cys Ala Ser Ile
                245                 250                 255

Ala Tyr Leu Met Pro Lys Ser Glu Asn Ser Ile Glu Gln Asp Leu Leu
            260                 265                 270

Leu Ser Val Thr
        275

<210>  115
<211>  18
<212>  DNA
<213>  künstliche Sequenz

<220>
<223>  Oligonukleotid

<400>  115
cttcgtaacc cagaccca                                                18

<210>  116
<211>  18
<212>  DNA
<213>  künstliche Sequenz

<220>
<223>  Oligonukleotid

<400>  116
cttgctcgta atgaccct                                                18

<210>  117
<211>  1233
<212>  DNA
<213>  Homo sapiens

<400>  117
gaagcagcca ccaccatctt gggagctctg ggagcaagga cccctgtaac acattcatcc      60

ttgaatgaca aaatgtctgg tccagcatgg tattataaca taaacatgaa gaggaagaga 120
catgagagat acgcacagtg aagagaccaa gctgggacac agtacgaagg tggcatctgc 180
acgccaagca gagggacctc agaagaaact gagccagcca gcaccccacc ttcgtctttg 240
acctccagcc tccagaacta aggatagagc tcttcatctc tgttagaaac gaccatcaaa 300
aagatacatc aattcattag aatcaaaagg acatgagtta tcagaattct ttctcctgaa 360
agaaagtgga gatcaaaggt aaaacttcta gagaatgaga tgaaggcaga tgaaagaagt 420
taacaagaca ttacatgact tgataatatt gcatgtatgc aaaaacctta tgaaatcaac 480
tgtgttctag cgaccacttg tttttctttt tgtcataata ctttttattc tcttgcaatg 540
atattgattc atctgcacct gacatcaact ctgcatttgt agaaggtgat aagaatacag 600
ggaaatggaa taagtggctt tgcctgcaat cccgcagcag cagaaatgtc catttcctct 660
ctcctgaata atactacatt ctccactggg ttccacaagt ttcgaggtaa aagcatgaac 720
atacacgaag tcaccatcac taccctcacc accaccacca ttatttccac catattcacc 780
cttttaatac gcaaacttcc tccaaggctt cctgaagtca cccagaaatg catttcccca 840
agagtgagtt gtgctaacat tgtatcctat ggaactctgg gaagctaccc agatcctcaa 900
ctcttggagt cttgctgact gcatgttcca ggctccacat ttaagctcca gtgactgctg 960
atgactgcat gacctaacac atgtcctcaa tcctttcttg gcctcagttt cttcaccagt 1020
gaattctgaa tgctggaatt ggcaatattt caggttcttt ccaactggaa atacccatgc 1080
taataatttt agtaagtcaa tagccataga aacctactga caaaatgagt attttaacag 1140
agacagttgt actttcttaa tttttagcag aagggaatgc atatgtataa tatctatgtt 1200
gccttctatg tgtaaaaata aatacacaga cac 1233

<210> 118
<211> 90
<212> PRT
<213> Homo sapiens

<400> 118

Met Ser Ile Ser Ser Leu Leu Asn Asn Thr Thr Phe Ser Thr Gly Phe
1               5                   10                  15

His Lys Phe Arg Gly Lys Ser Met Asn Ile His Glu Val Thr Ile Thr
            20                  25                  30

Thr Leu Thr Thr Thr Thr Ile Ile Ser Thr Ile Phe Thr Leu Leu Ile
        35                  40                  45

Arg Lys Leu Pro Pro Arg Leu Pro Glu Val Thr Gln Lys Cys Ile Ser
    50                  55                  60

Pro Arg Val Ser Cys Ala Asn Ile Val Ser Tyr Gly Thr Leu Gly Ser
65                  70                  75                  80

Tyr Pro Asp Pro Gln Leu Leu Glu Ser Cys
                85                  90

<210> 119
<211> 19
<212> DNA
<213> künstliche Sequenz

<220>
<223> Oligonukleotid

<400> 119
caccccacct tcgtctttg 19

<210> 120
<211> 24
<212> DNA
<213> künstliche Sequenz

<220>
<223> Oligonukleotid

<400> 120
gttttacctt tgatctccac tttc 24

<210> 121
<211> 4209
<212> DNA
<213> Homo sapiens

<400> 121
agttgcttga aagcaacgtg cctattcaca tggagaatct tccctttcct ttaaaattac 60

ttagtgcctc atcgctaaac gcccccagct ccacaccatg ggtgttggat atcttcctca 120

ccttggtgtt tgccctgggg ttcttcttcc tattactccc ctacttatct tacttccatt 180

gtgatgaccc accctcacca tcgcctggga agagaaagtg tccagtaggg cggaggcgga 240

ggcccagagg caggatgaaa aaccacagtc tgagagctgg tagagagtgc ccgagaggcc 300

tggaggagac ttcggacctt ctttcacaac tgcagagcct cctggggcca caccttgaca 360

aaggtgactt tggtcagctc tccggtccag accccccagg tgaggtgggc gaaagagcac 420

ctgatggagc ctcccagtcc tctcatgagc ctatggaaga tgctgctccc attctctccc 480

cgttagcttc ccccgatcct caagccaagc atcctcagga tctggcctcc accccatcac 540

caggcccaat gaccacctca gtctcctccc taagtgcctc ccagccacca gaaccttccc 600

ttcccctaga acacccctca cccgagccac ctgcactttt ccctcaccca ccacacaccc 660

ctgatcctct ggcctgctct ccgcctcctc caaaaggctt cactgctcct cccctgcggg 720

actccacact gataactcca tctcactgtg actcagtggc acttccactg ggcaccgtcc 780

ctcaaagctt gtctccacat gaggatttgg tggcttctgt cccagccatc tcaggccttg 840

gtggctcaaa cagtcatgtt tctgcctcct cccggtggca ggagactgcc agaacctcgt 900

gcgcctttaa ctcatcagtc cagcaagatc ctctttcccg ccacccacca gagacctgtc 960

agatggaagc tggtagcctg tttttgctca gctctgatgg ccagaatgtc gtggggatac 1020

aagtcacaga aacagccaag gtcaacattt gggaagaaaa agaaaatgtt ggatcattta 1080
caaatcaaat gaccccagaa aagcacttaa attctttggg gaatttggct aaatcattgg 1140
atgctgagca ggacaccaca aacccaaaac ccttctggaa catgggagag aactcgaaac 1200
agctgcccgg acctcagaag tgctcagatc ctaggctctt gcaggaaagt ttttggaaga 1260
attatagcca gcttttctgg ggcctcccct ctctgcacag cgagtccctg gtggctaacg 1320
cctgggtaac tgacaggtct tatactttac agtctcctcc tttcttgttc aatgaaatgt 1380
ccaatgtctg cccaattcaa agggagacta caatgtcccc actgcttttc caggcccagc 1440
ccctgtccca ccgccaaccc tttatttcat ccacacccca attcctgccc acacctatgg 1500
ctcaggccga ggctcaggcc catcttcagt cttctttccc agtcctatct cctgcttttc 1560
catccctgat taagaacact ggagtagctt gccctgcatc gcagaataaa gtgcaagctc 1620
tctccctacc tgaaactcag caccctgaat ggcctttgtt gaggaaacaa ctagaaggta 1680
ggttggcttt accctctagg gtccaaaaat ctcaggacgt ctttagtgtc tccactccta 1740
accttcccca ggaaagtttg acatccattc tgcctgagaa ctttccagtc agtcctgaac 1800
tccggagaca actggagcaa cacataaaaa agtggatcat ccaacactgg ggcaacctgg 1860
gaaggatcca agagtctctg gatctgatgc agcttcggga cgaatcacca gggacaagtc 1920
aggccaaggg caaacccagt ccctggcagt cctccacgtc cacaggtgaa agcagcaagg 1980
aggcacagaa ggtgaagttc cagctagaga gggacctgtg cccacatctg gggcaaattc 2040
tgggtgagac cccacaaaat ctatccaggg acatgaaaag cttcccacgg aaggttctgg 2100
gggtgacttc tgaggagtcg gaaaggaact tgaggaagcc cttgaggagt gactcgggaa 2160
gtgatttatt aagatgcaca gagaggactc atatagaaaa catcctgaaa gcccacatgg 2220
gcaggaactt gggccagacc aacgagggct tgatccccgt gcgtgtgcgt cgatcctggc 2280
ttgctgtcaa ccaggctctt cccgtgtcca acacccatgt gaaaaccagc aatctagcag 2340
ccccgaaaag tgggaaagcc tgtgtgaaca cagcccaggt gctttccttc ctcgagccgt 2400
gtactcagca ggggttggga gcccatattg tgaggttttg ggccaaacac aggtggggtc 2460
tacccctcag ggtcctcaag cccattcagt gctttaaact ggaaaaggtt tcatccttgt 2520
cccttacgca gcttgctggt ccctcctcag ccacctgtga atctggggct ggctcagaag 2580
ttgaggtgga catgttcctt agaaagccac caatggcaag tctgagaaag caggtgctga 2640
ccaaagcatc tgatcacatg ccagagagtc ttctggcctc ctcacctgca tggaagcagt 2700
tccagagggc accgcgagga atcccatctt ggaatgatca tgggcccttg aagcctcctc 2760
cagctggaca ggagggcagg tggccatcta agcccctcac gtacagcctc acaggcagca 2820
cccagcagag caggagctta ggagcccaat cttcaaaggc tggagagaca agggaggcag 2880
tgccacaatg cagagtcccc ttggaaacct gtatgctggc aaacctccaa gccacaagtg 2940
aggatgtgca tggtttcgag gctccaggga ccagcaaaag ctctctacac cctagagtgt 3000
ctgtctccca agatccaaga aagctgtgtc ttatggagga ggttgttagt gaatttgagc 3060

ctggaatggc cacaaagtca gagacccagc ctcaagtttg tgccgctgtt gtgctccttc 3120
cagatgggca agcatctgtt gtgccccacg cttcagagaa tttggtttct caagtgcccc 3180
agggccatct ccagagcatg cctactggga acatgcgggc ttcccaggag ctacatgacc 3240
tcatggcagc cagaaggagc aaactggtgc aagaggagcc cagaaaccca aactgtcaag 3300
gctcatgcaa gagccaaagg ccaatgtttc cccctattca caagagtgag aagtctagga 3360
agcccaactt agaaaaacat gaagaaaggc ttgaaggatt gaggactcct caacttaccc 3420
cagtcaggaa aacagaagac acccatcagg atgaaggcgt ccagctactg ccatcaaaga 3480
aacagcctcc ttcagtaagc cactttggag aaaacatcaa gcaatttttt cagtggattt 3540
tttcaaagaa aaaaagcaag ccagcaccag tcactgctga gagccaaaaa acagtaaaaa 3600
acagatcatg tgtgtacagc agcagtgctg aagctcaggg tctcatgacg gcagttggac 3660
aaatgctgga caagaaaatg tcactttgcc atgcgcacca tgcctcgaag gtaaatcagc 3720
acaaacagaa gtttcaagcc ccagtctgtg ggtttccctg caaccacagg cacctcttct 3780
actcagaaca tggcagaata ctgagctatg cagccagcag tcaacaagcc actctcaaga 3840
gccagggttg tcccaacaga gacaggcaaa tcagaaatca acagcccttg aaaagtgtgc 3900
ggtgcaacaa tgagcaatgg ggcctgcgac atccccaaat cttgcacccc aagaaagctg 3960
tatccccagt cagtccccct cagcactggc cgaagacatc cggtgcctct agccaccatc 4020
accactgtcc aaggcactgt cttctttggg aaggtatctg atttggtcag tcacaaattc 4080
ttttttagcc ttccctggag aaaaacaagt ccccaagaaa aaattcactc tatgtagaga 4140
aaaaatattt tctctcatgt tagtaaatgc agaacattta atattccaca atatatatgg 4200
ttttttatt 4209

<210> 122
<211> 1343
<212> PRT
<213> Homo sapiens

<400> 122

Met Glu Asn Leu Pro Phe Pro Leu Lys Leu Leu Ser Ala Ser Ser Leu
1               5                   10                  15

Asn Ala Pro Ser Ser Thr Pro Trp Val Leu Asp Ile Phe Leu Thr Leu
            20                  25                  30

Val Phe Ala Leu Gly Phe Phe Phe Leu Leu Leu Pro Tyr Leu Ser Tyr
        35                  40                  45

Phe His Cys Asp Asp Pro Pro Ser Pro Ser Pro Gly Lys Arg Lys Cys
    50                  55                  60

Pro Val Gly Arg Arg Arg Arg Pro Arg Gly Arg Met Lys Asn His Ser
65                  70                  75                  80

Leu Arg Ala Gly Arg Glu Cys Pro Arg Gly Leu Glu Glu Thr Ser Asp
                85                  90                  95

Leu Leu Ser Gln Leu Gln Ser Leu Leu Gly Pro His Leu Asp Lys Gly
            100                 105                 110

Asp Phe Gly Gln Leu Ser Gly Pro Asp Pro Pro Gly Glu Val Gly Glu
        115                 120                 125

Arg Ala Pro Asp Gly Ala Ser Gln Ser Ser His Glu Pro Met Glu Asp
    130                 135                 140

Ala Ala Pro Ile Leu Ser Pro Leu Ala Ser Pro Asp Pro Gln Ala Lys
145                 150                 155                 160

His Pro Gln Asp Leu Ala Ser Thr Pro Ser Pro Gly Pro Met Thr Thr
                165                 170                 175

Ser Val Ser Ser Leu Ser Ala Ser Gln Pro Pro Glu Pro Ser Leu Pro
            180                 185                 190

Leu Glu His Pro Ser Pro Glu Pro Pro Ala Leu Phe Pro His Pro Pro
        195                 200                 205

His Thr Pro Asp Pro Leu Ala Cys Ser Pro Pro Pro Pro Lys Gly Phe
    210                 215                 220

Thr Ala Pro Pro Leu Arg Asp Ser Thr Leu Ile Thr Pro Ser His Cys
225                 230                 235                 240

Asp Ser Val Ala Leu Pro Leu Gly Thr Val Pro Gln Ser Leu Ser Pro
                245                 250                 255

His Glu Asp Leu Val Ala Ser Val Pro Ala Ile Ser Gly Leu Gly Gly
            260                 265                 270

Ser Asn Ser His Val Ser Ala Ser Ser Arg Trp Gln Glu Thr Ala Arg
        275                 280                 285

Thr Ser Cys Ala Phe Asn Ser Ser Val Gln Gln Asp Pro Leu Ser Arg
    290                 295                 300

His Pro Pro Glu Thr Cys Gln Met Glu Ala Gly Ser Leu Phe Leu Leu
305                 310                 315                 320

Ser Ser Asp Gly Gln Asn Val Val Gly Ile Gln Val Thr Glu Thr Ala
                325                 330                 335

Lys Val Asn Ile Trp Glu Glu Lys Glu Asn Val Gly Ser Phe Thr Asn
            340                 345                 350

Gln Met Thr Pro Glu Lys His Leu Asn Ser Leu Gly Asn Leu Ala Lys
        355                 360                 365

Ser Leu Asp Ala Glu Gln Asp Thr Thr Asn Pro Lys Pro Phe Trp Asn
    370                 375                 380

Met Gly Glu Asn Ser Lys Gln Leu Pro Gly Pro Gln Lys Cys Ser Asp
385                 390                 395                 400

Pro Arg Leu Leu Gln Glu Ser Phe Trp Lys Asn Tyr Ser Gln Leu Phe
                405                 410                 415

Trp Gly Leu Pro Ser Leu His Ser Glu Ser Leu Val Ala Asn Ala Trp
            420                 425                 430

Val Thr Asp Arg Ser Tyr Thr Leu Gln Ser Pro Pro Phe Leu Phe Asn
        435                 440                 445

Glu Met Ser Asn Val Cys Pro Ile Gln Arg Glu Thr Thr Met Ser Pro
    450                 455                 460

Leu Leu Phe Gln Ala Gln Pro Leu Ser His Arg Gln Pro Phe Ile Ser
465                 470                 475                 480

Ser Thr Pro Gln Phe Leu Pro Thr Pro Met Ala Gln Ala Glu Ala Gln
                485                 490                 495

Ala His Leu Gln Ser Ser Phe Pro Val Leu Ser Pro Ala Phe Pro Ser
            500                 505                 510

Leu Ile Lys Asn Thr Gly Val Ala Cys Pro Ala Ser Gln Asn Lys Val
        515                 520                 525

Gln Ala Leu Ser Leu Pro Glu Thr Gln His Pro Glu Trp Pro Leu Leu
    530                 535                 540

Arg Lys Gln Leu Glu Gly Arg Leu Ala Leu Pro Ser Arg Val Gln Lys
545                 550                 555                 560

Ser Gln Asp Val Phe Ser Val Ser Thr Pro Asn Leu Pro Gln Glu Ser
                565                 570                 575

Leu Thr Ser Ile Leu Pro Glu Asn Phe Pro Val Ser Pro Glu Leu Arg
            580                 585                 590

Arg Gln Leu Glu Gln His Ile Lys Lys Trp Ile Ile Gln His Trp Gly
        595                 600                 605

Asn Leu Gly Arg Ile Gln Glu Ser Leu Asp Leu Met Gln Leu Arg Asp
    610                 615                 620

Glu Ser Pro Gly Thr Ser Gln Ala Lys Gly Lys Pro Ser Pro Trp Gln
625                 630                 635                 640

Ser Ser Thr Ser Thr Gly Glu Ser Ser Lys Glu Ala Gln Lys Val Lys
                645                 650                 655

Phe Gln Leu Glu Arg Asp Leu Cys Pro His Leu Gly Gln Ile Leu Gly
            660                 665                 670

Glu Thr Pro Gln Asn Leu Ser Arg Asp Met Lys Ser Phe Pro Arg Lys
        675                 680                 685

Val Leu Gly Val Thr Ser Glu Glu Ser Glu Arg Asn Leu Arg Lys Pro
    690                 695                 700

Leu Arg Ser Asp Ser Gly Ser Asp Leu Leu Arg Cys Thr Glu Arg Thr
705                 710                 715                 720

His Ile Glu Asn Ile Leu Lys Ala His Met Gly Arg Asn Leu Gly Gln
                725                 730                 735

Thr Asn Glu Gly Leu Ile Pro Val Arg Val Arg Arg Ser Trp Leu Ala
            740                 745                 750

Val Asn Gln Ala Leu Pro Val Ser Asn Thr His Val Lys Thr Ser Asn
        755                 760                 765

Leu Ala Ala Pro Lys Ser Gly Lys Ala Cys Val Asn Thr Ala Gln Val
    770                 775                 780

Leu Ser Phe Leu Glu Pro Cys Thr Gln Gln Gly Leu Gly Ala His Ile
785                 790                 795                 800

Val Arg Phe Trp Ala Lys His Arg Trp Gly Leu Pro Leu Arg Val Leu
                805                 810                 815

Lys Pro Ile Gln Cys Phe Lys Leu Glu Lys Val Ser Ser Leu Ser Leu
            820                 825                 830

Thr Gln Leu Ala Gly Pro Ser Ser Ala Thr Cys Glu Ser Gly Ala Gly
        835                 840                 845

Ser Glu Val Glu Val Asp Met Phe Leu Arg Lys Pro Pro Met Ala Ser
    850                 855                 860

Leu Arg Lys Gln Val Leu Thr Lys Ala Ser Asp His Met Pro Glu Ser
865                 870                 875                 880

Leu Leu Ala Ser Ser Pro Ala Trp Lys Gln Phe Gln Arg Ala Pro Arg
                885                 890                 895

Gly Ile Pro Ser Trp Asn Asp His Gly Pro Leu Lys Pro Pro Pro Ala
            900                 905                 910

Gly Gln Glu Gly Arg Trp Pro Ser Lys Pro Leu Thr Tyr Ser Leu Thr
        915                 920                 925

Gly Ser Thr Gln Gln Ser Arg Ser Leu Gly Ala Gln Ser Ser Lys Ala
    930                 935                 940

Gly Glu Thr Arg Glu Ala Val Pro Gln Cys Arg Val Pro Leu Glu Thr
945                 950                 955                 960

Cys Met Leu Ala Asn Leu Gln Ala Thr Ser Glu Asp Val His Gly Phe
                965                 970                 975

Glu Ala Pro Gly Thr Ser Lys Ser Ser Leu His Pro Arg Val Ser Val
            980                 985                 990

Ser Gln Asp Pro Arg Lys Leu Cys Leu Met Glu Glu Val Val Ser Glu
        995                 1000                1005

Phe Glu Pro Gly Met Ala Thr Lys Ser Glu Thr Gln Pro Gln Val
    1010                1015                1020

Cys Ala Ala Val Val Leu Leu Pro Asp Gly Gln Ala Ser Val Val
    1025                1030                1035

Pro His Ala Ser Glu Asn Leu Val Ser Gln Val Pro Gln Gly His
    1040                1045                1050

Leu Gln Ser Met Pro Thr Gly Asn Met Arg Ala Ser Gln Glu Leu
    1055                1060                1065

His Asp Leu Met Ala Ala Arg Arg Ser Lys Leu Val Gln Glu Glu
    1070                1075                1080

Pro Arg Asn Pro Asn Cys Gln Gly Ser Cys Lys Ser Gln Arg Pro
    1085                1090                1095

Met Phe Pro Pro Ile His Lys Ser Glu Lys Ser Arg Lys Pro Asn
    1100                1105                1110

Leu Glu Lys His Glu Glu Arg Leu Glu Gly Leu Arg Thr Pro Gln
    1115                1120                1125

Leu Thr Pro Val Arg Lys Thr Glu Asp Thr His Gln Asp Glu Gly
    1130                1135                1140

Val Gln Leu Leu Pro Ser Lys Lys Gln Pro Pro Ser Val Ser His
    1145                1150                1155

Phe Gly Glu Asn Ile Lys Gln Phe Phe Gln Trp Ile Phe Ser Lys
    1160                1165                1170

Lys Lys Ser Lys Pro Ala Pro Val Thr Ala Glu Ser Gln Lys Thr
    1175                1180                1185

Val Lys Asn Arg Ser Cys Val Tyr Ser Ser Ser Ala Glu Ala Gln
    1190                1195                1200

Gly Leu Met Thr Ala Val Gly Gln Met Leu Asp Lys Lys Met Ser
    1205                1210                1215

Leu Cys His Ala His His Ala Ser Lys Val Asn Gln His Lys Gln
    1220                1225                1230

Lys Phe Gln Ala Pro Val Cys Gly Phe Pro Cys Asn His Arg His
    1235                1240                1245

Leu Phe Tyr Ser Glu His Gly Arg Ile Leu Ser Tyr Ala Ala Ser
    1250                1255                1260

Ser Gln Gln Ala Thr Leu Lys Ser Gln Gly Cys Pro Asn Arg Asp
    1265                1270                1275

Arg Gln Ile Arg Asn Gln Gln Pro Leu Lys Ser Val Arg Cys Asn
    1280                1285                1290

Asn Glu Gln Trp Gly Leu Arg His Pro Gln Ile Leu His Pro Lys
    1295                1300                1305

Lys Ala Val Ser Pro Val Ser Pro Pro Gln His Trp Pro Lys Thr
    1310                1315                1320

Ser Gly Ala Ser Ser His His His His Cys Pro Arg His Cys Leu
    1325                1330                1335

Leu Trp Glu Gly Ile
    1340

<210>  123
<211>  24
<212>  DNA
<213>  künstliche Sequenz

<220>
<223>  Oligonukleotid

<400>  123
ctattactcc cctacttatc ttac                                              24

<210>  124
<211>  18
<212>  DNA
<213>  künstliche Sequenz

<220>
<223>  Oligonukleotid

<400>  124
tttcgcccac ctcacctg                                                    18


<210>  125
<211>  3136
<212>  DNA
<213>  Homo sapiens

<400>  125
gtcgccgccg ctaccgccgc cgccgccgca gggcccgccg ctgggatgcc gagcgcccgc      60

gccgccgctg cctctgtcct ccgcgcgctg ctcagctgaa ggcgcacagg attcaattac     120

tggacttgtc aactctgcca gtgtacgtgc catttctctt ccactatgag aggaccgatt     180

gtattgcaca tttgtctggc tttctgtagc cttctgcttt tcagcgttgc cacacaatgt     240

ctggccttcc ccaaaataga aaggaggagg gagatagcac atgttcatgc ggaaaaaggg     300

cagtccgata agatgaacac cgatgaccta gaaaatagct ctgttacctc aaagcagact     360

ccccaactgg tggtctctga agatccaatg atgatgtcag cagtaccatc ggcaacatca     420

ttaaataaag cattctcgat taacaaagaa acccagcctg gacaagctgg gctcatgcaa     480

acagaacgcc ctggtgtttc cacacctact gagtcaggtg tcccctcagc tgaagaagta     540

tttggttcca gccagccaga gagaatatct cctgaaagtg gacttgccaa ggccatgtta     600

accattgcta tcactgcgac tccttctctg actgttgatg aaaaggagga actccttaca     660

agcactaact ttcagcccat tgtagaagag atcacagaaa ccacaaaagg ttttctgaag     720

tatatggata atcaatcatt tgcaactgaa agtcaggaag gagttggttt gggacattca     780

ccttcatcct atgtgaatac taaggaaatg ctaaccacca atccaaagac tgagaaattt     840

gaagcagaca cagaccacag gacaacttct tttcctggtg ctgagtccac agcaggcagt     900

gagcctggaa gcctcacccc tgataaggag aagccttcgc agatgacagc tgataacacc     960

caggctgctg ccaccaagca accactcgaa acttccgagt acaccctgag tgttgagcca    1020

gaaactgata gtctgctggg agccccagaa gtcacagtga gtgtcagcac agctgttcca    1080

gctgcctctg ccttaagtga tgagtgggat gacaccaaat tagagagtgt aagccggata    1140

aggaccccca agcttggaga caatgaagag actcaggtga gaacggagat gtctcagaca    1200

gcacaagtaa gccatgaggg tatggaagga ggccagcctt ggacagaggc tgcacaggtg    1260

gctctggggc tgcctgaagg ggaaacacac acgggcacag ccctgctaat agcgcatggg    1320

aatgagagat cacctgcttt cactgatcaa agttccttta cccccacaag tctgatggaa    1380

gacatgaaag tttccattgt gaacttgctc caaagtacgg gagacttcac ggaatccacc    1440

aaggaaaacg atgccctgtt tttcttagaa accactgttt ctgtctctgt atatgagtct    1500

gaggcagacc aactgttggg aaatacaatg aaagacatca tcactcaaga gatgacaaca    1560

gctgttcaag agccagatgc cactttatcc atggtgacac aagagcaggt tgctaccctc    1620

gagcttatca gagacagtgg caagactgag gaagaaaagg aggacccctc tcctgtgtct 1680
gacgttcctg gtgttactca gctgtcaaga agatgggagc ctctggccac tacaatttca 1740
actacagtcg tccctttgtc ttttgaagtt actcccactg tggaagaaca aatggacaca 1800
gtcacagggc caaatgagga gttcacacca gttctgggat ctccagtgac acctcctgga 1860
ataatggtgg gggaacccag catttcccct gcacttcctg ctttggaggc atcctctgag 1920
agaagaactg ttgttccatc tattactcgt gttaatacag ctgcctcata tggcctggac 1980
caacttgaat ctgaagaggg acaagaagat gaggatgaag aggatgaaga agatgaagat 2040
gaagaagagg aagatgagga agaagatgag gaagataaag atgcagactc gctggatgag 2100
ggcttggatg gtgacactga gctgccaggt tttaccctcc ctggtatcac atcccaggaa 2160
ccaggcttag aggagggaaa catggacctg ttggagggag ctacctacca ggtgccagat 2220
gccctcgagt gggaacagca gaatcaaggc ctggtgagaa gctggatgga aaaattaaaa 2280
gacaaggctg gttacatgtc tgggatgctg gtgcctgtag gggttgggat agctggagcc 2340
ttgttcatct tgggagccct ctacagcatt aaggttatga atcgccgaag gagaaatggc 2400
ttcaaaaggc ataaaagaaa gcagagagaa ttcaacagca tgcaagatcg agtaatgctc 2460
ttagccgaca gctctgaaga tgaattttga attggactgg gttttaattg ggatattcaa 2520
cgatgctact attctaattt ttattttgga gcagaaaaaa aaaaagaaca acctgccaca 2580
ttgctgctat caggccgtta gtcctagtgt ctgctgggtg ctgggtagta gatttttctt 2640
gtactgagca gaaatggcat gttgtatact aaacgtatca tgcagtattt ggttttattc 2700
tgtagtgaat tttccacaac cgtgggctac aactcataaa tatgcaacat atatgttttt 2760
cagtaggagt tgctacatta ggcagagtaa atattttgta gttttccaca gtgtcttttc 2820
cttggtttga attacctgca ttgagaataa tgattgttgc caccaaggca tgcttgactc 2880
tgagatataa atcttaacaa agaataactt ctcaagatat actctaccta cttgaaacca 2940
cagggttgtg ggccatggta catactgcat ttgcatcaaa ctagcagtaa ctcagaatga 3000
aatcattttc attaagaagc tctctcagca tattaggatt atatgtagat ttgtatgtat 3060
tttgcattat gtacttcagt ctcctagttt tattattctc accttccgtt ttattcttgg 3120
cgaggaaaaa aatgca 3136

<210> 126
<211> 774
<212> PRT
<213> Homo sapiens

<400> 126

Met Arg Gly Pro Ile Val Leu His Ile Cys Leu Ala Phe Cys Ser Leu
1               5                   10                  15

Leu Leu Phe Ser Val Ala Thr Gln Cys Leu Ala Phe Pro Lys Ile Glu
            20                  25                  30

Arg Arg Arg Glu Ile Ala His Val His Ala Glu Lys Gly Gln Ser Asp
        35                  40                  45

Lys Met Asn Thr Asp Asp Leu Glu Asn Ser Ser Val Thr Ser Lys Gln
    50                  55                  60

Thr Pro Gln Leu Val Val Ser Glu Asp Pro Met Met Met Ser Ala Val
65                  70                  75                  80

Pro Ser Ala Thr Ser Leu Asn Lys Ala Phe Ser Ile Asn Lys Glu Thr
                85                  90                  95

Gln Pro Gly Gln Ala Gly Leu Met Gln Thr Glu Arg Pro Gly Val Ser
            100                 105                 110

Thr Pro Thr Glu Ser Gly Val Pro Ser Ala Glu Glu Val Phe Gly Ser
        115                 120                 125

Ser Gln Pro Glu Arg Ile Ser Pro Glu Ser Gly Leu Ala Lys Ala Met
    130                 135                 140

Leu Thr Ile Ala Ile Thr Ala Thr Pro Ser Leu Thr Val Asp Glu Lys
145                 150                 155                 160

Glu Glu Leu Leu Thr Ser Thr Asn Phe Gln Pro Ile Val Glu Glu Ile
                165                 170                 175

Thr Glu Thr Thr Lys Gly Phe Leu Lys Tyr Met Asp Asn Gln Ser Phe
            180                 185                 190

Ala Thr Glu Ser Gln Glu Gly Val Gly Leu Gly His Ser Pro Ser Ser
        195                 200                 205

Tyr Val Asn Thr Lys Glu Met Leu Thr Thr Asn Pro Lys Thr Glu Lys
    210                 215                 220

Phe Glu Ala Asp Thr Asp His Arg Thr Thr Ser Phe Pro Gly Ala Glu
225                 230                 235                 240

Ser Thr Ala Gly Ser Glu Pro Gly Ser Leu Thr Pro Asp Lys Glu Lys
                245                 250                 255

Pro Ser Gln Met Thr Ala Asp Asn Thr Gln Ala Ala Ala Thr Lys Gln
            260                 265                 270

Pro Leu Glu Thr Ser Glu Tyr Thr Leu Ser Val Glu Pro Glu Thr Asp
        275                 280                 285

Ser Leu Leu Gly Ala Pro Glu Val Thr Val Ser Val Ser Thr Ala Val
    290                 295                 300

Pro Ala Ala Ser Ala Leu Ser Asp Glu Trp Asp Asp Thr Lys Leu Glu
305                 310                 315                 320

Ser Val Ser Arg Ile Arg Thr Pro Lys Leu Gly Asp Asn Glu Glu Thr
                325                 330                 335

Gln Val Arg Thr Glu Met Ser Gln Thr Ala Gln Val Ser His Glu Gly
            340                 345                 350

Met Glu Gly Gly Gln Pro Trp Thr Glu Ala Ala Gln Val Ala Leu Gly
        355                 360                 365

Leu Pro Glu Gly Glu Thr His Thr Gly Thr Ala Leu Leu Ile Ala His
    370                 375                 380

Gly Asn Glu Arg Ser Pro Ala Phe Thr Asp Gln Ser Ser Phe Thr Pro
385                 390                 395                 400

Thr Ser Leu Met Glu Asp Met Lys Val Ser Ile Val Asn Leu Leu Gln
                405                 410                 415

Ser Thr Gly Asp Phe Thr Glu Ser Thr Lys Glu Asn Asp Ala Leu Phe
            420                 425                 430

Phe Leu Glu Thr Thr Val Ser Val Ser Val Tyr Glu Ser Glu Ala Asp
        435                 440                 445

Gln Leu Leu Gly Asn Thr Met Lys Asp Ile Ile Thr Gln Glu Met Thr
    450                 455                 460

Thr Ala Val Gln Glu Pro Asp Ala Thr Leu Ser Met Val Thr Gln Glu
465                 470                 475                 480

Gln Val Ala Thr Leu Glu Leu Ile Arg Asp Ser Gly Lys Thr Glu Glu
                485                 490                 495

Glu Lys Glu Asp Pro Ser Pro Val Ser Asp Val Pro Gly Val Thr Gln
            500                 505                 510

Leu Ser Arg Arg Trp Glu Pro Leu Ala Thr Thr Ile Ser Thr Thr Val
        515                 520                 525

Val Pro Leu Ser Phe Glu Val Thr Pro Thr Val Glu Glu Gln Met Asp
    530                 535                 540

Thr Val Thr Gly Pro Asn Glu Glu Phe Thr Pro Val Leu Gly Ser Pro
545                 550                 555                 560

Val Thr Pro Pro Gly Ile Met Val Gly Glu Pro Ser Ile Ser Pro Ala
                565                 570                 575

Leu Pro Ala Leu Glu Ala Ser Ser Glu Arg Arg Thr Val Val Pro Ser
            580                 585                 590

Ile Thr Arg Val Asn Thr Ala Ala Ser Tyr Gly Leu Asp Gln Leu Glu
        595                 600                 605

Ser Glu Glu Gly Gln Glu Asp Glu Asp Glu Glu Asp Glu Glu Asp Glu
    610                 615                 620

Asp Glu Glu Glu Glu Asp Glu Glu Glu Asp Glu Glu Asp Lys Asp Ala
625                 630                 635                 640

Asp Ser Leu Asp Glu Gly Leu Asp Gly Asp Thr Glu Leu Pro Gly Phe
                645                 650                 655

Thr Leu Pro Gly Ile Thr Ser Gln Glu Pro Gly Leu Glu Glu Gly Asn
            660                 665                 670

Met Asp Leu Leu Glu Gly Ala Thr Tyr Gln Val Pro Asp Ala Leu Glu
        675                 680                 685

Trp Glu Gln Gln Asn Gln Gly Leu Val Arg Ser Trp Met Glu Lys Leu
    690                 695                 700

Lys Asp Lys Ala Gly Tyr Met Ser Gly Met Leu Val Pro Val Gly Val
705                 710                 715                 720

Gly Ile Ala Gly Ala Leu Phe Ile Leu Gly Ala Leu Tyr Ser Ile Lys
                725                 730                 735

Val Met Asn Arg Arg Arg Arg Asn Gly Phe Lys Arg His Lys Arg Lys
            740                 745                 750

Gln Arg Glu Phe Asn Ser Met Gln Asp Arg Val Met Leu Leu Ala Asp
        755                 760                 765

Ser Ser Glu Asp Glu Phe
    770

<210> 127
<211> 18
<212> DNA
<213> künstliche Sequenz

<220>
<223> Oligonukleotid

<400> 127
ccctccctgg tatcacat 18

<210> 128
<211> 18
<212> DNA
<213> künstliche Sequenz

<220>
<223>  Oligonukleotid

<400>  128
caccagcatc ccagacat                                                    18


<210>  129
<211>  3627
<212>  DNA
<213>  Homo sapiens

<400>  129
   gggactgggg ggttcccaga tccttgaagc tcactccgcc tcctcactct cactgcattt      60
   cccaccttcc tgtgggcctt gcggcatctt catcactgag gcacctggtt acgcttcacc     120
   tcttgtttcc tgccctcact gcattccctc acctctacct ttttatcctt ccaccctagg     180
   cttctctcct ccctcttccc tcactcctga ctcttcctct tcccagcgga cggctggagg     240
   accgctcagt ctctcctctc tcacttccct tcctctctct caccttcacc acccaacacc     300
   tccctccctg cctctttctt tctgctccct cattctctcc ccaccactct cttctcgtgg     360
   cccccttgcc cgcgcgccct cttcccttcc ccttgcctca ctctctcagc tttcttccca     420
   cagttgagct cgggcagctc tttctgggga tagctatggg gctttggggg aagaaaggga     480
   cagtggctcc ccatgaccag agtccaagac gaagacctaa aaaagggctt atcaagaaaa     540
   aaatggtgaa gagggaaaaa cagaagcgca atatggagga actgaagaag gaagtggtca     600
   tggatgatca caaattaacc ttggaagagc tgagcaccaa gtactccgtg gacctgacaa     660
   agggccatag ccaccaaagg gcaaaggaaa tcctgactcg aggtggaccc aatactgtta     720
   ccccaccccc caccactcca gaatgggtca aattctgtaa gcaactgttc ggaggcttct     780
   ccctcctact atggactggg gccattctct gctttgtggc ctacagcatc cagatatatt     840
   tcaatgagga gcctaccaaa gacaacctct acctgagcat cgtactgtcc gtcgtggtca     900
   tcgtcactgg ctgcttctcc tattatcagg aggccaagag ctccaagatc atggagtctt     960
   ttaagaacat ggtgcctcag caagctctgg taattcgagg aggagagaag atgcaaatta    1020
   atgtacaaga ggtggtgttg ggagacctgg tggaaatcaa gggtggagac cgagtccctg    1080
   ctgacctccg gcttatctct gcacaaggat gtaaggtgga caactcatcc ttgactgggg    1140
   agtcagaacc ccagagccgc tcccctgact tcacccatga gaaccctctg gagacccgaa    1200
   acatctgctt cttttccacc aactgtgtgg aaggaaccgc ccggggtatt gtgattgcta    1260
   cgggagactc cacagtgatg ggcagaattg cctccctgac gtcaggcctg gcggttggcc    1320
   agacacctat cgctgctgag atcgaacact tcatccatct gatcactgtg gtggccgtct    1380
   tccttggtgt cacttttttt gcgctctcac ttctcttggg ctatggttgg ctggaggcta    1440
   tcatttttct cattggcatc attgtggcca atgtgcctga ggggctgttg gccacagtca    1500
   ctgtgtgcct gaccctcaca gccaagcgca tggcgcggaa gaactgcctg gtgaagaacc    1560
   tggaggcggt ggagacgctg ggctccacgt ccaccatctg ctcagacaag acgggcaccc    1620
   tcacccagaa ccgcatgacc gtcgcccaca tgtggtttga tatgaccgtg tatgaggccg 1680
   acaccactga agaacagact ggaaaaacat ttaccaagag ctctgatacc tggtttatgc 1740
   tggcccgaat cgctggcctc tgcaaccggg ctgactttaa ggctaatcag gagatcctgc 1800
   ccattgctaa gagggccaca acaggtgatg cttccgagtc agccctcctc aagttcatcg 1860
   agcagtctta cagctctgtg gcggagatga gagagaaaaa ccccaaggtg gcagagattc 1920
   cctttaattc taccaacaag taccagatgt ccatccacct tcgggaggac agctcccaga 1980
   cccacgtact gatgatgaag ggtgctccgg agaggatctt ggagttttgt tctacctttc 2040
   ttctgaatgg gcaggagtac tcaatgaacg atgaaatgaa ggaagccttc caaaatgcct 2100
   acttagaact gggaggtctg ggggaacgtg tgctaggctt ctgcttcttg aatctgccta 2160
   gcagcttctc caagggattc ccatttaata cagatgaaat aaatttcccc atggacaacc 2220
   tttgttttgt gggcctcata tccatgattg accctccccg agctgcagtg cctgatgctg 2280
   tgagcaagtg tcgcagtgca ggaattaagg tgatcatggt aacaggagat catcccatta 2340
   cagctaaggc cattgccaag ggtgtgggca tcatctcaga aggcactgag acggcagagg 2400
   aagtcgctgc ccggcttaag atccctatca gcaaggtcga tgccagtgct gccaaagcca 2460
   ttgtggtgca tggtgcagaa ctgaaggaca tacagtccaa gcagcttgat cagatcctcc 2520
   agaaccaccc tgagatcgtg tttgctcgga cctcccctca gcagaagctc atcattgtcg 2580
   agggatgtca gaggctggga gccgttgtgg ccgtgacagg tgacggggtg aacgactccc 2640
   ctgcgctgaa gaaggctgac attggcattg ccatgggcat ctctggctct gacgtctcta 2700
   agcaggcagc cgacatgatc ctgctggatg acaactttgc ctccatcgtc acgggggtgg 2760
   aggagggccg cctgatcttt gacaacctga agaaatccat catgtacacc ctgaccagca 2820
   acatccccga gatcacgccc ttcctgatgt tcatcatcct cggtataccc ctgcctctgg 2880
   gaaccataac catcctctgc attgatctcg gcactgacat ggtccctgcc atctccttgg 2940
   cttatgagtc agctgaaagc gacatcatga agaggcttcc aaggaaccca aagacggata 3000
   atctggtgaa ccaccgtctc attggcatgg cctatggaca gattgggatg atccaggctc 3060
   tggctggatt ctttacctac tttgtaatcc tggctgagaa tggttttagg cctgttgatc 3120
   tgctgggcat ccgcctccac tgggaagata aatacttgaa tgacctggag gacagctacg 3180
   gacagcagtg gacctatgag caacgaaaag ttgtggagtt cacatgccaa acggcctttt 3240
   ttgtcaccat cgtggttgtg cagtgggcgg atctcatcat ctccaagact cgccgcaact 3300
   cacttttcca gcagggcatg agaaacaaag tcttaatatt tgggatcctg gaggagacac 3360
   tcttggctgc atttctgtcc tacactccag gcatggacgt ggccctgcga atgtacccac 3420
   tcaagataac ctggtggctc tgtgccattc cctacagtat tctcatcttc gtctatgatg 3480
   aaatcagaaa actcctcatc cgtcagcacc cggatggctg ggtggaaagg gagacgtact 3540
   actaaactca gcagatgaag agcttcatgt gacacagggg tgttgtgaga gctgggatgg 3600
   ggccagagat tataagtttg acacaac 3627

<210>  130
<211>  1029
<212>  PRT
<213>  Homo sapiens

<400>  130
   Met Gly Leu Trp Gly Lys Lys Gly Thr Val Ala Pro His Asp Gln Ser
   1               5                   10                  15
   Pro Arg Arg Arg Pro Lys Lys Gly Leu Ile Lys Lys Lys Met Val Lys
               20                  25                  30
   Arg Glu Lys Gln Lys Arg Asn Met Glu Glu Leu Lys Lys Glu Val Val
           35                  40                  45
   Met Asp Asp His Lys Leu Thr Leu Glu Glu Leu Ser Thr Lys Tyr Ser
       50                  55                  60
   Val Asp Leu Thr Lys Gly His Ser His Gln Arg Ala Lys Glu Ile Leu
   65                  70                  75                  80
   Thr Arg Gly Gly Pro Asn Thr Val Thr Pro Pro Pro Thr Thr Pro Glu
                   85                  90                  95
   Trp Val Lys Phe Cys Lys Gln Leu Phe Gly Gly Phe Ser Leu Leu Leu
               100                 105                 110
   Trp Thr Gly Ala Ile Leu Cys Phe Val Ala Tyr Ser Ile Gln Ile Tyr
           115                 120                 125
   Phe Asn Glu Glu Pro Thr Lys Asp Asn Leu Tyr Leu Ser Ile Val Leu
       130                 135                 140
   Ser Val Val Val Ile Val Thr Gly Cys Phe Ser Tyr Tyr Gln Glu Ala
   145                 150                 155                 160
   Lys Ser Ser Lys Ile Met Glu Ser Phe Lys Asn Met Val Pro Gln Gln
                   165                 170                 175
   Ala Leu Val Ile Arg Gly Gly Glu Lys Met Gln Ile Asn Val Gln Glu
               180                 185                 190
   Val Val Leu Gly Asp Leu Val Glu Ile Lys Gly Gly Asp Arg Val Pro
           195                 200                 205
   Ala Asp Leu Arg Leu Ile Ser Ala Gln Gly Cys Lys Val Asp Asn Ser
       210                 215                 220
   Ser Leu Thr Gly Glu Ser Glu Pro Gln Ser Arg Ser Pro Asp Phe Thr
   225                 230                 235                 240
   His Glu Asn Pro Leu Glu Thr Arg Asn Ile Cys Phe Phe Ser Thr Asn
                   245                 250                 255
   Cys Val Glu Gly Thr Ala Arg Gly Ile Val Ile Ala Thr Gly Asp Ser
               260                 265                 270
   Thr Val Met Gly Arg Ile Ala Ser Leu Thr Ser Gly Leu Ala Val Gly
           275                 280                 285
   Gln Thr Pro Ile Ala Ala Glu Ile Glu His Phe Ile His Leu Ile Thr
       290                 295                 300
   Val Val Ala Val Phe Leu Gly Val Thr Phe Phe Ala Leu Ser Leu Leu
   305                 310                 315                 320
   Leu Gly Tyr Gly Trp Leu Glu Ala Ile Ile Phe Leu Ile Gly Ile Ile
                   325                 330                 335
   Val Ala Asn Val Pro Glu Gly Leu Leu Ala Thr Val Thr Val Cys Leu
               340                 345                 350
   Thr Leu Thr Ala Lys Arg Met Ala Arg Lys Asn Cys Leu Val Lys Asn
           355                 360                 365
   Leu Glu Ala Val Glu Thr Leu Gly Ser Thr Ser Thr Ile Cys Ser Asp
       370                 375                 380
   Lys Thr Gly Thr Leu Thr Gln Asn Arg Met Thr Val Ala His Met Trp
   385                 390                 395                 400
   Phe Asp Met Thr Val Tyr Glu Ala Asp Thr Thr Glu Glu Gln Thr Gly
                   405                 410                 415
   Lys Thr Phe Thr Lys Ser Ser Asp Thr Trp Phe Met Leu Ala Arg Ile
               420                 425                 430
   Ala Gly Leu Cys Asn Arg Ala Asp Phe Lys Ala Asn Gln Glu Ile Leu
           435                 440                 445
   Pro Ile Ala Lys Arg Ala Thr Thr Gly Asp Ala Ser Glu Ser Ala Leu
       450                 455                 460
   Leu Lys Phe Ile Glu Gln Ser Tyr Ser Ser Val Ala Glu Met Arg Glu
   465                 470                 475                 480
   Lys Asn Pro Lys Val Ala Glu Ile Pro Phe Asn Ser Thr Asn Lys Tyr
                   485                 490                 495
   Gln Met Ser Ile His Leu Arg Glu Asp Ser Ser Gln Thr His Val Leu
               500                 505                 510
   Met Met Lys Gly Ala Pro Glu Arg Ile Leu Glu Phe Cys Ser Thr Phe
           515                 520                 525
   Leu Leu Asn Gly Gln Glu Tyr Ser Met Asn Asp Glu Met Lys Glu Ala
       530                 535                 540
   Phe Gln Asn Ala Tyr Leu Glu Leu Gly Gly Leu Gly Glu Arg Val Leu
   545                 550                 555                 560
   Gly Phe Cys Phe Leu Asn Leu Pro Ser Ser Phe Ser Lys Gly Phe Pro
                   565                 570                 575
   Phe Asn Thr Asp Glu Ile Asn Phe Pro Met Asp Asn Leu Cys Phe Val
               580                 585                 590
   Gly Leu Ile Ser Met Ile Asp Pro Pro Arg Ala Ala Val Pro Asp Ala
           595                 600                 605
   Val Ser Lys Cys Arg Ser Ala Gly Ile Lys Val Ile Met Val Thr Gly
       610                 615                 620
   Asp His Pro Ile Thr Ala Lys Ala Ile Ala Lys Gly Val Gly Ile Ile
   625                 630                 635                 640
   Ser Glu Gly Thr Glu Thr Ala Glu Glu Val Ala Ala Arg Leu Lys Ile
                   645                 650                 655
   Pro Ile Ser Lys Val Asp Ala Ser Ala Ala Lys Ala Ile Val Val His
               660                 665                 670
   Gly Ala Glu Leu Lys Asp Ile Gln Ser Lys Gln Leu Asp Gln Ile Leu
           675                 680                 685
   Gln Asn His Pro Glu Ile Val Phe Ala Arg Thr Ser Pro Gln Gln Lys
       690                 695                 700
   Leu Ile Ile Val Glu Gly Cys Gln Arg Leu Gly Ala Val Val Ala Val
   705                 710                 715                 720
   Thr Gly Asp Gly Val Asn Asp Ser Pro Ala Leu Lys Lys Ala Asp Ile
                   725                 730                 735
   Gly Ile Ala Met Gly Ile Ser Gly Ser Asp Val Ser Lys Gln Ala Ala
               740                 745                 750
   Asp Met Ile Leu Leu Asp Asp Asn Phe Ala Ser Ile Val Thr Gly Val
           755                 760                 765
   Glu Glu Gly Arg Leu Ile Phe Asp Asn Leu Lys Lys Ser Ile Met Tyr
       770                 775                 780
   Thr Leu Thr Ser Asn Ile Pro Glu Ile Thr Pro Phe Leu Met Phe Ile
   785                 790                 795                 800
   Ile Leu Gly Ile Pro Leu Pro Leu Gly Thr Ile Thr Ile Leu Cys Ile
                   805                 810                 815
   Asp Leu Gly Thr Asp Met Val Pro Ala Ile Ser Leu Ala Tyr Glu Ser
               820                 825                 830
   Ala Glu Ser Asp Ile Met Lys Arg Leu Pro Arg Asn Pro Lys Thr Asp
           835                 840                 845
   Asn Leu Val Asn His Arg Leu Ile Gly Met Ala Tyr Gly Gln Ile Gly
       850                 855                 860
   Met Ile Gln Ala Leu Ala Gly Phe Phe Thr Tyr Phe Val Ile Leu Ala
   865                 870                 875                 880
   Glu Asn Gly Phe Arg Pro Val Asp Leu Leu Gly Ile Arg Leu His Trp
                   885                 890                 895
   Glu Asp Lys Tyr Leu Asn Asp Leu Glu Asp Ser Tyr Gly Gln Gln Trp
               900                 905                 910
   Thr Tyr Glu Gln Arg Lys Val Val Glu Phe Thr Cys Gln Thr Ala Phe
           915                 920                 925
   Phe Val Thr Ile Val Val Val Gln Trp Ala Asp Leu Ile Ile Ser Lys
       930                 935                 940
   Thr Arg Arg Asn Ser Leu Phe Gln Gln Gly Met Arg Asn Lys Val Leu
   945                 950                 955                 960
   Ile Phe Gly Ile Leu Glu Glu Thr Leu Leu Ala Ala Phe Leu Ser Tyr
                   965                 970                 975
   Thr Pro Gly Met Asp Val Ala Leu Arg Met Tyr Pro Leu Lys Ile Thr
               980                 985                 990
   Trp Trp Leu Cys Ala Ile Pro Tyr Ser Ile Leu Ile Phe Val Tyr Asp
           995                 1000                1005
   Glu Ile Arg Lys Leu Leu Ile Arg Gln His Pro Asp Gly Trp Val
       1010                1015                1020
   Glu Arg Glu Thr Tyr Tyr
       1025

<210>  131
<211>  21
<212>  DNA
<213>  künstliche Sequenz

<220>
<223>  Oligonukleotid

<400>  131
tgtaatcctg gctgagaatg g                                                21


<210>  132
<211>  18
<212>  DNA
<213>  künstliche Sequenz

<220>
<223>  Oligonukleotid

<400>  132
aagtgagttg cggcgagt                                                    18


<210>  133
<211>  279
<212>  DNA
<213>  Homo sapiens

<400>  133
atgtatgtaa aaattgcaaa acatctcaat gatgtttatg ccccccagaa ggtactgtgt      60

cacgggatct catatattct ggctgtcatt gtcataataa gccactcttg gtcatatgga     120

aaagcattca gctgctccct gcctttgctc acagcgtgtg gtactctctt agaagctatt     180

cctgtcctat ttaggcagtt attcctgctt cttgtgttgg acctgaagtc aacagggcca     240

gcaatagaga agaaagatga tgtgaaggag agcaactga                            279


<210>  134
<211>  92
<212>  PRT
<213>  Homo sapiens

<400>  134

Met Tyr Val Lys Ile Ala Lys His Leu Asn Asp Val Tyr Ala Pro Gln
1               5                   10                  15


Lys Val Leu Cys His Gly Ile Ser Tyr Ile Leu Ala Val Ile Val Ile
            20                  25                  30


Ile Ser His Ser Trp Ser Tyr Gly Lys Ala Phe Ser Cys Ser Leu Pro
        35                  40                  45


Leu Leu Thr Ala Cys Gly Thr Leu Leu Glu Ala Ile Pro Val Leu Phe
    50                  55                  60


Arg Gln Leu Phe Leu Leu Leu Val Leu Asp Leu Lys Ser Thr Gly Pro
65                  70                  75                  80


Ala Ile Glu Lys Lys Asp Asp Val Lys Glu Ser Asn
                85                  90

<210> 135
<211> 20
<212> DNA
<213> künstliche Sequenz

<220>
<223> Oligonukleotid

<400> 135
tgctccctgc ctttgctcac 20

<210> 136
<211> 24
<212> DNA
<213> künstliche Sequenz

<220>
<223> Oligonukleotid

<400> 136
ggtacttggt ctcgaacgat gatc 24

<210> 137
<211> 1569
<212> DNA
<213> Homo sapiens

<400> 137
   atgcctgtag ggggtggccc tgagagtgtg ggcaggtgca atggctgtca atgccacata 60
   aagggcaagg ggatctacat cctaaacagt gaaagaccag tgcccggaga ctacatctac 120
   atcaggaaga agaagcagca aaattctgac ccacagccca agaggggtcg gggcagcaga 180
   acctcagcca cagccaatca cagcggggtc cttcggggag gggcgtggcc tgacaacttc 240
   ggcgacgcgg ctggaccaat ccggacggag gagagcgaag ctcctctgca ctgggcccag 300
   gtgcgctcct cagcgtctcc gggtggcggg gcgcgcggga tggaggagtc ttgggaggct 360
   gcgcccggag gccaagccgg ggcagagctc ccaatggagc ccgtgggaag cctggtcccc 420
   acgctggagc agccgcaggt gcccgcgaag gtgcgacaac ctgaaggtcc cgaaagcagc 480
   ccaagtccgg ccggggccgt ggagaaggcg gcgggcgcag gcctggagcc ctcgagcaag 540
   aaaaagccgc cttcgcctcg ccccgggtcc ccgcgcgtgc cgccgctcag cctgggctac 600
   ggggtctgcc ccgagccgcc gtcaccgggc cctgccttgg tcaagctgcc ccggaatggc 660
   gaggcgcccg gggctgagcc tgcgcccagc gcctgggcgc ccatggagct gcaggtagat 720
   gtgcgcgtga agcccgtggg cgcggccggt ggcagcagca cgccatcgcc caggccctcc 780
   acgcgcttcc tcaaggtgcc ggtgcccgag tcccctgcct tctcccgcca cgcggacccg 840
   gcgcaccagc tcctgctgcg cgcaccatcc cagggcggca cgtggggccg ccgctcgccg 900
   ctggctgcag cccggacgga gagcggctgc gacgcagagg gccgggccag ccccgcggaa 960
   ggaagcgccg gctccccggg ctcccccacg tgctgccgct gcaaggagct ggggctggag 1020
   aaggaggatg cggcgctgtt gccccgcgcg gggttggacg gcgacgagaa gctgccccgg 1080
   gccgtaacgc ttacggggct acccatgtac gtgaagtccc tgtactgggc cctggcgttc 1140
   atggctgtgc tcctggcagt ctctggggtt gtcattgtgg tcctggcctc aagagcagga 1200
   gccagatgcc agcagtgccc cccaggctgg gtgttgtccg aggagcactg ttactacttc 1260
   tctgcagaag cgcaggcctg ggaagccagc caggctttct gctcagccta ccacgctacc 1320
   ctccccctgc taagccacac ccaggacttc ctgggcagat acccagtctc caggcactcc 1380
   tgggtggggg cctggcgagg cccccagggc tggcactgga tcgacgaggc cccactcccg 1440
   ccccagctac tccctgagga cggcgaggac aatctggata tcaactgtgg ggccctggag 1500
   gaaggcacgc tggtggctgc aaactgcagc actccaagac cctgggtctg tgccaagggg 1560
   acccagtga 1569

<210> 138
<211> 522
<212> PRT
<213> Homo sapiens

<400> 138

<210>  139
<211>  18
<212>  DNA
<213>  künstliche Sequenz

<220>
<223>  Oligonukleotid

<400>  139
gagaaggagg atgcggcg                                                    18

<210>  140
<211>  21
<212>  DNA
<213>  künstliche Sequenz

<220>
<223>  Oligonukleotid

<400>  140
ggaccacaat gacaacccca g                                                21

<210>  141
<211>  2217
<212>  DNA
<213>  Homo sapiens

<400>  141
atggtttgca cgttcgattc tgagcttctg aattgtcaaa ggaaagatga atataatcag      60

ttccagactt atcgggccca taaaataaaa gccaaaagaa gcatagccac tcctgaaaac     120

ctgaagaaat tattgccacg tgttcccaaa aacagtgccc tgagtgatga aatgacaaag     180

cttcacaaag gagctaagcc atgcaaatca aatacatttg gatgttttcc tattcatcag     240

gctgtacttt caggttccaa agaatgcatg gaaataatat tgaagtttgg tgaagagcac     300

gggtacagca gacagtgtca catcaacttt gtggataacg ggaaagccag ccctctccat     360

ctggctgtgc aaaatggtga cttggaaatg atgaaaatgt gcctggacaa tggtgtacaa     420

atagacctag tggagatgca acagatcaaa gagctggtaa tggatgaaga caacgatggg     480

tgtactcctc tacattatgc atgtagacag gggggccctg gttctgtaaa taacctactt     540

ggctttaatg tgtccattca ttccaaaagc aaagataaga aatcacctct gcattttgca 600
gccagttatg ggcgtatcaa tacctgtcag aggctcctac aagacataag tgatacgagg 660
cttctgaatg aaggggacct tcatggaatg actcctctcc atctggcagc aaagaatgga 720
catgataaag tagttcagct tcttctgaaa aaaggtgcat tgtttctcag atgggatgaa 780
tgtcttaagg tttttagtca ttattctcca aacaataaat gtccaatttt ggaaatgatc 840
gaatacctcc ctgaatgcat gaagaaagtt ctacccttct tttctaatgt tcacgtaaga 900
cctgctccaa accagaatca aataaaccat ggagaacaca ggttggctta cggatttata 960
gcccatatga taaatctagg attttactgt cttggtctca taccaatgac ctttcttgtt 1020
gtcagaataa aaccaggaat ggctttcaac tctgctggaa tcatcaataa aactagtgat 1080
cattcagaaa tactagataa catgaattca agtctaataa caatttgtat gattttagtt 1140
ttttgctcaa gtatattagg gtatgtcaaa gaagtggttc aaattttcca acagaaaagg 1200
aattacttta tggatattag cagtagtact gaatggatta tcaacacgat gggccccatt 1260
ttagtgctgc ccttgttcac tgaaatagca gcccatctgc aatttgagaa ttgtggaatt 1320
ttcattgtta tattggaggt aatttttaaa actttgttga ggtctgcagt tgtatttttc 1380
ttccttcttt tggcttttgg actcagcttt tacgtcctcc tgaatttaca gtccttccta 1440
gaaccatttc tgaagaataa attggcacat ccagttctgt cctttgcaca gcttatttcc 1500
ttcacagtat ttgccccaat tgtcctcatg aatttactta ttggtttggc agttggtgac 1560
attgctgagg tccagaaaca tgcatcattg aagaggatag ctatgcagaa gctgccatgc 1620
tgttgcatac gcaaagtgga tcggaaatcc accgccgtat gtcccaacaa acccagatgt 1680
gatgggacat tatttcaagt cctactcgct ctaggccccc taccccctaga agaaaataga 1740
aacataaaaa gttttcttcc tactgagatc actgttaaga ggactcacga acaccttcct 1800
tctgcaggtt ttggtcatca tgggaaacat accttgtcct tgcttttggt agaagagtgg 1860
cttcctctga atgtagtaca ctcctcttgc tctgccttca gagtggttgg ccagatcttt 1920
cccattagac attttcagtg gattcatgtg aatgagccgc acactggcaa tttaaaagag 1980
aaattggctg ctccatacat cactcaccag atcaagccat tcttgcgagc agctggtttt 2040
tgcacagtga aggtggtcca gagagatgac atctctgtgt ggagtgtgga tttcaggtgg 2100
ctcaatgcat gggaagcagc gattcgaaag cagtctctca gacaatctga gatggaggaa 2160
ctgagctgct cgctgctgct gcgtgtcact gatgtgcaca caagaagctt gtattag 2217

<210> 142
<211> 738
<212> PRT
<213> Homo sapiens

<400> 142


<210>  143
<211>  20
<212>  DNA
<213>  künstliche Sequenz

<220>
<223>  Oligonukleotid

<400>  143
ttccttactc tccgctttcc                                                20


<210>  144
<211>  20
<212>  DNA
<213>  künstliche Sequenz

<220>
<223>  Oligonukleotid

<400> 144
aactttgtgg ataacgggaa 20

<210> 145
<211> 1155
<212> DNA
<213> Homo sapiens

<400> 145
atgcagtctc tcatctcgcc ggtgaccaag gcgatcctgg tggccctctt catcttcgcc 60
atcctcctca tcctctacgt gatcctctgg gacgcaccgg ggagagcggg tgagtgcgct 120
cgtgcgggcg ctttgggggg ccacggttgg ggagccccaa cttcggggag gacgcggaat 180
ccggacgcgg gactgaaccc gaggattcac ggagcccggg gctcccctat ggggcacggg 240
aagcggcaga tgcgcgtgca gagaggtccg tcccacccac cccctgggcg ccttgggtcc 300
aaggcgcata ggcgctcccg cctgtggccg ccaccggtgc agcagaacgc gggctctcgg 360
gtgggtccaa tgcgctatgg cacaccaggc gctatcgggt ccctagccct ctgctccggc 420
ggtggggacc ccgcactcaa gttccctata acctccatgg acaaacacgg aaaaatcatg 480
tcttggaaga acagcatcgc cctacagata cagactaggc actttgcaca tgaaacaaga 540
gtcccagaaa tttctagaag caaatctcgc attcgtgacc gccagaccta cgggatgtac 600
cactttggga attttggaga agaaagaata aaggcagaaa tgaggataca gaaagcatgt 660
cacttgaaga tcaagaagtc aagcttggat gccaatggta aagtggatga tggtgaggat 720
gatgatggtg aggatgatga tggtgaggat gatgatggtg atgatgatgg tgaggatgat 780
gatggtgagg atgatgatgg tgaggatgat gatggtgagg atgatggtga ggatgatgat 840
ggtgatgatg atggtgagga tgatgatggt gatgatgatg gtgatgatga tggtgaggat 900
gatgatggtg aggatgatga tggtgacagt gaggatgatg gtgaggatgg tgatgatgat 960
ggtgaggatg atgatggtga cagtgaggat gatggcgatg atggtgatga tgatggtgag 1020
gatgatgatc atggtgatga tgtgaggatg atgatgatga tggtgatgac agtgacgatg 1080
atgaagaatg ttgttggtaa ttacagactt cctgagctac caacttggac atctgtacaa 1140
cgatacaaat tttga 1155

<210> 146
<211> 384
<212> PRT
<213> Homo sapiens

<400> 146

Met Gln Ser Leu Ile Ser Pro Val Thr Lys Ala Ile Leu Val Ala Leu
1               5                   10                  15

Phe Ile Phe Ala Ile Leu Leu Ile Leu Tyr Val Ile Leu Trp Asp Ala
            20                  25                  30

Pro Gly Arg Ala Gly Glu Cys Ala Arg Ala Gly Ala Leu Gly Gly His
        35                  40                  45

Gly Trp Gly Ala Pro Thr Ser Gly Arg Thr Arg Asn Pro Asp Ala Gly
    50                  55                  60

Leu Asn Pro Arg Ile His Gly Ala Arg Gly Ser Pro Met Gly His Gly
65                  70                  75                  80

Lys Arg Gln Met Arg Val Gln Arg Gly Pro Ser His Pro Pro Pro Gly
                85                  90                  95

Arg Leu Gly Ser Lys Ala His Arg Arg Ser Arg Leu Trp Pro Pro Pro
            100                 105                 110

Val Gln Gln Asn Ala Gly Ser Arg Val Gly Pro Met Arg Tyr Gly Thr
        115                 120                 125

Pro Gly Ala Ile Gly Ser Leu Ala Leu Cys Ser Gly Gly Gly Asp Pro
    130                 135                 140

Ala Leu Lys Phe Pro Ile Thr Ser Met Asp Lys His Gly Lys Ile Met
145                 150                 155                 160

Ser Trp Lys Asn Ser Ile Ala Leu Gln Ile Gln Thr Arg His Phe Ala
                165                 170                 175

His Glu Thr Arg Val Pro Glu Ile Ser Arg Ser Lys Ser Arg Ile Arg
            180                 185                 190

Asp Arg Gln Thr Tyr Gly Met Tyr His Phe Gly Asn Phe Gly Glu Glu
        195                 200                 205

Arg Ile Lys Ala Glu Met Arg Ile Gln Lys Ala Cys His Leu Lys Ile
    210                 215                 220

Lys Lys Ser Ser Leu Asp Ala Asn Gly Lys Val Asp Asp Gly Glu Asp
225                 230                 235                 240

Asp Asp Gly Glu Asp Asp Asp Gly Glu Asp Asp Asp Gly Asp Asp Asp
                245                 250                 255

Gly Glu Asp Asp Asp Gly Glu Asp Asp Asp Gly Glu Asp Asp Asp Gly
            260                 265                 270

Glu Asp Asp Gly Glu Asp Asp Asp Gly Asp Asp Asp Gly Glu Asp Asp
        275                 280                 285

Asp Gly Asp Asp Asp Gly Asp Asp Asp Gly Glu Asp Asp Asp Gly Glu
    290                 295                 300

Asp Asp Asp Gly Asp Ser Glu Asp Asp Gly Glu Asp Gly Asp Asp Asp
305                 310                 315                 320

Gly Glu Asp Asp Asp Gly Asp Ser Glu Asp Asp Gly Asp Asp Gly Asp
                325                 330                 335

Asp Asp Gly Glu Asp Asp Asp His Gly Asp Asp Val Arg Met Met Met
            340                 345                 350

Met Met Val Met Thr Val Thr Met Met Lys Asn Val Val Gly Asn Tyr
        355                 360                 365

Arg Leu Pro Glu Leu Pro Thr Trp Thr Ser Val Gln Arg Tyr Lys Phe
    370                 375                 380

<210>  147
<211>  21
<212>  DNA
<213>  künstliche Sequenz

<220>
<223>  Oligonukleotid

<400>  147
tccatgctgc cagcttcata c                                                21

<210>  148
<211>  21
<212>  DNA
<213>  künstliche Sequenz

<220>
<223>. Oligonukleotid

<400>  148
ctcacaagtg atgagattga g                                                21

<210>  149
<211>  4384
<212>  DNA
<213>  Homo sapiens

<400>  149
aaacagacgc ataactgtgc attgttcttt gggattttga gagccttcat ctcaatttca      60

actttaaagc agcttaatct ttaaggaaca tatctctgat ctgggtaatt tgtagaactt     120

aatttgaggg tcattacatg tgaggatagc aggagttgaa gatgccaagg acctgaaggg     180

ctactggagg gacaggtgaa gtgatttgaa gatgtagcat tttgaatctc tttctggccc     240

atcctctgct tcacaccaga atcattgtga cctgtagacc tgcaaaacaa aggaccaaag     300

gttagcatgc agaagtgaaa gtgtcaataa taaccaaacc actccatcaa gttaggtctg     360

gggaaaagca gcagcaaaaa tgagttctta cttctgggca caaaatgaaa gtaacagacc     420

tgatttactc tgcgggcagc cagctgacta ccttgttgaa gagaaacatt tcacaacgct     480

tgtatgcttc attgttgttt tgggagggct tttgaagatg tgtttaaaga attgtgaagt     540

cattgttttg acgattcttt ctctatcagg attcgtgata ggacacatgg cctacaattc     600

tgttgaggtg caccaaattg tctaccctct tctaagaaca tcaagttttt cactttattc 660
ttacttttca cctttaatta tatttatggt tgctttggat gtagaatttt atacactcaa 720
gaaaatgttt tggcaggtct tgttaactgg attaattagc ttttctacag caagcatcat 780
aattggatat gtcgttataa aattcaataa agattcatgg gatttgcaat cttgcctact 840
ctttagcatc acccttggca ttatagatcc tcttcgttct gtgaattcac taaaaactat 900
tggcatttct aaaatataca ttgatctcat tagaggagaa tcattgatca tttgtagcat 960
cgcatcaatt ttttttggaa attttcgggg caacagaatc cacttttcta tttttagaga 1020
tttacatgta ggcattgaac tcagctatga cattttggga agcataatat ttggatattg 1080
gtgtgcaaaa atcattcagt gtatattggc tgacgttttt agcaatatgc tgactaatat 1140
cattctctgc ttttcaatgg tgtacatgac tttctatatt gtggaatttt taggaatgtc 1200
aggcactctt gccttagccg ctgtaggact gaatttagat tctttaactt ttaaaccgaa 1260
gatcgaactt gtaattacta agttcttaag aattttttca tctgtatatg aacatttaat 1320
atatgctttc tttggcattg tgattggatg tggagaactc agccactatg aatttcacac 1380
tatacctttc atattcattt tatttacaac agtgaatttg gtaaggttgc ttactatttt 1440
gttagtgagc cctattttga tgcattcaaa ttatgaatat aattggcgat ggggagttgt 1500
aatcacgtgg tctggaatta aaggagtttt taatttactc tgggctcctg atgtttataa 1560
tctcgctgaa cgaaaagtgg aagtaccaca aatgtttata ctctatgtac aagtaatatc 1620
attattgaca atgggaataa attcatacgt gatgactcag tcagccagga agttagattt 1680
gtgtgttctt tccctcccaa gacaaatgat cttgcaaaat gccactcagc acatacagga 1740
gatagtacag aacacaataa ctttatttaa aacagaaaaa attttgacaa atgttaactg 1800
gaccttagta gaagataaaa cgaggatcga atacattcct ttttcccacg tttcacataa 1860
tgatatgaag acagaatcca caacagatga agctttaatg gaggaagcca gattgcatgt 1920
agctgcaata caaatgagta gctttgaaaa acagcgtaac aatggaattc ttgaaataga 1980
ggcagcccgg atattaattg gtgcagcaaa atgctattac tccatccaag gaaaattcat 2040
gagtatttat gatgtttcaa cttatatgag aactagaagt tggcttataa agtttaaaaa 2100
tgttttaact ttcttggaat attgtataga aaagatacat tttattccac ctgagagtaa 2160
tacatttctg acttttatat ttcacatagt attttctgaa gaatttgaat atacaggaca 2220
gattataaat ttgatatata tttatcctat gataatacat ctgtggccaa tggcaagagg 2280
tttaaatgta tcagcactga tatcaataaa ctactatttt atgtttttat atgtattaga 2340
atcaacattg aagataataa ttttgaaaag gaaatatttt caacaatgtt ggaatacttt 2400
ggaatttttt atcctggtta ttggaatcat tgatatcttt tgtgtatact ttgtgaaatt 2460
gagaccagac aacttggctc ttatacagct tacagtaata atgggatatt taagaataat 2520
taggtttctt cctctcttca agataatagt accaatactg ataagaattg cagatgtgca 2580
gatcaaaaag cgcctcagct tgatgtatag tattacaaaa ggctatatca aaagtcaaga 2640

agatgccaaa cttctaataa aacaaatagc tgtctgtgaa tcaatatatc agaaactatg 2700
tgaaattttg gaaaccaaca aacaggatgc tgtcaaagaa ttagtactca tggagcatga 2760
gggtcgtgat gttgtcattg ctttgaagac taaacaggca atccggaatg tgattgctaa 2820
agctctaaaa aatctcacct tcctttgttc aagaggcatt attgataagc atgaagtcat 2880
tgagataaat aaggtacttc ttaaaaaatt aaaagcacta aataactttc caaaggcaat 2940
cccaccccca actcctgaca tataccttca caacatcatt tggctggaag gtaaagatgt 3000
tctcattgac ttcttcaagg aaagagccaa acttgcctgt tttgactctg gagataccat 3060
ttgtaaagga ggtgaaatgc cacaaggaat ctacttaatt atttcaggaa tggcaatttt 3120
gcatagttta tctcctacct ttggaataga gagtaatcaa aggtgtgata gagggtccag 3180
agacatgttt acagagttct gtactactgg ggacataatt ggagagctaa gctgtctgct 3240
taagcgtgaa attgaatata ccgtcatctg tgaaactagt ttacaggcct gctttatctc 3300
cctggaggat ttatatgaag gctttgatgc cttctggcca tctctggaat ataaaatatg 3360
gctaaagctt gctctcagta ctgcctatca gtattttgaa tcaagtctta ttgatgagga 3420
cttaaggttt cagaactgtg tgatgttcaa tcaagcatat gtggaaactt tatcaagcta 3480
tagtgacatg attattgata atatgaccat gaaatttgtt atcattgtgt atggcagtgt 3540
aattgatact aagacagagg aaccatattt tgcaccttgc attataccta caacctgtga 3600
gcaggttcag ggaacttctg atttaagcaa gctgctgata atccaagcat ctgagcttac 3660
ccaaagaaat agtaacacca atgtcatggc ctcagtcaac acggtctttg aacaaccagg 3720
aaagaatata aatggaagac aaaagatgag ttgaaaactg gataccattt tagaaaaggg 3780
tattaatgat acaaatatga tgtgtggagt caggttaaag accaaactac tttcctcgct 3840
caaatactaa aggattatct gcaaggagtt tacttagaag ctactgaaac aggttactgc 3900
tgcatttagt ttataagcaa tggatggact tctgtaaaac ttcttaattt taagtagttg 3960
cattatattt gggatgttaa aaaagtcttc aggataatat aaaatacact gaaacatatg 4020
tcctaccaaa tgaaaccctg tttccagcta agagcaaatt ttaacatagt gcattataaa 4080
aagtgttgta taactgatat gttactctct aaagcataga acctgtaatt ttcatttgtg 4140
aaattgttat aattagtgcc tccctaatat tttcccgagt atagctattc tccccttccc 4200
agtttggtaa atattgaaaa acagaattat attccacaat cttagtaact ttcagtaagt 4260
aagtaacttt tgctttcagt gaaatttagg agaaattaat attctcatat tgcatagtac 4320
tgtttgatgt cacctttcat tttattttta aaaatcaaat aaagttgagt tttatggttg 4380
tcta 4384

<210> 150
<211> 1124
<212> PRT
<213> Homo sapiens

<400> 150

Met Ser Ser Tyr Phe Trp Ala Gln Asn Glu Ser Asn Arg Pro Asp Leu
1               5                   10                  15

Leu Cys Gly Gln Pro Ala Asp Tyr Leu Val Glu Glu Lys His Phe Thr
            20                  25                  30

Thr Leu Val Cys Phe Ile Val Val Leu Gly Gly Leu Leu Lys Met Cys
        35                  40                  45

Leu Lys Asn Cys Glu Val Ile Val Leu Thr Ile Leu Ser Leu Ser Gly
    50                  55                  60

Phe Val Ile Gly His Met Ala Tyr Asn Ser Val Glu Val His Gln Ile
65                  70                  75                  80

Val Tyr Pro Leu Leu Arg Thr Ser Ser Phe Ser Leu Tyr Ser Tyr Phe
                85                  90                  95

Ser Pro Leu Ile Ile Phe Met Val Ala Leu Asp Val Glu Phe Tyr Thr
            100                 105                 110

Leu Lys Lys Met Phe Trp Gln Val Leu Leu Thr Gly Leu Ile Ser Phe
        115                 120                 125

Ser Thr Ala Ser Ile Ile Ile Gly Tyr Val Val Ile Lys Phe Asn Lys
    130                 135                 140

Asp Ser Trp Asp Leu Gln Ser Cys Leu Leu Phe Ser Ile Thr Leu Gly
145                 150                 155                 160

Ile Ile Asp Pro Leu Arg Ser Val Asn Ser Leu Lys Thr Ile Gly Ile
                165                 170                 175

Ser Lys Ile Tyr Ile Asp Leu Ile Arg Gly Glu Ser Leu Ile Ile Cys
            180                 185                 190

Ser Ile Ala Ser Ile Phe Phe Gly Asn Phe Arg Gly Asn Arg Ile His
        195                 200                 205

Phe Ser Ile Phe Arg Asp Leu His Val Gly Ile Glu Leu Ser Tyr Asp
    210                 215                 220

Ile Leu Gly Ser Ile Ile Phe Gly Tyr Trp Cys Ala Lys Ile Ile Gln
225                 230                 235                 240

Cys Ile Leu Ala Asp Val Phe Ser Asn Met Leu Thr Asn Ile Ile Leu
                245                 250                 255

Cys Phe Ser Met Val Tyr Met Thr Phe Tyr Ile Val Glu Phe Leu Gly
            260                 265                 270

Met Ser Gly Thr Leu Ala Leu Ala Ala Val Gly Leu Asn Leu Asp Ser
        275                 280                 285

Leu Thr Phe Lys Pro Lys Ile Glu Leu Val Ile Thr Lys Phe Leu Arg
    290                 295                 300

Ile Phe Ser Ser Val Tyr Glu His Leu Ile Tyr Ala Phe Phe Gly Ile
305                 310                 315                 320

Val Ile Gly Cys Gly Glu Leu Ser His Tyr Glu Phe His Thr Ile Pro
                325                 330                 335

Phe Ile Phe Ile Leu Phe Thr Thr Val Asn Leu Val Arg Leu Leu Thr
            340                 345                 350

Ile Leu Leu Val Ser Pro Ile Leu Met His Ser Asn Tyr Glu Tyr Asn
        355                 360                 365

Trp Arg Trp Gly Val Val Ile Thr Trp Ser Gly Ile Lys Gly Val Phe
    370                 375                 380

Asn Leu Leu Trp Ala Pro Asp Val Tyr Asn Leu Ala Glu Arg Lys Val
385                 390                 395                 400

Glu Val Pro Gln Met Phe Ile Leu Tyr Val Gln Val Ile Ser Leu Leu
                405                 410                 415

Thr Met Gly Ile Asn Ser Tyr Val Met Thr Gln Ser Ala Arg Lys Leu
            420                 425                 430

Asp Leu Cys Val Leu Ser Leu Pro Arg Gln Met Ile Leu Gln Asn Ala
        435                 440                 445

Thr Gln His Ile Gln Glu Ile Val Gln Asn Thr Ile Thr Leu Phe Lys
    450                 455                 460

Thr Glu Lys Ile Leu Thr Asn Val Asn Trp Thr Leu Val Glu Asp Lys
465                 470                 475                 480

Thr Arg Ile Glu Tyr Ile Pro Phe Ser His Val Ser His Asn Asp Met
                485                 490                 495

Lys Thr Glu Ser Thr Thr Asp Glu Ala Leu Met Glu Glu Ala Arg Leu
            500                 505                 510

His Val Ala Ala Ile Gln Met Ser Ser Phe Glu Lys Gln Arg Asn Asn
        515                 520                 525

Gly Ile Leu Glu Ile Glu Ala Ala Arg Ile Leu Ile Gly Ala Ala Lys
    530                 535                 540

Cys Tyr Tyr Ser Ile Gln Gly Lys Phe Met Ser Ile Tyr Asp Val Ser
545                 550                 555                 560

Thr Tyr Met Arg Thr Arg Ser Trp Leu Ile Lys Phe Lys Asn Val Leu
                565                 570                 575

Thr Phe Leu Glu Tyr Cys Ile Glu Lys Ile His Phe Ile Pro Pro Glu
            580                 585                 590

Ser Asn Thr Phe Leu Thr Phe Ile Phe His Ile Val Phe Ser Glu Glu
        595                 600                 605

Phe Glu Tyr Thr Gly Gln Ile Ile Asn Leu Ile Tyr Ile Tyr Pro Met
    610                 615                 620

Ile Ile His Leu Trp Pro Met Ala Arg Gly Leu Asn Val Ser Ala Leu
625                 630                 635                 640

Ile Ser Ile Asn Tyr Tyr Phe Met Phe Leu Tyr Val Leu Glu Ser Thr
                645                 650                 655

Leu Lys Ile Ile Ile Leu Lys Arg Lys Tyr Phe Gln Gln Cys Trp Asn
            660                 665                 670

Thr Leu Glu Phe Phe Ile Leu Val Ile Gly Ile Ile Asp Ile Phe Cys
        675                 680                 685

Val Tyr Phe Val Lys Leu Arg Pro Asp Asn Leu Ala Leu Ile Gln Leu
    690                 695                 700

Thr Val Ile Met Gly Tyr Leu Arg Ile Ile Arg Phe Leu Pro Leu Phe
705                 710                 715                 720

Lys Ile Ile Val Pro Ile Leu Ile Arg Ile Ala Asp Val Gln Ile Lys
                725                 730                 735

Lys Arg Leu Ser Leu Met Tyr Ser Ile Thr Lys Gly Tyr Ile Lys Ser
            740                 745                 750

Gln Glu Asp Ala Lys Leu Leu Ile Lys Gln Ile Ala Val Cys Glu Ser
        755                 760                 765

Ile Tyr Gln Lys Leu Cys Glu Ile Leu Glu Thr Asn Lys Gln Asp Ala
    770                 775                 780

Val Lys Glu Leu Val Leu Met Glu His Glu Gly Arg Asp Val Val Ile
785                 790                 795                 800

Ala Leu Lys Thr Lys Gln Ala Ile Arg Asn Val Ile Ala Lys Ala Leu
                805                 810                 815

Lys Asn Leu Thr Phe Leu Cys Ser Arg Gly Ile Ile Asp Lys His Glu
            820                 825                 830

Val Ile Glu Ile Asn Lys Val Leu Leu Lys Lys Leu Lys Ala Leu Asn
        835                 840                 845

Asn Phe Pro Lys Ala Ile Pro Pro Pro Thr Pro Asp Ile Tyr Leu His
    850                 855                 860

Asn Ile Ile Trp Leu Glu Gly Lys Asp Val Leu Ile Asp Phe Phe Lys
865                 870                 875                 880

Glu Arg Ala Lys Leu Ala Cys Phe Asp Ser Gly Asp Thr Ile Cys Lys
                885                 890                 895

Gly Gly Glu Met Pro Gln Gly Ile Tyr Leu Ile Ile Ser Gly Met Ala
            900                 905                 910

Ile Leu His Ser Leu Ser Pro Thr Phe Gly Ile Glu Ser Asn Gln Arg
        915                 920                 925

Cys Asp Arg Gly Ser Arg Asp Met Phe Thr Glu Phe Cys Thr Thr Gly
    930                 935                 940

Asp Ile Ile Gly Glu Leu Ser Cys Leu Leu Lys Arg Glu Ile Glu Tyr
945                 950                 955                 960

Thr Val Ile Cys Glu Thr Ser Leu Gln Ala Cys Phe Ile Ser Leu Glu
                965                 970                 975

Asp Leu Tyr Glu Gly Phe Asp Ala Phe Trp Pro Ser Leu Glu Tyr Lys
            980                 985                 990

Ile Trp Leu Lys Leu Ala Leu Ser Thr Ala Tyr Gln Tyr Phe Glu Ser
        995                 1000                1005

Ser Leu Ile Asp Glu Asp Leu Arg Phe Gln Asn Cys Val Met Phe
    1010                1015                1020

Asn Gln Ala Tyr Val Glu Thr Leu Ser Ser Tyr Ser Asp Met Ile
    1025                1030                1035

Ile Asp Asn Met Thr Met Lys Phe Val Ile Ile Val Tyr Gly Ser
    1040                1045                1050

Val Ile Asp Thr Lys Thr Glu Glu Pro Tyr Phe Ala Pro Cys Ile
    1055                1060                1065

Ile Pro Thr Thr Cys Glu Gln Val Gln Gly Thr Ser Asp Leu Ser
    1070                1075                1080

Lys Leu Leu Ile Ile Gln Ala Ser Glu Leu Thr Gln Arg Asn Ser
    1085                1090                1095

Asn Thr Asn Val Met Ala Ser Val Asn Thr Val Phe Glu Gln Pro
    1100                1105                1110

Gly Lys Asn Ile Asn Gly Arg Gln Lys Met Ser
    1115                1120

<210> 151
<211> 21
<212> DNA
<213> künstliche Sequenz

<220>
<223> Oligonukleotid

<400> 151
ctacaacctg tgagcaggtt c 21

<210> 152
<211> 21
<212> DNA
<213> künstliche Sequenz

<220>
<223> Oligonukleotid

<400> 152
cctgtttcag tggcttctaa g 21

<210> 153
<211> 1189
<212> DNA
<213> Homo sapiens

<400> 153
ctatgccttc tgaccccgtc ttggacttca actgggagaa tgtggagcca tttgaacagg 60

ctcctcttct ggagcatatt ttcttctgtc acttgtagaa aagctgtatt ggattgtgag 120

gcaatgaaaa caaatgaatt cccttctcca tgtttggact caaagactaa ggtggttatg 180

aagggtcaaa atgtatctat gttttgttcc cataagaaca aatcactgca gatcacctat 240

tcattgtttc gacgtaagac acacctggga acccaggatg gaaaaggtga acctgcgatt 300

tttaacctaa gcatcacaga agcccatgaa tcaggcccct acaaatgcaa agcccaagtt 360

accagctgtt caaaatacag tcgtgacttc agcttcacga ttgtcgaccc ggtgacttcc 420

ccagtgctga acattatggt cattcaaaca gaaacagacc gacatataac attacattgc 480

ctctcagtca atggctcgct gcccatcaat tacactttct ttgaaaacca tgttgccata 540

tcaccagcta tttccaagta tgacagggag cctgctgaat ttaacttaac caagaagaat 600

cctggagaag aggaagagta taggtgtgaa gctaaaaaca gattgcctaa ctatgcaaca 660

tacagtcacc ctgtcaccat gccctcaaca ggcggagaca gctgtccttt ctgtctgaag 720

ctactacttc cagggttatt actgttgctg gtggtgataa tcctaattct ggctttttgg 780

gtactgccca aatacaaaac aagaaaagct atgagaaata atgtgcccag ggaccgtgga 840

gacacagcca tggaagttgg aatctatgca aatatccttg aaaaacaagc aaaggaggaa 900

tctgtgccag aagtgggatc caggccgtgt gtttccacag cccaagatga ggccaaacac 960

tcccaggagc tacagtatgc cacccccgtg ttccaggagg tggcaccaag agagcaagaa 1020

gcctgtgatt cttataaatc tggatatgtc tattctgaat cctgacctca gatgatctgc 1080

ctgcctcggc ctcccaaagt gctggaacta caagcctgag ccaccgtgcc cggccctgaa 1140

tcgctttagt aaataaaggg tctccaagaa taaattcatc cgaacatgc 1189

<210> 154
<211> 341
<212> PRT
<213> Homo sapiens

<400> 154

Met Trp Ser His Leu Asn Arg Leu Leu Phe Trp Ser Ile Phe Ser Ser
1               5                   10                  15

Val Thr Cys Arg Lys Ala Val Leu Asp Cys Glu Ala Met Lys Thr Asn
            20                  25                  30

Glu Phe Pro Ser Pro Cys Leu Asp Ser Lys Thr Lys Val Val Met Lys
        35                  40                  45

Gly Gln Asn Val Ser Met Phe Cys Ser His Lys Asn Lys Ser Leu Gln
    50                  55                  60

Ile Thr Tyr Ser Leu Phe Arg Arg Lys Thr His Leu Gly Thr Gln Asp
65                  70                  75                  80

Gly Lys Gly Glu Pro Ala Ile Phe Asn Leu Ser Ile Thr Glu Ala His
                85                  90                  95

Glu Ser Gly Pro Tyr Lys Cys Lys Ala Gln Val Thr Ser Cys Ser Lys
            100                 105                 110

Tyr Ser Arg Asp Phe Ser Phe Thr Ile Val Asp Pro Val Thr Ser Pro
        115                 120                 125

Val Leu Asn Ile Met Val Ile Gln Thr Glu Thr Asp Arg His Ile Thr
    130                 135                 140

Leu His Cys Leu Ser Val Asn Gly Ser Leu Pro Ile Asn Tyr Thr Phe
145                 150                 155                 160

Phe Glu Asn His Val Ala Ile Ser Pro Ala Ile Ser Lys Tyr Asp Arg
                165                 170                 175

Glu Pro Ala Glu Phe Asn Leu Thr Lys Lys Asn Pro Gly Glu Glu Glu
            180                 185                 190

Glu Tyr Arg Cys Glu Ala Lys Asn Arg Leu Pro Asn Tyr Ala Thr Tyr
        195                 200                 205

Ser His Pro Val Thr Met Pro Ser Thr Gly Gly Asp Ser Cys Pro Phe
    210                 215                 220

Cys Leu Lys Leu Leu Leu Pro Gly Leu Leu Leu Leu Leu Val Val Ile
225                 230                 235                 240

Ile Leu Ile Leu Ala Phe Trp Val Leu Pro Lys Tyr Lys Thr Arg Lys
                245                 250                 255

Ala Met Arg Asn Asn Val Pro Arg Asp Arg Gly Asp Thr Ala Met Glu
            260                 265                 270

Val Gly Ile Tyr Ala Asn Ile Leu Glu Lys Gln Ala Lys Glu Glu Ser
        275                 280                 285

Val Pro Glu Val Gly Ser Arg Pro Cys Val Ser Thr Ala Gln Asp Glu
    290                 295                 300

Ala Lys His Ser Gln Glu Leu Gln Tyr Ala Thr Pro Val Phe Gln Glu
305                 310                 315                 320

Val Ala Pro Arg Glu Gln Glu Ala Cys Asp Ser Tyr Lys Ser Gly Tyr
                325                 330                 335

Val Tyr Ser Glu Ser
            340

<210>  155
<211>  21
<212>  DNA
<213>  künstliche Sequenz

<220>
<223>  Oligonukleotid

<400>  155
gaggaatctg tgccagaagt g                                                21

<210>  156
<211>  21
<212>  DNA
<213>  künstliche Sequenz

<220>
<223>  Oligonukleotid

<400>  156
acagagtgag actccatcct g                                                21

<210>  157
<211>  2713

<212>  DNA
<213>  Homo sapiens

<400>  157
gggcttggct ggggtgctca gcccaatttt ccgtgtaggg agcgggcggc ggcgggggag      60
gcagaggcgg aggcggagtc aagagcgcac cgccgcgccc gccgtgccgg gcctgagctg     120
gagccgggcg tgagtcgcag caggagccgc agccggagtc acagccgcag ccagagccgc     180
agccaaagcc tcagagagca ggagttggag cgcaggccct gctggatccg cgcctagctc     240
gccgccaggc accggccgga ggacgggccg tggtgtcagc tcactgcccg ggcgctgtgg     300
gaggcagcga gcccgcgacc ccccgggccg ggcaccgcca ggcgcggagc ccagatcgcc     360
cccctgccag gcctggtcac ggccagagca cgcaggagtt cccagggtct ggatctgcgc     420
gcaccctaat gacctgggga ctgaagagaa aaaaggaacg aggatttcat ctaaaagcat     480
aacgtgggca ctaggcgagg aggaaagtgg agaccacctg gcacggggca gaggtgcctg     540
gagcccacgc ttgagcatcg gagaccctgg catcctagca gccgcgacct tggctctgcc     600
ctgtctgagc tggaaacaca gcttagcttc tagacatcgc tggcacaggc ctggcacaag     660
taagcagtgt cctcacctgt ctgaaacggg acacggggtc ggaggaacca ggatctagcc     720
tggccccaag cggaactctc tggtggccca gaggtcgtca ctggggagcc cgcctcctgc     780
cctagcctca ctggtgcgga tgtgccgctg cccgccggag caccatgatg gcaggatgac     840
ctcagccgaa gtaggagcag cagctggtgg tgctcaggcg gctgggcccc ccgagtggcc     900
ccctggcagc cctcaggccc tccggcagcc tggccgggcc cgagtggcca tggcagcact     960
ggtgtggctg ctggcgggag ccagcatgtc aagcctcaac aagtggatct tcacagtgca    1020
cggctttggg cggcccctgc tgctgtcggc cctgcacatg ctggtggcag ccctggcatg    1080
ccaccggggg gcacggcgcc ccatgccagg cggcactcgc tgccgagtcc tactgctcag    1140
tctcaccttt ggcacgtcca tggcctgcgg caacgtgggc ctaagggctg tgcccctgga    1200
cctggcacaa ctggttacta ccaccacacc tctgttcacc ctggccctgt cggcgctgct    1260
gctgggccgc cgccaccacc cacttcagtt ggccgccatg ggtccgctct gcctgggggc    1320
cgcctgcagc ctggctggag agttccggac accccctacc ggctgtggct tcctgctcgc    1380
agccacctgc ctccgcggac tcaagtcggt tcagcaaagt gccctgctgc aggaggagag    1440
gctggacgcg gtgaccctgc tttacgccac ctcgctgccc agcttctgcc tgctggcggg    1500
tgcagccctg gtgctggagg ctggcgttgc cccaccgccc actgctggcg actctcgcct    1560
ctgggcctgc atcctgctca gctgcctcct gtctgttctc tataacctgg ccagcttctc    1620
cctgctggcc ctcacctctg ccctcaccgt ccacgtcctg ggcaacctca ccgtggtggg    1680
caacctcatc ctgtcccggc tgttgtttgg cagccgcctc agtgccctca gctacgtggg    1740
catcgcactc actctttcag gaatgttcct ttaccacaac tgcgagttcg tggcctcctg    1800
ggctgcccgt cgggggctgt ggcggaggga ccagcccagc aagggtcttt gagacctggg    1860
ggatctcagg agccacctgg gatggccctg gcctgaatcc agcctccgct gtggccatag    1920

aaggaatgga gaacagggct gggcatggtg gctcacgcct ataatcccag cacttccaga 1980
gtccgaggtg ggtggatcac ctgaggccag gagttcgaga ccagcctggc taacatggca 2040
aaacctcatc tctactaaaa atagaaaaat tagctgggca tggtggcgcg tgcctatagt 2100
cccagctaca tgggaggcta aggtgggagg atcacttgag ccctggagat cgaggctgca 2160
gtaagccaag atcgcatgct actgcactcc agcctgggag acagagcgag acgctgtctc 2220
aattaaaaaa aaaaaaaagt ggagaactgg cagtgacctc tactgggggc catggcaggg 2280
aggggagcct tctggaaggg ctgccttgga gattggaatg ggactcccca gggagacctg 2340
cgttccatcc ctgcctgcct cacccctgcc acagactctg cacaccactg gatggtgggt 2400
ccaagcctgg cacagtccct gtgcttgtca gagtcattat tatgattaat atcaattacg 2460
atgccaaaaa ttgctgggca aactttgaag acctcaactt gttacaatga cgatgatgat 2520
gattcttggc ggttacacaa tccttcctcc tgggggggag gcagctagga ggcccagcag 2580
gggggcttct atgctgctgg gctcccctag ggagttgggg tagtctgtgc caactccagg 2640
cagctgctgt ggcctcaccc ctgggccccc caattttggg tcatccatcc tcaaatacac 2700
tatttttgct tgt 2713

<210> 158
<211> 350
<212> PRT
<213> Homo sapiens

<400> 158

Met Cys Arg Cys Pro Pro Glu His His Asp Gly Arg Met Thr Ser Ala
1               5                   10                  15

Glu Val Gly Ala Ala Ala Gly Gly Ala Gln Ala Ala Gly Pro Pro Glu
            20                  25                  30

Trp Pro Pro Gly Ser Pro Gln Ala Leu Arg Gln Pro Gly Arg Ala Arg
        35                  40                  45

Val Ala Met Ala Ala Leu Val Trp Leu Leu Ala Gly Ala Ser Met Ser
    50                  55                  60

Ser Leu Asn Lys Trp Ile Phe Thr Val His Gly Phe Gly Arg Pro Leu
65                  70                  75                  80

Leu Leu Ser Ala Leu His Met Leu Val Ala Ala Leu Ala Cys His Arg
                85                  90                  95

Gly Ala Arg Arg Pro Met Pro Gly Gly Thr Arg Cys Arg Val Leu Leu
            100                 105                 110

Leu Ser Leu Thr Phe Gly Thr Ser Met Ala Cys Gly Asn Val Gly Leu
        115                 120                 125

Arg Ala Val Pro Leu Asp Leu Ala Gln Leu Val Thr Thr Thr Thr Pro
    130                 135                 140

Leu Phe Thr Leu Ala Leu Ser Ala Leu Leu Leu Gly Arg Arg His His
145                 150                 155                 160

Pro Leu Gln Leu Ala Ala Met Gly Pro Leu Cys Leu Gly Ala Ala Cys
                165                 170                 175

Ser Leu Ala Gly Glu Phe Arg Thr Pro Pro Thr Gly Cys Gly Phe Leu
            180                 185                 190

Leu Ala Ala Thr Cys Leu Arg Gly Leu Lys Ser Val Gln Gln Ser Ala
        195                 200                 205

Leu Leu Gln Glu Glu Arg Leu Asp Ala Val Thr Leu Leu Tyr Ala Thr
    210                 215                 220

Ser Leu Pro Ser Phe Cys Leu Leu Ala Gly Ala Ala Leu Val Leu Glu
225                 230                 235                 240

Ala Gly Val Ala Pro Pro Pro Thr Ala Gly Asp Ser Arg Leu Trp Ala
                245                 250                 255

Cys Ile Leu Leu Ser Cys Leu Leu Ser Val Leu Tyr Asn Leu Ala Ser
            260                 265                 270

Phe Ser Leu Leu Ala Leu Thr Ser Ala Leu Thr Val His Val Leu Gly
        275                 280                 285

Asn Leu Thr Val Val Gly Asn Leu Ile Leu Ser Arg Leu Leu Phe Gly
    290                 295                 300

Ser Arg Leu Ser Ala Leu Ser Tyr Val Gly Ile Ala Leu Thr Leu Ser
305                 310                 315                 320

Gly Met Phe Leu Tyr His Asn Cys Glu Phe Val Ala Ser Trp Ala Ala
                325                 330                 335

Arg Arg Gly Leu Trp Arg Arg Asp Gln Pro Ser Lys Gly Leu
            340                 345                 350

<210>  159
<211>  21
<212>  DNA
<213>  künstliche Sequenz

<220>
<223>  Oligonukleotid

<400>  159
caagtcggtt cagcaaagtg c                                                21

<210> 160
<211> 21
<212> DNA
<213> künstliche Sequenz

<220>
<223> Oligonukleotid

<400> 160
cctgaaagag tgagtgcgat g 21


<210> 161
<211> 963
<212> DNA
<213> Homo sapiens

<400> 161
gactacacaa ggactgaacc agaaggaaga ggacagagca aagccatgaa catcatccta 60

gaaatccttc tgcttctgat caccatcatc tactcctact tggagtcgtt ggtgaagttt 120

ttcattcctc agaggagaaa atctgtggct ggggagattg ttctcattac tggagctggg 180

catggaatag gcaggcagac tacttatgaa tttgcaaaac gacagagcat attggttctg 240

tgggatatta ataagcgcgg tgtggaggaa actgcagctg agtgccgaaa actaggcgtc 300

actgcgcatg cgtatgtggt agactgcagc aacagagaag agatctatcg ctctctaaat 360

caggtgaaga aagaagtggg tgatgtaaca atcgtggtga ataatgctgg gacagtatat 420

ccagccgatc ttctcagcac caaggatgaa gagattacca agacatttga ggtcaacatc 480

ctaggacatt tttggatcac aaaagcactt cttccatcga tgatggagag aaatcatggc 540

cacatcgtca cagtggcttc agtgtgcggc cacgaaggga ttccttacct catcccatat 600

tgttccagca aatttgccgc tgttggcttt cacagaggtc tgacatcaga acttcaggcc 660

ttgggaaaaa ctggtatcaa aacctcatgt ctctgcccag tttttgtgaa tactgggttc 720

accaaaaatc caagcacaag attatggcct gtattggaga cagatgaagt cgtaagaagt 780

ctgatagatg gaatacttac caataagaaa atgatttttg ttccatcgta tatcaatatc 840

tttctgagac tacagaatcc tgataatatt aaaaacattg gtttggcact agcagcagtc 900

aaacgaacaa gattaattac ctgtcttcct gtttctcaag aatatttacg tagtttttca 960

tag 963


<210> 162
<211> 305
<212> PRT
<213> Homo sapiens

<400> 162

<210> 163
<211> 21
<212> DNA
<213> künstliche Sequenz

<220>
<223> Oligonukleotid

<400> 163
ggtctgacat cagaacttca g 21

<210> 164
<211> 21
<212> DNA
<213> künstliche Sequenz

<220>
<223> Oligonukleotid

<400> 164
tgcatacatc tctggctgga g 21

<210> 165
<211> 6014
<212> DNA
<213> Homo sapiens

<400> 165
cacccggaag gagcggtgtg agcggtccaa ggagccccgc aggtttgcct cggagatgaa 60
gcagtgtgtc cggctgacgg tccatcccaa caatatctcc gtctctcagt acaacgtgct 120
gctggtcctg gagacgtaca atgtcccgga gctgtcagct ggcgtcaact gcacctttga 180
ggacctgtca gagatggatg ggctggtcgt gggcaatcag atccagtgct actcccctgc 240
agccaaggag gtgccccgga tcatcacaga gaatggggac caccatgtcg tacagcttca 300
gctcaaatca aaggagaccg gcatgacctt cgccagcacc agctttgtct tctacaattg 360
cagcgtccac aattcgtgcc tgtcctgcgt ggagagtcca taccgctgcc actggtgtaa 420
ataccggcat gtctgcaccc atgaccccaa gacctgctcc ttccaggaag gccgagtgaa 480
gctgcccgag gactgccccc agctgctgcg agtggacaag atcctggtgc ccgtggaggt 540
gatcaagcct atcacgctga aggccaagaa cctcccccag ccccagtctg ggcagcgtgg 600
ctacgaatgc atcctcaaca ttcagggcag cgagcagcga gtgcccgccc tgcgcttcaa 660
cagctccagc gtacagtgcc agaacacctc ttattcctat gaagggatgg agatcaacaa 720
cctgcccgtg gagttgacag tcgtgtggaa tgggcacttc aacattgaca acccagctca 780
gaataaagtt cacctctaca agtgtggagc catgcgtgag agctgcgggc tgtgcctcaa 840
ggctgaccca gacttcgcat gtggctggtg ccagggccca ggccagtgca ccctgcgcca 900
gcactgccct gcccaggaga gccagtggct ggagctgtct ggtgccaaaa gcaagtgcac 960
aaacccccgc atcacagaga taatcccggt gacaggcccc cgggaagggg gcaccaaggt 1020

cactatccga ggggagaacc tgggcctgga atttcgcgac atcgcctccc atgtcaaggt 1080
tgctggcgtg gagtgcagcc ctttagtgga tggttacatc cctgcagaac agatcgtgtg 1140
tgagatgggg gaggccaagc ccagccagca tgcaggcttc gtggagatct gcgtggctgt 1200
gtgtcggcct gaattcatgg cccggtcctc acagctctat tacttcatga cactgactct 1260
ctcagatctg aagcccagcc gggggcccat gtccggaggg acccaagtga ccatcacagg 1320
caccaacctg aatgccggaa gcaacgtggt ggtgatgttt ggaaagcagc cctgtctctt 1380
ccacaggcga tctccatcct acattgtctg caacaccaca tcctcagatg aggtgctaga 1440
gatgaaggtg tcggtgcagg tggacagggc caagatccac caggacctgg tctttcagta 1500
tgtggaagac cccaccatcg tgcggattga gccagaatgg agcattgtca gtggaaacac 1560
acccatcgcc gtatggggga cccacctgga cctcatacag aacccccaga tccgtgccaa 1620
gcatggaggg aaggagcaca tcaatatctg tgaggttctg aacgctactg agatgacctg 1680
tcaggcgccc gccctcgctc tgggtcctga ccaccagtca gacctgaccg agaggcccga 1740
ggagtttggc ttcatcctgg acaacgtcca gtccctgctc atcctcaaca agaccaactt 1800
cacctactat cccaacccgg tgtttgaggc ctttggtccc tcaggaatcc tggagctcaa 1860
gcctggcacg cccatcatcc taaagggcaa gaacctgatc ccgcctgtgg ctgggggcaa 1920
cgtgaagctg aactacactg tgctggttgg ggagaagccg tgcaccgtga ccgtgtcaga 1980
tgtccagctg ctctgcgagt cccccaacct catcggcagg cacaaagtga tggcccgtgt 2040
cggtggcatg gagtactccc cggggatggt gtacattgcc ccggacagcc cgctcagcct 2100
gcccgccatc gtcagcatcg cagtggctgg cggcctcctc atcattttca tcgtggccgt 2160
gctcattgcc tataaacgca agtcccgcga aagtgacctc acgctgaagc ggctgcagat 2220
gcagatggac aacctggagt cccgtgtggc cctggagtgc aaggaaggta ctgagtggcc 2280
ccatgctgga ggccatgtgt gtgtgcgtgt gtgcatatgt gtgtgcatgc acatctgtgt 2340
atgtgtatgc atatgtttca tatacaaaca agcaggctgg gcagcagtgg gcagtgctgg 2400
aggctggcgg tgtgtgtgtc tgtgcgaatg tgtgtgtgtg catgtgtgtg tgtgcacatc 2460
tgtatgtata tatgtttcat atacaagcaa gcaggccggg cagcagtgag cagtgctgga 2520
ggctgtatat gtgtctgtgt gcgtgcgcat ctgtgtatgt gtatatgttt catgtacaag 2580
caagcaggcc gggcagcagt gggcagtgct ggaggctctg tgtgtgcgtg tgcatgtgtg 2640
tgtatgtatg tgtatgtgtt ccatttacaa gcaagcaggc caggcaactg tgagcagtgc 2700
tggaggctgt gtgcgcgtgt gtgtgtgtat gtgtatgtgt ttcatttaca agcaagcagg 2760
ccaggcagct gtgagcagtg ctggaggctg tgtgtgtgtg tgtgtgagca cgcacgtgtg 2820
tgagcacgca cgtgtatgtg tatgtgtgtc atttacaagc aagcaggcca ggcagctgtg 2880
agcagtgctg gaggccgtgt gtgtgtgtgt gtgtgtgtgt gcgcgcgcgc ctgtatatgt 2940
gtatgtgttt catttacaag caagcaggcc aggcagctgt gggcagtgct ggaggctgtg 3000
tgtgtgtgca cgtgtgtgta tgcgtatgtg tttcatttac aagcaagcag gccaggcagc 3060

tgtgggcagt gctggaggct gtgtgtgtgt gtgtgtgtgt gtgtgtgtat atatgtgtat 3120
gtgtatgtgt ttcatttaca agcaagcagc ccaggcagct gtgggcagtg ctggaggctg 3180
tgtgtgtgtg tgtgtgtgtg tgtgtatgtg tttcatttac aagtgtgtgt gtgtgtgtgt 3240
atgtgtatgt gtatgtgttt catttacaag caagcaggcc aggcagctgt gggcaatgct 3300
ggaggctgtg catcctacct gcatacctgc aaagcctctc actctatagt ccctatgcct 3360
gtgtcccaga ccacacccat acccaagcag gccccaccct ggcaacacca gagaggccaa 3420
ggtctccttg ccctctcctt gaaggtgtag tgattagaat ctcttttatg tgtggcaggc 3480
acacagcttt gaatgttgga ggcgcttggt gacttaaagg aaagctgcag actgataaaa 3540
agccaactcc ctccttctgc tccctgtggg ccgagcaccc caactgggag ggggcagccg 3600
aggggagctc ccacccagga ttgtcacctt caccccacta gagcaccttc accccactag 3660
agcagcctcc atacctggaa tcctggttga gtgggttttg cactctactc gaggggaggt 3720
ctgggggtgt cttaacatga cgcatttcag caatctccag ctttcttcct ctagcaggaa 3780
ggtaaggctg tagggctgat ctgtgattta gaaggaaggg tgtttcaaag cttgtattaa 3840
aaaaattaca aacaccacca taaagtgaaa tcagctgcac taaatccaag aaggaaattt 3900
aggagtcaga ctcttgtaac ccccaggata tcattttgtg actcatcctg ggaggatctg 3960
agctggttct ttgctgtaga tttgtacatg gagtaaatcc ggccccatac ctggggctct 4020
cacttcacac cgattcccac cagggcagcc acggctcttt ttgatgggga agtggatcca 4080
ttccatcccc tctctacatc cttcagctgt caacacagca tccgccttgt gggactgtta 4140
attactgcct tttattatat ttacgctgct taattttttt ctccgcaatg tactctttcc 4200
tctaattagg tgtagtgatt agaatctctt ttatgtgtgg caggcacgca gctttgaatg 4260
ttggaggcgc ttggtgactt aaaggaaagc tgcagactga taaaaagcca acaccctcct 4320
tctgctccct gtgggccgag caccccaact gggagggagc agccgatggg agctcccacc 4380
caggattgtc agctgaggcc ccaggaggaa accttggctt cagactttag gggcgagcta 4440
tgctgtgcac gtaggaagaa ggggtcttac agcaaaggac ttgtcagact agccacagag 4500
gcactttgca gcttgcccag agccagccac tgaacgttta cagggctgca ctggcccaag 4560
ccaaggggtc tccttgaaga cttcacagca agccaggacg tcctctacac aaactcagaa 4620
gacacccagc tgggcccttc atgggcctaa gcttctgata tataaacata cccgtgtatt 4680
tacaaacact cccacacagg cccacacacc ctcactgaca tacactcatg gactcacaca 4740
tacactcaca tgcacacatg catgcacact cacatacact cactcgtgca ctcacacata 4800
catgcccaca catagtgaca tgctcacaca ctcatgcttt cacatacata cactcactga 4860
catacactca tgtgctcaca cgctcatgta ctcacattcg tacacacaca ctgacatata 4920
cttacacact cacacttgca catgcataca catgcactca catgcacaca tgcatgcaca 4980
ctcatacact cacgcactca acttgcaggc gtgcagacac atgcccacat actcatgcac 5040
tcacattcac acatgcgtgc acacatagac gcatgcactc acacatgcat acacacagac 5100

atacacatgc actcacattc gtacttgcat acacaccaac acacatatgc acactcacac 5160

tgacaagcat acacacacac tcatgcactc acacccacgc aggcactcac attcacacac 5220

atacacactc attgacatac attcattcac atccatgcac tcacattcac acatgcatac 5280

acactgacat tcacacttgc acatgcctac acactcactg acatacacac acacatgcag 5340

tcatacacac tccctgacat gctcacacac tgtcatactc acacactccc tgacatgctc 5400

acacactgtc acactcacac actcacatac actccctgac atacacactc agacaagtgc 5460

ccatgcaccc acacctatgc tcatgcacat gttcccacac tctcttataa gcatacacac 5520

ccatgttcct cactcaggac acacatgaat gttccccagg gcatcatgtg acatcgcaga 5580

ggacagatgg tggaaaagac atgagcaacc taatgggaag aggaaaatgg gaaacaatgc 5640

attggaagag gaagaaaaaa aataaataac caaaggtttt ggcaagtgca gtaccaggtg 5700

gagaagcttg acttttctat ccttgatcat tttattccct cccaagaagt cagtcacagg 5760

acctggaagg ccagaaaggg tacatgtggg agacggtctg aggaagtacc tcggtcacta 5820

caatattttt gcacatataa agggttgggg aggaaagaga cacaaacgta tttaacacag 5880

atttgctgga tggaagctgc gtgtgtgaac gtgtgtatga gtgagtgcat tttgattttt 5940

tttttttttt tttgcacagt taagagaaaa aatcaaacaa gcagaaaaaa aaaagaaaaa 6000

agacttatca cggt 6014

<210> 166
<211> 817
<212> PRT
<213> Homo sapiens

<400> 166

Met Lys Gln Cys Val Arg Leu Thr Val His Pro Asn Asn Ile Ser Val
1               5                   10                  15

Ser Gln Tyr Asn Val Leu Leu Val Leu Glu Thr Tyr Asn Val Pro Glu
            20                  25                  30

Leu Ser Ala Gly Val Asn Cys Thr Phe Glu Asp Leu Ser Glu Met Asp
        35                  40                  45

Gly Leu Val Val Gly Asn Gln Ile Gln Cys Tyr Ser Pro Ala Ala Lys
    50                  55                  60

Glu Val Pro Arg Ile Ile Thr Glu Asn Gly Asp His His Val Val Gln
65                  70                  75                  80

Leu Gln Leu Lys Ser Lys Glu Thr Gly Met Thr Phe Ala Ser Thr Ser
                85                  90                  95

Phe Val Phe Tyr Asn Cys Ser Val His Asn Ser Cys Leu Ser Cys Val
            100                 105                 110

Glu Ser Pro Tyr Arg Cys His Trp Cys Lys Tyr Arg His Val Cys Thr
        115                 120                 125

His Asp Pro Lys Thr Cys Ser Phe Gln Glu Gly Arg Val Lys Leu Pro
    130                 135                 140

Glu Asp Cys Pro Gln Leu Leu Arg Val Asp Lys Ile Leu Val Pro Val
145                 150                 155                 160

Glu Val Ile Lys Pro Ile Thr Leu Lys Ala Lys Asn Leu Pro Gln Pro
                165                 170                 175

Gln Ser Gly Gln Arg Gly Tyr Glu Cys Ile Leu Asn Ile Gln Gly Ser
            180                 185                 190

Glu Gln Arg Val Pro Ala Leu Arg Phe Asn Ser Ser Ser Val Gln Cys
        195                 200                 205

Gln Asn Thr Ser Tyr Ser Tyr Glu Gly Met Glu Ile Asn Asn Leu Pro
    210                 215                 220

Val Glu Leu Thr Val Val Trp Asn Gly His Phe Asn Ile Asp Asn Pro
225                 230                 235                 240

Ala Gln Asn Lys Val His Leu Tyr Lys Cys Gly Ala Met Arg Glu Ser
                245                 250                 255

Cys Gly Leu Cys Leu Lys Ala Asp Pro Asp Phe Ala Cys Gly Trp Cys
            260                 265                 270

Gln Gly Pro Gly Gln Cys Thr Leu Arg Gln His Cys Pro Ala Gln Glu
        275                 280                 285

Ser Gln Trp Leu Glu Leu Ser Gly Ala Lys Ser Lys Cys Thr Asn Pro
    290                 295                 300

Arg Ile Thr Glu Ile Ile Pro Val Thr Gly Pro Arg Glu Gly Gly Thr
305                 310                 315                 320

Lys Val Thr Ile Arg Gly Glu Asn Leu Gly Leu Glu Phe Arg Asp Ile
                325                 330                 335

Ala Ser His Val Lys Val Ala Gly Val Glu Cys Ser Pro Leu Val Asp
            340                 345                 350

Gly Tyr Ile Pro Ala Glu Gln Ile Val Cys Glu Met Gly Glu Ala Lys
        355                 360                 365

Pro Ser Gln His Ala Gly Phe Val Glu Ile Cys Val Ala Val Cys Arg
    370                 375                 380

Pro Glu Phe Met Ala Arg Ser Ser Gln Leu Tyr Tyr Phe Met Thr Leu
385                 390                 395                 400

Thr Leu Ser Asp Leu Lys Pro Ser Arg Gly Pro Met Ser Gly Gly Thr
                405                 410                 415

Gln Val Thr Ile Thr Gly Thr Asn Leu Asn Ala Gly Ser Asn Val Val
            420                 425                 430

Val Met Phe Gly Lys Gln Pro Cys Leu Phe His Arg Arg Ser Pro Ser
        435                 440                 445

Tyr Ile Val Cys Asn Thr Thr Ser Ser Asp Glu Val Leu Glu Met Lys
    450                 455                 460

Val Ser Val Gln Val Asp Arg Ala Lys Ile His Gln Asp Leu Val Phe
465                 470                 475                 480

Gln Tyr Val Glu Asp Pro Thr Ile Val Arg Ile Glu Pro Glu Trp Ser
                485                 490                 495

Ile Val Ser Gly Asn Thr Pro Ile Ala Val Trp Gly Thr His Leu Asp
            500                 505                 510

Leu Ile Gln Asn Pro Gln Ile Arg Ala Lys His Gly Gly Lys Glu His
        515                 520                 525

Ile Asn Ile Cys Glu Val Leu Asn Ala Thr Glu Met Thr Cys Gln Ala
    530                 535                 540

Pro Ala Leu Ala Leu Gly Pro Asp His Gln Ser Asp Leu Thr Glu Arg
545                 550                 555                 560

Pro Glu Glu Phe Gly Phe Ile Leu Asp Asn Val Gln Ser Leu Leu Ile
                565                 570                 575

Leu Asn Lys Thr Asn Phe Thr Tyr Tyr Pro Asn Pro Val Phe Glu Ala
            580                 585                 590

Phe Gly Pro Ser Gly Ile Leu Glu Leu Lys Pro Gly Thr Pro Ile Ile
        595                 600                 605

Leu Lys Gly Lys Asn Leu Ile Pro Pro Val Ala Gly Gly Asn Val Lys
    610                 615                 620

Leu Asn Tyr Thr Val Leu Val Gly Glu Lys Pro Cys Thr Val Thr Val
625                 630                 635                 640

Ser Asp Val Gln Leu Leu Cys Glu Ser Pro Asn Leu Ile Gly Arg His
                645                 650                 655

Lys Val Met Ala Arg Val Gly Gly Met Glu Tyr Ser Pro Gly Met Val
            660                 665                 670

Tyr Ile Ala Pro Asp Ser Pro Leu Ser Leu Pro Ala Ile Val Ser Ile
        675                 680                 685

Ala Val Ala Gly Gly Leu Leu Ile Ile Phe Ile Val Ala Val Leu Ile
    690                 695                 700

Ala Tyr Lys Arg Lys Ser Arg Glu Ser Asp Leu Thr Leu Lys Arg Leu
705                 710                 715                 720

Gln Met Gln Met Asp Asn Leu Glu Ser Arg Val Ala Leu Glu Cys Lys
                725                 730                 735

Glu Gly Thr Glu Trp Pro His Ala Gly Gly His Val Cys Val Arg Val
            740                 745                 750

Cys Ile Cys Val Cys Met His Ile Cys Val Cys Val Cys Ile Cys Phe
        755                 760                 765

Ile Tyr Lys Gln Ala Gly Trp Ala Ala Val Gly Ser Ala Gly Gly Trp
    770                 775                 780

Arg Cys Val Cys Leu Cys Glu Cys Val Cys Val His Val Cys Val Cys
785                 790                 795                 800

Thr Ser Val Cys Ile Tyr Val Ser Tyr Thr Ser Lys Gln Ala Gly Gln
                805                 810                 815

Gln

<210>  167
<211>  21
<212>  DNA
<213>  künstliche Sequenz

<220>
<223>  Oligonukleotid

<400>  167
gcaccaaggt cactatccga g                                                21

<210>  168
<211>  21
<212>  DNA
<213>  künstliche Sequenz

<220>
<223>  Oligonukleotid

<400>  168
tctgagagag tcagtgtcat g                                                21

<210>  169
<211>  2565
<212>  DNA
<213>  Homo sapiens

<400>  169
actgcgacgg taccggggcg gcggggaagg accgagaggc gggaggagca gcggctcagg      60
cgcctgcaaa ctggtggcct gaacgaggta gaccatgact gtggtttcag tggcgtcact     120
cgctgggctg ctcttcctga ggttttccta agccatcccc tggcggaacc gcccccagta     180
tggactccaa ttgccttgac agtgttttta gtggctgttg caacattatg taaagaacaa     240
ggaataacag ttgtaggaat ttgctgtgtg tatgaagtgt ttattgccca ggggtatact     300
ttgccattac tatgtactac tgctggacag tttctccgtg gaaagggtag cattccattt     360
tctatgctgc agacactagt aaaactcatt gtcttgatgt tcagtacatt attacttgtt     420
gtgattagag tccaggttat tcaatcccaa cttccagtat tcaccaggtt tgataaccca     480
gctgctgtaa gcccaactcc tacaaggcaa ctaactttta actacctcct tcctgtgaat     540
gcttggttgt tattaaatcc ttcagagctc tgctgtgatt ggaccatggg aacaatacca     600
cttatagagt cattactaga tattcgaaat ctggccacat ttactttctt ttgttttctg     660
gggatgttgg gagtattcag tatcagatac tctggtgatt cctccaagac tgttttaatg     720
ttgcctgcta aaactgacat gggtcaaaaa tttgagaaaa gtagtgaaga ttcaaagcag     780
tcaagaagag tggaaggaac tttccagaga aacctagaaa tcccaaacag tcttaaggat     840
aaatttgaac ttggtgctca tgcttttatg acagtattaa tctgttcagc tttgggactt     900
tctctagcag tgcgttgcca ctctgttgga tttgttgttg ccgagcgagt attatatgtt     960
cccagcatgg ggttctgtat tttggtagcc catggatggc agaaaatatc aacaaaaagt    1020
gtatttaaaa agctatcctg gatttgtctg tctatggtga tactcactca ttccttaaaa    1080
acattccaca gaaattggga ttgggagtct gaatatacat tgtttatgtc agccttgaag    1140
gtaaataaaa ataatgccaa actttggaat aatgtgggtc atgctctgga aaatgaaaag    1200
aactttgaga gagctttgaa atacttctta caggctaccc atgttcagcc agatgatatt    1260
ggtgcccata tgaatgtagg aagaacttat aaaaatttaa atagaaccaa agaagctgaa    1320
gaatcttaca tgatggctaa atcactgatg cctcaaatta ttcctggtaa aaaatatgca    1380
gccagaattg cccctaacca cctaaatgtt tatatcaatc tggctaacct gatccgagca    1440
aatgagtccc gactggaaga agcagatcag ctgtaccgtc aagcaataag catgaggccc    1500
gacttcaagc aggcttacat tagcagagga gaattgcttt taaaaatgaa taaacctctt    1560
aaagcaaagg aagcatatct taaagcacta gagctggaca gaaataatgc agatctttgg    1620
tacaacttgg caattgtaca tattgaactt aaagaaccaa atgaagccct aaaaaacttt    1680
aatcgtgctc tggaactaaa tccaaagcat aaactagcat tattcaactc tgctatagta    1740
atgcaagaat caggtgaggt taaactcaga cctgaagcta gaaaacgact tctaagttat    1800
ataaatgaag agccactaga tgctaatggg tatttcaatt tgggaatgct tgccatggat    1860

gacaaaaagg acaatgaagc agagatttgg atgaagaaag ccataaagtt acaagccgac 1920
ttccgaagtg ctttgtttaa tctggctctc ctgtattccc agactgcaaa ggaattaaag 1980
gctttgccaa ttttggagga gttactcaga tactaccctg atcatatcaa gggcctcatt 2040
ttaaaaggag acattctgat gaatcaaaag aaagatatac taggagcaaa aaaatgtttt 2100
gaaaggattt tggagatgga tccaagcaat gtgcaaggaa aacacaatct ttgtgttgtt 2160
tattttgaag aaaaagactt attaaaagct gaaagatgcc ttcttgaaac actggcatta 2220
gcaccacatg aagaatatat tcagcgccat ttgaatatag tcagggataa gatttcctca 2280
tctagtttta tagagccaat attcccaacc agtaagattt caagtgtgga aggaaagaaa 2340
attccaactg aaagtgtaaa agaaattaga ggtgaatcca gacaaacaca aatagtaaaa 2400
acaagtgata ataaaagtca gtctaaatcc aacaaacaat taggaaaaaa tggagacgaa 2460
gagacacccc acaaaacaac aaaagacatc aaagaaattg agaagaaaag agttgctgct 2520
ttaaaaagac tagaagagat tgaacgtatt ttaaatggtg aataa 2565

<210> 170
<211> 733
<212> PRT
<213> Homo sapiens

<400> 170

Met Leu Gln Thr Leu Val Lys Leu Ile Val Leu Met Phe Ser Thr Leu
1               5                   10                  15

Leu Leu Val Val Ile Arg Val Gln Val Ile Gln Ser Gln Leu Pro Val
            20                  25                  30

Phe Thr Arg Phe Asp Asn Pro Ala Ala Val Ser Pro Thr Pro Thr Arg
        35                  40                  45

Gln Leu Thr Phe Asn Tyr Leu Leu Pro Val Asn Ala Trp Leu Leu Leu
    50                  55                  60

Asn Pro Ser Glu Leu Cys Cys Asp Trp Thr Met Gly Thr Ile Pro Leu
65                  70                  75                  80

Ile Glu Ser Leu Leu Asp Ile Arg Asn Leu Ala Thr Phe Thr Phe Phe
                85                  90                  95

Cys Phe Leu Gly Met Leu Gly Val Phe Ser Ile Arg Tyr Ser Gly Asp
            100                 105                 110

Ser Ser Lys Thr Val Leu Met Leu Pro Ala Lys Thr Asp Met Gly Gln
        115                 120                 125

Lys Phe Glu Lys Ser Ser Glu Asp Ser Lys Gln Ser Arg Arg Val Glu
    130                 135                 140

Gly Thr Phe Gln Arg Asn Leu Glu Ile Pro Asn Ser Leu Lys Asp Lys
145                 150                 155                 160

Phe Glu Leu Gly Ala His Ala Phe Met Thr Val Leu Ile Cys Ser Ala
                165                 170                 175

Leu Gly Leu Ser Leu Ala Val Arg Cys His Ser Val Gly Phe Val Val
            180                 185                 190

Ala Glu Arg Val Leu Tyr Val Pro Ser Met Gly Phe Cys Ile Leu Val
        195                 200                 205

Ala His Gly Trp Gln Lys Ile Ser Thr Lys Ser Val Phe Lys Lys Leu
    210                 215                 220

Ser Trp Ile Cys Leu Ser Met Val Ile Leu Thr His Ser Leu Lys Thr
225                 230                 235                 240

Phe His Arg Asn Trp Asp Trp Glu Ser Glu Tyr Thr Leu Phe Met Ser
                245                 250                 255

Ala Leu Lys Val Asn Lys Asn Asn Ala Lys Leu Trp Asn Asn Val Gly
            260                 265                 270

His Ala Leu Glu Asn Glu Lys Asn Phe Glu Arg Ala Leu Lys Tyr Phe
        275                 280                 285

Leu Gln Ala Thr His Val Gln Pro Asp Asp Ile Gly Ala His Met Asn
    290                 295                 300

Val Gly Arg Thr Tyr Lys Asn Leu Asn Arg Thr Lys Glu Ala Glu Glu
305                 310                 315                 320

Ser Tyr Met Met Ala Lys Ser Leu Met Pro Gln Ile Ile Pro Gly Lys
                325                 330                 335

Lys Tyr Ala Ala Arg Ile Ala Pro Asn His Leu Asn Val Tyr Ile Asn
            340                 345                 350

Leu Ala Asn Leu Ile Arg Ala Asn Glu Ser Arg Leu Glu Glu Ala Asp
        355                 360                 365

Gln Leu Tyr Arg Gln Ala Ile Ser Met Arg Pro Asp Phe Lys Gln Ala
    370                 375                 380

Tyr Ile Ser Arg Gly Glu Leu Leu Leu Lys Met Asn Lys Pro Leu Lys
385                 390                 395                 400

Ala Lys Glu Ala Tyr Leu Lys Ala Leu Glu Leu Asp Arg Asn Asn Ala
                405                 410                 415

Asp Leu Trp Tyr Asn Leu Ala Ile Val His Ile Glu Leu Lys Glu Pro
            420                 425                 430

Asn Glu Ala Leu Lys Asn Phe Asn Arg Ala Leu Glu Leu Asn Pro Lys
        435                 440                 445

His Lys Leu Ala Leu Phe Asn Ser Ala Ile Val Met Gln Glu Ser Gly
    450                 455                 460

Glu Val Lys Leu Arg Pro Glu Ala Arg Lys Arg Leu Leu Ser Tyr Ile
465                 470                 475                 480

Asn Glu Glu Pro Leu Asp Ala Asn Gly Tyr Phe Asn Leu Gly Met Leu
                485                 490                 495

Ala Met Asp Asp Lys Lys Asp Asn Glu Ala Glu Ile Trp Met Lys Lys
            500                 505                 510

Ala Ile Lys Leu Gln Ala Asp Phe Arg Ser Ala Leu Phe Asn Leu Ala
        515                 520                 525

Leu Leu Tyr Ser Gln Thr Ala Lys Glu Leu Lys Ala Leu Pro Ile Leu
    530                 535                 540

Glu Glu Leu Leu Arg Tyr Tyr Pro Asp His Ile Lys Gly Leu Ile Leu
545                 550                 555                 560

Lys Gly Asp Ile Leu Met Asn Gln Lys Lys Asp Ile Leu Gly Ala Lys
                565                 570                 575

Lys Cys Phe Glu Arg Ile Leu Glu Met Asp Pro Ser Asn Val Gln Gly
            580                 585                 590

Lys His Asn Leu Cys Val Val Tyr Phe Glu Glu Lys Asp Leu Leu Lys
        595                 600                 605

Ala Glu Arg Cys Leu Leu Glu Thr Leu Ala Leu Ala Pro His Glu Glu
    610                 615                 620

Tyr Ile Gln Arg His Leu Asn Ile Val Arg Asp Lys Ile Ser Ser Ser
625                 630                 635                 640

Ser Phe Ile Glu Pro Ile Phe Pro Thr Ser Lys Ile Ser Ser Val Glu
                645                 650                 655

Gly Lys Lys Ile Pro Thr Glu Ser Val Lys Glu Ile Arg Gly Glu Ser
            660                 665                 670

Arg Gln Thr Gln Ile Val Lys Thr Ser Asp Asn Lys Ser Gln Ser Lys
        675                 680                 685

Ser Asn Lys Gln Leu Gly Lys Asn Gly Asp Glu Glu Thr Pro His Lys
    690                 695                 700

Thr Thr Lys Asp Ile Lys Glu Ile Glu Lys Lys Arg Val Ala Ala Leu
705                 710                 715                 720

Lys Arg Leu Glu Glu Ile Glu Arg Ile Leu Asn Gly Glu
                725                 730

<210>  171
<211>  21
<212>  DNA
<213>  künstliche Sequenz

<220>
<223>  Oligonukleotid

<400>  171
aggcttacat tagcagagga g                                                21

<210>  172
<211>  21
<212>  DNA
<213>  künstliche Sequenz

<220>
<223>  Oligonukleotid

<400>  172
cgttttctag cttcaggtct g                                                21

<210>  173
<211>  3296
<212>  DNA
<213>  Homo sapiens

<400>  173
tgaattcaaa acagttactc tgaatggtct ttgctaagaa caatttaatg attaagtaag      60

gtcagtgtcc ttggaagtcc aaactctagc cagatttccc tggtctacac ccctagggat     120

aaggtaaatg tttaagcaca cagtgaactt cctgaggccc ccaaatctaa tggaactagc     180

tattgagggc taaaagagga tggttttttt agaaaactcg aagcaaatct ctcaggctgg     240

ggatatttca aagactacta ctattattat taataacaat tgcaatattt gttgagtccc     300

taaatgaagc taaaactttg ttctaataaa tttaatcttt acagcaacct atgaggtaga     360

taatattgtc attcccatga gggagctaag gatcagagaa ggtaagtcac ttgtctaagg     420

tcacatagct agcatgttat gcaatcagga gtcaaacctg gtttgtctga atctgaagtc     480

catctgctct gtgcactttt ataccgtctg ctttttcctt tattcctaac cttcttccat     540

tctgattccc actgagtagt ggacaggaac cactgaagtt tgcctgacac catcaaccag     600

gccctagtca cctggctttg cctttgccct gctgtgtgat cttagctccc tgcccaggcc     660

cacagccatg gccatggccc agaaactcag ccacctcctg ccgagtctgc ggcaggtcat     720

ccaggagcct cagctatctc tgcagccaga gcctgtcttc acggtggatc gagctgaggt     780

gccgccgctc ttctggaagc cgtacatcta tgcgggctac cggccgctgc atcagacctg 840
gcgcttctat ttccgcacgc tgttccagca gcacaacgag gccgtgaatg tctggaccca 900
cctgctggcg gccctggtac tgctgctgcg gctggccctc tttgtggaga ccgtggactt 960
ctggggagac ccacacgccc tgcccctctt catcattgtc cttgcctctt tcacctacct 1020
ctccttcagt gccttggctc acctcctgca ggccaagtct gagttctggc attacagctt 1080
cttcttcctg gactatgtgg gggtggccgt gtaccagttt ggcagtgcct tggcacactt 1140
ctactatgct atcgagcccg cctggcatgc ccaggtgcag gctgtttttc tgcccatggc 1200
tgcctttctc gcctggcttt cctgcattgg ctcctgctat aacaagtaca tccagaaacc 1260
aggcctgctg ggccgcacat gccaggaggt gccctccgtc ctggcctacg cactggacat 1320
tagtcctgtg gtgcatcgta tcttcgtgtc ctccgacccc accacggatg atccagctct 1380
tctctaccac aagtgccagg tggtcttctt tctgctggct gctgccttct tctctacctt 1440
catgcccgag cgctggttcc ctggcagctg ccatgtcttc gggcagggcc accaactttt 1500
ccacatcttc ttggtgctgt gcacgctggc tcagctggag gctgtggcac tggactatga 1560
ggcccgacgg cccatctatg agcctctgca cacgcactgg cctcacaact tttctggcct 1620
cttcctgctc acggtgggca gcagcatcct cactgcattc ctcctgagcc agctggtaca 1680
gcgcaaactt gatcagaaga ccaagtgaag ggggatggca tctggtaggg agggaggtat 1740
agttggggga caggggtctg ggtttggctc caggtgggaa caaggcctgg taaagttgtt 1800
tgtgtctggc ccacagtgac tctctgtgca cgactcaact gccaagggca tcactggcca 1860
attcttggat ttagggattg gctaggagtt gctggggtcc actcctgggc ctgccccagc 1920
tccttgccca gggagaggga aagagttaac ggtgtgggcc actccagctt gcccttccac 1980
tgccactcac tggggtgagg ctgggggtca gcttggtgag gattggggct tctagattgt 2040
ctaggcagga ggtgaaactt aggccagagt cagatttgag ctgagccagg ggaggccttg 2100
gcaacctact tctactcaga tttcattgct ggatgcggaa ggggtaggcc caaaatatat 2160
acaggatctt actgtccctt gaagcccagc cacaagtgtt ggagctgcag agagacccca 2220
aaggtagtag attgtgccag atacaaatgg gtcccatcca gtgcttcata ctccttcagt 2280
cactatccca gacagtgagc cccagatctc ctagctctgg cttctgtgtc ccacacggcc 2340
tgttcccagc ttctctcctg gttcccttgt tacggattca tttatccatt cagtgtttcc 2400
tgggcctctg ctcagaggca ggtcaccact gggccctgtg gatcaatgca agatgacaaa 2460
ggcttttttt tttttttttt tttttttttt ttttgaggag tttcgctctt gttggctagg 2520
ctggagtaaa atggtgcgat ctcggctcac tgcacctccg cctcccaggt tcaagcgatt 2580
ttcctgcctc agcctcccga gtagctgggg ttacaggcat gcaccaccat gcctggctaa 2640
ttttctgtat ttttagtaga gacggggttt ctccatgttg gtcaggctgg tcttgaactc 2700
ctgacctcag gtgatctgcc cgtctcggcc tcccaaagtg ctgggattac cggcatgagc 2760
cactgcgcct ggccgacaaa ggctttgata tcagaatgaa ctgtcaaggg aggtgctgga 2820

gagggattaa cctgtgctgc ctgggaccct cagggtctta ggttggggag tgtgaatagg 2880
agtttgcaga tggagaatag gaagggcatt ccaggcagag ggaaacctgt gcagagacca 2940
agaggtgtgg aaggaaaagt ggggttgggg ctgggtggtc tggattatgg cctggatgca 3000
ataaagtact gtgacagtag ccacctcttt gttttttgtc tcctgtttcc gggaggggcc 3060
cctgctcaca ttactggagg ttttccggag gaagctgggg cccctgggag tggacacagg 3120
gtgcagggag cagttcttgt tttatctttg ctgggggatg gggttggggc cttatatacc 3180
atatctatat atacaaaatt tgtttggcaa gggagtgggc ggcagtttta ttactaaagt 3240
tttataagta gttaaaataa tgtgtttaaa atatgataat cccactttat gatctg 3296

<210> 174
<211> 346
<212> PRT
<213> Homo sapiens

<400> 174

Met Ala Met Ala Gln Lys Leu Ser His Leu Leu Pro Ser Leu Arg Gln
1               5                   10                  15

Val Ile Gln Glu Pro Gln Leu Ser Leu Gln Pro Glu Pro Val Phe Thr
            20                  25                  30

Val Asp Arg Ala Glu Val Pro Pro Leu Phe Trp Lys Pro Tyr Ile Tyr
        35                  40                  45

Ala Gly Tyr Arg Pro Leu His Gln Thr Trp Arg Phe Tyr Phe Arg Thr
    50                  55                  60

Leu Phe Gln Gln His Asn Glu Ala Val Asn Val Trp Thr His Leu Leu
65                  70                  75                  80

Ala Ala Leu Val Leu Leu Leu Arg Leu Ala Leu Phe Val Glu Thr Val
                85                  90                  95

Asp Phe Trp Gly Asp Pro His Ala Leu Pro Leu Phe Ile Ile Val Leu
            100                 105                 110

Ala Ser Phe Thr Tyr Leu Ser Phe Ser Ala Leu Ala His Leu Leu Gln
        115                 120                 125

Ala Lys Ser Glu Phe Trp His Tyr Ser Phe Phe Phe Leu Asp Tyr Val
    130                 135                 140

Gly Val Ala Val Tyr Gln Phe Gly Ser Ala Leu Ala His Phe Tyr Tyr
145                 150                 155                 160

Ala Ile Glu Pro Ala Trp His Ala Gln Val Gln Ala Val Phe Leu Pro
                165                 170                 175

Met Ala Ala Phe Leu Ala Trp Leu Ser Cys Ile Gly Ser Cys Tyr Asn
        180                 185                 190

Lys Tyr Ile Gln Lys Pro Gly Leu Leu Gly Arg Thr Cys Gln Glu Val
    195                 200                 205

Pro Ser Val Leu Ala Tyr Ala Leu Asp Ile Ser Pro Val Val His Arg
    210                 215                 220

Ile Phe Val Ser Ser Asp Pro Thr Thr Asp Asp Pro Ala Leu Leu Tyr
225                 230                 235                 240

His Lys Cys Gln Val Val Phe Phe Leu Leu Ala Ala Ala Phe Phe Ser
                245                 250                 255

Thr Phe Met Pro Glu Arg Trp Phe Pro Gly Ser Cys His Val Phe Gly
            260                 265                 270

Gln Gly His Gln Leu Phe His Ile Phe Leu Val Leu Cys Thr Leu Ala
        275                 280                 285

Gln Leu Glu Ala Val Ala Leu Asp Tyr Glu Ala Arg Arg Pro Ile Tyr
    290                 295                 300

Glu Pro Leu His Thr His Trp Pro His Asn Phe Ser Gly Leu Phe Leu
305                 310                 315                 320

Leu Thr Val Gly Ser Ser Ile Leu Thr Ala Phe Leu Leu Ser Gln Leu
                325                 330                 335

Val Gln Arg Lys Leu Asp Gln Lys Thr Lys
            340                 345

<210>  175
<211>  2858
<212>  DNA
<213>  Homo sapiens

<400>  175
agtggcgggg aagcaaagca caggagcgct gtggtgccag cggccgggct agggacgact      60

ggcgggtttg cgctggaccc gaccccgagg gcgggcgcaa gggggcgggc gctgccgtac     120

tcaggccgcg gggccagggc gggccggccg gcggggcatt taaaccccgc tgacagccag     180

tccagcccgg gacacgcgcc cagctctgta gcctcctccg tcgactcagc cttaggtacc     240

ggtcaggcaa aatgcggtcc tccctggctc cgggagtctg gttcttccgg gccttctcca     300

gggacagctg gttccgaggc ctcatcctgc tgctgacctt cctaatttac gcctgctatc     360

acatgtccag gaagcctatc agtatcgtca agagccgtct gcaccagaac tgctcggagc     420

agatcaaacc catcaatgat actcacagtc tcaatgacac catgtggtgc agctgggccc     480

catttgacaa ggacaactat aaggagttac tagggggcgt ggacaacgcc ttcctcatcg     540

cctatgccat cggcatgttc atcagtgggg tttttgggga gcggcttccg ctccgttact 600
acctctcagc tggaatgctg ctcagtggcc ttttcacctc gctctttggc ctgggatatt 660
tctggaacat ccacgagctc tggtactttg tggtcatcca ggtctgtaat ggactcgtcc 720
agaccacagg ctggccctct gtggtgacct gtgttggcaa ctggttcggg aaggggaagc 780
gggggttcat catgggcatc tggaattccc acacatctgt gggcaacatc ctgggctccc 840
tgatcgccgg catctgggtg aacgggcagt ggggcctgtc gttcatcgtg cctggcatca 900
ttactgccgt catgggcgtc atcaccttcc tcttcctcat cgaacaccca gaagatgtgg 960
actgcgcccc tcctcagcac cacggtgagc cagctgagaa ccaggacaac cctgaggacc 1020
ctgggaacag tccctgctct atcagggaga gcggccttga gactgtggcc aaatgctcca 1080
aggggccatg cgaagagcct gctgccatca gcttctttgg ggcgctccgg atcccaggcg 1140
tggtcgagtt ctctctgtgt ctgctgtttg ccaagctggt cagttacacc ttcctctact 1200
ggctgcccct ctacatcgcc aatgtggctc actttagtgc caaggaggct ggggacctgt 1260
ctacactctt cgatgttggt ggcatcatag gcggcatcgt ggcagggctc gtctctgact 1320
acaccaatgg cagggccacc acttgctgtg tcatgctcat cttggctgcc cccatgatgt 1380
tcctgtacaa ctacattggc caggacggga ttgccagctc catagtgatg ctgatcatct 1440
gtgggggcct ggtcaatggc ccatacgcgc tcatcaccac tgctgtctct gctgatctgg 1500
ggactcacaa gagcctgaag ggcaacgcca aagccctgtc cacggtcacg gccatcattg 1560
acggcaccgg ctccataggt gcggctctgg ggcctctgct ggctgggctc atctccccca 1620
cgggctggaa caatgtcttc tacatgctca tctctgccga cgtcctagcc tgcttgctcc 1680
tttgccggtt agtatacaaa gagatcttgg cctggaaggt gtccctgagc agaggcagcg 1740
ggtataaaga aatatgaggc cccaattgga acagcagcat ggagggtccc agttgggtcc 1800
ccaacgtgct ccccatgggc aagacaatgg aaacttccac aagcagggaa ggcaaaccct 1860
ctttattgaa cattagccag cccagcccag accccagggc tgcctaagga cacagagatt 1920
ctccatggga aggggactgc caagcatgag gaaatagaag attcaggggc ctgagctctg 1980
gaagctgcaa gcaaaaggga tgggactagg gctgagttgt gtctccattt tgataaggaa 2040
aggatatgct cagactcttg cttgttcaga ttccaagaca gaaggcttca caaggccaac 2100
gcctggaaaa tgggcatctc tccttcccat gttaagcttt aacctctgta atctgcctgt 2160
atctataggt gggcatctca ctccaccaaa ggagcccagc ctctctttgt ccctctatcc 2220
atgcaacagt cttctctgtg catttcccca agctgggccc tcttctactc tccatttagg 2280
cctgttgata actccattac ccgcccatca ctgctgttcc tccagggcca gcactcgggc 2340
gaggcagggg agctgccttc ggtacataat ttgaaggggc actccctctt gggcacatgc 2400
cggccctgag tgcctccctt gcctcactct gatcctggcc ccataatgtc ctcagtggaa 2460
ggtgatgggg gccggtgctg tggggagagt agaaagaggg gttggcatga ctaaaaatac 2520
cagtatgtgt attaagtatt ttgagaatga aatgccaagg agtgcctact atatgccagc 2580

tctaggaatg gagtagacag tggacacaag aaggacttac gccctgagca caggtgccaa 2640

tggtgacaag actggcaaga cgtgagggca tgaatggttc attcaggcag ctgctgcaga 2700

tgtggtcacc tggtgccatc tgctgctccc ttttccactt ttctatgtcc tccttccacc 2760

ccaagtcccg gatcactcgc tgttttctgg ctagctcttg gcatctccat ctgagcctaa 2820

agttgcccac tggcaccaat agattctgtt tgacctgc 2858

<210> 176
<211> 501
<212> PRT
<213> Homo sapiens

<400> 176

Met Arg Ser Ser Leu Ala Pro Gly Val Trp Phe Phe Arg Ala Phe Ser
1               5                   10                  15

Arg Asp Ser Trp Phe Arg Gly Leu Ile Leu Leu Leu Thr Phe Leu Ile
            20                  25                  30

Tyr Ala Cys Tyr His Met Ser Arg Lys Pro Ile Ser Ile Val Lys Ser
        35                  40                  45

Arg Leu His Gln Asn Cys Ser Glu Gln Ile Lys Pro Ile Asn Asp Thr
    50                  55                  60

His Ser Leu Asn Asp Thr Met Trp Cys Ser Trp Ala Pro Phe Asp Lys
65                  70                  75                  80

Asp Asn Tyr Lys Glu Leu Leu Gly Gly Val Asp Asn Ala Phe Leu Ile
                85                  90                  95

Ala Tyr Ala Ile Gly Met Phe Ile Ser Gly Val Phe Gly Glu Arg Leu
            100                 105                 110

Pro Leu Arg Tyr Tyr Leu Ser Ala Gly Met Leu Leu Ser Gly Leu Phe
        115                 120                 125

Thr Ser Leu Phe Gly Leu Gly Tyr Phe Trp Asn Ile His Glu Leu Trp
    130                 135                 140

Tyr Phe Val Val Ile Gln Val Cys Asn Gly Leu Val Gln Thr Thr Gly
145                 150                 155                 160

Trp Pro Ser Val Val Thr Cys Val Gly Asn Trp Phe Gly Lys Gly Lys
                165                 170                 175

Arg Gly Phe Ile Met Gly Ile Trp Asn Ser His Thr Ser Val Gly Asn
            180                 185                 190

Ile Leu Gly Ser Leu Ile Ala Gly Ile Trp Val Asn Gly Gln Trp Gly
        195                 200                 205

Leu Ser Phe Ile Val Pro Gly Ile Ile Thr Ala Val Met Gly Val Ile
    210                 215                 220

Thr Phe Leu Phe Leu Ile Glu His Pro Glu Asp Val Asp Cys Ala Pro
225                 230                 235                 240

Pro Gln His His Gly Glu Pro Ala Glu Asn Gln Asp Asn Pro Glu Asp
                245                 250                 255

Pro Gly Asn Ser Pro Cys Ser Ile Arg Glu Ser Gly Leu Glu Thr Val
            260                 265                 270

Ala Lys Cys Ser Lys Gly Pro Cys Glu Glu Pro Ala Ala Ile Ser Phe
        275                 280                 285

Phe Gly Ala Leu Arg Ile Pro Gly Val Val Glu Phe Ser Leu Cys Leu
    290                 295                 300

Leu Phe Ala Lys Leu Val Ser Tyr Thr Phe Leu Tyr Trp Leu Pro Leu
305                 310                 315                 320

Tyr Ile Ala Asn Val Ala His Phe Ser Ala Lys Glu Ala Gly Asp Leu
                325                 330                 335

Ser Thr Leu Phe Asp Val Gly Gly Ile Ile Gly Gly Ile Val Ala Gly
            340                 345                 350

Leu Val Ser Asp Tyr Thr Asn Gly Arg Ala Thr Thr Cys Cys Val Met
        355                 360                 365

Leu Ile Leu Ala Ala Pro Met Met Phe Leu Tyr Asn Tyr Ile Gly Gln
    370                 375                 380

Asp Gly Ile Ala Ser Ser Ile Val Met Leu Ile Ile Cys Gly Gly Leu
385                 390                 395                 400

Val Asn Gly Pro Tyr Ala Leu Ile Thr Thr Ala Val Ser Ala Asp Leu
                405                 410                 415

Gly Thr His Lys Ser Leu Lys Gly Asn Ala Lys Ala Leu Ser Thr Val
            420                 425                 430

Thr Ala Ile Ile Asp Gly Thr Gly Ser Ile Gly Ala Ala Leu Gly Pro
        435                 440                 445

Leu Leu Ala Gly Leu Ile Ser Pro Thr Gly Trp Asn Asn Val Phe Tyr
    450                 455                 460

Met Leu Ile Ser Ala Asp Val Leu Ala Cys Leu Leu Leu Cys Arg Leu
465                 470                 475                 480

Val Tyr Lys Glu Ile Leu Ala Trp Lys Val Ser Leu Ser Arg Gly Ser
                485                 490                 495

Gly Tyr Lys Glu Ile
            500

<210> 177
<211> 21
<212> DNA
<213> künstliche Sequenz

<220>
<223> Oligonukleotid

<400> 177
tctacatcgc caatgtggct c 21

<210> 178
<211> 21
<212> DNA
<213> künstliche Sequenz

<220>
<223> Oligonukleotid

<400> 178
cagatgatca gcatcactat g 21

<210> 179
<211> 4892
<212> DNA
<213> Homo sapiens

<400> 179
atagaaacct taaaagggca acacaaagtt ttgaatagaa gaggccaagc agcctcgccc 60

agaagctgat gtttgtgaat gtactgggcc ttctaaagcg gcgcttcaca caccttttca 120

cttcttggca caggtaggaa aggatgatat tacaagggtc aaaatggggg taaacagaag 180

aggctgctcc tgcagaaggc ttcctgcaga agcccttgca cttggagggc tgggaagacc 240

catgctgtat ctgcatccct gtcattcgtt tcacggcatc cagttgggaa gctctgctta 300

aagctttgtc tggcacgttt tcttagctac atttttccac tccagctgag actgcctcac 360

tgagttgtca acacttggtc ttcttcagca gtgaggaacc aacaagacag gaggctgggt 420

caatactcaa cttggcaaac tccaggaaat ggtgcttaaa acgtttggct tcttgaatgg 480

aattcatggt actgctccca gcctgcacct gggttctcca acttgagaca atttctcccc 540

gcatcccccc acccttccct ggctttcact cactagcaag tgggctgctt ctactttctt 600

tctcacattc atttcttagg tccattctca gagcggttag gattactgtt taattagcct 660

cataatcata tctatgatgg caaaatcaag aaacaattta aacatgattc ttaaaagtaa 720

ggagataaat accagagaca tagaaggtga aagaatttgc ctctaggaag caggaattta 780

aatttgggga agacagggtg gagcaaggga tataaatcta gtccattttt cttttctttt 840

cttttctttt ttttttattt ttaagataga gtctcactct gtcgcccagg ctggagtgca 900
gtggcatgat cttggctcac tgcaacctct gcctccttcg ttcaagcgat tctcccacct 960
cagcctccct agtaactggg attacaagtg actgccacta tgcccagcta attttgtatt 1020
tttagtagag acgaggtttc accatgttgg ccaggctggt ctcgaactcc tgacctcagg 1080
ggatccgccc agctcagcct cccaaagtgc taggattcca ggcgtgagcc actgcacctg 1140
gcggaatcta gtccattttc cactttgcta ccacacatct gcagggtttc ttgcttgctt 1200
aaaagctttc attggctccc aatctctgat aatatcaaga gcaagttcct gaacaactca 1260
ttcaagaccc atcacccctc gcatctgtca actctgcccc ttgaatatta cacctcattt 1320
tactacacca catctagttc cctgaatatg caaacagatt tcatacattt gcacctttat 1380
acatgttatt gcttttgcct gggagagtat tctcttgctg ctataataat agctaatgac 1440
actgtgctaa gtactttctg tgatttataa ctgttaattc ttacatcaac cctatggtaa 1500
atgttactgt tatctccatt ttataaacaa gaaaactaag acttaaagag tttaagtgat 1560
ttgcagaaat atgtagtatt tggtgaggct aaatttgaac ccagcaatct gactccagga 1620
ctaacataat attacctatt catccttcta aaatgtttcc cagacactaa atttgaacag 1680
gattaaaaga tttaaatgtt tttaagtctt aaaagggcaa ggagaaaata caagtgaatt 1740
gcttttaatc tcaaactaag catgaaacaa cggctgaaat tacaaaggaa aagtgacaga 1800
tctgactgtt gaacttttaa cttcttttat ccaaaaaaaa accccataga ataaaattta 1860
aaaacaagta aaaattacaa aaaatttgca atatacatga cagataagca taacattaaa 1920
gagaacttag aaagaaaaaa tagcctacta aaaatgagca aaatgcaaat tcgtcatcat 1980
gagagaaaaa tgtaaatggc caaacatttt taagagaagt aaaaacttaa aacgataatg 2040
caccatcaaa ttgagaataa aataatactc agagctagta atttgggcca gtgacccttg 2100
gagtaggatg tatagcaact aaagaaactc atacattact gagaagggtg taaattggct 2160
caacgattct ggagagcaat ttgacagaat gtagtgaaag cgtcaaaaat gttcacacac 2220
tttgacttaa aaattacatt cctggaaatt tataatacaa acattttcta taaaaggtca 2280
gatggcaaat actttgggct ttgaaggcca catatgtctc tgtggctttt cttttgtgtg 2340
tgtgtttaaa aaaaaaaaaa actgcccccc ctccccccac ccttgttagg ccattcttgc 2400
attactataa agaaatacct gaggctggtt aatttatatg aaaagaggtt taactggctc 2460
atggttctgc aggctgtaca ggaagcataa tgccatctgc ttctgggggg gcctcaggaa 2520
gcttccaatc atgctggaag gtgatgggga gcagatgtct cacatggtga gaacgggagc 2580
aagaaggagt tgggggggag gagccacata aacaatgaga tccctgtgag ctcagagtga 2640
gagcacactt atcaccaagg agatggccca agctattcat gagggatccg cccctatgat 2700
ccaaacacct cccaccaggc tccacctcca acactggaga ttatatctca acatgaaatt 2760
tgaatgggac atccaaactg tatcaccccc aaaatgtaaa gtctcatcac agtacatttg 2820
gtaatggcca aaagagaaac caaactaaat gtccgagaat aaaaattagt tacaactaga 2880

tacacggagg caagttttta aaaagtgtta aaattttaaa atgttgcaga atggtatcta 2940
ttggataaaa tagtatttat gatttattaa gtggaaaata cagtttacaa aataatatgg 3000
tgtgatcccg aaaacaacat aatcatgtgt gtataaatgc atagaaaaaa atctggaaag 3060
atataaacag atatttatag tggtctaggg caggggatgg aattgtagat atttgctttt 3120
tgttttatgt atatgtttcc cataatgaaa tgtattgttt atataattaa aaaatatacg 3180
aaactttgct tgggggacaa caaagcacct catttgttaa tttgggaaaa tcttttatta 3240
caatctctgt aaggagttgg ttgctctctc ttctgtactc cctgattaca taatgctctt 3300
ctgagcactt ttatttaata gcagaatggt tgatatcatt atttagttaa ggtttcctct 3360
attatcgaac atctgagttc ccagtacact agtctcccct tatctgtggt tttgcttcca 3420
aggtttcagt tatgatcaac caagatctga aaatattaaa tgaaaaattc cagaaataaa 3480
acaattcata agttttacat tgtgcaccat cctgctgtat cctgtccagg ccatgggtca 3540
tccctcttgt tcagtgtgtc cacactgtag atgctcccct gtctgttagt cactttgtag 3600
ttggcttggt tgtcagacct actgtcaagg tattgcagta cttatgtcca agtaacactt 3660
atttaactta ataatggccc ctaaacacaa gagtagtaat gttggcaatt tgggtatgcc 3720
aaagaaaagt cataaagtgc ttcttttaag tgaaaaccca aaagtttttg aattagtaag 3780
gaaagaaaaa aatccatatg ctgaggtcgc taagatctat gataagaatg aatcttctac 3840
ctgtgaaatt gtgaaggaaa aagaaattca cgttagtttt gctgttgtac ctcaaactgc 3900
aaaagttatg gccacagtgt ataactttta ttaaaatata tttgtataac tgttcttatt 3960
ttacttttct gttttatttt tagagacagg gcttcattct gtcacccagg ctggagtgca 4020
aaggtgcaat catagctcac tgcagcctca aactctttgg ctcaagtgat cttcctgcct 4080
cagcctccca agtagctggg actgcaggtg tgcatcacta cgcccagcta attttttaat 4140
tttttgtgca gatggagtct gactctgttg cccaggaact cctggcctca agtaatcctc 4200
ccgcctcggt tttccaaaga gctgggatta caggcatgag ccactgtgcc tggctattct 4260
attttattag cagtaattgt tgttaatctc ttattgtgcc ttatttatat taataactta 4320
atcatagata gatatgtata ggaaaaaaca ttgtatataa agggttcagt actatctgca 4380
gtttcagata tccatgaggg gtcttgaaac gtatccccca caggtaaggg gggacttgta 4440
tttctctgtt ataaatatgc tggttattct ccacttgttg tgttttagtg ccatcttctg 4500
ctcttctctg ctagactctg tgcctcagaa ggtggaattt ttcataaact attccagctg 4560
gggtctcatg ccagttggtt ttgaccaatg ggtaacacca tcagtagatt ggaggatgga 4620
aaaggaaaaa aggttaggat atgtttcacc acctcttttc ctgcttctgg ctgggttctg 4680
atggtggctt tgtcccttga aggctcctcc tgcaaggcag ccctgctcca ctgtgccagc 4740
cttcactggg ctctactaac gtgattccct ccccttattt cttcaggcct agctgtgcta 4800
actcctaggt acctccatgt ttcttgtttc ctttcatcca accctaaccc taacttctat 4860
aaatagttcc cgcaataaag tctcttcagc tg 4892

<210> 180
<211> 95
<212> PRT
<213> Homo sapiens

<400> 180

<210> 181
<211> 15
<212> DNA
<213> künstliche Sequenz

<220>
<223> Oligonukleotid

<400> 181
gctcaacgat tctgg                                                        15

<210> 182
<211> 15
<212> DNA
<213> künstliche Sequenz

<220>
<223> Oligonukleotid

<400> 182
atgtggcctt caaag                                                        15

<210> 183
<211> 501
<212> DNA
<213> Homo sapiens

<400> 183
atgaacagaa gcatctatga ccgacagttg ctctgtgtcc ttctagcctc gcaggagttt      60

ccagctcatg agggcagagg agatgaagag aggccgatcg acgtgagggt tgtgcaggcg     120

gcccctctga ggtgtgactc cactcctcct gagggtgctg taggagacat ctgcaaaaaa     180

gaagatgctg gcaatatgcc atcaacctca gaggggagta tttaccctga aatggctcac 240
ttcctgagga acaaacttgc tggatctagt gtacggaaac ctgattctgg gttcctttgg 300
gaaggagcat tacgggcctg gttatttctc atcctaatag ttctcaccca catcatgtgg 360
gtcccattag tacaggtatc tccgaatgct ccactcttcc attacattga gtcaattgct 420
catgaccttg ggcctccaat tggggctatt ttcctgctat ccatctcctg gtctatagta 480
aaagagccaa tgagcagata a 501

<210> 184
<211> 166
<212> PRT
<213> Homo sapiens

<400> 184

<210> 185
<211> 20
<212> DNA
<213> künstliche Sequenz

<220>
<223>  Oligonukleotid

<400>  185
ctgagggtgc tgtaggagac                                                    20


<210>  186
<211>  15
<212>  DNA
<213>  künstliche Sequenz

<220>
<223>  Oligonukleotid

<400>  186
ggcccgtaat gctcc                                                         15


<210>  187
<211>  3978
<212>  DNA
<213>  Homo sapiens

<400>  187
   agactagggg cgagtttgga gcaagtaact gtcagtgagg ttgcagttgg tctgggctgt      60
   ttggctgtga gcgaaatagc tgccccccac ttctcacttg cacaccacgg gatactcctc     120
   ctgaggctcc ggatgattca gatggactgt gaaaaacaac aagatggatg atcatatgga     180
   gattgcttct aacataaatc tgcataaaaa tttttctgaa acatggctgg aatatttaag     240
   gagttttttt tcagtactga ggacctccct gaagtcattc taacattgtc tttgatcagc     300
   tccattggag catttttgaa ccggcacttg gaagactttc caattcctgt ccctgtgata     360
   ttatttttac ttggatgcag ttttgaagta ttaagcttta catcttcaca ggtccaaaga     420
   tacgcaaacg ccatacaatg gatgagtcca gacttatttt ttcgtatatt tacaccagta     480
   gttttcttta ctactgcatt tgacatggat acgtacatgc ttcaaaagtt attttggcag     540
   atacttttaa tttcaattcc cggctttttg gttaattata tcttagttct ttggcatctg     600
   gcatctgtaa atcaattact tttgaagcct acccaatggt tattattttc agctatcctt     660
   gtgagttcag atcccatgct aaccgcagct gctataagag accttgggct ttctagaagc     720
   ctcatcagtt taattaatgg agaaagtctg atgacctctg ttatatcatt aattacattt     780
   actagtatta tggattttga ccaaagacta caaagtaaaa gaaaccatac cttagctgaa     840
   gagatcgtgg gtggaatttg ttcatatatt atagcaagtt tcttgtttgg aattctaagt     900
   tcaaaactga ttcaattttg gatgtcaact gtttttggtg atgatgtcaa tcatataagt     960
   ctcatctttt caattctgta tctcatcttt tatatttgtg agttagttgg aatgtcagga    1020
   atatttactc tggccattgt gggacttctt ttaaattcta caagttttaa agcagcaatt    1080
   gaagaaacac ttcttcttga atttctgacc cttcttttaa taagccctgt tttgtctcga    1140
   gttggtcatg agttcagttg gcgctggata ttcataatgg tctgtagtga aatgaagggg    1200
   atgcctaata taaacatggc ccttctgctt gcctactctg atctttattt tggatctgac    1260
   aaagaaaaat ctcaaatatt atttcatgga gtgttagtat gcctaataac ccttgttgtc 1320
   aatagattta ttttgccagt ggcagttact atactaggtc ttcgtgatgc cacatcaaca 1380
   aaatataaat cggtttgttg cacatttcaa cactttcaag agctaaccaa gtctgcagcc 1440
   tctgccctta aatttgacaa agatcttgct aatgctgatt ggaacatgat tgagaaagca 1500
   attacacttg aaaacccata catgttgaac gaagaagaaa caacagaaca tcagaaggtg 1560
   aaatgtccac actgtaacaa ggaaatagat gagatcttta acactgaagc aatggagctg 1620
   gccaacaggc gtctcttgtc agcacaaata gcaagctacc agagacaata caggaatgag 1680
   attctgtccc agagtgctgt ccaggtgttg gttggtgcag cagaaagttt tggtgagaag 1740
   aagggaaaat gtatgagtct tgatacaata aagaattatt ctgaaagcca aaaaacagtt 1800
   acctttgcta gaaaactact acttaattgg gtgtataata ccagaaagga aaaagagggc 1860
   ccatcaaaat acttcttttt tcgtatatgc catacaatag tatttactga ggaatttgaa 1920
   catgttggat accttgtgat attaatgaat atatttccct ttataatctc ttggatatcc 1980
   cagttaaatg taatctacca cagcgaatta aaacacacta actactgttt tcttacactt 2040
   tatattctag aggcactact taagatagca gcaatgagga aggacttttt ttcacatgcc 2100
   tggaacatat tcgagttagc aattacatta attggcatct tacatgtaat acttattgaa 2160
   atagacacca ttaagtatat ttttaatgag actgaagtaa tagtctttat aaaagttgtt 2220
   caattttttc gtatactacg cattttcaag ctcatagcac caaagttgct gcaaataata 2280
   gataaaagaa tgagtcatca gaagaccttt tggtatggaa tactaaaagg ctatgtccaa 2340
   ggcgaagcag acataatgac cataattgat cagattacaa gttctaaaca gattaaacag 2400
   atgttattaa agcaagtgat aaggaatatg gaacatgcta taaaagagct aggctactta 2460
   gagtatgatc acccagaaat tgctgtcact gtgaaaacaa aggaagaaat taatgttatg 2520
   ctcaatatgg ctacagaaat tcttaaggct tttggcttaa aaggaattat tagtaaaact 2580
   gaaggtgctg gaattaataa gttaatcatg gccaaaaaga aagaggtgct tgattctcaa 2640
   tctattatca ggcctcttac tgttgaagaa gttctatatc atattccgtg gctagataaa 2700
   aacaaagatt atataaactt cattcaggaa aaagccaaag ttgtaacatt tgattgtgga 2760
   aatgatatat ttgaagaagg tgatgagccc aaaggaatct atatcattat ttcaggcatg 2820
   gtaaagcttg aaaaatcaaa gccaggttta gggattgatc aaatggtgga gtcaaaggag 2880
   aaagattttc cgataattga cacagactat atgctcagtg gagaaataat aggagagata 2940
   aactgcttaa ctaatgaacc tatgaaatat tctgccacct gcaaaactgt agtggagaca 3000
   tgttttattc ccaaaactca cttgtatgat gcttttgagc aatgctctcc tctcattaaa 3060
   caaaaaatgt ggctaaaact tggactcgct attacagcca gaaaaatcag agaacactta 3120
   tcttatgagg attggaacta caatatgcaa ctaaagctct ctaatattta tgtagtagat 3180
   ataccaatga gtaccaaaac tgatatttat gatgaaaatc taatctatgt tatcctcata 3240
   catggagctg tagaagattg tctgttacga aaaacttata gagcaccttt cttaattcct 3300
   ataacatgcc atcagataca aagtattgaa gatttcacaa aagtagtgat tattcaaact 3360
   ccgattaaca tgaaaacatt cagaaggaat attagaaagt ttgttcctaa acataaaagt 3420
   tatcttacac caggattaat aggttcagtt ggaacattgg aagaaggcat tcaagaagaa 3480
   agaaatgtta aggaggatgg agcacacagt gccgccactg ccaggagtcc ccagccttgc 3540
   tccctgctgg ggacaaagtt caactgtaag gagtccccta gaataaacct aaggaaagtc 3600
   aggaaagagt aagactgtta agaagaccga agcatgtatt aatgctgtgg ctatgagagg 3660
   cctcctgctg cagaaacaca cttccctaca tcaagaagga gtaacttcag gttggatcct 3720
   gtgtggatga tcttggtgct aagcagaaaa gaaatttgga ccttgaaacc agcagttcaa 3780
   catatatact ttttgcaaaa tttccttgat ttaaaatatt tgttatttta aatatacaaa 3840
   acatttagaa aaatcttaga gtaaatttta gtcttaaagc cagaaaataa gtttatagcc 3900
   atctagatat tttgcatatt gctcttacag caataatggt ttggttcact ttatgaaaaa 3960
   taaaatgtat taaaatat 3978

<210> 188
<211> 1129
<212> PRT
<213> Homo sapiens

<400> 188

Met Ala Gly Ile Phe Lys Glu Phe Phe Phe Ser Thr Glu Asp Leu Pro
1               5                   10                  15

Glu Val Ile Leu Thr Leu Ser Leu Ile Ser Ser Ile Gly Ala Phe Leu
            20                  25                  30

Asn Arg His Leu Glu Asp Phe Pro Ile Pro Val Pro Val Ile Leu Phe
        35                  40                  45

Leu Leu Gly Cys Ser Phe Glu Val Leu Ser Phe Thr Ser Ser Gln Val
    50                  55                  60

Gln Arg Tyr Ala Asn Ala Ile Gln Trp Met Ser Pro Asp Leu Phe Phe
65                  70                  75                  80

Arg Ile Phe Thr Pro Val Val Phe Phe Thr Thr Ala Phe Asp Met Asp
                85                  90                  95

Thr Tyr Met Leu Gln Lys Leu Phe Trp Gln Ile Leu Leu Ile Ser Ile
            100                 105                 110

Pro Gly Phe Leu Val Asn Tyr Ile Leu Val Leu Trp His Leu Ala Ser
        115                 120                 125

Val Asn Gln Leu Leu Leu Lys Pro Thr Gln Trp Leu Leu Phe Ser Ala
    130                 135                 140

Ile Leu Val Ser Ser Asp Pro Met Leu Thr Ala Ala Ala Ile Arg Asp
145                 150                 155                 160

Leu Gly Leu Ser Arg Ser Leu Ile Ser Leu Ile Asn Gly Glu Ser Leu
                165                 170                 175

Met Thr Ser Val Ile Ser Leu Ile Thr Phe Thr Ser Ile Met Asp Phe
            180                 185                 190

Asp Gln Arg Leu Gln Ser Lys Arg Asn His Thr Leu Ala Glu Glu Ile
        195                 200                 205

Val Gly Gly Ile Cys Ser Tyr Ile Ile Ala Ser Phe Leu Phe Gly Ile
    210                 215                 220

Leu Ser Ser Lys Leu Ile Gln Phe Trp Met Ser Thr Val Phe Gly Asp
225                 230                 235                 240

Asp Val Asn His Ile Ser Leu Ile Phe Ser Ile Leu Tyr Leu Ile Phe
                245                 250                 255

Tyr Ile Cys Glu Leu Val Gly Met Ser Gly Ile Phe Thr Leu Ala Ile
            260                 265                 270

Val Gly Leu Leu Leu Asn Ser Thr Ser Phe Lys Ala Ala Ile Glu Glu
        275                 280                 285

Thr Leu Leu Leu Glu Phe Leu Thr Leu Leu Leu Ile Ser Pro Val Leu
    290                 295                 300

Ser Arg Val Gly His Glu Phe Ser Trp Arg Trp Ile Phe Ile Met Val
305                 310                 315                 320

Cys Ser Glu Met Lys Gly Met Pro Asn Ile Asn Met Ala Leu Leu Leu
                325                 330                 335

Ala Tyr Ser Asp Leu Tyr Phe Gly Ser Asp Lys Glu Lys Ser Gln Ile
            340                 345                 350

Leu Phe His Gly Val Leu Val Cys Leu Ile Thr Leu Val Val Asn Arg
        355                 360                 365

Phe Ile Leu Pro Val Ala Val Thr Ile Leu Gly Leu Arg Asp Ala Thr
    370                 375                 380

Ser Thr Lys Tyr Lys Ser Val Cys Cys Thr Phe Gln His Phe Gln Glu
385                 390                 395                 400

Leu Thr Lys Ser Ala Ala Ser Ala Leu Lys Phe Asp Lys Asp Leu Ala
                405                 410                 415

Asn Ala Asp Trp Asn Met Ile Glu Lys Ala Ile Thr Leu Glu Asn Pro
            420                 425                 430

Tyr Met Leu Asn Glu Glu Glu Thr Thr Glu His Gln Lys Val Lys Cys
        435                 440                 445

Pro His Cys Asn Lys Glu Ile Asp Glu Ile Phe Asn Thr Glu Ala Met
    450                 455                 460

Glu Leu Ala Asn Arg Arg Leu Leu Ser Ala Gln Ile Ala Ser Tyr Gln
465                 470                 475                 480

Arg Gln Tyr Arg Asn Glu Ile Leu Ser Gln Ser Ala Val Gln Val Leu
                485                 490                 495

Val Gly Ala Ala Glu Ser Phe Gly Glu Lys Lys Gly Lys Cys Met Ser
            500                 505                 510

Leu Asp Thr Ile Lys Asn Tyr Ser Glu Ser Gln Lys Thr Val Thr Phe
        515                 520                 525

Ala Arg Lys Leu Leu Leu Asn Trp Val Tyr Asn Thr Arg Lys Glu Lys
    530                 535                 540

Glu Gly Pro Ser Lys Tyr Phe Phe Phe Arg Ile Cys His Thr Ile Val
545                 550                 555                 560

Phe Thr Glu Glu Phe Glu His Val Gly Tyr Leu Val Ile Leu Met Asn
                565                 570                 575

Ile Phe Pro Phe Ile Ile Ser Trp Ile Ser Gln Leu Asn Val Ile Tyr
            580                 585                 590

His Ser Glu Leu Lys His Thr Asn Tyr Cys Phe Leu Thr Leu Tyr Ile
        595                 600                 605

Leu Glu Ala Leu Leu Lys Ile Ala Ala Met Arg Lys Asp Phe Phe Ser
    610                 615                 620

His Ala Trp Asn Ile Phe Glu Leu Ala Ile Thr Leu Ile Gly Ile Leu
625                 630                 635                 640

His Val Ile Leu Ile Glu Ile Asp Thr Ile Lys Tyr Ile Phe Asn Glu
                645                 650                 655

Thr Glu Val Ile Val Phe Ile Lys Val Val Gln Phe Phe Arg Ile Leu
            660                 665                 670

Arg Ile Phe Lys Leu Ile Ala Pro Lys Leu Leu Gln Ile Ile Asp Lys
        675                 680                 685

Arg Met Ser His Gln Lys Thr Phe Trp Tyr Gly Ile Leu Lys Gly Tyr
    690                 695                 700

Val Gln Gly Glu Ala Asp Ile Met Thr Ile Ile Asp Gln Ile Thr Ser
705                 710                 715                 720

Ser Lys Gln Ile Lys Gln Met Leu Leu Lys Gln Val Ile Arg Asn Met
                725                 730                 735

Glu His Ala Ile Lys Glu Leu Gly Tyr Leu Glu Tyr Asp His Pro Glu
            740                 745                 750

Ile Ala Val Thr Val Lys Thr Lys Glu Glu Ile Asn Val Met Leu Asn
        755                 760                 765

Met Ala Thr Glu Ile Leu Lys Ala Phe Gly Leu Lys Gly Ile Ile Ser
    770                 775                 780

Lys Thr Glu Gly Ala Gly Ile Asn Lys Leu Ile Met Ala Lys Lys Lys
785                 790                 795                 800

Glu Val Leu Asp Ser Gln Ser Ile Ile Arg Pro Leu Thr Val Glu Glu
                805                 810                 815

Val Leu Tyr His Ile Pro Trp Leu Asp Lys Asn Lys Asp Tyr Ile Asn
            820                 825                 830

Phe Ile Gln Glu Lys Ala Lys Val Val Thr Phe Asp Cys Gly Asn Asp
        835                 840                 845

Ile Phe Glu Glu Gly Asp Glu Pro Lys Gly Ile Tyr Ile Ile Ile Ser
    850                 855                 860

Gly Met Val Lys Leu Glu Lys Ser Lys Pro Gly Leu Gly Ile Asp Gln
865                 870                 875                 880

Met Val Glu Ser Lys Glu Lys Asp Phe Pro Ile Ile Asp Thr Asp Tyr
                885                 890                 895

Met Leu Ser Gly Glu Ile Ile Gly Glu Ile Asn Cys Leu Thr Asn Glu
            900                 905                 910

Pro Met Lys Tyr Ser Ala Thr Cys Lys Thr Val Val Glu Thr Cys Phe
        915                 920                 925

Ile Pro Lys Thr His Leu Tyr Asp Ala Phe Glu Gln Cys Ser Pro Leu
    930                 935                 940

Ile Lys Gln Lys Met Trp Leu Lys Leu Gly Leu Ala Ile Thr Ala Arg
945                 950                 955                 960

Lys Ile Arg Glu His Leu Ser Tyr Glu Asp Trp Asn Tyr Asn Met Gln
                965                 970                 975

Leu Lys Leu Ser Asn Ile Tyr Val Val Asp Ile Pro Met Ser Thr Lys
            980                 985                 990

Thr Asp Ile Tyr Asp Glu Asn Leu Ile Tyr Val Ile Leu Ile His Gly
        995                 1000                1005

Ala Val Glu Asp Cys Leu Leu Arg Lys Thr Tyr Arg Ala Pro Phe
    1010                1015                1020

Leu Ile Pro Ile Thr Cys His Gln Ile Gln Ser Ile Glu Asp Phe
    1025                1030                1035

Thr Lys Val Val Ile Ile Gln Thr Pro Ile Asn Met Lys Thr Phe
    1040                1045                1050

Arg Arg Asn Ile Arg Lys Phe Val Pro Lys His Lys Ser Tyr Leu
    1055                1060                1065

Thr Pro Gly Leu Ile Gly Ser Val Gly Thr Leu Glu Glu Gly Ile
    1070                1075                1080

Gln Glu Glu Arg Asn Val Lys Glu Asp Gly Ala His Ser Ala Ala
    1085                1090                1095

Thr Ala Arg Ser Pro Gln Pro Cys Ser Leu Leu Gly Thr Lys Phe
    1100                1105                1110

Asn Cys Lys Glu Ser Pro Arg Ile Asn Leu Arg Lys Val Arg Lys
    1115                1120                1125

Glu

<210>  189
<211>  17
<212>  DNA
<213>  künstliche Sequenz

<220>
<223>  Oligonukleotid

<400>  189
tttgaaccgg cacttgg                                                    17

<210>  190
<211>  23
<212>  DNA
<213>  künstliche Sequenz

<220>
<223>  Oligonukleotid

<400> 190
tcaaatgcag tagtaaagaa aac 23

<210> 191
<211> 2898
<212> DNA
<213> Homo sapiens

<400> 191
atgtgggtgc ggtgtgcact cctggttgca cgcgactgtg gctgtgctga gcgcgtgtgc 60

ccgtctgtgg tgcgtgaccg cgtgtgtgtt gtgggggcgg ggaaaattca tacaaaagaa 120

aaaaatatag cacatctctt ggaaatgaaa tacttcaagt ttaatatctc tcttgctaat 180

gcagaattta tcagccaaga cagctggctg gcctgggtgg ggtttgttaa agttgtcaag 240

tataaggcct actgtaagag ataccaagtg acttttagaa gacagtgtga gggtaaaact 300

gattactatg cttggaaaca cttagtggta caggataaaa ataagtctaa cacacacaaa 360

tacagaatga ttatttgtgt gataaataca gataccattt gtgagatggc ttatgcccat 420

atagaatggg acatgatagt ctgtgcagct tatgcacacg aacttccaaa atacggtgta 480

aaggttggcc tgacaaatga tgctgcagca tgttgtactg gcctgctgct ggcatgcagg 540

cttctcagta ggtttggcat ggacaagatc tataaaggcc aagtggaggt aaccagagat 600

gaatacaacg tgggaagcac tgatggtcag ccaggtgcct ttacctgctg tttggatgca 660

ggccttgcca gaaccaccac tgacaataaa gtttttgggg ctctgagagt gctgtggatg 720

gaggtttctc tatccctcac agtgcctaac gattccctga gtaaagggaa gcctggcccc 780

aggaaggagc agctgcctgc aagagggagc ctgagccgtg gagtcctggg agcctttgag 840

gtgggcagcc agggcgtgga ggcagcagca agcccaaacg gtcaatacgg gcccagctgg 900

ggcctggcgg cggagggcac ggagggagct aggccacagg caccaaagcg ggatttgtcc 960

tatagcagga ctgactctca cagagactgt tctcctgtct gtcacaacat gtccctgagg 1020

ggtcaccttg tccccaagaa gccctcaaag gagaagcagg gacagcagaa actggacagc 1080

aagttttatg agagctgggc cacagccttg ctcacagcta tattcccggt gcttggcatc 1140

ttggtgcttg ttgaatcttt gctgatgaat gacccaatgc gtgaatgcat cctcagcacc 1200

tctggcttct cagggcctcg cgccaggctc ctgggggtcc tggccctggg cgggcttcct 1260

ctccatcttg gtgcacctgt tattgtaatg gcgtggattg tccttgcttt gctattcaca 1320

cggagcagga ccagggctga tcctgcagac gtgctgcccc ctggtgcatt tgagaagact 1380

cgcatgcatg cactgccccc gcctcttggt ttgactttag atgacggtga agtgatcacc 1440

acaagattgc tcactgatgc ttctgtgcaa aaagtcgtgg tccggatatc tgaatcctcc 1500

tcctgcctcc acaatgggct gctatccggt aacggctgtg aggtccatta ccgcagggcg 1560

aggctcttcc aggacgctca gatgcctgct cagagcccag cttatcgggg ggatctgcga 1620

gctcctgtca acgccctgag aattcagaac cggagtcagc tcagcccagg tggaaagatc 1680

aagtggcggc agcacaggca gctggaaggt acccacagaa agaaatcgag cactatgttc 1740

agaaagatcc actccatctt taactccagc ccacagagaa agacggcggc cgagagcccc 1800
ttctacgaag gagccagccc cgcagtgaag ctgattcgaa gcagttccat gtatgtggtc 1860
ggggaccacg gggagaaatt cagcgagtcc ttaaagaagt acaaaagcac cagtagcatg 1920
gacaccagcc tgtactacct gcggcaggag gaggaccggg cgtggatgta ttcgcgcacc 1980
caggactgcc tgcagtacct gcaggagctg ctggccttgc gcaaaaaata tctcagcagc 2040
ttcagtgatc tgaagcccca ccgcacccag gggatttcct caacctcctc caaatcctcc 2100
aagggaggga aaaagactcc tgtccggtct actcccaaag aaataaagaa agcaacccca 2160
aagaaatact cgcagttcag tgctgatgtg gccgaggcca ttgccttctt tgactccatc 2220
attgcagagc tggatacaga gagacgaccc cgggctgctg aggccagcct gccaaatgaa 2280
gatgtggact ttgacgtggc caccagctcc agggagcaca gcttgcattc taactggatc 2340
ctgcgggcac cgcgcagaca ctccgaggat atcgctgccc acactgtgca tactgtagac 2400
ggccagtttc gaaggagcac cgagcacagg accgtgggca ctcagaggag actcgagagg 2460
caccccattt atttgcccaa ggctgtggaa ggggccttca acacctggaa atttaagccc 2520
aaagcctgca aaaaagacct ggggagctcc agacagatcc ttttcaactt ctcaggagaa 2580
gatatggagt gggatgcaga gctctttgcg ttggagcccc agttgtctcc tggggaggac 2640
tactatgaga cagagaaccc caaaggacag tggctgcttc gagaaagact ttgggagcgg 2700
acgactgggt ccctgagaag ctgtccgctt tcagcgcagc atgaggtatt tggtagagtt 2760
gaaaatgcca attgtaacac agtcaaccca ctcagcacac tgcctgctgg tgccgtgcca 2820
gtgccaaaca gacctgtggc ttcccagggg acaggtctca ggacactctc agagcttgag 2880
tttctctgcg tgggctga 2898

<210> 192
<211> 965
<212> PRT
<213> Homo sapiens

<400> 192

Met Trp Val Arg Cys Ala Leu Leu Val Ala Arg Asp Cys Gly Cys Ala
1               5                   10                  15

Glu Arg Val Cys Pro Ser Val Val Arg Asp Arg Val Cys Val Val Gly
            20                  25                  30

Ala Gly Lys Ile His Thr Lys Glu Lys Asn Ile Ala His Leu Leu Glu
        35                  40                  45

Met Lys Tyr Phe Lys Phe Asn Ile Ser Leu Ala Asn Ala Glu Phe Ile
    50                  55                  60

Ser Gln Asp Ser Trp Leu Ala Trp Val Gly Phe Val Lys Val Val Lys
65                  70                  75                  80

Tyr Lys Ala Tyr Cys Lys Arg Tyr Gln Val Thr Phe Arg Arg Gln Cys
                85                  90                  95

Glu Gly Lys Thr Asp Tyr Tyr Ala Trp Lys His Leu Val Val Gln Asp
            100                 105                 110

Lys Asn Lys Ser Asn Thr His Lys Tyr Arg Met Ile Ile Cys Val Ile
        115                 120                 125

Asn Thr Asp Thr Ile Cys Glu Met Ala Tyr Ala His Ile Glu Trp Asp
    130                 135                 140

Met Ile Val Cys Ala Ala Tyr Ala His Glu Leu Pro Lys Tyr Gly Val
145                 150                 155                 160

Lys Val Gly Leu Thr Asn Asp Ala Ala Ala Cys Cys Thr Gly Leu Leu
                165                 170                 175

Leu Ala Cys Arg Leu Leu Ser Arg Phe Gly Met Asp Lys Ile Tyr Lys
            180                 185                 190

Gly Gln Val Glu Val Thr Arg Asp Glu Tyr Asn Val Gly Ser Thr Asp
        195                 200                 205

Gly Gln Pro Gly Ala Phe Thr Cys Cys Leu Asp Ala Gly Leu Ala Arg
    210                 215                 220

Thr Thr Thr Asp Asn Lys Val Phe Gly Ala Leu Arg Val Leu Trp Met
225                 230                 235                 240

Glu Val Ser Leu Ser Leu Thr Val Pro Asn Asp Ser Leu Ser Lys Gly
                245                 250                 255

Lys Pro Gly Pro Arg Lys Glu Gln Leu Pro Ala Arg Gly Ser Leu Ser
            260                 265                 270

Arg Gly Val Leu Gly Ala Phe Glu Val Gly Ser Gln Gly Val Glu Ala
        275                 280                 285

Ala Ala Ser Pro Asn Gly Gln Tyr Gly Pro Ser Trp Gly Leu Ala Ala
    290                 295                 300

Glu Gly Thr Glu Gly Ala Arg Pro Gln Ala Pro Lys Arg Asp Leu Ser
305                 310                 315                 320

Tyr Ser Arg Thr Asp Ser His Arg Asp Cys Ser Pro Val Cys His Asn
                325                 330                 335

Met Ser Leu Arg Gly His Leu Val Pro Lys Lys Pro Ser Lys Glu Lys
            340                 345                 350

Gln Gly Gln Gln Lys Leu Asp Ser Lys Phe Tyr Glu Ser Trp Ala Thr
        355                 360                 365

Ala Leu Leu Thr Ala Ile Phe Pro Val Leu Gly Ile Leu Val Leu Val
    370                 375                 380

Glu Ser Leu Leu Met Asn Asp Pro Met Arg Glu Cys Ile Leu Ser Thr
385                 390                 395                 400

Ser Gly Phe Ser Gly Pro Arg Ala Arg Leu Leu Gly Val Leu Ala Leu
                405                 410                 415

Gly Gly Leu Pro Leu His Leu Gly Ala Pro Val Ile Val Met Ala Trp
            420                 425                 430

Ile Val Leu Ala Leu Leu Phe Thr Arg Ser Arg Thr Arg Ala Asp Pro
        435                 440                 445

Ala Asp Val Leu Pro Pro Gly Ala Phe Glu Lys Thr Arg Met His Ala
    450                 455                 460

Leu Pro Pro Pro Leu Gly Leu Thr Leu Asp Asp Gly Glu Val Ile Thr
465                 470                 475                 480

Thr Arg Leu Leu Thr Asp Ala Ser Val Gln Lys Val Val Val Arg Ile
                485                 490                 495

Ser Glu Ser Ser Ser Cys Leu His Asn Gly Leu Leu Ser Gly Asn Gly
            500                 505                 510

Cys Glu Val His Tyr Arg Arg Ala Arg Leu Phe Gln Asp Ala Gln Met
        515                 520                 525

Pro Ala Gln Ser Pro Ala Tyr Arg Gly Asp Leu Arg Ala Pro Val Asn
    530                 535                 540

Ala Leu Arg Ile Gln Asn Arg Ser Gln Leu Ser Pro Gly Gly Lys Ile
545                 550                 555                 560

Lys Trp Arg Gln His Arg Gln Leu Glu Gly Thr His Arg Lys Lys Ser
                565                 570                 575

Ser Thr Met Phe Arg Lys Ile His Ser Ile Phe Asn Ser Ser Pro Gln
            580                 585                 590

Arg Lys Thr Ala Ala Glu Ser Pro Phe Tyr Glu Gly Ala Ser Pro Ala
        595                 600                 605

Val Lys Leu Ile Arg Ser Ser Ser Met Tyr Val Val Gly Asp His Gly
    610                 615                 620

Glu Lys Phe Ser Glu Ser Leu Lys Lys Tyr Lys Ser Thr Ser Ser Met
625                 630                 635                 640

Asp Thr Ser Leu Tyr Tyr Leu Arg Gln Glu Glu Asp Arg Ala Trp Met
                645                 650                 655

Tyr Ser Arg Thr Gln Asp Cys Leu Gln Tyr Leu Gln Glu Leu Leu Ala
            660                 665                 670

Leu Arg Lys Lys Tyr Leu Ser Ser Phe Ser Asp Leu Lys Pro His Arg
        675                 680                 685

Thr Gln Gly Ile Ser Ser Thr Ser Ser Lys Ser Ser Lys Gly Gly Lys
    690                 695                 700

Lys Thr Pro Val Arg Ser Thr Pro Lys Glu Ile Lys Lys Ala Thr Pro
705                 710                 715                 720

Lys Lys Tyr Ser Gln Phe Ser Ala Asp Val Ala Glu Ala Ile Ala Phe
                725                 730                 735

Phe Asp Ser Ile Ile Ala Glu Leu Asp Thr Glu Arg Arg Pro Arg Ala
            740                 745                 750

Ala Glu Ala Ser Leu Pro Asn Glu Asp Val Asp Phe Asp Val Ala Thr
        755                 760                 765

Ser Ser Arg Glu His Ser Leu His Ser Asn Trp Ile Leu Arg Ala Pro
    770                 775                 780

Arg Arg His Ser Glu Asp Ile Ala Ala His Thr Val His Thr Val Asp
785                 790                 795                 800

Gly Gln Phe Arg Arg Ser Thr Glu His Arg Thr Val Gly Thr Gln Arg
                805                 810                 815

Arg Leu Glu Arg His Pro Ile Tyr Leu Pro Lys Ala Val Glu Gly Ala
            820                 825                 830

Phe Asn Thr Trp Lys Phe Lys Pro Lys Ala Cys Lys Lys Asp Leu Gly
        835                 840                 845

Ser Ser Arg Gln Ile Leu Phe Asn Phe Ser Gly Glu Asp Met Glu Trp
    850                 855                 860

Asp Ala Glu Leu Phe Ala Leu Glu Pro Gln Leu Ser Pro Gly Glu Asp
865                 870                 875                 880

Tyr Tyr Glu Thr Glu Asn Pro Lys Gly Gln Trp Leu Leu Arg Glu Arg
                885                 890                 895

Leu Trp Glu Arg Thr Thr Gly Ser Leu Arg Ser Cys Pro Leu Ser Ala
            900                     905                     910

Gln His Glu Val Phe Gly Arg Val Glu Asn Ala Asn Cys Asn Thr Val
        915                     920                     925

Asn Pro Leu Ser Thr Leu Pro Ala Gly Ala Val Pro Val Pro Asn Arg
    930                     935                     940

Pro Val Ala Ser Gln Gly Thr Gly Leu Arg Thr Leu Ser Glu Leu Glu
945                     950                     955                     960

Phe Leu Cys Val Gly
                965

<210>  193
<211>  22
<212>  DNA
<213>  künstliche Sequenz

<220>
<223>  Oligonukleotid

<400>  193
cgagaggcac cccatttatt tg                                                22

<210>  194
<211>  26
<212>  DNA
<213>  künstliche Sequenz

<220>
<223>  Oligonukleotid

<400>  194
ttctctgtct catagtagtc ctcccc                                            26

<210>  195
<211>  1363
<212>  DNA
<213>  Homo sapiens

<400>  195
aacaggcccc atgctgctct ggacggctgt gctgctcttt ggtaagtcaa cgagcatggg      60

catcccctct tggagcacta aggaccttcc ctgtgttggg aaaactgtct ggctgtacct     120

ccaagcctgg ccaaaccctg tgtttgaagg agatgccctg actctgcgat gtcagggatg     180

gaagaataca ccactgtctc aggtgaagtt ctacagagat ggaaaattcc ttcatttctc     240

taaggaaaac cagactctgt ccatgggagc agcaacagtg cagagccgtg gccagtacag     300

ctgctctggg caggtgatgt atattccaca gacattcaca caaacttcag agactgccat     360

ggttcaagtc caagagctgt ttccacctcc tgtgctgagt gccatcccct ctcctgagcc     420

ccgagagggt agcctggtga ccctgagatg tcagacaaag ctgcaccccc tgaggtcagc     480

cttgaggctc cttttctcct tccacaagga cggccacacc ttgcaggaca ggggccctca     540

cccagaactc tgcatcccgg gagccaagga gggagactct gggctttact ggtgtgaggt 600
ggcccctgag ggtggccagg tccagaagca gagcccccag ctggaggtca gagtgcaggc 660
tcctgtatcc cgtcctgtgc tcactctgca ccacgggcct gctgaccctg ctgtggggga 720
catggtgcag ctcctctgtg aggcacagag gggctcccct ccgatcctgt attccttcta 780
ccttgatgag aagattgtgg ggaaccactc agctccctgt ggtggaacca cctccctcct 840
cttcccagtg aagtcagaac aggatgctgg gaactactcc tgcgaggctg agaacagtgt 900
ctccagagag aggagtgagc ccaagaagct gtctctgaag ggttctcaag tcttgttcac 960
tcccgccagc aactggctgg ttccttggct tcctgcgagc ctgcttggcc tgatggttat 1020
tgctgctgca cttctggttt atgtgagatc ctggagaaaa gctgggcccc ttccatccca 1080
gataccaccc acagctccag gtggagagca gtgcccacta tatgccaacg tgcatcacca 1140
gaaagggaaa gatgaaggtg ttgtctactc tgtggtgcat agaacctcaa agaggagtga 1200
agccaggtct gctgagttca ccgtggggag aaagcacaaa gcttcaccca aattccaccc 1260
caccctggat ctccacacca agcggctcag ggttaatggt cgagttcagg aagcttatgt 1320
ggccttggtc aacacctgct ccctcacccc cagcctgaag tga 1363

<210> 196
<211> 450
<212> PRT
<213> Homo sapiens

<400> 196

Met Leu Leu Trp Thr Ala Val Leu Leu Phe Gly Lys Ser Thr Ser Met
1               5                   10                  15

Gly Ile Pro Ser Trp Ser Thr Lys Asp Leu Pro Cys Val Gly Lys Thr
            20                  25                  30

Val Trp Leu Tyr Leu Gln Ala Trp Pro Asn Pro Val Phe Glu Gly Asp
        35                  40                  45

Ala Leu Thr Leu Arg Cys Gln Gly Trp Lys Asn Thr Pro Leu Ser Gln
    50                  55                  60

Val Lys Phe Tyr Arg Asp Gly Lys Phe Leu His Phe Ser Lys Glu Asn
65                  70                  75                  80

Gln Thr Leu Ser Met Gly Ala Ala Thr Val Gln Ser Arg Gly Gln Tyr
                85                  90                  95

Ser Cys Ser Gly Gln Val Met Tyr Ile Pro Gln Thr Phe Thr Gln Thr
            100                 105                 110

Ser Glu Thr Ala Met Val Gln Val Gln Glu Leu Phe Pro Pro Pro Val
        115                 120                 125

Leu Ser Ala Ile Pro Ser Pro Glu Pro Arg Glu Gly Ser Leu Val Thr
    130                 135                 140

Leu Arg Cys Gln Thr Lys Leu His Pro Leu Arg Ser Ala Leu Arg Leu
145                 150                 155                 160

Leu Phe Ser Phe His Lys Asp Gly His Thr Leu Gln Asp Arg Gly Pro
                165                 170                 175

His Pro Glu Leu Cys Ile Pro Gly Ala Lys Glu Gly Asp Ser Gly Leu
            180                 185                 190

Tyr Trp Cys Glu Val Ala Pro Glu Gly Gly Gln Val Gln Lys Gln Ser
        195                 200                 205

Pro Gln Leu Glu Val Arg Val Gln Ala Pro Val Ser Arg Pro Val Leu
    210                 215                 220

Thr Leu His His Gly Pro Ala Asp Pro Ala Val Gly Asp Met Val Gln
225                 230                 235                 240

Leu Leu Cys Glu Ala Gln Arg Gly Ser Pro Pro Ile Leu Tyr Ser Phe
                245                 250                 255

Tyr Leu Asp Glu Lys Ile Val Gly Asn His Ser Ala Pro Cys Gly Gly
            260                 265                 270

Thr Thr Ser Leu Leu Phe Pro Val Lys Ser Glu Gln Asp Ala Gly Asn
        275                 280                 285

Tyr Ser Cys Glu Ala Glu Asn Ser Val Ser Arg Glu Arg Ser Glu Pro
    290                 295                 300

Lys Lys Leu Ser Leu Lys Gly Ser Gln Val Leu Phe Thr Pro Ala Ser
305                 310                 315                 320

Asn Trp Leu Val Pro Trp Leu Pro Ala Ser Leu Leu Gly Leu Met Val
                325                 330                 335

Ile Ala Ala Ala Leu Leu Val Tyr Val Arg Ser Trp Arg Lys Ala Gly
            340                 345                 350

Pro Leu Pro Ser Gln Ile Pro Pro Thr Ala Pro Gly Gly Glu Gln Cys
        355                 360                 365

Pro Leu Tyr Ala Asn Val His His Gln Lys Gly Lys Asp Glu Gly Val
    370                 375                 380

Val Tyr Ser Val Val His Arg Thr Ser Lys Arg Ser Glu Ala Arg Ser
385                 390                 395                 400

Ala Glu Phe Thr Val Gly Arg Lys His Lys Ala Ser Pro Lys Phe His
                405                 410                 415

Pro Thr Leu Asp Leu His Thr Lys Arg Leu Arg Val Asn Gly Arg Val
            420                 425                 430

Gln Glu Ala Tyr Val Ala Leu Val Asn Thr Cys Ser Leu Thr Pro Ser
        435                 440                 445

Leu Lys
    450

<210>  197
<211>  19
<212>  DNA
<213>  künstliche Sequenz

<220>
<223>  Oligonukleotid

<400>  197
gtcagggatg gaagaatac                                                    19

<210>  198
<211>  18
<212>  DNA
<213>  künstliche Sequenz

<220>
<223>  Oligonukleotid

<400>  198
acaggaggtg gaaacagc                                                     18

<210>  199
<211>  534
<212>  DNA
<213>  Homo sapiens

<400>  199
acaattgtgt cttcttccag atgtcatcgc tataaggagt ggggctttca tcacctcctt      60

gacgtaggat gtgtacatgg ctctccaggt cagagttgct ccaagcaagg ttgttttgca     120

gaagtttctt ctatgtgtca ttcttttcta cactgtgtac tatgtgtccc tgagcatggg     180

ctgcgtgatg tttgaggtgc atgagttgaa tgtcctggct ccatttgatt tcaaaacaaa     240

tccctcatgg ctcaacataa actataaagt tcttttagtt tcaacagagg tcacctactt     300

tgtttgtgga ttgttttttg ttccagttgt ggaagaatgg gtttgggatt atgctatttc     360

agtcactatt cttcatgttg ccatcacttc aactgttatg ttggaattcc ccttgacatc     420

acattggtgg gctgctttag gtatatcaaa attgcttgtt tagattctct aatgcacaga     480

aataatgtta aatagaataa ctgtggaaat atattttatt ttctcataga tttt           534

<210>  200
<211>  128
<212>  PRT

<213>  Homo sapiens

<400>  200

Met Ala Leu Gln Val Arg Val Ala Pro Ser Lys Val Val Leu Gln Lys
1               5                   10                  15

Phe Leu Leu Cys Val Ile Leu Phe Tyr Thr Val Tyr Tyr Val Ser Leu
            20                  25                  30

Ser Met Gly Cys Val Met Phe Glu Val His Glu Leu Asn Val Leu Ala
        35                  40                  45

Pro Phe Asp Phe Lys Thr Asn Pro Ser Trp Leu Asn Ile Asn Tyr Lys
    50                  55                  60

Val Leu Leu Val Ser Thr Glu Val Thr Tyr Phe Val Cys Gly Leu Phe
65                  70                  75                  80

Phe Val Pro Val Val Glu Glu Trp Val Trp Asp Tyr Ala Ile Ser Val
                85                  90                  95

Thr Ile Leu His Val Ala Ile Thr Ser Thr Val Met Leu Glu Phe Pro
            100                 105                 110

Leu Thr Ser His Trp Trp Ala Ala Leu Gly Ile Ser Lys Leu Leu Val
        115                 120                 125

<210>  201
<211>  20
<212>  DNA
<213>  künstliche Sequenz

<220>
<223>  Oligonukleotid

<400>  201
tcaaacatca cgcagcccat                                                20

<210>  202
<211>  22
<212>  DNA
<213>  künstliche Sequenz

<220>
<223>  Oligonukleotid

<400>  202
tggggctttc atcacctcct tg                                             22

<210>  203
<211>  615
<212>  DNA
<213>  Homo sapiens

<400>  203
ggggatgtga tgtcaggctt gattgtgggc atattattgg tgccccagtc cattgcttat      60

tccctgctgg ctggccaaga acctgtctat ggtctgtaca catctttttt tgccagcatc 120
atttattttc tcttgggtac ctcccgtcac atctctgtgg gcatttttgg agtactgtgc 180
cttatgattg gtgagacagt tgaccgagaa ctacagaaag ctggctatga caatgcccat 240
agtgctcctt ccttaggaat ggtttcaaat gggagcacat tattaaatca tacatcagac 300
aggatatgtg acaaaagttg ctatgcaatt atggttggca gcactgtaac ctttatagct 360
ggagtttatc agtgattgtt ttgttaatgt ggaagcaaca ttttctatga ttaatctgct 420
gttacctgtt ttgactgagc tactacaaaa agaaaaatca ctgaattgct atgggtttct 480
gaaatatcca aaaaattaac ctgaagcagg gggaaaaatg acatcacacc attagcaggt 540
attgtgtgaa acttctaaaa atgaaactga catttatctg acttattagg aataaatact 600
ctctaatgaa ctctc 615

<210> 204
<211> 121
<212> PRT
<213> Homo sapiens

<400> 204

Met Ser Gly Leu Ile Val Gly Ile Leu Leu Val Pro Gln Ser Ile Ala
1               5                   10                  15

Tyr Ser Leu Leu Ala Gly Gln Glu Pro Val Tyr Gly Leu Tyr Thr Ser
            20                  25                  30

Phe Phe Ala Ser Ile Ile Tyr Phe Leu Leu Gly Thr Ser Arg His Ile
        35                  40                  45

Ser Val Gly Ile Phe Gly Val Leu Cys Leu Met Ile Gly Glu Thr Val
    50                  55                  60

Asp Arg Glu Leu Gln Lys Ala Gly Tyr Asp Asn Ala His Ser Ala Pro
65                  70                  75                  80

Ser Leu Gly Met Val Ser Asn Gly Ser Thr Leu Leu Asn His Thr Ser
                85                  90                  95

Asp Arg Ile Cys Asp Lys Ser Cys Tyr Ala Ile Met Val Gly Ser Thr
            100                 105                 110

Val Thr Phe Ile Ala Gly Val Tyr Gln
        115                 120

<210> 205
<211> 20
<212> DNA
<213> künstliche Sequenz

<220>
<223> Oligonukleotid

<400> 205
taaatcatac atcagacagg                                                    20


<210> 206
<211> 20
<212> DNA
<213> künstliche Sequenz

<220>
<223> Oligonukleotid

<400> 206
aaaacaggta acagcagatt                                                    20


<210> 207
<211> 513
<212> DNA
<213> Homo sapiens

<400> 207
atggcggcgg ccgctctcgc gagaattcgg cccgtcgggc tccaagcccg gcgcctggcg      60

tcggagggaa agactcgagc cgaaagcccc atctctgacc ctagcaactc atacccttct     120

ggcttccctt tagcaaagcg cctggacgtc atcccctctt cagatacccc aggcctcgtc     180

ctggccactg gcttgactat tgcaggagag cctgataaga tgggacacgg ctccaccttg     240

cattcagcaa gtcgttatcc tgcaactacg atgcaccagg aagaggatgt ggtgaggcca     300

gcttttccat atgcagttag gcatcgaagg gaagatctgc tgtacctaag tggggtgggc     360

atttcatttt tagggaccgt ctttgttaaa ataatttggg acctcataaa gcctccagcc     420

attcctgatc aggacatagc ttacaacagc agcctggtgc ccataacctg gacagcctgg     480

agtgaagtca cactcccaga cttgatgttc taa                                  513


<210> 208
<211> 170
<212> PRT
<213> Homo sapiens

<400> 208

Met Ala Ala Ala Ala Leu Ala Arg Ile Arg Pro Val Gly Leu Gln Ala
1               5                   10                  15


Arg Arg Leu Ala Ser Glu Gly Lys Thr Arg Ala Glu Ser Pro Ile Ser
            20                  25                  30


Asp Pro Ser Asn Ser Tyr Pro Ser Gly Phe Pro Leu Ala Lys Arg Leu
        35                  40                  45


Asp Val Ile Pro Ser Ser Asp Thr Pro Gly Leu Val Leu Ala Thr Gly
    50                  55                  60


Leu Thr Ile Ala Gly Glu Pro Asp Lys Met Gly His Gly Ser Thr Leu
65                  70                  75                  80

His Ser Ala Ser Arg Tyr Pro Ala Thr Thr Met His Gln Glu Glu Asp
                85                  90                  95

Val Val Arg Pro Ala Phe Pro Tyr Ala Val Arg His Arg Arg Glu Asp
            100                 105                 110

Leu Leu Tyr Leu Ser Gly Val Gly Ile Ser Phe Leu Gly Thr Val Phe
        115                 120                 125

Val Lys Ile Ile Trp Asp Leu Ile Lys Pro Pro Ala Ile Pro Asp Gln
    130                 135                 140

Asp Ile Ala Tyr Asn Ser Ser Leu Val Pro Ile Thr Trp Thr Ala Trp
145                 150                 155                 160

Ser Glu Val Thr Leu Pro Asp Leu Met Phe
                165                 170

<210>  209
<211>  20
<212>  DNA
<213>  künstliche Sequenz

<220>
<223>  Oligonukleotid

<400>  209
tgagccctag atatacttgg                                                20

<210>  210
<211>  18
<212>  DNA
<213>  künstliche Sequenz

<220>
<223>  Oligonukleotid

<400>  210
cagtcagcct ccatttct                                                  18

<210>  211
<211>  508
<212>  DNA
<213>  Homo sapiens

<400>  211
tgagccctag atatacttgg cttgcattta ggggccatga tgtttagaga tgaataatgc      60

cttacatgct ggagtcaccc tcagtttgtc aaagtgttca cactgtgaga ggctcacaga     120

aatggaggct gactgaagga agagcagatt cacatctttc atcccttctt tatgctcatg     180

cttctaattt ttgttcccat gttttcttgc cctcctcttt cttagcattt attttgtctg     240

tttctctttc ccctcttctg gctccctctc catctctcct gagcacagaa atgcggctac     300

tgtatttaat ccacagtggc cccctctggc cccctctttg tgtctcctga gcacaggccc     360

tggccccctc tccatctctc ctgacctcct gatccgccca cctcggccag ttattgctgt     420

tttataagga aaatgttttc tagtaccaca cttgtctccc tggaagggat agaagaagga    480

gggaaggaag tagggaggca gggaagag                                        508

<210>  212
<211>  97
<212>  PRT
<213>  Homo sapiens

<400>  212

Met Pro Tyr Met Leu Glu Ser Pro Ser Val Cys Gln Ser Val His Thr
1               5                   10                  15

Val Arg Gly Ser Gln Lys Trp Arg Leu Thr Glu Gly Arg Ala Asp Ser
            20                  25                  30

His Leu Ser Ser Leu Leu Tyr Ala His Ala Ser Asn Phe Cys Ser His
        35                  40                  45

Val Phe Leu Pro Leu Leu Phe Leu Ala Phe Ile Leu Ser Val Ser Leu
    50                  55                  60

Ser Pro Leu Leu Ala Pro Ser Pro Ser Leu Leu Ser Thr Glu Met Arg
65                  70                  75                  80

Leu Leu Tyr Leu Ile His Ser Gly Pro Leu Trp Pro Pro Leu Cys Val
                85                  90                  95

Ser

<210>  213
<211>  25
<212>  DNA
<213>  künstliche Sequenz

<220>
<223>  Oligonukleotid

<400>  213
ctgtatttaa tccacagtgg ccccc                                           25

<210>  214
<211>  27
<212>  DNA
<213>  künstliche Sequenz

<220>
<223>  Oligonukleotid

<400>  214
tccctacttc cttccctcct tcttcta                                         27

<210>  215
<211>  1321
<212>  DNA
<213>  Homo sapiens

<400> 215
cagtgcccag gcaagcccag gagttgacat ttctctgccc agccatgggc ctcaccctgc 60

tcttgctgct gctcctggga ctagaaggtc agggcatagt tggcagcctc cctgaggtgc 120

tgcaggcacc cgtgggaagc tccattctgg tgcagtgcca ctacaggctc caggatgtca 180

aagctcagaa ggtgtggtgc cggttcttgc cggaggggtg ccagcccctg gtgtcctcag 240

ctgtggatcg cagagctcca gcgggcaggc gtacgtttct cacagacctg ggtgggggcc 300

tgctgcaggt ggaaatggtt accctgcagg aagaggatgc tggcgagtat ggctgcatgg 360

tggatggggc cagggggccc cagattttgc acagagtctc tctgaacata ctgcccccag 420

aggaagaaga agagacccat aagattggca gtctggctga gaacgcattc tcagaccctg 480

caggcagtgc caaccctttg gaacccagcc aggatgagaa gagcatcccc ttgatctggg 540

gtgctgtgct cctggtaggt ctgctggtgg cagcggtggt gctgtttgct gtgatggcca 600

agaggaaaca agggaacagg cttggtgtct gtggccgatt cctgagcagc agagtttcag 660

gcatgaatcc ctcctcagtg gtccaccacg tcagtgactc tggaccggct gctgaattgc 720

ctttggatgt accacacatt aggcttgact caccaccttc atttgacaat accacctaca 780

ccagcctacc tcttgattcc ccatcaggaa aaccttcact cccagctcca tcctcattgc 840

cccctctacc tcctaaggtc ctggtctgct ccaagcctgt gacatatgcc acagtaatct 900

tcccgggagg gaacaagggt ggagggacct cgtgtgggcc agcccagaat ccacctaaca 960

atcagactcc atccagctaa gctgctcatc acactttaaa ctcatgagga ccatccctag 1020

gggttctgtg catccatcca gccagctcat gccctaggat ccttaggata tctgagcaac 1080

cagggacttt aagatctaat ccaatgtcct aactttacta gggaaagtga cgctcagaca 1140

tgactgagat gtcttgggga agacctccct gcacccaact cccccactgg ttcttctacc 1200

attacacact gggctaaata aaccctaata atgatgtgca aactcttaat ggctgaatgg 1260

gaaaggaaac tgcccaagtt tgactaattg cttggcctgt gaatggaaaa gactctggtc 1320

t 1321

<210> 216
<211> 311
<212> PRT
<213> Homo sapiens

<400> 216

Met Gly Leu Thr Leu Leu Leu Leu Leu Leu Leu Gly Leu Glu Gly Gln
1               5                   10                  15

Gly Ile Val Gly Ser Leu Pro Glu Val Leu Gln Ala Pro Val Gly Ser
            20                  25                  30

Ser Ile Leu Val Gln Cys His Tyr Arg Leu Gln Asp Val Lys Ala Gln
        35                  40                  45

Lys Val Trp Cys Arg Phe Leu Pro Glu Gly Cys Gln Pro Leu Val Ser
    50                  55                  60

Ser Ala Val Asp Arg Arg Ala Pro Ala Gly Arg Arg Thr Phe Leu Thr
65                  70                  75                  80

Asp Leu Gly Gly Gly Leu Leu Gln Val Glu Met Val Thr Leu Gln Glu
                85                  90                  95

Glu Asp Ala Gly Glu Tyr Gly Cys Met Val Asp Gly Ala Arg Gly Pro
            100                 105                 110

Gln Ile Leu His Arg Val Ser Leu Asn Ile Leu Pro Pro Glu Glu Glu
        115                 120                 125

Glu Glu Thr His Lys Ile Gly Ser Leu Ala Glu Asn Ala Phe Ser Asp
    130                 135                 140

Pro Ala Gly Ser Ala Asn Pro Leu Glu Pro Ser Gln Asp Glu Lys Ser
145                 150                 155                 160

Ile Pro Leu Ile Trp Gly Ala Val Leu Leu Val Gly Leu Leu Val Ala
                165                 170                 175

Ala Val Val Leu Phe Ala Val Met Ala Lys Arg Lys Gln Gly Asn Arg
            180                 185                 190

Leu Gly Val Cys Gly Arg Phe Leu Ser Ser Arg Val Ser Gly Met Asn
        195                 200                 205

Pro Ser Ser Val Val His His Val Ser Asp Ser Gly Pro Ala Ala Glu
    210                 215                 220

Leu Pro Leu Asp Val Pro His Ile Arg Leu Asp Ser Pro Pro Ser Phe
225                 230                 235                 240

Asp Asn Thr Thr Tyr Thr Ser Leu Pro Leu Asp Ser Pro Ser Gly Lys
                245                 250                 255

Pro Ser Leu Pro Ala Pro Ser Ser Leu Pro Pro Leu Pro Pro Lys Val
            260                 265                 270

Leu Val Cys Ser Lys Pro Val Thr Tyr Ala Thr Val Ile Phe Pro Gly
        275                 280                 285

Gly Asn Lys Gly Gly Gly Thr Ser Cys Gly Pro Ala Gln Asn Pro Pro
    290                 295                 300

Asn Asn Gln Thr Pro Ser Ser
305                 310

<210> 217
<211> 18
<212> DNA
<213> künstliche Sequenz

<220>
<223> Oligonukleotid

<400> 217
aggaagaaga agagaccc 18

<210> 218
<211> 18
<212> DNA
<213> künstliche Sequenz

<220>
<223> Oligonukleotid

<400> 218
catcacagca aacagcac 18

<210> 219
<211> 3874
<212> DNA
<213> Homo sapiens

<400> 219
gagaactggg gcggcgcggc gcggcgcggt gcatttccag gcgctgctct ccgtcgcaga 60
gaaccctgag ctcggcgcgc cgagagtccc agcagggcaa gggggcgcgg cgtcctggtc 120
ctcgagcttg ggagacagat gcgcatgggc gtgggggcat gcggacctaa gctcgggtga 180
agctctcggg aagggcaaga ctgcggcgac gagatgcgag cagaggagcc ctgcgccccc 240
ggggccccca gcgccctggg agcccagcgc acgccgggcc ccgagctgcg cctgtccagc 300
cagctgctgc ccgagctctg taccttcgtg gtgcgcgtgc tgttctacct ggggcctgtc 360
tacctagctg gctacctggg gctcagcata acctggttgc tgctcggcgc cctgctgtgg 420
atgtggtggc gcaggaaccg ccgcgggaag cttgggcgcc tggccgccgc cttcgaattc 480
cttgacaatg aacgcgagtt catcagccgc gagctgcggg gccagcacct gccagcctgg 540
atccacttcc cggacgtgga gcgggtcgag tgggccaaca agatcatctc tcagacctgg 600
ccctacctaa gcatgatcat ggaaagcaag ttccgggaga aacttgagcc caagatccga 660
gagaagagca tccacctgag gacctttacc tttaccaagc tctactttgg acagaagtgt 720
cccagggtca acggtgtcaa ggcacacact aatacgtgca accgaagacg tgtgactgtg 780
gacctgcaga tctgctacat cggggactgt gagatcagtg tggagctgca gaagattcag 840
gctggtgtga acgggatcca gttgcagggc accctgcggg tcatcctgga gcccctccta 900
gtggacaagc cctttgtggg agccgtgact gtgttcttcc ttcagaagca gcacctacag 960
atcaactgga ctggcctgac caacctgctg gatgcgccgg gaatcaatga tgtgtcagac 1020
agcttactgg aggacctcat tgccacccac ctggtgctgc ccaaccgtgt gactgtgcct 1080
gtgaagaagg ggctggatct gaccaacctg cgcttccctc tgccctgtgg ggtgatcaga 1140

gtgcacttgc tggaggcaga gcagctggcc cagaaggaca actttctggg gctccgaggc 1200
aagtcagatc cctacgccaa ggtgagcatc ggcctacagc atttccggag taggaccatc 1260
tacaggaacc tgaaccccac ctggaacgaa gtgtttgagt tcatggtgta cgaagtccct 1320
ggacaggacc tggaggtaga cctgtatgat gaggataccg acagggatga cttcctgggc 1380
agcctgcaga tctgccttgg agatgtcatg accaacagag tggtggatga gtggtttgtc 1440
ctgaatgaca caaccagcgg gcggctgcac ctgcggctgg agtggctttc attgcttact 1500
gaccaagaag ttctgactga ggaccatggt ggcctttcca ctgccattct cgtggtcttc 1560
ttggagagtg cctgcaactt gccgagaaac ccttttgact acctgaatgg tgaatatcga 1620
gccaaaaaac tctccaggtt tgccagagtg aaacaaggtc agcaaagacc cttcttccta 1680
tgtcaaacta tctgtaggca agaagacaca tacaagtaag acctgtcccc acaacaagga 1740
ccctgtgtgg agccaggtgt tctccttctt tgtgcacaat gtggccactg agcggctcca 1800
tctgaaggtt tgatggaaga agggctcttg aaacagagtt aagaggtttt taagccaggc 1860
gggctgggaa gcttgaagtg caccttgagc aggttctcct ggcagcgttt aaagtcagcc 1920
ccttgtatgt aagagaggac actgaggccc cacaaggcct catctcctta aggctagtgc 1980
ctgaggtcac tgtatagggg gatgtgggag gataaatcct caagtccctt gactttccct 2040
gcaaaagggt ctttatattt gctacacagt acccagagca gcctatctac acaggacatt 2100
aataatggtg tactttaaaa aatatatgtt tcatttaatc ttcacaaaag atctgtagag 2160
taagcaaaga gaggcaaaaa caatgtcttg tccaagatct catgaccaac aagtggtgga 2220
gctgggatct tttagggccc tgagccctgc ctggagagca gcacagctca tcagtcccca 2280
aagccccctg gctctgggca tttgacagac tagctcatac agatcataat tgcctctact 2340
ctgagtcact atcttccctg acagaagaca aggaccaggt ctggcctgat cccattctag 2400
ttttcagaat aggaccagat gcccatagaa gcacagtaca gactgaagta aacccaaact 2460
tggctggggc tcagatacta gtagtggagt ggtggggctt ggttatcctc ttgttttgtg 2520
actggaccac tgcccaggtg cttgatgatg accaggagtg tgctctggga atgctggagg 2580
tccccctgtg ccagatcctc ccctatgctg acctcactct tgagcagcgc tttcagctgg 2640
accactcagg cctggacagc ctcatctcca tgaggctggt gcttgcagtt cctgcaagtg 2700
gaggaacgag agctggggag cccatacaca ggacctgaag ccctaaagaa aggccctctg 2760
ctcatcaaga aagtggctac caaccagggt cccaaagccc aacctcagga agaaggccct 2820
acagatttgc catgtccccc agaccctgct tctgatacta aggacgtatc caggagtacc 2880
acaaccacca ccagtgctac caccgttgcc actgagccca catcccaaga gacaggccca 2940
gagcctaaag gcaaggacag tgccaaaagg ttctgtgagc ccatcgggga gaagaagagt 3000
ccagccacca tcttcctgac tgtcccaggt ccccactctc cagggcccat caagtcaccc 3060
agacccatga aatgccctgc ctccccattc gcatggccgc ccaagaggct ggctcccagc 3120
atgtcctcgc tcaactcctt ggcctcttct tgctttgacc tggcagatat cagcctcaac 3180

attgagtatg cacctctctg cttaatcttt tctaaaatcg cctgtatgaa aaatacctcg 3240

ctggatggaa aagtagatat gaacttacat ttctgtgcaa gttgtttttt cacaaaatat 3300

cttcctaaga ggcagcatgg tgtggtagaa agaacacagg acaagggaga gagagccaaa 3360

caggctgttt atggctctag ctgcgtactg actataaaat agatgctgga ctctggttga 3420

ggtggggacc tcaggcgacg gcagctgggt gagattcagc tcacagtgcg ctatgtgtgt 3480

ctgcggcgct gcctcagcgt gctaatcaat ggctgcagaa acctaacacc atgtaccagc 3540

agtggagctg atccctacgt ccgtgtctac ttgttgccag aaaggaagtg ggcatgtcgt 3600

aagaagactt cagtgaagcg gaagaccttg gaacccctgt ttgatgagac atttgaattt 3660

tttgttccca tggaagaagt aaagaagagg tcactagatg ttgcagtgaa aaatagtagg 3720

ccacttggct cacacagaag aaaggagtta ggaaaagtac tgattgactt atcaaaagaa 3780

gatctgatta agggcttttc acaatggtaa gtgtgccctt tcattttatc actgttatcc 3840

tgctattcaa gacagttttc ccttttcagt actg 3874

<210> 220
<211> 501
<212> PRT
<213> Homo sapiens

<400> 220

Met Arg Ala Glu Glu Pro Cys Ala Pro Gly Ala Pro Ser Ala Leu Gly
1               5                   10                  15

Ala Gln Arg Thr Pro Gly Pro Glu Leu Arg Leu Ser Ser Gln Leu Leu
            20                  25                  30

Pro Glu Leu Cys Thr Phe Val Val Arg Val Leu Phe Tyr Leu Gly Pro
        35                  40                  45

Val Tyr Leu Ala Gly Tyr Leu Gly Leu Ser Ile Thr Trp Leu Leu Leu
    50                  55                  60

Gly Ala Leu Leu Trp Met Trp Trp Arg Arg Asn Arg Arg Gly Lys Leu
65                  70                  75                  80

Gly Arg Leu Ala Ala Ala Phe Glu Phe Leu Asp Asn Glu Arg Glu Phe
                85                  90                  95

Ile Ser Arg Glu Leu Arg Gly Gln His Leu Pro Ala Trp Ile His Phe
            100                 105                 110

Pro Asp Val Glu Arg Val Glu Trp Ala Asn Lys Ile Ile Ser Gln Thr
        115                 120                 125

Trp Pro Tyr Leu Ser Met Ile Met Glu Ser Lys Phe Arg Glu Lys Leu
    130                 135                 140

Glu Pro Lys Ile Arg Glu Lys Ser Ile His Leu Arg Thr Phe Thr Phe
145                 150                 155                 160

Thr Lys Leu Tyr Phe Gly Gln Lys Cys Pro Arg Val Asn Gly Val Lys
                165                 170                 175

Ala His Thr Asn Thr Cys Asn Arg Arg Arg Val Thr Val Asp Leu Gln
            180                 185                 190

Ile Cys Tyr Ile Gly Asp Cys Glu Ile Ser Val Glu Leu Gln Lys Ile
        195                 200                 205

Gln Ala Gly Val Asn Gly Ile Gln Leu Gln Gly Thr Leu Arg Val Ile
    210                 215                 220

Leu Glu Pro Leu Leu Val Asp Lys Pro Phe Val Gly Ala Val Thr Val
225                 230                 235                 240

Phe Phe Leu Gln Lys Gln His Leu Gln Ile Asn Trp Thr Gly Leu Thr
                245                 250                 255

Asn Leu Leu Asp Ala Pro Gly Ile Asn Asp Val Ser Asp Ser Leu Leu
            260                 265                 270

Glu Asp Leu Ile Ala Thr His Leu Val Leu Pro Asn Arg Val Thr Val
        275                 280                 285

Pro Val Lys Lys Gly Leu Asp Leu Thr Asn Leu Arg Phe Pro Leu Pro
    290                 295                 300

Cys Gly Val Ile Arg Val His Leu Leu Glu Ala Glu Gln Leu Ala Gln
305                 310                 315                 320

Lys Asp Asn Phe Leu Gly Leu Arg Gly Lys Ser Asp Pro Tyr Ala Lys
                325                 330                 335

Val Ser Ile Gly Leu Gln His Phe Arg Ser Arg Thr Ile Tyr Arg Asn
            340                 345                 350

Leu Asn Pro Thr Trp Asn Glu Val Phe Glu Phe Met Val Tyr Glu Val
        355                 360                 365

Pro Gly Gln Asp Leu Glu Val Asp Leu Tyr Asp Glu Asp Thr Asp Arg
    370                 375                 380

Asp Asp Phe Leu Gly Ser Leu Gln Ile Cys Leu Gly Asp Val Met Thr
385                 390                 395                 400

Asn Arg Val Val Asp Glu Trp Phe Val Leu Asn Asp Thr Thr Ser Gly
                405                 410                 415

Arg Leu His Leu Arg Leu Glu Trp Leu Ser Leu Leu Thr Asp Gln Glu
            420                     425                     430

Val Leu Thr Glu Asp His Gly Gly Leu Ser Thr Ala Ile Leu Val Val
        435                     440                     445

Phe Leu Glu Ser Ala Cys Asn Leu Pro Arg Asn Pro Phe Asp Tyr Leu
    450                     455                     460

Asn Gly Glu Tyr Arg Ala Lys Lys Leu Ser Arg Phe Ala Arg Val Lys
465                     470                     475                     480

Gln Gly Gln Gln Arg Pro Phe Phe Leu Cys Gln Thr Ile Cys Arg Gln
                485                     490                     495

Glu Asp Thr Tyr Lys
            500

<210>  221
<211>  20
<212>  DNA
<213>  künstliche Sequenz

<220>
<223>  Oligonukleotid

<400>  221
tggggcctgt ctacctagct                                                   20

<210>  222
<211>  19
<212>  DNA
<213>  künstliche Sequenz

<220>
<223>  Oligonukleotid

<400>  222
tcttgttggc ccactcgac                                                    19

<210>  223
<211>  1020
<212>  DNA
<213>  Homo sapiens

<400>  223
agacacagga cctgctgggc cacagaaagg aggctctggg tagacgcact agattactgg      60

ataaatcact tcaatttccc aatgaatttt atattgttta tttttatacc tggagttttt     120

tccttaaaaa gtagcacttt gaagcctact attgaagcat tgcctaatgt gctaccttta     180

aatgaagatg ttaataagca ggaagaaaag aatgaagatc atactcccaa ttatgctcct     240

gctaatgaga aaaatggcaa ttattataaa gatataaaac aatatgtgtt cacaacacaa     300

aatccaaatg gcactgagtc tgaaatatct gtgagagcca caactgacct gaattttgct     360

ctaaaaaacg ataaaactgt caatgcaact acatatgaaa aatccaccat tgaagaagaa     420

acaactacta gcgaaccctc tcataaaaat attcaaagat caaccccaaa cgtgcctgca 480

ttttggacaa tgttagctaa agctataaat ggaacagcag tggtcatgga tgataaagat 540

caattatttc acccaattcc agagtctgat gtgaatgcta cacagggaga aaatcagcca 600

gatctagagg atctgaagat caaaataatg ctgggaatct cgttgatgac cctcctcctc 660

tttgtggtcc tcttggcatt ctgtagtgct acactgtaca aactgaggca tctgagttat 720

aaaagttgtg agagtcagta ctctgtcaac ccagagctgg ccacgatgtc ttactttcat 780

ccatcagaag gtgtttcaga tacatccttt tccaagagtg cagagagcag cacatttttg 840

ggtaccactt cttcagatat gagaagatca ggcacaagaa catcagaatc taagataatg 900

acggatatca tttccatagg ctcagataat gagatgcatg aaaacgatga gtcggttacc 960

cggtgaagaa atcaaggaac ccggtgaaga aatcttattg atgaataaat aactttaatt 1020

<210> 224
<211> 294
<212> PRT
<213> Homo sapiens

<400> 224

Met Asn Phe Ile Leu Phe Ile Phe Ile Pro Gly Val Phe Ser Leu Lys
1               5                   10                  15

Ser Ser Thr Leu Lys Pro Thr Ile Glu Ala Leu Pro Asn Val Leu Pro
            20                  25                  30

Leu Asn Glu Asp Val Asn Lys Gln Glu Glu Lys Asn Glu Asp His Thr
        35                  40                  45

Pro Asn Tyr Ala Pro Ala Asn Glu Lys Asn Gly Asn Tyr Tyr Lys Asp
    50                  55                  60

Ile Lys Gln Tyr Val Phe Thr Thr Gln Asn Pro Asn Gly Thr Glu Ser
65                  70                  75                  80

Glu Ile Ser Val Arg Ala Thr Thr Asp Leu Asn Phe Ala Leu Lys Asn
                85                  90                  95

Asp Lys Thr Val Asn Ala Thr Thr Tyr Glu Lys Ser Thr Ile Glu Glu
            100                 105                 110

Glu Thr Thr Thr Ser Glu Pro Ser His Lys Asn Ile Gln Arg Ser Thr
        115                 120                 125

Pro Asn Val Pro Ala Phe Trp Thr Met Leu Ala Lys Ala Ile Asn Gly
    130                 135                 140

Thr Ala Val Val Met Asp Asp Lys Asp Gln Leu Phe His Pro Ile Pro
145                 150                 155                 160

Glu Ser Asp Val Asn Ala Thr Gln Gly Glu Asn Gln Pro Asp Leu Glu
                165                 170                 175

Asp Leu Lys Ile Lys Ile Met Leu Gly Ile Ser Leu Met Thr Leu Leu
            180                 185                 190

Leu Phe Val Val Leu Leu Ala Phe Cys Ser Ala Thr Leu Tyr Lys Leu
        195                 200                 205

Arg His Leu Ser Tyr Lys Ser Cys Glu Ser Gln Tyr Ser Val Asn Pro
    210                 215                 220

Glu Leu Ala Thr Met Ser Tyr Phe His Pro Ser Glu Gly Val Ser Asp
225                 230                 235                 240

Thr Ser Phe Ser Lys Ser Ala Glu Ser Ser Thr Phe Leu Gly Thr Thr
                245                 250                 255

Ser Ser Asp Met Arg Arg Ser Gly Thr Arg Thr Ser Glu Ser Lys Ile
            260                 265                 270

Met Thr Asp Ile Ile Ser Ile Gly Ser Asp Asn Glu Met His Glu Asn
        275                 280                 285

Asp Glu Ser Val Thr Arg
    290

<210>  225
<211>  24
<212>  DNA
<213>  künstliche Sequenz

<220>
<223>  Oligonukleotid

<400>  225
tgaatgctac acagggagaa aatc                                              24

<210>  226
<211>  21
<212>  DNA
<213>  künstliche Sequenz

<220>
<223>  Oligonukleotid

<400>  226
tgaaagtaag acatcgtggc c                                                 21

<210>  227
<211>  309
<212>  DNA
<213>  Homo sapiens

<400>  227
atgaccacag ccacccctct gggggatacc accttcttct cactgaacat gaccaccagg      60

ggagaagact tcctgtataa gagttctgga gccattgttg ctgccgttgt ggtggttgtc 120

atcatcatct tcaccgtggt tctgatcctg ctgaagatgt acaacaggaa aatgaggacg 180

aggcgggaac tagagcccaa gggccccaag ccaaccgccc cttctgccgt gggcccaaac 240

agcaacggca gccaacaccc agcaactgtg accttcagtc ctgttgacgt ccaggtggag 300

acgcgatga 309

<210> 228
<211> 102
<212> PRT
<213> Homo sapiens

<400> 228

Met Thr Thr Ala Thr Pro Leu Gly Asp Thr Thr Phe Phe Ser Leu Asn
1               5                   10                  15

Met Thr Thr Arg Gly Glu Asp Phe Leu Tyr Lys Ser Ser Gly Ala Ile
            20                  25                  30

Val Ala Ala Val Val Val Val Val Ile Ile Ile Phe Thr Val Val Leu
        35                  40                  45

Ile Leu Leu Lys Met Tyr Asn Arg Lys Met Arg Thr Arg Arg Glu Leu
    50                  55                  60

Glu Pro Lys Gly Pro Lys Pro Thr Ala Pro Ser Ala Val Gly Pro Asn
65                  70                  75                  80

Ser Asn Gly Ser Gln His Pro Ala Thr Val Thr Phe Ser Pro Val Asp
                85                  90                  95

Val Gln Val Glu Thr Arg
            100

<210> 229
<211> 19
<212> DNA
<213> künstliche Sequenz

<220>
<223> Oligonukleotid

<400> 229
ggggatacca ccttcttct 19

<210> 230
<211> 18
<212> DNA
<213> künstliche Sequenz

<220>
<223> Oligonukleotid

<400> 230
agttgctggg tgttggct 18

<210> 231
<211> 2510
<212> DNA
<213> Homo sapiens

<400> 231
gactttttaa taatagtcgt tctgactgat gtgaaatgga gtctctttgt ggttctgatt 60

tgcatctctg atgatgcatg atgttgacca gtttttaata tgtttgttga ctgcttgtat 120

gtcttctttt aagaagtgtc tgttcatatc ctttgccctt tcgcttctat gcaccaataa 180

cacccaggct gagagtcaaa ccaagaacac aatcctgact acagtagcca taaagaaaat 240

gaaatacctg ggaatacacc taatcaaaaa catgaaagca ctctctagag ggagaactac 300

aaaacattgc tgaaagaaat cagagatgat tctctgaaaa agaagtcaga ttagaaatga 360

ttctctgaaa aagaaatcat ctctgatttc tttcagcagt gtgttttttg tttgtttgtt 420

tgttttgaga cagagtcttg ctctgtcgcc aaggctggag ggcaatggca tgatttcagc 480

tcactacaac ctcctgctcc tgggttcgag cgattctcct acctcagcct cccgagtagc 540

tgggattaca ggaggctgag aaaatgttag aaattggggg agacaagttt cccttagaga 600

gcaggaagtt actaagtagt cctggaaaga acatcagttg cagatgtgac ccctctgaga 660

ttaatatatc tgatgaaatg cctaaaacta cagtttggaa agctctcagt atgaattctg 720

gaaatgcaaa ggaaaagagt ctcttcaact aagagtcttt gctgggatgg aagatttggg 780

ccgtgtggtg cctcagggaa gttctggtta cagagaaaat ggcgagtctc tcagagaaga 840

agcaagacca agtctggccc tgtccttggt catctcaaag ccatgccgaa gcattcagtt 900

attcttggtg tgcattggaa ggcatccagc tatccccata ccagcagcca gtcaccagat 960

gtgaatgtgg aagcagaaga ccacctcctg ttggttcttc tcctcttcct tctttttctc 1020

tttagaacgg ccaccattga agacctagct tcccattttc cagacgtttt ctctgaaatt 1080

ctctgctggc ctgccaagcc atatggattc attctgccac tgaggagtcc ttcagtgagg 1140

tccctcttcc taaaggacag agtggggagt aggaggggaa cagagaggac atcctctctg 1200

gctctccagt gctcttagtg tctacaggct cctaggcagc cctgggcctt ggtttgatta 1260

cctcccctgg gggatgctgg tcagacccag aggttgtcag gaggtcagct accaggaaga 1320

tccatgatct gggcattggc agtgcctgcc accacagcca ggaagatgcc tctgacctgg 1380

gtgcatctcc atcactcctt agcagcagcc tgcataactg gcaagaatct tggatgatac 1440

aagagccaag aagggacatt tgagttgtgt cgcttagata ggaaagggat ccagggaaaa 1500

tcaacagtaa gtgaggatga gcagtgtctc ttggttttca ttgaggatag agtaagagat 1560

tgagtttaga ttgcaacaga aggaattagt ttagatacca ggaagaactt cctagcctga 1620

agatttgtca tagtgtctgc tttctagata tctgggaaag atttgataat agttgtttgt 1680

gaatagaaag gaggatatga tgtttttatt ggccattttg cgggactctt cgacttcttg 1740

ctgctgtctc ttgaggatac attccaattc catcctggcg agatccaagt gcttacgtac 1800

tgtctcctta gctgccttag agtaaacgat catcagttca atggaccaaa atcaccttca 1860
gccatgtggt ttcttcatca tcatggattt cttttggttg acaaacattc tggctctcag 1920
atgcaaaaag tcacactggg aaatgaactg taagtggtga aattagtttt ggtatttaat 1980
ttaaaactac atttatagtt tttctcttct cttctatgtt gcaatgaatg taaagtattt 2040
gggatccagt gcttataaac ctttccttcc tttgtgcaca gaatgtaact agcaagccca 2100
ttagcaccca gataattcta tcatgttagt ttcccatcct ggaaaatctt tgtacagtgg 2160
gaagttcccc gatgtgtttt tctttcttag gtgaagggtt ggctatatca ctttattgaa 2220
ttttgcattc cttagacttt taaaatatac taatgtattc tagtcttact ctaaagacct 2280
ttgatgttaa aggaatcctt catttatttc atattcccta tctcataggg ccacaattat 2340
tttaatacag agatgatttt caaaatattt taacaactgg tacaggacag atgccagcca 2400
ctcagaaggg atgcctgctg taaacaagca gtatgtatgg ttgtaccaat gcctattggc 2460
tgaacattat gctactttca gatattaaaa tggtgttcct ttgaatcgtg 2510

<210> 232
<211> 164
<212> PRT
<213> Homo sapiens

<400> 232

<210> 233
<211> 20
<212> DNA
<213> künstliche Sequenz

<220>
<223> Oligonukleotid

<400> 233
gaggctgaga aaatgttaga                                                20

<210> 234
<211> 18
<212> DNA
<213> künstliche Sequenz

<220>
<223> Oligonukleotid

<400> 234
tccatcccag caaagact                                                  18

<210> 235
<211> 1977
<212> DNA
<213> Homo sapiens

<400> 235
cgtgggcttg aggacctgga gagagtagat cctgaagaac tttttcagtc tgctgaagag      60

cttggaagac tggagacaga aggcagagtc tcaggctctg aaggtataag gagtgtgagt     120

tcctgtgaga aacactcatt tgattgtgaa aagacttgaa ttctatgcta agcagggttc     180

caagtagcta aatgaatgat ctcagcaagt ctctcttgct gctgctgcta ctcgtttaca     240

tttattgatt acttacgatg attcaggtac tgttgtaagt gctttacatg ctgttatacg     300

agactcttgg gagaaatcac tttaatgaag cttgagacac atggcattgc catgcaatga     360

tttttccccc ctcttcacgg gatcagaggg aactaataga atgtgacaat gattctttag     420

cagggactgc tgaggcttct ggttcctttt taagatctgc agtgaaagaa gatgagaaac     480

atggatatgc ccttcttttg gtccccctct tcctttattt gatctctact tccttctata     540

aatatattag ggctacattg tccctttgta tttcaaacaa ggcaaaaaga ggttgtaatt     600

acactttact gcaatcctca gtttctccag ggaacaggaa tgcaaaggct ttgaaggcct     660

ctctatttgc tgacatggtc agctgggtgc catgggccaa gtccttctgt tgccctcctc     720

tgtcaccaag taagctaggt cctttctgag gctcaggttt gctgtgatga tgatcacttt     780

taggcagaag gttagaggcc tcatgagtgc tatatggact ttattaggct ttagatttga     840

tggggaataa gggatgtgat ttgtcttttg ggaactcatc tttgattcat cattgtctct     900

tggtatcttg gaatttccat gtcattacag tctacagaat gaaagagtaa cctgtcccag 960

aggagaggca ggtgaaagac tccacagcat gctcattctc attctgtctt ctcagtgaca 1020

ccgaggttta ctgagtgccc actatgtgcc aagcactgtg ctcagggctt tctttgtatg 1080

catgatctca gtgaatctca ccaagcctca tctggaaaac ggggacaaat taacaacagg 1140

atggcaaatt gaaaaacacg taaccatgtt ctacagatgg aaaggggtgc ttggttatta 1200

tgaaggcccc ctcgcaagcg tgtgggacat gggtgtgttc tctgggttgt actgatcaga 1260

tcaaggacct cccccaccct tctcacactc tgcccacttc cgccctttgc ttatcagacc 1320

cttagccagt gactcattcc agaaccagaa ccttggtgaa atctcaaccg acaccagaga 1380

tcggtgtctt cagtcctaga ctgatggaga aaatccagaa tatatactag aagctccaaa 1440

tgctctgggt ttcagctcct ctgtgctgtg gacactgact ttggctcaga actccgattt 1500

agtacaaaag gctcattttt atttcagggg cactcttcct aaagcaaacc taataaatga 1560

aatatggaat tcacagatac acacacacat taaaaaatta acctagtgta tctgtgagga 1620

gtaggcagaa attcactgta taaaagaatg cttcatttca tagagaattt gtgttaagat 1680

tccattagat agtacatttc tcaaagattt ttgaggttgt atttgcttta ccaaaacttg 1740

gtttatgtaa gtggaaaaag catgttgcaa aataacttgg tgtctatgat tcagtttatg 1800

taaaataata aatgtatgta ggaatacgtg tgttgaaaga tgtacatcaa tttgctaaca 1860

atggttatct ctgacgtggt gggatttgag atgtgttttt ctttttggtt gtatttttct 1920

ctattgtttg acttaacaca gaacatgttt ggttacaaca ataaagttat tgaagac 1977

<210> 236
<211> 130
<212> PRT
<213> Homo sapiens

<400> 236

Met Ile Phe Pro Pro Ser Ser Arg Asp Gln Arg Glu Leu Ile Glu Cys
1               5                   10                  15

Asp Asn Asp Ser Leu Ala Gly Thr Ala Glu Ala Ser Gly Ser Phe Leu
            20                  25                  30

Arg Ser Ala Val Lys Glu Asp Glu Lys His Gly Tyr Ala Leu Leu Leu
        35                  40                  45

Val Pro Leu Phe Leu Tyr Leu Ile Ser Thr Ser Phe Tyr Lys Tyr Ile
    50                  55                  60

Arg Ala Thr Leu Ser Leu Cys Ile Ser Asn Lys Ala Lys Arg Gly Cys
65                  70                  75                  80

Asn Tyr Thr Leu Leu Gln Ser Ser Val Ser Pro Gly Asn Arg Asn Ala
                85                  90                  95

Lys Ala Leu Lys Ala Ser Leu Phe Ala Asp Met Val Ser Trp Val Pro
            100                 105                 110

Trp Ala Lys Ser Phe Cys Cys Pro Pro Leu Ser Pro Ser Lys Leu Gly
        115                 120                 125

Pro Phe
    130

<210> 237
<211> 18
<212> DNA
<213> künstliche Sequenz

<220>
<223> Oligonukleotid

<400> 237
atgattcttt agcaggga                                                    18

<210> 238
<211> 18
<212> DNA
<213> künstliche Sequenz

<220>
<223> Oligonukleotid

<400> 238
ctctttttgc cttgtttg                                                    18

<210> 239
<211> 1293
<212> DNA
<213> Homo sapiens

<400> 239
aggccgaggg gttcggcgac gcggagggag ggagagtctg ggccgcgcgg gagccgcagg      60

gcgccctagc cttcgcagaa acgatggcgg aggaagaagg accacctgta gagctgcgcc     120

aaagaaaaaa gccaaagtct tcagaaaata aggaatctgc caaagaagag aaaatcagtg     180

acattccaat tcctgaaaga gctccaaaac atgtattatt tcaacgcttt gcaaagattt     240

tcattggctg tcttgcagcg gttactagtg gtatgatgta tgctctctac ttatcagcat     300

accatgaacg gaaattctgg ttttccaaca ggcaggagct tgaacgggaa atcacgtttc     360

agggtgacag tgccatttat tactcctatt ataaagatat gttaaaggca ccttcatttg     420

aaagaggtgt ttacgaactg acacacaata acaaaactgt atctctgaag actataaatg     480

cagtgcagca aatgtctctg tatccggaac ttattgctag cattttatat caagccactg     540

gtagcaatga gattattgag ccagtgtatt tctatattgg cattgttttt ggattgcaag     600

gaatatatgt tactgcttta tttgttacaa gttggcttat gagtggaaca tggctagcag     660

gaatgcttac tgttgcgtgg ttcgttatta acagttgcac agacccctgg tacagtgtgg     720

gaggtgacaa cacaggatat taataccagg aggcaggaat cattgggacc gtcttggagg     780

ctggctacca cattcaatta actttgctat taatttcatg taatccctat atctgtcttc 840

atatttgaag aggaaaagat actttctcat gtaaacataa tggttttaaa gaataagact 900

ctcttatgct acttaaacaa aagaataaga ctctctttag agatcttagt gagaattgta 960

agaaataaaa taaacagaag tctgactgcc ttatttgatg tcactgatgt atgttgtatt 1020

gctggagtag aagttaaata gaaaaattga cctggtatat tctactcaaa tgtatctttt 1080

gacaattgaa atgttcttaa tagctaagtt ttaaaaaatg cgtttgtttg ctttttgttt 1140

atattttatt ggtatgtatc ttgtactgca aaatacattt taatgccatg aaagaatatg 1200

ctgtctcttt attcatcagc tttatagctt ttatttatat atgacttctt agaaaagtat 1260

aaaaagatat taaagtcatt ccattatatt atg 1293

<210> 240
<211> 219
<212> PRT
<213> Homo sapiens

<400> 240

Met Ala Glu Glu Glu Gly Pro Pro Val Glu Leu Arg Gln Arg Lys Lys
1               5                   10                  15

Pro Lys Ser Ser Glu Asn Lys Glu Ser Ala Lys Glu Glu Lys Ile Ser
            20                  25                  30

Asp Ile Pro Ile Pro Glu Arg Ala Pro Lys His Val Leu Phe Gln Arg
        35                  40                  45

Phe Ala Lys Ile Phe Ile Gly Cys Leu Ala Ala Val Thr Ser Gly Met
    50                  55                  60

Met Tyr Ala Leu Tyr Leu Ser Ala Tyr His Glu Arg Lys Phe Trp Phe
65                  70                  75                  80

Ser Asn Arg Gln Glu Leu Glu Arg Glu Ile Thr Phe Gln Gly Asp Ser
                85                  90                  95

Ala Ile Tyr Tyr Ser Tyr Tyr Lys Asp Met Leu Lys Ala Pro Ser Phe
            100                 105                 110

Glu Arg Gly Val Tyr Glu Leu Thr His Asn Asn Lys Thr Val Ser Leu
        115                 120                 125

Lys Thr Ile Asn Ala Val Gln Gln Met Ser Leu Tyr Pro Glu Leu Ile
    130                 135                 140

Ala Ser Ile Leu Tyr Gln Ala Thr Gly Ser Asn Glu Ile Ile Glu Pro
145                 150                 155                 160

Val Tyr Phe Tyr Ile Gly Ile Val Phe Gly Leu Gln Gly Ile Tyr Val
                165                 170                 175

Thr Ala Leu Phe Val Thr Ser Trp Leu Met Ser Gly Thr Trp Leu Ala
            180                 185                 190

Gly Met Leu Thr Val Ala Trp Phe Val Ile Asn Ser Cys Thr Asp Pro
        195                 200                 205

Trp Tyr Ser Val Gly Gly Asp Asn Thr Gly Tyr
    210                 215

<210>  241
<211>  19
<212>  DNA
<213>  künstliche Sequenz

<220>
<223>  Oligonukleotid

<400>  241
accgctgcaa gacagccaa                                                    19

<210>  242
<211>  20
<212>  DNA
<213>  künstliche Sequenz

<220>
<223>  Oligonukleotid

<400>  242
gcagaaacga tggcggagga                                                   20

<210>  243
<211>  1291
<212>  DNA
<213>  Homo sapiens

<400>  243
atcatgtatt ccattgccac tggaggcttg gttttgatgg cagtgtttta tacacagaaa      60

gacagctgca tggaaaacaa aattctgctg ggagtaaatg gaggcctgtg cctgcttata     120

tcattggtag ccatctcacc ctgggtccaa aatcgacagc cacactcggg gctcttacaa     180

tcaggggtca taagctgcta tgtcacctac ctcaccttct cagctctgtc cagcaaacct     240

gcagaagtag ttctagatga acatgggaaa aatgttacaa tctgtgtgcc tgactttggt     300

caagacctgt acagagatga aaacttggtg actatactgg ggaccagcct cttaatcgga     360

tgtatcttgt attcatgttt gacatcaaca acaagatcga gttctgacgc tctgcagggg     420

cgatacgcag ctcctgaatt ggagatagct cgctgttgtt tttgcttcag tcctggtgga     480

gaggacactg aagagcagca gccggggaag gagggaccac gggtcattta tgacgagaag     540

aaaggcaccg tctacatcta ctcctacttc cacttcgtgt tcttcctagc ttccctgtat     600

gtgatgatga ccgtcaccaa ctggttcaac tacgaaagtg ccaacatcga gagcttcttc     660

agcgggagct ggtccatctt ctgggtcaag atggcctcct gctggatatg cgtgctgttg     720

tacctgtgta cgctggtcgc tcccctctgc tgccccaccc gggagttctc tgtgtgatga 780
tatcggcggt cccctgggct ttgtgggcct acagcctgga aagtgccatc ttttgaacag 840
tgtccccggg gcagggactg gcgccctgtg cctgagtggg tctgaaaaag ctttgagaga 900
gaaaaaaaaa aatctcctga ttagcttttt acttttgaaa ttcaaaaaga aactaccagt 960
ttgtcccaaa ggaattgaaa ttttcaacca aactgatcat ggttgaaata tcttacccct 1020
aggaactgga taccagttat gttgacttcc ttctgcatgt ttttgccaaa acagaatttg 1080
gggcacagca tcttttcaca gggataaaaa tatcttgtgg ggccagtcat tctcatcctc 1140
ggaatagaaa aacatgccaa aatcttgagt ccccagcgcc taacagaatc cagacccctc 1200
tcactcactt ccgcctctta gagccttgtc cccagggggc tttgaggaca ggactcagcc 1260
tgcagggccc ctggtattta tagggtccaa g 1291

<210> 244
<211> 257
<212> PRT
<213> Homo sapiens

<400> 244

Met Tyr Ser Ile Ala Thr Gly Gly Leu Val Leu Met Ala Val Phe Tyr
1               5                   10                  15

Thr Gln Lys Asp Ser Cys Met Glu Asn Lys Ile Leu Leu Gly Val Asn
            20                  25                  30

Gly Gly Leu Cys Leu Leu Ile Ser Leu Val Ala Ile Ser Pro Trp Val
        35                  40                  45

Gln Asn Arg Gln Pro His Ser Gly Leu Leu Gln Ser Gly Val Ile Ser
    50                  55                  60

Cys Tyr Val Thr Tyr Leu Thr Phe Ser Ala Leu Ser Ser Lys Pro Ala
65                  70                  75                  80

Glu Val Val Leu Asp Glu His Gly Lys Asn Val Thr Ile Cys Val Pro
                85                  90                  95

Asp Phe Gly Gln Asp Leu Tyr Arg Asp Glu Asn Leu Val Thr Ile Leu
            100                 105                 110

Gly Thr Ser Leu Leu Ile Gly Cys Ile Leu Tyr Ser Cys Leu Thr Ser
        115                 120                 125

Thr Thr Arg Ser Ser Ser Asp Ala Leu Gln Gly Arg Tyr Ala Ala Pro
    130                 135                 140

Glu Leu Glu Ile Ala Arg Cys Cys Phe Cys Phe Ser Pro Gly Gly Glu
145                 150                 155                 160

Asp Thr Glu Glu Gln Gln Pro Gly Lys Glu Gly Pro Arg Val Ile Tyr
                165                 170                 175

Asp Glu Lys Lys Gly Thr Val Tyr Ile Tyr Ser Tyr Phe His Phe Val
            180                 185                 190

Phe Phe Leu Ala Ser Leu Tyr Val Met Met Thr Val Thr Asn Trp Phe
        195                 200                 205

Asn Tyr Glu Ser Ala Asn Ile Glu Ser Phe Phe Ser Gly Ser Trp Ser
    210                 215                 220

Ile Phe Trp Val Lys Met Ala Ser Cys Trp Ile Cys Val Leu Leu Tyr
225                 230                 235                 240

Leu Cys Thr Leu Val Ala Pro Leu Cys Cys Pro Thr Arg Glu Phe Ser
                245                 250                 255

Val

<210>   245
<211>   18
<212>   DNA
<213>   künstliche Sequenz

<220>
<223>   Oligonukleotid

<400>   245
agtcaggcac acagattg                                                    18

<210>   246
<211>   18
<212>   DNA
<213>   künstliche Sequenz

<220>
<223>   Oligonukleotid

<400>   246
ttctgctggg agtaaatg                                                    18

<210>   247
<211>   2412
<212>   DNA
<213>   Homo sapiens

<400>   247
gaacccaggc atcctgggct ccagctgaaa ccattgcatg tggctttccc catccctggc      60

cccgtgactc agtccctctg aagggagcag ccctcttttt tggcaatcac cagggaggtg     120

gggggaggag gaggggagct aggtggtgac atcacagtcg aaggttataa aagcttccag     180

ccaaacggca ttgaagttga agatacaacc tgacagcaca gcctgagatc ttggggatcc     240

ctcagcctaa cacccacaga cgtcagctgg tggattcccg ctgcatcaag gcctacccac     300

tgtctccatg ctgggctctc cctgccttct gtggctcctg gccgtgacct tcttggttcc 360
cagagctcag cccttggccc ctcaagactt tgaagaagag gaggcagatg agactgagac 420
ggcgtggccg cctttgccgg ctgtcccctg cgactacgac cactgccgac acctgcaggt 480
gccctgcaag gagctacaga gggtcgggcc ggcggcctgc ctgtgcccag gactctccag 540
ccccgcccag ccgcccgacc cgccgcgcat gggagaagtg cgcattgcgg ccgaagaggg 600
ccgcgcagtg gtccactggt gtgccccctt ctccccggtc ctccactact ggctgctgct 660
ttgggacggc agcgaggctg cgcagaaggg gcccccgctg aacgctacgg tccgcagagc 720
cgaactgaag gggctgaagc cagggggcat ttatgtcgtt tgcgtagtgg ccgctaacga 780
ggccggggca agccgcgtgc cccaggctgg aggagagggc ctcgaggggg ccgacatccc 840
tgccttcggg ccttgcagcc gccttgcggt gccgcccaac ccccgcactc tggtccacgc 900
ggccgtcggg gtgggcacgg ccctggccct gctaagctgt gccgccctgg tgtggcactt 960
ctgcctgcgc gatcgctggg gctgcccgcg ccgagccgcc gcccgagccg caggggcgct 1020
ctgaaagggg cctgggggca tctcgggcac agacagcccc acctggggcg ctcagcctgg 1080
cccccgggaa agaggaaaac ccgctgcctc cagggagggc tggacggcga gctgggagcc 1140
agccccaggc tccagggcca cggcggagtc atggttctca ggactgagcg cttgtttagg 1200
tccggtactt ggcgctttgt ttcctggctg aggtctggga aggaatagaa aggggccccc 1260
aatttttttt taagcggcca gataataaat aatgtaacct ttgcggttta agaggataaa 1320
atggaggata ttattatgtg ggtatttata tgacctttgt aaccatttaa aaatgtaaaa 1380
acgacctgac ttagtaatgc gaacctatag tagcagctac tccagaggct gaaatgggag 1440
gatctcttga gcccaggagt tggagtccag tccagccagg gcaacacagc cagacgccct 1500
tgttttttat tttgttttgt tttggttttt tgttttttga ggagtttccc tctgtcacac 1560
aagctggagg gcaatggcgc catctcagct cactgcaacg tccacctcct gggttcaagc 1620
gattctcctg cctcagcatc ctaattagtt gggattacag gcgcccacca ccatgcccgg 1680
ctaatttttg tgttttttta gtagagacgg ggtttcacca tgttgtcagg ctggtctcaa 1740
actcctgacc tcaggtactc cacccgcctt ggtctctcaa agtgctggga ttacaggcat 1800
aagccactgt gcccaggcag acccccttct ttaaagatgt aaaacccggc cgggcgcggt 1860
ggctcacgcc tgtaatccca gcactttggg aggctgaggc gggcagatca cgaagtcagg 1920
agatcgagac catcctggct aacacggtga aaccccgtct ctactaaaaa tacaaaaatt 1980
agccgggcat ggtggtgggt acctgtagtc ccagctactc cggaggctga ggcaggagaa 2040
tggcgtgaac ccgggaggcg gatcttgcag tgagcggaga ttgcaccact gcactccagc 2100
ctgggtgaca gagcaagact ccctctcaaa agaaaaagaa aaaagatgta aaaaccattc 2160
ttagtttgtg ggccttacaa atcaggccac tggcccattg cttgtagtta gttgatccat 2220
gtcatgcacc ctaaaaatgg ctctgtcact gtgagtggct tcagtaggat tttgagaata 2280
agtttatatt cttgctaggt aaaacaaaac aaaaacgaca gtaataccaa ggaatctccc 2340

ccccccttta ccctccattt gtgtttattg catatccact ataacaacat taaaggacct 2400

ttaaaaggaa gt 2412

<210> 248
<211> 238
<212> PRT
<213> Homo sapiens

<400> 248

Met Leu Gly Ser Pro Cys Leu Leu Trp Leu Leu Ala Val Thr Phe Leu
1               5                   10                  15

Val Pro Arg Ala Gln Pro Leu Ala Pro Gln Asp Phe Glu Glu Glu Glu
            20                  25                  30

Ala Asp Glu Thr Glu Thr Ala Trp Pro Pro Leu Pro Ala Val Pro Cys
        35                  40                  45

Asp Tyr Asp His Cys Arg His Leu Gln Val Pro Cys Lys Glu Leu Gln
    50                  55                  60

Arg Val Gly Pro Ala Ala Cys Leu Cys Pro Gly Leu Ser Ser Pro Ala
65                  70                  75                  80

Gln Pro Pro Asp Pro Pro Arg Met Gly Glu Val Arg Ile Ala Ala Glu
                85                  90                  95

Glu Gly Arg Ala Val Val His Trp Cys Ala Pro Phe Ser Pro Val Leu
            100                 105                 110

His Tyr Trp Leu Leu Leu Trp Asp Gly Ser Glu Ala Ala Gln Lys Gly
        115                 120                 125

Pro Pro Leu Asn Ala Thr Val Arg Arg Ala Glu Leu Lys Gly Leu Lys
    130                 135                 140

Pro Gly Gly Ile Tyr Val Val Cys Val Val Ala Ala Asn Glu Ala Gly
145                 150                 155                 160

Ala Ser Arg Val Pro Gln Ala Gly Gly Glu Gly Leu Glu Gly Ala Asp
                165                 170                 175

Ile Pro Ala Phe Gly Pro Cys Ser Arg Leu Ala Val Pro Pro Asn Pro
            180                 185                 190

Arg Thr Leu Val His Ala Ala Val Gly Val Gly Thr Ala Leu Ala Leu
        195                 200                 205

Leu Ser Cys Ala Ala Leu Val Trp His Phe Cys Leu Arg Asp Arg Trp
    210                 215                 220

Gly Cys Pro Arg Arg Ala Ala Ala Arg Ala Ala Gly Ala Leu
225                 230                 235

<210> 249
<211> 18
<212> DNA
<213> künstliche Sequenz

<220>
<223> Oligonukleotid

<400> 249
atccctcagc ctaacacc                                                    18

<210> 250
<211> 18
<212> DNA
<213> künstliche Sequenz

<220>
<223> Oligonukleotid

<400> 250
gccgtctcag tctcatct                                                    18

<210> 251
<211> 1024
<212> DNA
<213> Homo sapiens

<400> 251
gagcgccagg ggttccagct gcacgtccca ggctctccag cgcgcggcag gccggggcgg      60

gacgaggaga gctgcgggga caacgcctgt ggctgggtcc ggaggtgcgg gtgcggcgcg     120

ggacaagcgg gcagcatgct cagggcggtc gggagcctac tgcgccttgg ccgcgggcta     180

acagtccgct gcggccccgg ggcgcctctc gaggccacgc gacggcccgc accggctctt     240

ccgccccggg gtctcccctg ctactccagc ggcggggccc ccagcaattc tgggccccaa     300

ggtcacgggg agattcaccg agtccccacg cagcgcaggc cttcgcagtt cgacaagaaa     360

atcctgctgt ggacagggcg tttcaaatcg atggaggaga tcccgcctcg gatcccgcca     420

gaaatgatag acaccgcaag aaacaaagct cgagtgaaag cttgttacat aatgattgga     480

ctcacaatta tcgcctgctt tgctgtgata gtgtcagcca aaagggctgt agaacgacat     540

gaatccttaa caagttggaa cttggcaaag aaagctaagt ggcgtgaaga agctgcattg     600

gctgcacagg ctaaagctaa atgatattct aagtgacaaa gtgttcacct gaataccatc     660

cctgtcatca gcaacagtag aagatgggaa aaatagaata tttaccaaaa tatctgccat     720

ggttttattt tggtaacaag aagcacaatg tcttttttat ttttattttt tagtaaactt     780

ttactgaagt ataccatgca ttcaaaaagt ggacaaaact gtatacagtc tgatagatat     840

ttatgtcgtg aacacctgtg taaccactgc caaagtgaag atgtagaata ttggcaacac     900

ttcacagcct cattcctgcc ttttctcagc cattacctcc caaacatagc agtttttctg     960

agtttcatca cctttgattc attttgcctg tttttgaact ttatataaat ggatttatac    1020

atta                                                                    1024

<210>  252
<211>  162
<212>  PRT
<213>  Homo sapiens

<400>  252

Met Leu Arg Ala Val Gly Ser Leu Leu Arg Leu Gly Arg Gly Leu Thr
1               5                   10                  15

Val Arg Cys Gly Pro Gly Ala Pro Leu Glu Ala Thr Arg Arg Pro Ala
            20                  25                  30

Pro Ala Leu Pro Pro Arg Gly Leu Pro Cys Tyr Ser Ser Gly Gly Ala
        35                  40                  45

Pro Ser Asn Ser Gly Pro Gln Gly His Gly Glu Ile His Arg Val Pro
    50                  55                  60

Thr Gln Arg Arg Pro Ser Gln Phe Asp Lys Lys Ile Leu Leu Trp Thr
65                  70                  75                  80

Gly Arg Phe Lys Ser Met Glu Glu Ile Pro Pro Arg Ile Pro Pro Glu
                85                  90                  95

Met Ile Asp Thr Ala Arg Asn Lys Ala Arg Val Lys Ala Cys Tyr Ile
            100                 105                 110

Met Ile Gly Leu Thr Ile Ile Ala Cys Phe Ala Val Ile Val Ser Ala
        115                 120                 125

Lys Arg Ala Val Glu Arg His Glu Ser Leu Thr Ser Trp Asn Leu Ala
    130                 135                 140

Lys Lys Ala Lys Trp Arg Glu Glu Ala Ala Leu Ala Ala Gln Ala Lys
145                 150                 155                 160

Ala Lys


<210>  253
<211>  19
<212>  DNA
<213>  künstliche Sequenz

<220>
<223>  Oligonukleotid

<400>  253
attatcgcct gctttgctg                                                    19


<210>  254
<211>  22

<212>  DNA
<213>  künstliche Sequenz

<220>
<223>  Oligonukleotid

<400>  254
ttcccatctt ctactgttgc tg                                              22


<210>  255
<211>  852
<212>  DNA
<213>  Homo sapiens

<400>  255
ttagggcgag tttaaggcac tgtggcagct gtgagataaa gtctggttcc tccccagctg      60

gctcaggaaa tgttcgcgga tacaacggcg gccccctctg ggcatacctg cctgtggagc     120

ggagagtgga cggtgtgagg gggaccggga gaggcaccaa atctggcctg ggggcccgag     180

aagcttcctc tcagtgacca caatatgaat gggaacagca agatggcaaa agcttgctga     240

gtggtacagc gccagcctgg gtagtggcct ccccagcaag ttgcatgtca ctagcttcct     300

gtggctgtca ctcctgggcc caggcacctc cgaagatcag cacctcctca tgggctcaag     360

cgaggacagg agcccgtcac ccatgagctc tcaagggcag agccactgtc ctgtctcgat     420

ggctccaccg tgactccagt ggactttgga cagtggggag caggcccaac agggccactc     480

ggatgtggtc actctggatt tgggtggatc agcaccaagc tagactcatc cccagccccc     540

aggtgctgtt gctgctcctg cgtgaggccc catccacagc tgcagctgtg gcagggtggc     600

tagtggtggc cagcatggcc ctgctgcagc tccacgctgt ggggggcgtg gccctgacca     660

gcagccaccc ctccatgtgg gccacagggg aggagcttag gaagccgcct tggcaaggtt     720

ccgcaggctc tgcgtctggt gtggaagagc tcacggggaa gcactcctgc ccaggacccg     780

aggagccggc caccgttcag aaggccccag cttgaaggcc tggagagccg cccagcagca     840

caacacaggg aa                                                         852


<210>  256
<211>  110
<212>  PRT
<213>  Homo sapiens

<400>  256

<210>  257
<211>  18
<212>  DNA
<213>  künstliche Sequenz

<220>
<223>  Oligonukleotid

<400>  257
ttgctgttcc cattcata                                                    18

<210>  258
<211>  19
<212>  DNA
<213>  künstliche Sequenz

<220>
<223>  Oligonukleotid

<400>  258
gataaagtct ggttcctcc                                                   19

<210>  259
<211>  4231
<212>  DNA
<213>  Homo sapiens

<400>  259
gcggccgcct ttgcaaggtt gctggacaga tggaactgga agggcagccg tctgccgccc      60

acgaacacct tctcaagcac tttgagtgac cacggcttgc aagctggtgg ctggcccccc     120

gagtcccggg ctctgaggca cggccgtcga cttaagcgtt gcatcctgtt acctggagac     180

cctctgagct ctcacctgct acttctgccg ctgcttctgc acagagcccg ggcgaggacc     240

cctccaggat gcaggtcccg aacagcaccg gcccggacaa cgcgacgctg cagatgctgc     300

ggaacccggc gatcgcggtg gccctgcccg tggtgtactc gctggtggcg gcggtcagca     360

tcccgggcaa cctcttctct ctgtgggtgc tgtgccggcg catggggccc agatccccgt     420

cggtcatctt catgatcaac ctgagcgtca cggacctgat gctggccagc gtgttgcctt     480

tccaaatcta ctaccattgc aaccgccacc actgggtatt cggggtgctg ctttgcaacg     540

tggtgaccgt ggccttttac gcaaacatgt attccagcat cctcaccatg acctgtatca     600

gcgtggagcg cttcctgggg gtcctgtacc cgctcagctc caagcgctgg cgccgccgtc     660

gttacgcggt ggccgcgtgt gcagggacct ggctgctgct cctgaccgcc ctgtccccgc     720

tggcgcgcac cgatctcacc tacccggtgc acgccctggg catcatcacc tgcttcgacg 780
tcctcaagtg gacgatgctc cccagcgtgg ccatgtgggc cgtgttcctc ttcaccatct 840
tcatcctgct gttcctcatc ccgttcgtga tcaccgtggc ttgttacacg gccaccatcc 900
tcaagctgtt gcgcacggag gaggcgcacg gccgggagca gcggaggcgc gcggtgggcc 960
tggccgcggt ggtcttgctg gcctttgtca cctgcttcgc ccccaacaac ttcgtgctcc 1020
tggcgcacat cgtgagccgc ctgttctacg gcaagagcta ctaccacgtg tacaagctca 1080
cgctgtgtct cagctgcctc aacaactgtc tggacccgtt tgtttattac tttgcgtccc 1140
gggaattcca gctgcgcctg cgggaatatt tgggctgccg ccgggtgccc agagacaccc 1200
tggacacgcg ccgcgagagc ctcttctccg ccaggaccac gtccgtgcgc tccgaggccg 1260
gtgcgcaccc tgaagggatg gagggagcca ccaggcccgg cctccagagg caggagagtg 1320
tgttctgagt cccgggggcg cagcttggag agccgggggc gcagcttgga gatccagggg 1380
cgcatggaga ggccacggtg ccagaggttc agggagaaca gctgcgttgc tcccaggcac 1440
tgcagaggcc cggtggggaa gggtctccag gctttattcc tcccaggcac tgcagaggca 1500
ccggtgagga agggtctcca ggcttcactc agggtagaga aacaagcaaa gcccagcagc 1560
gcacagggtg cttgttatcc tgcagagggt gcctctgcct ctctgtgtca ggggacagct 1620
tgtgtcacca cgcccggcta atttttgtat tttttttagt agagctgggc tgtcaccccc 1680
gagctcctta gacactcctc acacctgtcc atacccgagg gtggatattc aaccagcccc 1740
accgcctacc cgactcggtt tctggatatc ctccgtgggc gaactgcgag ccccattccc 1800
agctcttctc cctgctgaca tcgtccctta gttgtggttc tggccttctc cattctcctc 1860
caggggttct ggtctccgta gcccggtgca cgccgaaatt tctgtttatt tcactcaggg 1920
gcactgtggt tgctgtggtt ggaattcttc tttcagagga gcgcctgggg ctcctgcaag 1980
tcagctactc tccgtgccca cttcccccca cacacacacc ccaccctgtt gctgaccaag 2040
gtgatttttg gcacatttgt tctggcctgg cttggtggga ccccacccct attctgcttc 2100
tgtgagtccc tgatagagaa ggaggtccca tcaggcccct ggaacacact caggcttccc 2160
tgactcagga caaggaccac gggaggccca ggtgcggaaa ggaggctccg tgagatgggg 2220
tccagcccat cccaacacaa gggtgcagct tgattcggga gttccccacc tcctgcccat 2280
tctccgcgtc cttttacccc atggagagcc tcagccatgg caagtccatc tggagtccag 2340
gaagcaggca actggcctga cccatgagac cgtttggaga ccaagcagca gatgcaggtg 2400
tggaccccag gaacctacag gggtgtcagc cgctgagccc cctccctgct gtgtgggtgg 2460
tgagcaggct gggtctttgt ctgtcttctt ctacacggca tgtgcctgca ccagccccaa 2520
cacctgagct ggtttagcgc aaagaagagc tctgactctc caggggtgct gggacatcac 2580
gtggaattgg atcccaggct ctcttgggcg agaaagacca ttctggaggt gggagtggga 2640
gagctgcctg tctgcccacg ggctctgcgt ctccgcagtg ggtggccttg gatgcccggc 2700
ccctcccttt ctgtgcactg gggacgctga tggaggctga agctgctgtt cggaggccct 2760

ctattggtgc ctctctcctg ccgtcatcac tatggcagga aaacagagat ggtttagtaa 2820
tgaattatca ttcccaaacc cgtgtccacc tggaacatca ggatgggacc atgtttgaaa 2880
atcgggtctt tccaaatgta attaagtaag gcgaggccat actgcattta caatgggccc 2940
aatccagtgt ccctatgaga gacggaagag gagacacaga cacaaagcag gaggccacat 3000
aaagacagag gcagagactg aagtgatgct gccccaagcc cagggatgcc tggagtcccc 3060
aggagctggg agaggcagga aggaccctcc cctagagtct ctggagggaa ctggatacaa 3120
ttgcagagtg cactaaacag ttgccccaga aagacatgtc ttgttttaaa gcccagaacc 3180
tgaaattatt atagatttta ttcggtaata aggaactttg catgtgtaat tacttaagga 3240
tatgaagatg agattgtgct ggattattaa gcaccctaaa tgccatgaca ggtgtccttc 3300
caagagacag aagaggagac acagacacag agcaggagga cacgtggaga cagaggcaga 3360
ctggagtgat gcggccacaa gcccagggac acctggagcc cccaggagct gggagaggca 3420
ggaaggatcc tcccctagag cctccagggg gaactggagg atgcgtaaga gacccagaac 3480
ttccacagaa ggaggaaaat taacctcctg cttctctaga ctgttccaaa gctgaaccct 3540
agaaagcaaa gctgatacag aagcatccag gctgcaggag tacaggtcgc aagtgctgag 3600
cgtgggcctt gggtgtgtct catgggggaa aaaaaactgt gaaaaacctc agagtagcat 3660
cttcacagta acgcacggac gatccctaaa ctgccttgta aacaaaaatg agagcttgag 3720
tcagaggaag ccgagacaat atccttcctc gacaacgtgc gagaaccctg acgtccccca 3780
gcaaaggaag acgttgcaag caggcaaaat gcgtcgattt tttttttttg tcagtatgat 3840
gatttttgca gccacttggc tatggagagc agccgacacc ccctcttaca gccgtggatg 3900
tttcctggaa gctgactcag tctgttcact ggttgagctt tgagtgaaaa gataacacag 3960
gtctattgac tcacacacat gttttaagat ggaaaacttt acttctgttc ttggcaggac 4020
atggagagag ggagggattc caaaaagtct cagcctccat caaggcgtgg cagctcatgc 4080
cggtaatctc agcactttgg gaggctcagg cgggaggact gattgagtcc gggtgttcaa 4140
gggccaacct aggcaacaca gtgagaactc atctctgtaa aaaataaaaa taaaacatta 4200
aaaaaaaaca tgagctttga agtgcacagg g 4231

<210> 260
<211> 359
<212> PRT
<213> Homo sapiens

<400> 260

Met Gln Val Pro Asn Ser Thr Gly Pro Asp Asn Ala Thr Leu Gln Met
1               5                   10                  15

Leu Arg Asn Pro Ala Ile Ala Val Ala Leu Pro Val Val Tyr Ser Leu
            20                  25                  30

Val Ala Ala Val Ser Ile Pro Gly Asn Leu Phe Ser Leu Trp Val Leu
        35                  40                  45

Cys Arg Arg Met Gly Pro Arg Ser Pro Ser Val Ile Phe Met Ile Asn
    50                  55                  60

Leu Ser Val Thr Asp Leu Met Leu Ala Ser Val Leu Pro Phe Gln Ile
65                  70                  75                  80

Tyr Tyr His Cys Asn Arg His His Trp Val Phe Gly Val Leu Leu Cys
                85                  90                  95

Asn Val Val Thr Val Ala Phe Tyr Ala Asn Met Tyr Ser Ser Ile Leu
            100                 105                 110

Thr Met Thr Cys Ile Ser Val Glu Arg Phe Leu Gly Val Leu Tyr Pro
        115                 120                 125

Leu Ser Ser Lys Arg Trp Arg Arg Arg Arg Tyr Ala Val Ala Ala Cys
    130                 135                 140

Ala Gly Thr Trp Leu Leu Leu Leu Thr Ala Leu Ser Pro Leu Ala Arg
145                 150                 155                 160

Thr Asp Leu Thr Tyr Pro Val His Ala Leu Gly Ile Ile Thr Cys Phe
                165                 170                 175

Asp Val Leu Lys Trp Thr Met Leu Pro Ser Val Ala Met Trp Ala Val
            180                 185                 190

Phe Leu Phe Thr Ile Phe Ile Leu Leu Phe Leu Ile Pro Phe Val Ile
        195                 200                 205

Thr Val Ala Cys Tyr Thr Ala Thr Ile Leu Lys Leu Leu Arg Thr Glu
    210                 215                 220

Glu Ala His Gly Arg Glu Gln Arg Arg Arg Ala Val Gly Leu Ala Ala
225                 230                 235                 240

Val Val Leu Leu Ala Phe Val Thr Cys Phe Ala Pro Asn Asn Phe Val
                245                 250                 255

Leu Leu Ala His Ile Val Ser Arg Leu Phe Tyr Gly Lys Ser Tyr Tyr
            260                 265                 270

His Val Tyr Lys Leu Thr Leu Cys Leu Ser Cys Leu Asn Asn Cys Leu
        275                 280                 285

Asp Pro Phe Val Tyr Tyr Phe Ala Ser Arg Glu Phe Gln Leu Arg Leu
    290                 295                 300

Arg Glu Tyr Leu Gly Cys Arg Arg Val Pro Arg Asp Thr Leu Asp Thr
305                 310                 315                 320

Arg Arg Glu Ser Leu Phe Ser Ala Arg Thr Thr Ser Val Arg Ser Glu
                325                 330                 335

Ala Gly Ala His Pro Glu Gly Met Glu Gly Ala Thr Arg Pro Gly Leu
            340                 345                 350

Gln Arg Gln Glu Ser Val Phe
        355

<210>  261
<211>  19
<212>  DNA
<213>  künstliche Sequenz

<220>
<223>  Oligonukleotid

<400>  261
cctgttacct ggagaccct                                                    19

<210>  262
<211>  18
<212>  DNA
<213>  künstliche Sequenz

<220>
<223>  Oligonukleotid

<400>  262
accagcgagt acaccacg                                                     18

<210>  263
<211>  717
<212>  DNA
<213>  Homo sapiens

<400>  263
   ggccgggctg gggcttcagc gggaggcagc agaggggaag tggtcagcgt ggcgaatgac      60
   ggaagaaact cgcattgtct actggatcaa ggacagacag ctcaccaacc gtgacagcac     120
   catactggaa cttcaaaaag ttctgaaaac atgttgtgct cagagcatga aaattttctg     180
   ctgtctttgg aactttgtct acaaacagtt agaagatgca gcccaagggc tcaccatggg     240
   tggcgatgtt gaagaacatg aagaccttac tgctgatagc accatcttca aatttgtgga     300
   agcttataca gagtgggagg tgaagaggtg gtcagacaac aatctgataa tgaaacaaac     360
   aaatgtgaag agaagacgct tagatgatgt tggccctgaa ttggaaaagg ctgtctggga     420
   gctcggctgc ccacccagca ttcagtgtct gctacctcct gtctgttatg cttgtgtctg     480
   gttttttcaa gttttaattt tttttttaat tcttagtttt tgtgggtaca tagtaggtgt     540
   atatatttat gggttacatg agatgttttg atacaggcat gcaatatgta ataatcacct     600
   catggagaat ggggtaccca tcacatcaag catttatcct ttgtgttaca aacggtccag     660
   ttagactctt ttagttatta ttaaaatgta caattaaatt atttttgact atagtca        717

<210> 264
<211> 171
<212> PRT
<213> Homo sapiens

<400> 264

Met Thr Glu Glu Thr Arg Ile Val Tyr Trp Ile Lys Asp Arg Gln Leu
1               5                   10                  15

Thr Asn Arg Asp Ser Thr Ile Leu Glu Leu Gln Lys Val Leu Lys Thr
            20                  25                  30

Cys Cys Ala Gln Ser Met Lys Ile Phe Cys Cys Leu Trp Asn Phe Val
        35                  40                  45

Tyr Lys Gln Leu Glu Asp Ala Ala Gln Gly Leu Thr Met Gly Gly Asp
    50                  55                  60

Val Glu Glu His Glu Asp Leu Thr Ala Asp Ser Thr Ile Phe Lys Phe
65                  70                  75                  80

Val Glu Ala Tyr Thr Glu Trp Glu Val Lys Arg Trp Ser Asp Asn Asn
                85                  90                  95

Leu Ile Met Lys Gln Thr Asn Val Lys Arg Arg Arg Leu Asp Asp Val
            100                 105                 110

Gly Pro Glu Leu Glu Lys Ala Val Trp Glu Leu Gly Cys Pro Pro Ser
        115                 120                 125

Ile Gln Cys Leu Leu Pro Pro Val Cys Tyr Ala Cys Val Trp Phe Phe
    130                 135                 140

Gln Val Leu Ile Phe Phe Leu Ile Leu Ser Phe Cys Gly Tyr Ile Val
145                 150                 155                 160

Gly Val Tyr Ile Tyr Gly Leu His Glu Met Phe
                165                 170

<210> 265
<211> 18
<212> DNA
<213> künstliche Sequenz

<220>
<223> Oligonukleotid

<400> 265
ttcaacatcg ccacccat 18

<210> 266
<211> 20
<212> DNA
<213> künstliche Sequenz

<220>
<223>  Oligonukleotid

<400>  266
cagcagaggg gaagtggtca                                                    20


<210>  267
<211>  390
<212>  DNA
<213>  Homo sapiens

<400>  267
atggaagtga tattaccaga caaacctcag gtagatgcac tggcctttct agctgctgtc      60

accatgctgt ggataacgct gcccatgagt ccttttgcag aagcagagaa attggcatgg     120

gatctggagg ttggaggttt agctggacag ccccttaaag ttttcactcc acgtaaaaaa     180

ggttctgggg aagtgggtga tgcttctcag tcgcccagca gaagcaatga tggccagcat     240

tcctgcattg gccacagcag agatctctgc tgctacactg ctcagaccct cataatctcc     300

tacacatcaa atggtctttc tcctttagca actccaccct tccaccctat tcctggaaac     360

tgctacgaca gtgttgatta taaaatatag                                      390


<210>  268
<211>  129
<212>  PRT
<213>  Homo sapiens

<400>  268

Met Glu Val Ile Leu Pro Asp Lys Pro Gln Val Asp Ala Leu Ala Phe
1               5                   10                  15


Leu Ala Ala Val Thr Met Leu Trp Ile Thr Leu Pro Met Ser Pro Phe
            20                  25                  30


Ala Glu Ala Glu Lys Leu Ala Trp Asp Leu Glu Val Gly Gly Leu Ala
        35                  40                  45


Gly Gln Pro Leu Lys Val Phe Thr Pro Arg Lys Lys Gly Ser Gly Glu
    50                  55                  60


Val Gly Asp Ala Ser Gln Ser Pro Ser Arg Ser Asn Asp Gly Gln His
65                  70                  75                  80


Ser Cys Ile Gly His Ser Arg Asp Leu Cys Cys Tyr Thr Ala Gln Thr
                85                  90                  95


Leu Ile Ile Ser Tyr Thr Ser Asn Gly Leu Ser Pro Leu Ala Thr Pro
            100                 105                 110


Pro Phe His Pro Ile Pro Gly Asn Cys Tyr Asp Ser Val Asp Tyr Lys
        115                 120                 125

Ile

<210> 269
<211> 2856
<212> DNA
<213> Homo sapiens

<400> 269
atggccaaaa gaaatctcag cactgtgaca gagttcattc ttgtagtctt cacagatcac 60
cctgaactgg cagttccact cttcctagtg tttctcagtt tctatcttgt cacttttctg 120
gggaatgggg ggatgatcat tctaatccaa gtggatgccc aactccacac ccccgtgtac 180
ttcttcctga gccaccttgc tttcctggat gcctgctgtg cctcagtaat cacccctcag 240
attctggcca cactggccac agacaagaca gttatctcct atggctgccg tgctgtgcag 300
ttctctttct tcaccatatg tgcaggcaca gagtgttacc tgctgtcagt gatggcctat 360
gaccgctttg ttgccattag caatccactg cactgtaaca tgaccatgac tccaggtacc 420
tgcagggtct ttttggccag tgccttcatc tgtggggtgt caggggccat tctgcatacc 480
acgtgcacct tcaccctctc cttctgttgt gacaatcaga tcaacttctt cttctgtgac 540
ctcccacccc tgctgaagct cgcctgcagc agcatgacac aaactgagat tgtcattctc 600
ctttgtgcaa aatgcatgtt cctagccaat gtcatggtta tcctgatctg ctacatgctc 660
attatcagag ccattttgag ggtgaagtcg gcaggaggcc tcctgatagc atctgctcat 720
ttcgatgcat atgtatatga gacaggcatc aactacaaca cagtttatgg ctcaggaaag 780
gcagtagggt ggtcctggag gagcctgcgg gaaaccaacc acatgagacc aggaaatact 840
tcaaaacact cagcagccca gctgcatcaa tgcctcatcc agcaagttgg caggtggccc 900
ttgcagagca tgcccttccc cgtttctgca gggccacctt ataagtcagt gcagcctctc 960
cctggagacc cccggcctct cctgtgcatc accggattat ttctgacttt gaagatgatg 1020
gggtgtgggc ccaggaggcc cagggacagg aagtctgact tcttcataaa cacagaccct 1080
ggtgcagggt caccagaaga acagaggtgt ggatgggaag ggcatccttc ccactcctat 1140
accctggggc tgtctctgcc agtcaacttc ggcctgaaat gtccatggtg gacactatct 1200
ggacccccag ctacctgcca acgtccagac ctgcagacac cttctccacc aaaggagata 1260
tgttcatccg ggctgcgacc ccttacacac agcgctggac cagacagaag tcaagttcca 1320
gcagcctccg gagcagccac tatgctgaca aaggggctgc ccgacatcac tgtgggactg 1380
cagatttatg actcctgcat ctcagggatc caggctctgg ggagcaccct ggccctgctg 1440
tccaatcagc ttccacccac aaccaactat gcttgtggct cccagcaaca tctcctgggc 1500
gtggttggag ggatgacctt cctggagtca gagcccatgt ctgagctgct ctccatctac 1560
agagtccctc agggccaaag actcaccaaa aactttgaag taaaagaact tgtctgcaca 1620
tatctggtag gacagcttcc ttatggcctg gtcagttatg acaacagcaa ctttgagtgg 1680
ctggatcagc agctgcagaa gcagatcggg ggcgagggac ttcctgttgg cgctgcgccc 1740

agccgtgtag ccaggcaaca gtctgatgag gaagctgtgg gaggagtgca gggatacagg 1800
tggtctggat taggggcttc catccaaagt gccagagaag gggcttggca tcgcacaggg 1860
ctggagaaca tgaccactgc ccacctgtct gccttcaaac ttcctgatct aactgccact 1920
taccaagcct acctggcagc caaagccctg tgggttgcct atcagaactt gatgtcctgc 1980
tctgagagag agggaccatt cctgggaggc acgtatgcca atgcatggga agccaggctt 2040
tctcaggtta acttcaccac caaagcccaa gaagaggttt tcttcgccaa agatggggaa 2100
gtgctgacaa cgtttgacat taaaaacatc tatgttctcc cagacctgtc aggacagaca 2160
gccattgttg gacactttga cttcagagca ccttctggaa aagagcttct gttggatgac 2220
agcgcaattg tctgggcaga aggaccctta aagattagag ctgagagaac cctaagaacc 2280
aagaccacac agcacctctc acatcccaag ctccaggagt cccttcctct gtctgcaacg 2340
aaaaacgtcc tgtggaaacc aggaagtcaa ccctatttga gaagtcaaaa tgctgctaca 2400
aaagccttcc ctgacccaga agagaaatcg caatgtcacc agtttctctt tctcccttca 2460
gatagtgttg catgtcagaa gtgctctgac aaccagtggc ccaatgtgca gaagggcgag 2520
tgcatcccca aaacccttga cttcttgttc tatcacaagc cccttgacac agcgttggct 2580
gtctgcacag ccctgctctt tctccttgcc ctggccatct taggcatctt ccatgttgtc 2640
tgctcctgtg tctgggtgtc cttcatacct gcccacatgc atgcccacag caaagacacc 2700
atggccatgg aggtctttgt catcttggca tcagcaggag gcctcatgtc ctccctcttc 2760
ttttccaaat gctacatcat ccttctccat cctgaaaaga acacaaaaga ccaaatgttt 2820
ggccggcatc atcgcaagtg ggaaaaactg aagtga 2856

<210> 270
<211> 951
<212> PRT
<213> Homo sapiens

<400> 270

Met Ala Lys Arg Asn Leu Ser Thr Val Thr Glu Phe Ile Leu Val Val
1               5                   10                  15

Phe Thr Asp His Pro Glu Leu Ala Val Pro Leu Phe Leu Val Phe Leu
            20                  25                  30

Ser Phe Tyr Leu Val Thr Phe Leu Gly Asn Gly Gly Met Ile Ile Leu
        35                  40                  45

Ile Gln Val Asp Ala Gln Leu His Thr Pro Val Tyr Phe Phe Leu Ser
    50                  55                  60

His Leu Ala Phe Leu Asp Ala Cys Cys Ala Ser Val Ile Thr Pro Gln
65                  70                  75                  80

Ile Leu Ala Thr Leu Ala Thr Asp Lys Thr Val Ile Ser Tyr Gly Cys
                85                  90                  95

Arg Ala Val Gln Phe Ser Phe Phe Thr Ile Cys Ala Gly Thr Glu Cys
            100                 105                 110

Tyr Leu Leu Ser Val Met Ala Tyr Asp Arg Phe Val Ala Ile Ser Asn
        115                 120                 125

Pro Leu His Cys Asn Met Thr Met Thr Pro Gly Thr Cys Arg Val Phe
    130                 135                 140

Leu Ala Ser Ala Phe Ile Cys Gly Val Ser Gly Ala Ile Leu His Thr
145                 150                 155                 160

Thr Cys Thr Phe Thr Leu Ser Phe Cys Cys Asp Asn Gln Ile Asn Phe
                165                 170                 175

Phe Phe Cys Asp Leu Pro Pro Leu Leu Lys Leu Ala Cys Ser Ser Met
            180                 185                 190

Thr Gln Thr Glu Ile Val Ile Leu Leu Cys Ala Lys Cys Met Phe Leu
        195                 200                 205

Ala Asn Val Met Val Ile Leu Ile Cys Tyr Met Leu Ile Ile Arg Ala
    210                 215                 220

Ile Leu Arg Val Lys Ser Ala Gly Gly Leu Leu Ile Ala Ser Ala His
225                 230                 235                 240

Phe Asp Ala Tyr Val Tyr Glu Thr Gly Ile Asn Tyr Asn Thr Val Tyr
                245                 250                 255

Gly Ser Gly Lys Ala Val Gly Trp Ser Trp Arg Ser Leu Arg Glu Thr
            260                 265                 270

Asn His Met Arg Pro Gly Asn Thr Ser Lys His Ser Ala Ala Gln Leu
        275                 280                 285

His Gln Cys Leu Ile Gln Gln Val Gly Arg Trp Pro Leu Gln Ser Met
    290                 295                 300

Pro Phe Pro Val Ser Ala Gly Pro Pro Tyr Lys Ser Val Gln Pro Leu
305                 310                 315                 320

Pro Gly Asp Pro Arg Pro Leu Leu Cys Ile Thr Gly Leu Phe Leu Thr
                325                 330                 335

Leu Lys Met Met Gly Cys Gly Pro Arg Arg Pro Arg Asp Arg Lys Ser
            340                 345                 350

Asp Phe Phe Ile Asn Thr Asp Pro Gly Ala Gly Ser Pro Glu Glu Gln
        355                 360                 365

Arg Cys Gly Trp Glu Gly His Pro Ser His Ser Tyr Thr Leu Gly Leu
    370                 375                 380

Ser Leu Pro Val Asn Phe Gly Leu Lys Cys Pro Trp Trp Thr Leu Ser
385                 390                 395                 400

Gly Pro Pro Ala Thr Cys Gln Arg Pro Asp Leu Gln Thr Pro Ser Pro
                405                 410                 415

Pro Lys Glu Ile Cys Ser Ser Gly Leu Arg Pro Leu Thr His Ser Ala
            420                 425                 430

Gly Pro Asp Arg Ser Gln Val Pro Ala Ala Ser Gly Ala Ala Thr Met
        435                 440                 445

Leu Thr Lys Gly Leu Pro Asp Ile Thr Val Gly Leu Gln Ile Tyr Asp
    450                 455                 460

Ser Cys Ile Ser Gly Ile Gln Ala Leu Gly Ser Thr Leu Ala Leu Leu
465                 470                 475                 480

Ser Asn Gln Leu Pro Pro Thr Thr Asn Tyr Ala Cys Gly Ser Gln Gln
                485                 490                 495

His Leu Leu Gly Val Val Gly Gly Met Thr Phe Leu Glu Ser Glu Pro
            500                 505                 510

Met Ser Glu Leu Leu Ser Ile Tyr Arg Val Pro Gln Gly Gln Arg Leu
        515                 520                 525

Thr Lys Asn Phe Glu Val Lys Glu Leu Val Cys Thr Tyr Leu Val Gly
    530                 535                 540

Gln Leu Pro Tyr Gly Leu Val Ser Tyr Asp Asn Ser Asn Phe Glu Trp
545                 550                 555                 560

Leu Asp Gln Gln Leu Gln Lys Gln Ile Gly Gly Glu Gly Leu Pro Val
                565                 570                 575

Gly Ala Ala Pro Ser Arg Val Ala Arg Gln Gln Ser Asp Glu Glu Ala
            580                 585                 590

Val Gly Gly Val Gln Gly Tyr Arg Trp Ser Gly Leu Gly Ala Ser Ile
        595                 600                 605

Gln Ser Ala Arg Glu Gly Ala Trp His Arg Thr Gly Leu Glu Asn Met
    610                 615                 620

Thr Thr Ala His Leu Ser Ala Phe Lys Leu Pro Asp Leu Thr Ala Thr
625                 630                 635                 640

Tyr Gln Ala Tyr Leu Ala Ala Lys Ala Leu Trp Val Ala Tyr Gln Asn
                645                 650                 655

Leu Met Ser Cys Ser Glu Arg Glu Gly Pro Phe Leu Gly Gly Thr Tyr
            660                 665                 670

Ala Asn Ala Trp Glu Ala Arg Leu Ser Gln Val Asn Phe Thr Thr Lys
        675                 680                 685

Ala Gln Glu Glu Val Phe Phe Ala Lys Asp Gly Glu Val Leu Thr Thr
    690                 695                 700

Phe Asp Ile Lys Asn Ile Tyr Val Leu Pro Asp Leu Ser Gly Gln Thr
705                 710                 715                 720

Ala Ile Val Gly His Phe Asp Phe Arg Ala Pro Ser Gly Lys Glu Leu
                725                 730                 735

Leu Leu Asp Asp Ser Ala Ile Val Trp Ala Glu Gly Pro Leu Lys Ile
            740                 745                 750

Arg Ala Glu Arg Thr Leu Arg Thr Lys Thr Thr Gln His Leu Ser His
        755                 760                 765

Pro Lys Leu Gln Glu Ser Leu Pro Leu Ser Ala Thr Lys Asn Val Leu
    770                 775                 780

Trp Lys Pro Gly Ser Gln Pro Tyr Leu Arg Ser Gln Asn Ala Ala Thr
785                 790                 795                 800

Lys Ala Phe Pro Asp Pro Glu Glu Lys Ser Gln Cys His Gln Phe Leu
                805                 810                 815

Phe Leu Pro Ser Asp Ser Val Ala Cys Gln Lys Cys Ser Asp Asn Gln
            820                 825                 830

Trp Pro Asn Val Gln Lys Gly Glu Cys Ile Pro Lys Thr Leu Asp Phe
        835                 840                 845

Leu Phe Tyr His Lys Pro Leu Asp Thr Ala Leu Ala Val Cys Thr Ala
    850                 855                 860

Leu Leu Phe Leu Leu Ala Leu Ala Ile Leu Gly Ile Phe His Val Val
865                 870                 875                 880

Cys Ser Cys Val Trp Val Ser Phe Ile Pro Ala His Met His Ala His
                885                 890                 895

Ser Lys Asp Thr Met Ala Met Glu Val Phe Val Ile Leu Ala Ser Ala
            900                 905                 910

Gly Gly Leu Met Ser Ser Leu Phe Phe Ser Lys Cys Tyr Ile Ile Leu
        915                 920                 925

Leu His Pro Glu Lys Asn Thr Lys Asp Gln Met Phe Gly Arg His His
    930                 935                 940

Arg Lys Trp Glu Lys Leu Lys
945                 950

<210> 271
<211> 956
<212> DNA
<213> Homo sapiens

<400> 271
gccgcgctgt atggggccag cggacacttc gccccaggca ccactgtgcc cctggccctg 60

ccacctggtg gcaatggctc agccacacct gacaatggca ccacccctgg ggccgagggc 120

tggcggcagt tgctgggcct actccccgag cacatggcgg agaagctgtg tgaggcctgg 180

gcctttgggc agagccacca gacgggcgtc gtggcactgg gcctactcac ctgcctgctg 240

gcaatgctgc tggctggccg catcaggctc cggaggatcg atgccttctg cacctgcctg 300

tgggccctgc tgctggggct gcacctggct gagcagcacc tgcaggccgc ctcgcctagc 360

tggctagaca cgctcaagtt cagcaccaca tctttgtgct gcctggttgg cttcacggcg 420

gctgtggcca caaggaaggc aacgggccca cggaggttcc ggccccgaag gttcttccca 480

ggagactctg ccggcctttt ccccaccagc cccagcttgg ccatccctca cccgagtgtc 540

ggaggctctc cagcgtctct gttcatcccc agcccgccca gcttcctgcc cctcgccaac 600

caagcagctc ttccggtctc ctcgacggac ctcaccctcc tcatttgcct ggccgcctca 660

gccgggccct ctctctggga accataccct ctctgactcg agcagactcc ggctatctgt 720

tcagcggtag ccgcccacca tctcaggtgt ctcgatctgg gggagtttcc tgttttcaga 780

ttacttctct cttcttgtcg gggaagctgc ccctccgtcc catcctttcc cagggccttc 840

cgggggcggc tcggtgggcc tccagtccgg ctctctggcc acgggaggcc ctcatcagcc 900

tgccggtcaa cctgagggac gaagtgtgtt gtccggcacc cctggagagg cccaaa 956

<210> 272
<211> 231
<212> PRT
<213> Homo sapiens

<400> 272

Ala Ala Leu Tyr Gly Ala Ser Gly His Phe Ala Pro Gly Thr Thr Val
1               5                   10                  15

Pro Leu Ala Leu Pro Pro Gly Gly Asn Gly Ser Ala Thr Pro Asp Asn
            20                  25                  30

Gly Thr Thr Pro Gly Ala Glu Gly Trp Arg Gln Leu Leu Gly Leu Leu
        35                  40                  45

Pro Glu His Met Ala Glu Lys Leu Cys Glu Ala Trp Ala Phe Gly Gln
    50                  55                  60

Ser His Gln Thr Gly Val Val Ala Leu Gly Leu Leu Thr Cys Leu Leu
65                  70                  75                  80

Ala Met Leu Leu Ala Gly Arg Ile Arg Leu Arg Arg Ile Asp Ala Phe
                85                  90                  95

Cys Thr Cys Leu Trp Ala Leu Leu Leu Gly Leu His Leu Ala Glu Gln
            100                 105                 110

His Leu Gln Ala Ala Ser Pro Ser Trp Leu Asp Thr Leu Lys Phe Ser
        115                 120                 125

Thr Thr Ser Leu Cys Cys Leu Val Gly Phe Thr Ala Ala Val Ala Thr
    130                 135                 140

Arg Lys Ala Thr Gly Pro Arg Arg Phe Arg Pro Arg Arg Phe Phe Pro
145                 150                 155                 160

Gly Asp Ser Ala Gly Leu Phe Pro Thr Ser Pro Ser Leu Ala Ile Pro
                165                 170                 175

His Pro Ser Val Gly Gly Ser Pro Ala Ser Leu Phe Ile Pro Ser Pro
            180                 185                 190

Pro Ser Phe Leu Pro Leu Ala Asn Gln Ala Ala Leu Pro Val Ser Ser
        195                 200                 205

Thr Asp Leu Thr Leu Leu Ile Cys Leu Ala Ala Ser Ala Gly Pro Ser
    210                 215                 220

Leu Trp Glu Pro Tyr Pro Leu
225                 230

<210>  273
<211>  1806
<212>  DNA
<213>  Homo sapiens

<400>  273
gaggaggcgc gcgtcgccgc cccgcgtccc gcctgcggcc cgcgcccccg gcgtcaccgc      60

ctcctgcccg cctgcccgcc tgcccgcctg cccgcctacc cgcctacccg cctacccgcc     120

tacccccctg ccggcctgcc gtccttccac gcggagagcc atggagggag tgagcgcgct     180

gctggcccgc tgccccacgg ccggcctggc cggcggcctg ggggtcacgg cgtgcgccgc     240

ggccggcgtg ttgctctacc ggatcgcgcg gaggatgaag ccaacgcaca cgatggtcaa     300

ctgctggttc tgcaaccagg atacgctggt gccctatggg aaccgcaact gctgggactg 360
tccccactgc gagcagtaca acggcttcca ggagaacggc gactacaaca agccgatccc 420
cgcccagtac ttggagcacc tgaaccacgt ggtgagcagc gcgcccagcc tgcgcgaccc 480
ttcgcagccg cagcagtggg tgagcagcca agtcctgctg tgcaagaggt gcaaccacca 540
ccagaccacc aagatcaagc agctggccgc cttcgctccc cgcgaggagg gcaggtatga 600
cgaggaggtc gaggtgtacc ggcatcacct ggagcagatg tacaagctgt gccggccgtg 660
ccaagcggct gtggagtact acatcaagca ccagaaccgc cagctgcgcg ccctgttgct 720
cagccaccag ttcaagcgcc gggaggccga ccagacccac gcacagaact tctcctccgc 780
cgtgaagtcc ccggtccagg tcatcctgct ccgtgccctc gccttcctgg cctgcgcctt 840
cctactgacc accgcgctgt atggggccag cggacacttc gccccaggca ccactgtgcc 900
cctggccctg ccacctggtg gcaatggctc agccacacct gacaatggca ccacccctgg 960
ggccgagggc tggcggcagt tgctgggcct actccccgag cacatggcgg agaagctgtg 1020
tgaggcctgg gcctttgggc agagccacca gacgggcgtc gtggcactgg gcctactcac 1080
ctgcctgctg gcaatgctgc tggctggccg catcaggctc cggaggatcg atgccttctg 1140
cacctgcctg tgggccctgc tgctggggct gcacctggct gagcagcacc tgcaggccgc 1200
ctcgcctagc tggctagaca cgctcaagtt cagcaccaca tctttgtgct gcctggttgg 1260
cttcacggcg gctgtggcca caaggaaggc aacgggccca cggaggttcc ggccccgaag 1320
gttcttccca ggagactctg ccggcctttt ccccaccagc cccagcttgg ccatccctca 1380
cccgagtgtc ggaggctctc cagcgtctct gttcatcccc agcccgccca gcttcctgcc 1440
cctcgccaac caagcagctc ttccggtctc ctcgacggac ctcaccctcc tcatttgcct 1500
ggccgcctca gccgggccct ctctctggga accataccct ctctgactcg agcagactcc 1560
ggctatctgt tcagcggtag ccgcccacca tctcaggtgt ctcgatctgg gggagtttcc 1620
tgttttcaga ttacttctct cttcttgtcg gggaagctgc ccctccgtcc catcctttcc 1680
cagggccttc cgggggcggc tcggtgggcc tccagtccgg ctctctggcc acgggaggcc 1740
ctcatcagcc tgccggtcaa cctgagggac gaagtgtgtt gtccggcacc cctggagagg 1800
cccaaa 1806

<210> 274
<211> 461
<212> PRT
<213> Homo sapiens

<400> 274

Met Glu Gly Val Ser Ala Leu Leu Ala Arg Cys Pro Thr Ala Gly Leu
1               5                   10                  15

Ala Gly Gly Leu Gly Val Thr Ala Cys Ala Ala Ala Gly Val Leu Leu
            20                  25                  30

Tyr Arg Ile Ala Arg Arg Met Lys Pro Thr His Thr Met Val Asn Cys
        35                  40                  45

Trp Phe Cys Asn Gln Asp Thr Leu Val Pro Tyr Gly Asn Arg Asn Cys
    50                  55                  60

Trp Asp Cys Pro His Cys Glu Gln Tyr Asn Gly Phe Gln Glu Asn Gly
65                  70                  75                  80

Asp Tyr Asn Lys Pro Ile Pro Ala Gln Tyr Leu Glu His Leu Asn His
                85                  90                  95

Val Val Ser Ser Ala Pro Ser Leu Arg Asp Pro Ser Gln Pro Gln Gln
            100                 105                 110

Trp Val Ser Ser Gln Val Leu Leu Cys Lys Arg Cys Asn His His Gln
        115                 120                 125

Thr Thr Lys Ile Lys Gln Leu Ala Ala Phe Ala Pro Arg Glu Glu Gly
    130                 135                 140

Arg Tyr Asp Glu Glu Val Glu Val Tyr Arg His His Leu Glu Gln Met
145                 150                 155                 160

Tyr Lys Leu Cys Arg Pro Cys Gln Ala Ala Val Glu Tyr Tyr Ile Lys
                165                 170                 175

His Gln Asn Arg Gln Leu Arg Ala Leu Leu Leu Ser His Gln Phe Lys
            180                 185                 190

Arg Arg Glu Ala Asp Gln Thr His Ala Gln Asn Phe Ser Ser Ala Val
        195                 200                 205

Lys Ser Pro Val Gln Val Ile Leu Leu Arg Ala Leu Ala Phe Leu Ala
    210                 215                 220

Cys Ala Phe Leu Leu Thr Thr Ala Leu Tyr Gly Ala Ser Gly His Phe
225                 230                 235                 240

Ala Pro Gly Thr Thr Val Pro Leu Ala Leu Pro Pro Gly Gly Asn Gly
                245                 250                 255

Ser Ala Thr Pro Asp Asn Gly Thr Thr Pro Gly Ala Glu Gly Trp Arg
            260                 265                 270

Gln Leu Leu Gly Leu Leu Pro Glu His Met Ala Glu Lys Leu Cys Glu
        275                 280                 285

Ala Trp Ala Phe Gly Gln Ser His Gln Thr Gly Val Val Ala Leu Gly
    290                 295                 300

Leu Leu Thr Cys Leu Leu Ala Met Leu Leu Ala Gly Arg Ile Arg Leu
305                 310                 315                 320

Arg Arg Ile Asp Ala Phe Cys Thr Cys Leu Trp Ala Leu Leu Leu Gly
                325                 330                 335

Leu His Leu Ala Glu Gln His Leu Gln Ala Ala Ser Pro Ser Trp Leu
            340                 345                 350

Asp Thr Leu Lys Phe Ser Thr Thr Ser Leu Cys Cys Leu Val Gly Phe
        355                 360                 365

Thr Ala Ala Val Ala Thr Arg Lys Ala Thr Gly Pro Arg Arg Phe Arg
    370                 375                 380

Pro Arg Arg Phe Phe Pro Gly Asp Ser Ala Gly Leu Phe Pro Thr Ser
385                 390                 395                 400

Pro Ser Leu Ala Ile Pro His Pro Ser Val Gly Gly Ser Pro Ala Ser
                405                 410                 415

Leu Phe Ile Pro Ser Pro Pro Ser Phe Leu Pro Leu Ala Asn Gln Ala
            420                 425                 430

Ala Leu Pro Val Ser Ser Thr Asp Leu Thr Leu Leu Ile Cys Leu Ala
        435                 440                 445

Ala Ser Ala Gly Pro Ser Leu Trp Glu Pro Tyr Pro Leu
    450                 455                 460

<210>  275
<211>  600
<212>  DNA
<213>  Homo sapiens

<220>
<221>  misc_feature
<222>  (460)..(461)
<223>  any

<220>
<221>  misc_feature
<222>  (530)..(531)
<223>  any

<220>
<221>  misc_feature
<222>  (574)..(575)
<223>  any

<220>
<221>  misc_feature
<222>  (577)..(578)
<223>  any

<400>  275
tcaagtctga cttgcatcta cactgcgggc aagatgcggc tgcaagaccg catcgccacg      60

ttcttcttcc caaaaggcat gatgctcacc acggctgcgc tgatgctctt cttcttacac     120

ctgggcatct tcatcagaga cgtgcacaac ttctgcatca cctaccacta tgaccacatg     180

agctttcact acacggtcgt cctgatgttc tcccaggtga tcagcatctg ctgggctgcc     240

atggggtcac tctatgctga gatgacagaa aacaagtacg tctgcttctc cgccctgacc     300

atcctgagtg agtggcagga gggggagggt gcaagaggga gcggggagct ttggaaccct     360

gagatgtggc aaggagtagc cagggaaggg tactggggct catggggggc tctgtccccc     420

gcccagtgct caacggagcc atgctcttca accgcctgtn cttggagttt ctggccatcg     480

agtaccggga ggagcaccac tgaggcctgg ggagtcggaa cagggctaan gagggggaag     540

caaaaggctg cctcgggtgt tttaataaag ctgntgntta tttccaaaaa aaaaaaaaaa     600

<210>  276
<211>  174
<212>  PRT
<213>  Homo sapiens

<220>
<221>  MISC_FEATURE
<222>  (128)..(129)
<223>  i

<220>
<221>  MISC_FEATURE
<222>  (151)..(152)
<223>  any

<220>
<221>  MISC_FEATURE
<222>  (166)..(168)
<223>  any

<400>  276

Met Met Leu Thr Thr Ala Ala Leu Met Leu Phe Phe Leu His Leu Gly
1               5                   10                  15

Ile Phe Ile Arg Asp Val His Asn Phe Cys Ile Thr Tyr His Tyr Asp
            20                  25                  30

His Met Ser Phe His Tyr Thr Val Val Leu Met Phe Ser Gln Val Ile
        35                  40                  45

Ser Ile Cys Trp Ala Ala Met Gly Ser Leu Tyr Ala Glu Met Thr Glu
    50                  55                  60

Asn Lys Tyr Val Cys Phe Ser Ala Leu Thr Ile Leu Ser Glu Trp Gln
65                  70                  75                  80

Glu Gly Glu Gly Ala Arg Gly Ser Gly Glu Leu Trp Asn Pro Glu Met
                85                  90                  95

Trp Gln Gly Val Ala Arg Glu Gly Tyr Trp Gly Ser Trp Gly Ala Leu
            100                 105                 110

Ser Pro Ala Gln Cys Ser Thr Glu Pro Cys Ser Ser Thr Ala Cys Xaa
        115                 120                 125

Trp Ser Phe Trp Pro Ser Ser Thr Gly Arg Ser Thr Thr Glu Ala Trp
    130                 135                 140

Gly Val Gly Thr Gly Leu Xaa Arg Gly Lys Gln Lys Ala Ala Ser Gly
145                 150                 155                 160

Val Leu Ile Lys Leu Xaa Xaa Ile Ser Lys Lys Lys Lys Lys
                165                 170

<210> 277
<211> 457
<212> DNA
<213> Homo sapiens

<400> 277
aaacactgca ggctacgaat cggtcattgc ataggttttc catgaatcag gaagattcag 60

tcctggtaaa ttcattccca ggaacatcgc tgccactgct attattctag cagctgttcc 120

catactccaa tgagtccagt taaacatttg ccttcttggg tcatgtaaag gtggcctgaa 180

gactgccaga agaggctgaa gaactgccaa agtcatcact atacagccga ggtatgggtg 240

gtaacctgca tgcctactcc agcctcccct gtatataaac ggcataacaa aagcaatgca 300

ggtgaggaca gttgtggtga acatgagcat ccgatgcacc tgaaaccaag ctgcttcacc 360

aagcaagaaa gcttttgacc aaactggctt gaagaaccgg gcaaccagta cacctatgct 420

aacagtagtc atccatgcca caaacattaa ggcacca 457

<210> 278
<211> 144
<212> PRT
<213> Homo sapiens

<400> 278

Met Phe Val Ala Trp Met Thr Thr Val Ser Ile Gly Val Leu Val Ala
1               5                   10                  15

Arg Phe Phe Lys Pro Val Trp Ser Lys Ala Phe Leu Leu Gly Glu Ala
            20                  25                  30

Ala Trp Phe Gln Val His Arg Met Leu Met Phe Thr Thr Thr Val Leu
        35                  40                  45

Thr Cys Ile Ala Phe Val Met Pro Phe Ile Tyr Arg Gly Gly Trp Ser
    50                  55                  60

Arg His Ala Gly Tyr His Pro Tyr Leu Gly Cys Ile Val Met Thr Leu
65                  70                  75                  80

Ala Val Leu Gln Pro Leu Leu Ala Val Phe Arg Pro Pro Leu His Asp
                85                  90                  95

Pro Arg Arg Gln Met Phe Asn Trp Thr His Trp Ser Met Gly Thr Ala
            100                 105                 110

Ala Arg Ile Ile Ala Val Ala Ala Met Phe Leu Gly Met Asn Leu Pro
        115                 120                 125

Gly Leu Asn Leu Pro Asp Ser Trp Lys Thr Tyr Ala Met Thr Asp Ser
    130                 135                 140

<210>  279
<211>  293
<212>  DNA
<213>  Homo sapiens

<400>  279
tttttttttt tttttttaag gctgaagcaa ataggaacgt atatttctca tgaatccaaa      60

gcaaagacac aggaagtgct ggcattcttt tggtggctgg tagctcttga ccttctcttc     120

aaggttgcca catgccttag cagcagctca tgacttcacg ttctcaccgt attcgaaggc     180

aggaagcatg gagtagctgg cagctgcgtt tgacacagac tgccctcgga ccccttctcc     240

gcgcagtgcg actcgcaatt gtctggagca cgttggcagc agccctcgtg ccg            293

<210>  280
<211>  45
<212>  PRT
<213>  Homo sapiens

<400>  280

Arg His Glu Gly Cys Cys Gln Arg Ala Pro Asp Asn Cys Glu Ser His
1               5                   10                  15

Cys Ala Glu Lys Gly Ser Glu Gly Ser Leu Cys Gln Thr Gln Leu Pro
            20                  25                  30

Ala Thr Pro Cys Phe Leu Pro Ser Asn Thr Val Arg Thr
        35                  40                  45

<210>  281
<211>  15
<212>  PRT
<213>  Homo sapiens

<400>  281

<210>   282
<211>   13
<212>   PRT
<213>   Homo sapiens

<400>   282

Met Ala Lys Gln Gly Glu Met Asn Thr Ser Thr Ser Cys
1               5                   10


<210>   283
<211>   13
<212>   PRT
<213>   Homo sapiens

<400>   283

Pro Lys Arg Gly Gly Arg Ala Gly Arg Glu His Ser Cys
1               5                   10


<210>   284
<211>   91
<212>   PRT
<213>   Homo sapiens

<400>   284

Arg Phe Gln Arg Asn Thr Gly Glu Met Ser Ser Asn Ser Thr Ala Leu
1               5                   10                  15


Ala Leu Val Arg Pro Ser Ser Ser Gly Leu Ile Asn Ser Asn Thr Asp
            20                  25                  30


Asn Asn Leu Ala Val Tyr Asp Leu Ser Arg Asp Ile Leu Asn Asn Phe
        35                  40                  45


Pro His Ser Ile Ala Arg Gln Lys Arg Ile Leu Val Asn Leu Ser Met
    50                  55                  60


Val Glu Asn Lys Leu Val Glu Leu Glu His Thr Leu Leu Ser Lys Gly
65                  70                  75                  80


Phe Arg Gly Ala Ser Pro His Arg Lys Ser Thr
                85                  90


<210>   285
<211>   15
<212>   PRT
<213>   Homo sapiens

<400>   285

Cys Lys Tyr Arg Arg Phe Gln Arg Asn Thr Gly Glu Met Ser Ser
1               5                   10                  15


<210>   286
<211>   14
<212>   PRT

<213>  Homo sapiens

<400>  286

Cys Lys Gly Phe Arg Gly Ala Ser Pro His Arg Lys Ser Thr
1               5                   10


<210>  287
<211>  19
<212>  PRT
<213>  Homo sapiens

<400>  287

Met Ala Cys Ile Tyr Pro Thr Thr Phe Tyr Thr Ser Leu Pro Thr Lys
1               5                   10                  15


Ser Leu Asn



<210>  288
<211>  121
<212>  PRT
<213>  Homo sapiens

<400>  288

Ala Pro Pro Ser Cys Arg Glu Cys Tyr Gln Ser Leu His Tyr Arg Gly
1               5                   10                  15


Glu Met Gln Gln Tyr Phe Thr Tyr His Thr His Ile Glu Arg Ser Cys
            20                  25                  30


Tyr Gly Asn Leu Ile Glu Glu Cys Val Glu Ser Gly Lys Ser Tyr Tyr
        35                  40                  45


Lys Val Lys Asn Leu Gly Val Cys Gly Ser Arg Asn Gly Ala Ile Cys
    50                  55                  60


Pro Arg Gly Lys Gln Trp Leu Cys Phe Thr Lys Ile Gly Gln Trp Gly
65                  70                  75                  80


Val Asn Thr Gln Val Leu Glu Asp Ile Lys Arg Glu Gln Ile Ile Ala
                85                  90                  95


Lys Ala Lys Ala Ser Lys Pro Thr Thr Pro Pro Glu Asn Arg Pro Arg
            100                 105                 110


His Phe His Ser Phe Ile Gln Lys Leu
        115                 120


<210>  289
<211>  15
<212>  PRT
<213>  Homo sapiens

<400>  289

Cys Glu Asn Arg Pro Arg His Phe His Ser Phe Ile Gln Lys Leu
1               5                   10                  15

<210>  290
<211>  13
<212>  PRT
<213>  Homo sapiens

<400>  290

Cys Ile Tyr Pro Thr Thr Phe Tyr Thr Ser Leu Pro Thr
1               5                   10

<210>  291
<211>  14
<212>  PRT
<213>  Homo sapiens

<400>  291

Cys Lys Glu Asp Glu Leu Val Arg Asp Ser Pro Ala Arg Lys
1               5                   10

<210>  292
<211>  12
<212>  PRT
<213>  Homo sapiens

<400>  292

Ala Leu Gly Thr Arg Leu Ser Gln His Thr Asp Val
1               5                   10

<210>  293
<211>  11
<212>  PRT
<213>  Homo sapiens

<400>  293

Asp Phe Asn Cys Pro Cys Leu Val His Tyr Asn
1               5                   10

<210>  294
<211>  53
<212>  PRT
<213>  Homo sapiens

<400>  294

Ser Ser Ser Val Asp Pro Glu Lys Phe Leu Asp Phe Ala Asn Met Thr
1               5                   10                  15

Pro Ser Gln Val Gln Leu Phe Leu Ala Lys Val Pro Cys Lys Glu Asp
            20                  25                  30

Glu Leu Val Arg Asp Ser Pro Ala Arg Lys Ala Val Ser Arg Tyr Leu
        35                  40                  45

Arg Cys Leu Ser Gln
    50

<210> 295
<211> 146
<212> PRT
<213> Homo sapiens

<400> 295

Arg Cys Leu Arg Pro Cys Phe Asp Gln Thr Val Phe Leu Gln Arg Arg
1               5                   10                  15

Tyr Trp Ser Asn Tyr Val Asp Leu Glu Gln Lys Leu Phe Asp Glu Thr
            20                  25                  30

Cys Cys Glu His Ala Arg Asp Phe Ala His Arg Cys Val Leu His Phe
        35                  40                  45

Phe Ala Ser Met Arg Ser Glu Leu Gln Ala Arg Gly Leu Arg Arg Gly
    50                  55                  60

Asn Ala Gly Arg Arg Leu Glu Leu Pro Ala Val Pro Glu Pro Pro Glu
65                  70                  75                  80

Gly Leu Asp Ser Gly Ser Gly Lys Ala His Leu Arg Ala Ile Ser Ser
                85                  90                  95

Arg Glu Gln Val Asp Arg Leu Leu Ser Thr Trp Tyr Ser Ser Lys Pro
            100                 105                 110

Pro Leu Asp Leu Ala Ala Ser Pro Gly Leu Cys Gly Gly Gly Leu Ser
        115                 120                 125

His Arg Ala Pro Thr Leu Ala Leu Gly Thr Arg Leu Ser Gln His Thr
    130                 135                 140

Asp Val
145

<210> 296
<211> 1035
<212> PRT
<213> Homo sapiens

<400> 296

Met Pro Cys Gly Phe Ser Pro Ser Pro Val Ala His His Leu Val Pro
1               5                   10                  15

Gly Pro Pro Asp Thr Pro Ala Gln Gln Leu Arg Cys Gly Trp Thr Val
            20                  25                  30

Gly Gly Trp Leu Leu Ser Leu Val Arg Gly Leu Leu Pro Cys Leu Pro
        35                  40                  45

Pro Gly Ala Arg Thr Ala Glu Gly Pro Ile Met Val Leu Ala Gly Pro
    50                  55                  60

Leu Ala Val Ser Leu Leu Leu Pro Ser Leu Thr Leu Leu Val Ser His
65                  70                  75                  80

Leu Ser Ser Ser Gln Asp Val Ser Ser Glu Pro Ser Ser Glu Gln Gln
                85                  90                  95

Leu Cys Ala Leu Ser Lys His Pro Thr Val Ala Phe Glu Asp Leu Gln
            100                 105                 110

Pro Trp Val Ser Asn Phe Thr Tyr Pro Gly Ala Arg Asp Phe Ser Gln
        115                 120                 125

Leu Ala Leu Asp Pro Ser Gly Asn Gln Leu Ile Val Gly Ala Arg Asn
    130                 135                 140

Tyr Leu Phe Arg Leu Ser Leu Ala Asn Val Ser Leu Leu Gln Ala Thr
145                 150                 155                 160

Glu Trp Ala Ser Ser Glu Asp Thr Arg Arg Ser Cys Gln Ser Lys Gly
                165                 170                 175

Lys Thr Glu Glu Glu Cys Gln Asn Tyr Val Arg Val Leu Ile Val Ala
            180                 185                 190

Gly Arg Lys Val Phe Met Cys Gly Thr Asn Ala Phe Ser Pro Met Cys
        195                 200                 205

Thr Ser Arg Gln Val Gly Asn Leu Ser Arg Thr Ile Glu Lys Ile Asn
    210                 215                 220

Gly Val Ala Arg Cys Pro Tyr Asp Pro Arg His Asn Ser Thr Ala Val
225                 230                 235                 240

Ile Ser Ser Gln Gly Glu Leu Tyr Ala Ala Thr Val Ile Asp Phe Ser
                245                 250                 255

Gly Arg Asp Pro Ala Ile Tyr Arg Ser Leu Gly Ser Gly Pro Pro Leu
            260                 265                 270

Arg Thr Ala Gln Tyr Asn Ser Lys Trp Leu Asn Glu Pro Asn Phe Val
        275                 280                 285

Ala Ala Tyr Asp Ile Gly Leu Phe Ala Tyr Phe Phe Leu Arg Glu Asn
    290                 295                 300

Ala Val Glu His Asp Cys Gly Arg Thr Val Tyr Ser Arg Val Ala Arg
305                 310                 315                 320

Val Cys Lys Asn Asp Val Gly Gly Arg Phe Leu Leu Glu Asp Thr Trp
                325                 330                 335

Thr Thr Phe Met Lys Ala Arg Leu Asn Cys Ser Arg Pro Gly Glu Val
            340                 345                 350

Pro Phe Tyr Tyr Asn Glu Leu Gln Ser Ala Phe His Leu Pro Glu Gln
        355                 360                 365

Asp Leu Ile Tyr Gly Val Phe Thr Thr Asn Val Asn Ser Ile Ala Ala
    370                 375                 380

Ser Ala Val Cys Ala Phe Asn Leu Ser Ala Ile Ser Gln Ala Phe Asn
385                 390                 395                 400

Gly Pro Phe Arg Tyr Gln Glu Asn Pro Arg Ala Ala Trp Leu Pro Ile
                405                 410                 415

Ala Asn Pro Ile Pro Asn Phe Gln Cys Gly Thr Leu Pro Glu Thr Gly
            420                 425                 430

Pro Asn Glu Asn Leu Thr Glu Arg Ser Leu Gln Asp Ala Gln Arg Leu
        435                 440                 445

Phe Leu Met Ser Glu Ala Val Gln Pro Val Thr Pro Glu Pro Cys Val
    450                 455                 460

Thr Gln Asp Ser Val Arg Phe Ser His Leu Val Val Asp Leu Val Gln
465                 470                 475                 480

Ala Lys Asp Thr Leu Tyr His Val Leu Tyr Ile Gly Thr Glu Ser Gly
                485                 490                 495

Thr Ile Leu Lys Ala Leu Ser Thr Ala Ser Arg Ser Leu His Gly Cys
            500                 505                 510

Tyr Leu Glu Glu Leu His Val Leu Pro Pro Gly Arg Arg Glu Pro Leu
        515                 520                 525

Arg Ser Leu Arg Ile Leu His Ser Ala Arg Ala Leu Phe Val Gly Leu
    530                 535                 540

Arg Asp Gly Val Leu Arg Val Pro Leu Glu Arg Cys Ala Ala Tyr Arg
545                 550                 555                 560

Ser Gln Gly Ala Cys Leu Gly Ala Arg Asp Pro Tyr Cys Gly Trp Asp
                565                 570                 575

Gly Lys Gln Gln Arg Cys Ser Thr Leu Glu Asp Ser Ser Asn Met Ser
            580                 585                 590

Leu Trp Thr Gln Asn Ile Thr Ala Cys Pro Val Arg Asn Val Thr Arg
        595                 600                 605

Asp Gly Gly Phe Gly Pro Trp Ser Pro Trp Gln Pro Cys Glu His Leu
    610                 615                 620

Asp Gly Asp Asn Ser Gly Ser Cys Leu Cys Arg Ala Arg Ser Cys Asp
625                 630                 635                 640

Ser Pro Arg Pro Arg Cys Gly Gly Leu Asp Cys Leu Gly Pro Ala Ile
                645                 650                 655

His Ile Ala Asn Cys Ser Arg Asn Gly Ala Trp Thr Pro Trp Ser Ser
            660                 665                 670

Trp Ala Leu Cys Ser Thr Ser Cys Gly Ile Gly Phe Gln Val Arg Gln
        675                 680                 685

Arg Ser Cys Ser Asn Pro Ala Pro Arg His Gly Gly Arg Ile Cys Val
    690                 695                 700

Gly Lys Ser Arg Glu Glu Arg Phe Cys Asn Glu Asn Thr Pro Cys Pro
705                 710                 715                 720

Val Pro Ile Phe Trp Ala Ser Trp Gly Ser Trp Ser Lys Cys Ser Ser
                725                 730                 735

Asn Cys Gly Gly Gly Met Gln Ser Arg Arg Arg Ala Cys Glu Asn Gly
            740                 745                 750

Asn Ser Cys Leu Gly Cys Gly Val Glu Phe Lys Thr Cys Asn Pro Glu
        755                 760                 765

Gly Cys Pro Glu Val Arg Arg Asn Thr Pro Trp Thr Pro Trp Leu Pro
    770                 775                 780

Val Asn Val Thr Gln Gly Gly Ala Arg Gln Glu Gln Arg Phe Arg Phe
785                 790                 795                 800

Thr Cys Arg Ala Pro Leu Ala Asp Pro His Gly Leu Gln Phe Gly Arg
                805                 810                 815

Arg Arg Thr Glu Thr Arg Thr Cys Pro Ala Asp Gly Ser Gly Ser Cys
            820                 825                 830

Asp Thr Asp Ala Leu Val Glu Val Leu Leu Arg Ser Gly Ser Thr Ser
        835                 840                 845

Pro His Thr Val Ser Gly Gly Trp Ala Ala Trp Gly Pro Trp Ser Ser
    850                 855                 860

Cys Ser Arg Asp Cys Glu Leu Gly Phe Arg Val Arg Lys Arg Thr Cys
865                 870                 875                 880

Thr Asn Pro Glu Pro Arg Asn Gly Gly Leu Pro Cys Val Gly Asp Ala
                885                 890                 895

Ala Glu Tyr Gln Asp Cys Asn Pro Gln Ala Cys Pro Val Arg Gly Ala
            900                 905                 910

Trp Ser Cys Trp Thr Ser Trp Ser Pro Cys Ser Ala Ser Cys Gly Gly
        915                 920                 925

Gly His Tyr Gln Arg Thr Arg Ser Cys Thr Ser Pro Ala Pro Ser Pro
    930                 935                 940

Gly Glu Asp Ile Cys Leu Gly Leu His Thr Glu Glu Ala Leu Cys Ala
945                 950                 955                 960

Thr Gln Ala Cys Pro Glu Gly Trp Ser Pro Trp Ser Glu Trp Ser Lys
                965                 970                 975

Cys Thr Asp Asp Gly Ala Gln Ser Arg Ser Arg His Cys Glu Glu Leu
            980                 985                 990

Leu Pro Gly Ser Ser Ala Cys Ala  Gly Asn Ser Ser Gln  Ser Arg Pro
        995                 1000                1005

Cys Pro  Tyr Ser Glu Ile Pro  Val Ile Leu Pro Ala  Ser Ser Met
    1010                1015                1020

Glu Glu  Ala Thr Asp Cys Ala  Gly Phe Asn Leu Ile
    1025                1030                1035

<210>  297
<211>  16
<212>  PRT
<213>  Homo sapiens

<400>  297

Cys Pro Tyr Asp Pro Arg His Asn Ser Thr Ala Val Ile Ser Ser Gln
1               5                   10                  15

<210>  298
<211>  11
<212>  PRT
<213>  Homo sapiens

<400>  298

Cys Pro Glu Val Arg Arg Asn Thr Pro Trp Thr
1               5                   10

<210>  299
<211>  35
<212>  PRT
<213>  Homo sapiens

<400>  299

Glu Arg Val Trp Ser Asp Asp His Lys Asp Phe Asp Cys Asn Thr Arg
1               5                   10                  15

Gln Pro Gly Cys Ser Asn Val Cys Phe Asp Glu Phe Phe Pro Val Ser
            20                  25                  30

His Val Arg
        35

<210>  300
<211>  38
<212>  PRT
<213>  Homo sapiens

<400>  300

His Ser Phe Tyr Pro Lys Tyr Ile Leu Pro Pro Val Val Lys Cys His
1               5                   10                  15

Ala Asp Pro Cys Pro Asn Ile Val Asp Cys Phe Ile Ser Lys Pro Ser
            20                  25                  30

Glu Lys Asn Ile Phe Thr
        35

<210>  301
<211>  15
<212>  PRT
<213>  Homo sapiens

<400>  301

Cys Leu Pro Asp Arg Pro Arg Asp His Val Lys Lys Thr Ile Leu
1               5                   10                  15

<210>  302
<211>  13
<212>  PRT
<213>  Homo sapiens

<400>  302

Glu Arg Val Trp Ser Asp Asp His Lys Asp Phe Asp Cys
1               5                   10

<210>  303
<211>  38
<212>  PRT
<213>  Homo sapiens

<400>  303

Asn Asn Asp Val Ser Cys Asp His Pro Ser Asn Thr Val Pro Ser Gly

1               5                   10                  15

Ser Asn Gln Asp Leu Gly Ala Gly Ala Gly Glu Asp Ala Arg Ser Asp
            20                  25                  30

Asp Ser Ser Ser Arg Ile
        35

<210>  304
<211>  15
<212>  PRT
<213>  Homo sapiens

<400>  304

Cys Asp His Pro Ser Asn Thr Val Pro Ser Gly Ser Asn Gln Asp
1               5                   10                  15

<210>  305
<211>  12
<212>  PRT
<213>  Homo sapiens

<400>  305

Cys Val Pro His Ser Arg Ser Arg Gly Pro Asn Leu
1               5                   10

<210>  306
<211>  12
<212>  PRT
<213>  Homo sapiens

<400>  306

Cys Glu Leu Ser Gln Thr Pro His Pro His Ser Arg
1               5                   10

<210>  307
<211>  14
<212>  PRT
<213>  Homo sapiens

<400>  307

Cys Leu Asp Ser Ala Gly Asn Asn Ala Gly Ile Gln Trp Gly
1               5                   10

<210>  308
<211>  14
<212>  PRT
<213>  Homo sapiens

<400>  308

Cys Asn Arg Val Ser Lys Asn Pro Glu Met Leu Gln Thr Gly
1               5                   10

<210>  309
<211>  2115

<212>  DNA
<213>  Homo sapiens

<400>  309
atgcgtatat gttatgaatg ccaaaatgaa agaacattgt ggcgatgtgt ttcccaggat      60
ggggctgact acagtgtggg cgtgtgtgtc cctgattctt gtgctgaaga ggatgtgact     120
ctgatgtctc ggctggatac tttaagattc agaaatactt catttttggc cccttccctc     180
tttcttttta caataaattc ttcctccttg tctggtggga gtgtgaccag atgtgctgct     240
ggaaagatcc ccctggacac atttgctgcc gtatgtctgt tcatcacctt gctgggtctc     300
atcctccctc cggctggaac agtctgcgtg gcagctaggg aatgggggtc agcctgcagg     360
acatcgcggg aacacgggga acctctggcc acttacggga gtctgccact gagcgaggcg     420
gagagcaatg aacaaagaag cagaatccca cggacacact gccgggcaca tctcctcctg     480
tcagcagcct ccagcagagg aaaaaggttt ctaggagccg tggctcatgc tctggagtgc     540
ttttcttggc agaagaatgt gccagccatc tggactacaa aggcaccagg tggcacctgc     600
tctgcactga atggcattcg tgtcttgagt cttctttgga tcatctcggg acacaccagt     660
cagatgactg catggctgtc tttgggatgg aaagatggag ggcacgaaag gccactggtc     720
atgtctgggc catcagtggg aatcggagac accagagaag ccacgagtgg ttggttaagt     780
gcaagttcgt ttttaaagat gcatcagaat tcagacaaag gaataacccc caaaggcata     840
ctcagatact ttctcagtca cctggtaagg ttgcagcctc ttcacctgta ttcaatgtgc     900
ttgttggttg gactgttctc tcttgttccc tggggacctg tctgggaaat gcccaaattc     960
cactgggata actgccggca agcatggtgg acgaatctgc tgttgctaaa taactttgtg    1020
tcggtcaaga atgcgtgcaa tggctggacc tggtaccttg ccaatgactt ccagttccac    1080
ctcaccacac cagtgattat cttcatccat gtaaagagta cacagatcct catcctcctt    1140
ggggccatgc tgttcttggc atctttcaca gccactgctc tgatcacctt ggcatataaa    1200
cttcctgtcg tggctccatc agaaaccagg acttcccggg gagggctgct gaatgccagg    1260
ctgttcaccc tgtgcccttt ggttcatgga aaaagtgggt atgaaacttt tggtctggat    1320
gggaaagctg attgccttct tgcttccaaa cttctgaacc tttcaacctg cactggaaat    1380
gaacaagtgt gccctaaatg tacctttggg cttgctgatt attccaatgg acatctcagg    1440
gatttggatt ccctttgcca tgtccagatc aaacataaca ttttggctta tttccttgta    1500
tttttcagtg aagaggcgat tgtattgtat ttcgtggagt actacacaaa gccctactgc    1560
cgatttgggc cagttcttgt gggcctcttt ctgagcattt acatgcacca aaaccaccag    1620
gaaaacattc tcagaaccaa gctgcagctc tctaccaagc cctccaccgg accctgtggg    1680
cggcggctgt gggctgagtc ctctttgcgt gccacggagg atatggaggt atggaagcgg    1740
ctccaggctt tgctgtcggg ttcacaccct gttcctttaa aggtgacaaa tcgaacacac    1800
aggagagcca agcagataaa aggcttcaat ggaaaagaat cttctccagg tctggtgaac    1860
cgtgtgcttt cttgggacat ctggagtttc ctgtccagca tcagttatgc tcgctacttg    1920

gtgcatccga ttctgatcat cctttacaat ggccttcagg aaacacttat tcaccacact 1980

gacaccaaca tgttctatct tttctctgga caccgtgtgc tgaccttcgt cactgggctg 2040

gccctgacgc tgttcattga gaaaccatgt caggaactga agcagcacct gctgggccat 2100

gaatgttctg gttaa 2115

<210> 310
<211> 704
<212> PRT
<213> Homo sapiens

<400> 310

Met Arg Ile Cys Tyr Glu Cys Gln Asn Glu Arg Thr Leu Trp Arg Cys
1               5                   10                  15

Val Ser Gln Asp Gly Ala Asp Tyr Ser Val Gly Val Cys Val Pro Asp
            20                  25                  30

Ser Cys Ala Glu Glu Asp Val Thr Leu Met Ser Arg Leu Asp Thr Leu
        35                  40                  45

Arg Phe Arg Asn Thr Ser Phe Leu Ala Pro Ser Leu Phe Leu Phe Thr
    50                  55                  60

Ile Asn Ser Ser Ser Leu Ser Gly Gly Ser Val Thr Arg Cys Ala Ala
65                  70                  75                  80

Gly Lys Ile Pro Leu Asp Thr Phe Ala Ala Val Cys Leu Phe Ile Thr
                85                  90                  95

Leu Leu Gly Leu Ile Leu Pro Pro Ala Gly Thr Val Cys Val Ala Ala
            100                 105                 110

Arg Glu Trp Gly Ser Ala Cys Arg Thr Ser Arg Glu His Gly Glu Pro
        115                 120                 125

Leu Ala Thr Tyr Gly Ser Leu Pro Leu Ser Glu Ala Glu Ser Asn Glu
    130                 135                 140

Gln Arg Ser Arg Ile Pro Arg Thr His Cys Arg Ala His Leu Leu Leu
145                 150                 155                 160

Ser Ala Ala Ser Ser Arg Gly Lys Arg Phe Leu Gly Ala Val Ala His
                165                 170                 175

Ala Leu Glu Cys Phe Ser Trp Gln Lys Asn Val Pro Ala Ile Trp Thr
            180                 185                 190

Thr Lys Ala Pro Gly Gly Thr Cys Ser Ala Leu Asn Gly Ile Arg Val
        195                 200                 205

Leu Ser Leu Leu Trp Ile Ile Ser Gly His Thr Ser Gln Met Thr Ala
    210                 215                 220

Trp Leu Ser Leu Gly Trp Lys Asp Gly Gly His Glu Arg Pro Leu Val
225                 230                 235                 240

Met Ser Gly Pro Ser Val Gly Ile Gly Asp Thr Arg Glu Ala Thr Ser
                245                 250                 255

Gly Trp Leu Ser Ala Ser Ser Phe Leu Lys Met His Gln Asn Ser Asp
            260                 265                 270

Lys Gly Ile Thr Pro Lys Gly Ile Leu Arg Tyr Phe Leu Ser His Leu
        275                 280                 285

Val Arg Leu Gln Pro Leu His Leu Tyr Ser Met Cys Leu Leu Val Gly
    290                 295                 300

Leu Phe Ser Leu Val Pro Trp Gly Pro Val Trp Glu Met Pro Lys Phe
305                 310                 315                 320

His Trp Asp Asn Cys Arg Gln Ala Trp Trp Thr Asn Leu Leu Leu Leu
                325                 330                 335

Asn Asn Phe Val Ser Val Lys Asn Ala Cys Asn Gly Trp Thr Trp Tyr
            340                 345                 350

Leu Ala Asn Asp Phe Gln Phe His Leu Thr Thr Pro Val Ile Ile Phe
        355                 360                 365

Ile His Val Lys Ser Thr Gln Ile Leu Ile Leu Leu Gly Ala Met Leu
    370                 375                 380

Phe Leu Ala Ser Phe Thr Ala Thr Ala Leu Ile Thr Leu Ala Tyr Lys
385                 390                 395                 400

Leu Pro Val Val Ala Pro Ser Glu Thr Arg Thr Ser Arg Gly Gly Leu
                405                 410                 415

Leu Asn Ala Arg Leu Phe Thr Leu Cys Pro Leu Val His Gly Lys Ser
            420                 425                 430

Gly Tyr Glu Thr Phe Gly Leu Asp Gly Lys Ala Asp Cys Leu Leu Ala
        435                 440                 445

Ser Lys Leu Leu Asn Leu Ser Thr Cys Thr Gly Asn Glu Gln Val Cys
    450                 455                 460

Pro Lys Cys Thr Phe Gly Leu Ala Asp Tyr Ser Asn Gly His Leu Arg
465                 470                 475                 480

Asp Leu Asp Ser Leu Cys His Val Gln Ile Lys His Asn Ile Leu Ala
                485                 490                 495

Tyr Phe Leu Val Phe Phe Ser Glu Glu Ala Ile Val Leu Tyr Phe Val
            500                 505                 510

Glu Tyr Tyr Thr Lys Pro Tyr Cys Arg Phe Gly Pro Val Leu Val Gly
        515                 520                 525

Leu Phe Leu Ser Ile Tyr Met His Gln Asn His Gln Glu Asn Ile Leu
    530                 535                 540

Arg Thr Lys Leu Gln Leu Ser Thr Lys Pro Ser Thr Gly Pro Cys Gly
545                 550                 555                 560

Arg Arg Leu Trp Ala Glu Ser Ser Leu Arg Ala Thr Glu Asp Met Glu
                565                 570                 575

Val Trp Lys Arg Leu Gln Ala Leu Leu Ser Gly Ser His Pro Val Pro
            580                 585                 590

Leu Lys Val Thr Asn Arg Thr His Arg Arg Ala Lys Gln Ile Lys Gly
        595                 600                 605

Phe Asn Gly Lys Glu Ser Ser Pro Gly Leu Val Asn Arg Val Leu Ser
    610                 615                 620

Trp Asp Ile Trp Ser Phe Leu Ser Ser Ile Ser Tyr Ala Arg Tyr Leu
625                 630                 635                 640

Val His Pro Ile Leu Ile Ile Leu Tyr Asn Gly Leu Gln Glu Thr Leu
                645                 650                 655

Ile His His Thr Asp Thr Asn Met Phe Tyr Leu Phe Ser Gly His Arg
            660                 665                 670

Val Leu Thr Phe Val Thr Gly Leu Ala Leu Thr Leu Phe Ile Glu Lys
        675                 680                 685

Pro Cys Gln Glu Leu Lys Gln His Leu Leu Gly His Glu Cys Ser Gly
    690                 695                 700

<210>  311
<211>  21
<212>  DNA
<213>  künstliche Sequenz

<220>
<223>  Oligonukleotid

<400>  311
aaccgtgtgc tttcttggga c                                                21

<210> 312
<211> 19
<212> DNA
<213> künstliche Sequenz

<220>
<223> Oligonukleotid

<400> 312
acattcatgg cccagcagg 19

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfimktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen.

An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitäts-Antigene) Klassen II bzw. I präsentiert werden, während B-Lymphozyten zirkulierende Antikörpermoleküle produzieren, die direkt an unprozessierte Antigene binden.

Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effelctormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Åntigene (CTA). CTA und sie kodierende Gene (Cancer/Testis-Gene oder CTG) sind durch ihr charakteristisches Expressionsmuster definiert (Tureci et al, Mol. Med. Today 3:342-9, 1997). Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumore z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Auswertung humaner Protein- und Nukleinsäuredatenbanken im Hinblick auf potenzielle, auf der Zelloberfläche zugängliche, krebsspezifische Antigene. Die Definition der dafür notwendigen Filterkriterien zusammen mit einer Hochdurchsatz-Methodik zur Analyse möglichst aller Proteine bilden den zentralen Bestandteil der Erfindung. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Datamining ist ein bekanntes Verfahren zur Identifizierung von-Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das erfindungsgemäße Konzept beruht jedoch darauf, Datamining zur elektronischen Extraktion aller Gene, die für auf der Zelloberfläche zugängliche, krebsspezifische Antigene kodieren, zu nutzen und diese sodann auf ektope Expression in Tumoren zu evaluieren.

Somit betrifft die Erfindung in einem Aspekt eine Strategie zur Identifizierung von differentiell in Tumoren exprimierten Genen. Diese kombiniert Datamining von öffentlichen Sequenzbanken (*"in silico"*) mit darauffolgenden evaluierenden labor-experimentellen ("wet bench") Untersuchungen.

Eine kombinierte Strategie basierend auf unterschiedlichen bioinformatischen Skripten ermöglichte erfindungsgemäß die Identifizierung von Genen, die für auf der Zelloberfläche zugängliche, krebsspezifische Antigene kodieren. Die Identifizierung und Bereitstellung dieser Tumor-assoziierten Gene und der dadurch kodierten Genprodukte erfolgte erfindungsgemäß unabhängig von einer immunogenen Wirkung.

Die erfindungsgemäß identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe ausgewählt ist, bestehend aus SEQ ID NOs: 1, 5, 9, 13, 17, 21, 25,-29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169,173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls. In einer weiteren bevorzugten Ausführungsform umfasst ein erfindungsgemäß identifiziertes Tumor-assoziiertes Antigen eine Aminosäuresequenz, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.

Die vorliegende Erfindung betrifft allgemein die Verwendung von erfindungsgemäß identifizierten Tumor-assoziierten Antigenen oder von Teilen davon, von dafür kodierenden Nukleinsäuren oder von Nukleinsäuren, die gegen die kodierenden Nukleinsäuren gerichtet sind, oder von Antikörpern, die gegen die erfindungsgemäß identifizierten Tumor-assoziierten Antigene oder Teile davon gerichtet sind, für die Therapie und Diagnose. Diese Nutzung kann einzelne, aber auch Kombinationen von mehreren dieser Antigene, funktionalen Fragmente, Nukleinsäuren, Antikörper etc..betreffen, in einer Ausführungsform auch in Kombination mit anderen Tumor-assoziierten Genen und Antigenen für eine Diagnose, Therapie und Verlaufskontrolle.

Die Eigenschaft der erfindungsgemäß identifizierten Tumor-assoziierten Antigene, dass sie auf oder an der Zelloberfläche lokalisiert sind, qualifiziert sie als geeignete Ziele oder Mittel für die Therapie und Diagnose. Besonders geeignet hierfür ist ein Teil der erfindungsgemäß identifizierten Tumor-assoziierten Antigene, der dem nicht-Transmembrananteil, insbesondere dem extrazellulären Anteil der Antigene entspricht oder davon umfasst wird. Somit ist erfindungsgemäß ein Teil der erfindungsgemäß identifizierten Tumor-assoziierten Antigene, der dem nicht-Transmembrananteil der Antigene entspricht oder davon umfasst ist, oder ein entsprechender Teil der für die erfindungsgemäß identifizierten Antigene kodierenden Nukleinsäuren für eine Therapie oder Diagnose bevorzugt. Ähnlich ist die Verwendung von Antikörpern bevorzugt, die gegen einen Teil der erfindungsgemäß identifizierten Tumor-assoziierten Antigene gerichtet sind, der dem nicht-Transmembrananteil der Antigene entspricht oder davon umfasst ist.

Bevorzugte Erkrankungen für eine Therapie und/oder Diagnose sind solche, bei denen eine selektive Expression oder abnormale Expression von einem oder mehreren der erfindungsgemäß identifizierten Tumor-assoziierten Antigenen vorliegt.

Die Erfindung betrifft auch Nukleinsäuren und Genprodukte, die tumorzellassoziiert exprimiert werden und die durch verändertes Spleißen (Spleißvarianten) von Genen bzw. durch veränderte Translation unter Nutzung alternativer offener Leserahmen entstehen. Diese Nukleinsäuren umfassen die Sequenzen gemäß SEQ ID NOs: 1, 5, 9, 13, 17, 21, 25, 29, 33, 37, 41, 45, 49, 53, 57, 61, 65, 69, 73, 77, 81, 85, 89, 93, 97,101, 105, 109, 113, 117,121, 125, 129, 133, 137, 141, 145, 149,153, 157, 161, 165, 169,173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls. Ferner umfassen die Genprodukte alle Sequenzen gemäß SEQ ID NOs: 2, 6, 10, 14, 18, 22, 26, 30, 34, 38, 42, 46, 50, 54, 58, 62, 66, 70, 74, 78, 82, 86, 90, 94, 98,102, 106, 110, 114, 18,122, 126, 130, 134, 138, 142,146, 150, 154, 158, 162, 166, 170, 174,176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls. Die erfindungsgemäßen Spleißvarianten sind erfindungsgemäß als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Ausspleißen von einzelnen oder mehreren Exons,
- über Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvarianten-spezifischen Oligonukleotiden erfolgen.

Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Für die Immunisierung eignen sich besonders die Aminosäuren, die deutliche Epitopunterschiede zu der/den Variante(n) des Genprodukts aufweisen, welche bevorzugt in gesunden Zellen gebildet wird/werden. Der Nachweis der Tumorzellen mit Antikörper kann dabei an einer vom Patienten isolierten Probe oder als Imaging mit intravenös applizierten Antikörpern erfolgen.

Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferiert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo- oder Polypeptide, die die Spleißvarianten-spezifischen Epitope beinhalten, oder Nukleinsäuren, die für diese kodieren. Oligo- oder Polypeptide, die die Spleißvarianten-spezifischen Epitope beinhalten, oder Nukleinsäuren, die für diese Polypeptide kodieren, sind auch für die Nutzung als pharmazeutisch wirksame Substanzen bei der aktiven Immuntherapie (Vakzinierung, Vakzintherapie) verwendbar.

Die aberrante Expression von Genen in Tumorzellen kann auf veränderte Methylierungsmuster ihrer Promotoren beruhen (De Smet C et al., Mol Cell Biol. 24(11):4781-90, 2004; De Smet C et al., Mol Cell Biol. 19(11):7327-35, 1999; De Smet C et al., Proc Natl Acad Sci U S A. 93(14):7149-53, 1996) Diese Methylierungsunterschiede können als indirekter Marker für den im Tumor veränderten Zustand des entsprechenden Gens genutzt werden. Dementsprechend können die Zunahme oder Abnahme von Basenmethylierungen im Promotorbereich für diagnostische Zwecke genutzt werden.

In einem Aspekt betrifft die Erfindung eine pharmazeutische Zusammensetzung, umfassend ein Mittel, das das erfindungsgemäß identifizierte Tumor-assozüerte Antigen erkennt und vorzugsweise selektiv für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein erfindungsgemäß identifiziertes Tumor-assoziiertes Antigen ist. Die Erkennung muss nicht direkt mit einer Hemmung von Aktivität oder Expression des Antigens einhergehen. In diesem Aspekt der Erfindung dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zelle lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein Helfer-T-Lymphozyt, der Effektorfunktionen von anderen Zellen, die spezifisch dieses Antigen erkennen, stärkt.

In einem Aspekt betrifft die Erfindung eine pharmazeutische Zusammensetzung, umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein erfindungsgemäß identifiziertes Tumor-assoziiertes Antigen ist.

Die Aktivität eines erfindungsgemäß identifizierten Tumor-assoziierten Antigens kann eine jegliche Aktivität eines Proteins oder Peptids sein. Somit können die erfindungsgemäßen Therapie- und Diagnosverfahren auch auf Hemmung oder Reduktion dieser Aktivität oder auf ein Testen dieser Aktivität abzielen.

Des weiteren betrifft die Erfindung eine pharmazeutische Zusammensetzung, die ein Mittel umfasst, das bei einer Verabreichung selektiv die Menge an Komplexen zwischen einem HLA-Molekül und einem Peptidepitop aus dem erfindungsgemäß identifizierten Tumor-assoziierten Antigen erhöht. Das Mittel umfasst in einer Ausführungsform einen oder mehrere Bestandteile, die aus der Gruppe ausgewählt sind bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren betrifft die Erfindung eine pharmazeutische Zusammensetzung, die einen oder mehrere Bestandteile umfasst, die aus der Gruppe ausgewählt sind bestehend aus (i) einem erfindungsgemäß identifizierten Tumor-assoziierten Antigen oder einem Teil davon, (ii) einer Nukleinsäure, die für ein erfindungsgemäß identifiziertes Tumor-assoziiertes Antigen oder einen Teil davon kodiert, (iii) einem Antikörper, der an ein erfindungsgemäß identifiziertes Tumor-assozüertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfindungsgemäß identifiziertes Tumor-assoziiertes Antigen oder einen Teil davon exprimiert, und (vi) isolierten Komplexen zwischen einem erfindungsgemäß identifizierten Tumor-assoziierten Antigen oder einem Teil davon und einem HLA-Molekül.

Eine Nukleinsäure, die für ein erfindungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer erfindungsgemäßen pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer erfindungsgemäßen pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers oder ein synthetischer Antikörper, die durch kombinatorische Techniken hergestellt werden können. Der Antikörper kann mit einem therapeutisch oder diagnostisch nützlichen Mittel gekoppelt sein.

Eine in einer erfindungsgemäßen pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das erfindungsgemäß identifizierte Tumor-assoziierte Antigen kodiert, umfassen.

In weiteren Ausführungsformen bindet ein durch eine erfindungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Zytokinausschüttung induziert.

Eine erfindungsgemäße pharmazeutische Zusammensetzung kann einen pharmazeutisch verträglichen Träger und/oder ein Adjuvans umfassen. Das Adjuvans kann aus Saponin, GM-CSF, CpG-Oligonukleotiden, RNA, einem Zytokin oder einem Chemokin ausgewählt sein. Eine erfindungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren betrifft die Erfindung Verfahren zur Behandlung, Diagnose oder Überwachung, d.h. Bestimmung der Regression, des Verlaufs und/oder des Ausbruchs, einer Erkrankung, die sich durch die Expression oder abnormale Expression eines oder mehrerer Tumor-assoziierter Antigene auszeichnet.

In einer Ausführungsform umfassen die erfindungsgemäßen Behandlungsverfahren die Verabreichung einer erfindungsgemäßen pharmazeutischen Zusammensetzung.

Die erfindungsgemäßen Diagnoseverfahren und/oder Verfahren zur Überwachung betreffen allgemein die Verwendung von Mitteln für den Nachweis und/oder die Bestimmung bzw. Überwachung der Menge (i) einer Nukleinsäure, die für das Tumor-assoziierte Antigen kodiert, oder eines Teils davon und/oder (ii) des Tumor-assoziierten Antigens oder eines Teils davon, und/oder (iii) eines Antikörpers gegen das Tumor-assoziierte Antigen oder einen Teil davon und/oder (iv) von cytotoxischen oder Helfer-T-Lymphozyten, die für das Tumor-assoziierte Antigen oder einen Teil davon spezifisch sind, in einer aus einem Patienten isolierten biologischen Probe.

In einem Aspekt betrifft die Erfindung ein Verfahren zur Diagnose einer Erkrankung, die sich durch die Expression oder abnormale Expression eines erfindungsgemäß identifizierten Tumor-assoziierten Antigens auszeichnet. Das Verfahren umfasst (i) den Nachweis einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

Die erfindungsgemäßen Diagnoseverfahren können auch veränderte Methylierungsmuster des Promotorbereiches des jeweiligen Tumor-assoziierten Genproduktes nutzen. Der Nachweis solcher Methylierungsmuster kann unter Nutzung von PCR-basierenden Verfahren, mit Hilfe von Restriktionsenzymen oder durch Sequenzierung erfolgen. Ein dazu geeignetes Test kann dabei folgendermaßen aufgebaut sein: (1) Extraktion von DNA aus Gewebeproben von Patienten z.B. unter Nutzung von paraffineingebettetem Material, (2) Behandlung der DNA mit Bisulfit-haltigen Reagenzien (z.B. wie beschrieben in Clark SJ et al., Nucleic Acids Res. 22(15):2990-7, 1994), (3) Amplifikation von DNA mit PCR, und (4) Analyse durch Bestimmung der Menge sequenzspezifischer Amplifikationsprodukte (z.B. durch quantitative PCR, Hybridisierungsverfahren wie Microarrayverfahren).

Die erfindungsgemäßen Diagnoseverfahren können auch eine Nutzung der erfindungsgemäß identifizierten Tumor-assoziierten Antigene als prognostische Marker betreffen, um eine Metastatisierung z.B. durch Testen des Migrationsverhalten von Zellen und daher einen verschlechterten Krankheitsverlauf zu prädizieren, wodurch unter anderem die Planung einer aggressiveren Therapie ermöglicht wird.

In einem weiteren Aspekt betrifft die Erfindung ein Verfahren zur Bestimmung der Regression, des Verlaufs oder des Ausbruchs einer Erkrankung, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (üi) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Bestimmung bzw. Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In bestimmten Ausführungsformen der erfindungsgemäßen Diagnoseverfahren erfolgt eine selektive Amplifikation des Promotorbereichs oder eines Teils davon einer Nukleinsäure, die für ein erfindungsgemäß identifiziertes Tumor-assoziiertes Antigen kodiert und in Form von genomischer DNA vorliegt, nach Behandlung mit einem bisulfithaltigen Reagenz. Die Nukleinsäure wird vorzugsweise vor einer Behandlung mit dem bisulfithaltigen Reagenz aus einer Probe eines zu untersuchenden Patienten isoliert. Die bei einer solchen Amplifikation verwendeten Oligonukleotide weisen vorzugsweise eine Sequenz auf, die an die mit dem bisulfithaltigen Reagenz behandelte Nukleinsäure binden, vorzugsweise dazu vollständig komplementär sind. Vorzugsweise sind die Oligonukleotide einem unterschiedlichen Grad einer Methylierung der Nukleinsäure angepaßt und bedingen differenzierbare Amplifikationsprodukte.

Ein Nachweis eines Tumor-assoziierten Antigens oder eines Teils davon oder eine Bestimmung bzw. Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In bestimmten Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Bestimmung bzw. Überwachung der Menge an Antikörpern kann erfindungsgemäß mit einem Protein oder Peptid erfolgen, das spezifisch an den Antikörper bindet.

Ein Nachweis von cytolytischen T-Zellen oder Helfer-T-Zellen oder die Bestimmung bzw. Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Bestimmung bzw. Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, der spezifische T-Lymphozyten identifizieren kann.

In einem weiteren Aspekt betrifft die Erfindung ein Verfahren zur Behandlung, Diagnose oder Überwachung einer Erkrankung, die sich durch die Expression oder abnormale Expression eines erfindungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Die Erfindung betrifft auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiven Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumor-assoziierte Antigen oder einen Teil davon exprimieren. Die Erfindung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die Erfindung ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat) und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die Erfindung betrifft auch ein Verfahren zur Behandlung einer Erkrankung, die sich durch die Expression oder abnormale Expression eines erfindungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die erfindungsgemäß verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegende Erfindung eine Nukleinsäure, die aus der Gruppe ausgewählt ist bestehend aus (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137,141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. Des weiteren betrifft die Erfindung eine Nukleinsäure, die für ein Protein oder Polypeptid kodiert, das eine Aminosäuresequenz umfasst, ausgewählt aus der Gruppe, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls,einem Teil oder Derivat davon.

In einem weiteren Aspekt betrifft die Erfindung Promotorsequenzen von erfindungsgemäßen Nukleinsäuren. Diese können funktionell mit einem anderen Gen vorzugsweise in einem Expressionsvektor verbunden werden, und somit die selektive Expression dieses Gens in entsprechenden Zellen gewährleisten.

In einem weiteren Aspekt betrifft die Erfindung ein rekombinantes Nukleinsäuremolekül, insbesondere DNA- oder RNA-Molekül, das eine erfindungsgemäße Nukleinsäure umfasst.

Die Erfindung betrifft auch Wirtszellen, die eine erfindungsgemäße Nukleinsäure oder ein rekombinantes Nukleinsäuremolekül, das eine erfindungsgemäße Nuldeinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die erfindungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die Erfindung Oligonukleotide, die mit einer erfindungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southem- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Erfindung ein Protein oder Polypeptid, das von einer Nukleinsäure kodiert wird, die aus der Gruppe ausgewählt ist bestehend aus (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153,157,161,165,169,173,175,179,183,187,191,195,199,203,207, 211,215,219,223, 227, 231, 235, 239, 243, 247, 251, 255, 259, 263, 267, 269, 271, 273, 275, 277, 279, 309 des Sequenzprotokolls, einem Teil oder Derivat davon, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform betrifft die Erfindung ein Protein oder Polypeptid, das eine Aminosäuresequenz umfasst, ausgewählt aus der Gruppe, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.

In einem weiteren Aspekt betrifft die Erfindung ein immunogenes Fragment eines erfindungsgemäß identifizierten Tumor-assoziierten Antigens. Das Fragment bindet vorzugsweise an einen menschlichen HLA-Rezeptor oder menschlichen Antikörper. Vorzugsweise umfasst ein erfindungsgemäßes Fragment eine Sequenz von mindestens 6, insbesondere mindestens 8, mindestens 10, mindestens 12, mindestens 15, mindestens 20, mindestens 30 oder mindestens 50 Aminosäuren.

In einem weiteren Aspekt betrifft die Erfindung ein Mittel, das an ein erfindungsgemäß identifiziertes Tumor-assoziiertes Antigen oder an einen Teil davon bindet. In einer bevorzugten Ausführungsform ist das Mittel ein Antikörper. In weiteren Ausfühnzngsformen ist der Antikörper ein chimärer, ein humanisierter oder mit kombinatorischen Techniken hergestellte Antikörper oder ein Fragment eines Antikörpers. Des weiteren betrifft die Erfindung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem therapeutischen oder diagnostischen Mittel. In einer Ausführungsform ist das therapeutische oder diagnostische Mittel ein Toxin.

In einem weiteren Aspekt betrifft die Erfindung einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

Die Erfindung betrifft insbesondere folgendes:
1. Pharmazeutische Zusammensetzung, umfassend ein Mittel, das die Expression oder Aktivität eines Tumor-assoziierten Antigens hemmt, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 2613, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
2. Pharmazeutische Zusammensetzung, umfassend ein Mittel mit tumorhemmender Aktivität, das selektiv ist für Zellen, die eine Expression oder abnormale Expression eines tumorassoziierten Antigens aufweisen, wobei das Tumor-assoziierte Antigen eine Sequenz aufweist, die von einer Nuldeinsäure kodiert wird, die aus der Gruppe ausgewählt ist, bestehend aus
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
3. Pharmazeutische Zusammensetzung nach Ziffer 2, wobei das Mittel die Induktion des Zelltods, die Reduktion des Zellwachstums, eine Schädigung der Zellmembran oder eine Sekretion von Zytokinen bewirkt.
4. Pharmazeutische Zusammensetzung nach Ziffer 1 oder 2, wobei das Mittel eine Antisense-Nukleinsäure ist, die selektiv mit der Nukleinsäure hybridisiert, die für das Tumor-assoziierte Antigen kodiert.
5. Pharmazeutische Zusammensetzung nach Ziffer 1 oder 2, wobei das Mittel ein Antikörper ist, der selektiv an das Tumor-assoziierte Antigen bindet.
6. Pharmazeutische Zusammensetzung nach Ziffer 2, wobei das Mittel ein komplementaktivierender Antikörper ist, der selektiv an das Tumor-assoziierte Antigen bindet.
7. Pharmazeutische Zusammensetzung, umfassend ein Mittel, das bei einer Verabreichung selektiv die Menge an Komplexen zwischen einem HLA-Molekül und einem Tumor-assoziierten Antigen oder einem Teil davon erhöht, wobei das Tumor-assoziierte Antigen eine Sequenz aufweist, die von einer Nukleinsäure kodiert wird, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
8. Pharmazeutische Zusammensetzung nach Ziffer 7, wobei das Mittel einen oder mehrere Bestandteile umfasst, die aus der Gruppe ausgewählt sind bestehend aus:
   (i) dem Tumor-assoziierten Antigen oder einem Teil davon,
   (ii) einer Nukleinsäure, die für das Tumor-assoziierte Antigen oder einen Teil davon kodiert,
   (iii) einer Wirtszelle, die das Tumor-assoziierte Antigen oder einen Teil davon exprimiert, und
   (iv) isolierten Komplexen zwischen dem Tumor-assoziierten Antigen oder einem Teil davon und einem HLA-Molekül.
9. Pharmazeutische Zusammensetzung nach Ziffer 1, 2 oder 7, wobei das Mittel mehrere Mittel umfasst, die jeweils selektiv die Expression oder Aktivität verschiedener Tumor-assoziierter Antigene hemmen, jeweils selektiv für Zellen sind, die verschiedene Tumor-assoziierte Antigene exprimieren oder die Menge an Komplexen zwischen HLA-Molekülen und verschiedenen Tumor-assoziierten Antigenen oder Teilen davon erhöhen, wobei mindestens eines der Tumor-assoziierten Antigene eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
10. Pharmazeutische Zusammensetzung umfassend einen oder mehrer Bestandteile, die aus der Gruppe ausgewählt sind bestehend aus:
   (i) einem Tumor-assoziierten Antigen oder einem Teil davon,
   (ii) einer Nukleinsäure, die für ein Tumor-assoziiertes Antigen oder einen Teil davon kodiert,
   (iii) einem Antikörper, der an ein Tumor-assoziiertes Antigen oder einen Teil davon bindet,
   (iv) einer Antisense-Nukleinsäure, die spezifisch mit einer Nukleinsäure, die für ein Tumor-assoziiertes Antigen kodiert, hybridisiert,
   (v) einer Wirtszelle, die ein Tumor-assoziiertes Antigen oder einen Teil davon exprimiert, und
   (vi) isolierten Komplexen zwischen einem Tumor-assoziierten Antigen oder einem Teil davon und einem HLA-Molekül,
   wobei das Tumor-assoziierte Antigen eine Sequenz aufweist, die von einer Nukleinsäure kodiert wird, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
11. Pharmazeutische Zusammensetzung nach Ziffer 8 oder 10, wobei die Nukleinsäure unter (ii) in einem Expressionsvektor vorliegt.
12. Pharmazeutische Zusammensetzung nach Ziffer 8 oder 10, wobei die Nukleinsäure unter (ii) funktionell mit einem Promotor verbunden ist.
13. Pharmazeutische Zusammensetzung nach Ziffer 8 oder 10, wobei die Wirtszelle das Tumor-assoziierte Antigen oder den Teil davon sekretiert.
14. Pharmazeutische Zusammensetzung nach Ziffer 8 oder 10, wobei die Wirtszelle zusätzlich ein HLA-Molekül exprimiert, das an das Tumor-assoziierte Antigen oder den Teil davon bindet.
15. Pharmazeutische Zusammensetzung nach Ziffer 14, wobei die Wirtszelle das HLA-Molekül und/oder das Tumor-assoziierte Antigen oder den Teil davon rekombinant exprimiert.
16. Pharmazeutische Zusammensetzung nach Ziffer 14, wobei die Wirtszelle das HLA-Molekül endogen exprimiert.
17. Pharmazeutische Zusammensetzung nach Ziffer 8, 10, 14 oder 16, wobei die Wirtszelle eine Antigen-präsentierende Zelle ist.
18. Pharmazeutische Zusammensetzung nach Ziffer 17, wobei die Antigen-präsentierende Zelle eine dendritische Zelle, ein Monozyt oder Makrophage ist.
19. Pharmazeutische Zusammensetzung nach einer der Ziffern 8, 10 und 13-18, wobei die Wirtszelle nicht-proliferativ ist.
20. Pharmazeutische Zusammensetzung nach Ziffer 5 oder 10, wobei der Antikörper ein monoklonaler Antikörper ist.
21. Pharmazeutische Zusammensetzung nach Ziffer 5 oder 10, wobei der Antikörper ein chimärer oder humanisierter Antikörper ist.
22. Pharmazeutische Zusammensetzung nach Ziffer 5 oder 10, wobei der Antikörper ein Fragment eines natürlichen Antikörpers ist.
23. Pharmazeutische Zusammensetzung nach Ziffer 5 oder 10, wobei der Antikörper mit einem therapeutischen oder diagnostischen Mittel gekoppelt ist.
24. Pharmazeutische Zusammensetzung nach Ziffer 4 oder 10, wobei die Antisense-Nukleinsäure eine Sequenz von 6-50 zusammenhängenden Nukleotiden aus der Nukleinsäure, die für das Tumor-assoziierte Antigen kodiert, umfasst.
25. Pharmazeutische Zusammensetzung nach einer der Ziffern 8 und 10-13, wobei das durch die pharmazeutische Zusammensetzung bereitgestellte Tumor-assoziierte Antigen oder der Teil davon an MHC-Moleküle auf der Oberfläche von Zellen bindet, die eine abnormale Menge des Tumor-assoziierten Antigens oder eines Teils davon exprimieren.
26. Pharmazeutische Zusammensetzung nach Ziffer 25, wobei die Bindung eine cytolytische Reaktion hervorruft und/ oder eine Zytokinausschüttung induziert
27. Pharmazeutische Zusammensetzung nach einer der Ziffern 1-26, ferner umfassend einen pharmazeutisch verträglichen Träger und/oder ein Adjuvans.
28. Pharmazeutische Zusammensetzung nach Ziffer 27, wobei das Adjuvans Saponin, GM-CSF, ein CpG-Oligonukleotid, Zytokin oder Chemokin ist.
29. Pharmazeutische Zusammensetzung nach einer der Ziffern 1-28, die zur Behandlung einer Erkrankung eingesetzt werden kann, die sich durch die Expression oder abnormale Expression eines Tumor-assoziierten Antigens auszeichnet.
30. Pharmazeutische Zusammensetzung nach Ziffer 29, wobei die Erkrankung Krebs ist.
31. Pharmazeutische Zusammensetzung nach Ziffer 29, wobei die Erkrankung ein Colon-, Rektal-, Nieren-, Brust-, Prostata-, Gebärmutter-, Ovarial-, Endometrial-, Speiseröhren-, Blut-, Leber-, Pankreas-, Haut-, Gehirn- oder Lungenkrebs, ein Lymphom oder Neuroblastom, ein Lungen-, Brust-, Prostata-, Colontumor, Nierenzell-, Zervix-, Colon- oder Mammakarzinom oder Metastasen der vorstehenden Krebsarten oder Tumore ist.
32. Pharmazeutische Zusammensetzung nach einer der Ziffern 1-31, wobei das Tumor-assoziierte Antigen eine Aminosäuresequenz umfasst, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.
33. Verfahren zur Diagnose einer Erkrankung, die sich durch die Expression oder abnormale Expression eines Tumor-assoziierten Antigens auszeichnet, umfassend
   (i) den Nachweis einer Nukleinsäure, die für das Tumor-assoziierte Antigen kodiert, oder eines Teils davon, und/oder
   (ii) den Nachweis des Tumor-assoziierten Antigens oder eines Teils davon, und/oder
   (iii) den Nachweis eines Antikörpers gegen das Tumor-assoziierte Antigen oder eines Teils davon und/oder
   (iv) den Nachweis von cytotoxischen oder Helfer-T-Lymphozyten, die für das Tumor-assoziierte Antigen oder einen Teil davon spezifisch sind, in einer aus einem Patienten isolierten biologischen Probe, wobei
   das Tumor-assoziierte Antigen eine Sequenz aufweist, die von einer Nukleinsäure kodiert wird, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
34. Verfahren nach Ziffer 33, wobei der Nachweis
   (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an die cytotoxischen oder Helfer-T-Lymphozyten bindet, und
   (ii) den Nachweis der Komplexbildung zwischen dem Mittel und der Nuldeinsäure oder dem Teil davon, dem Tumor-assoziierten Antigen oder dem Teil davon, dem Antikörper oder den cytotoxischen oder Helfer-T-Lymphozyten umfasst.
35. Verfahren nach Ziffer 33 oder 34, wobei der Nachweis mit dem Nachweis in einer vergleichbaren normalen biologischen Probe verglichen wird.
36. Verfahren nach einer der Ziffern 33-35, wobei sich die Erkrankung durch die Expression oder abnormale Expression mehrerer verschiedener Tumor-assoziierter Antigene auszeichnet und der Nachweis einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind, umfasst.
37. Verfahren nach einer der Ziffern 33-36, wobei der Nachweis der Nukleinsäure oder des Teils davon mit einer Polynukleotid-Sonde erfolgt, die spezifisch mit der Nukleinsäure oder dem Teil davon hybridisiert.
38. Verfahren nach Ziffer 37, wobei die Polynukleotid-Sonde eine Sequenz von 6-50 zusammenhängenden Nukleotiden aus der Nukleinsäure, die für das Tumor-assoziierte Antigen kodiert, umfasst.
39. Verfahren nach einer der Ziffern 33-36, wobei der Nachweis der Nukleinsäure oder des Teils davon durch selektive Amplifikation der Nukleinsäure oder des Teils davon erfolgt.
40. Verfahren nach einer der Ziffern 33-36, wobei das nachzuweisende Tumor-assoziierte Antigen oder der Teil davon in einem Komplex mit einem MHC-Molekül vorliegt.
41. Verfahren nach Ziffer 40, wobei das MHC-Molekül ein HLA-Molekül ist.
42. Verfahren nach einer der Ziffern 33-36 und 40-41, wobei der Nachweis des Tumor-assoziierten Antigens oder des Teils davon mit einem Antikörper erfolgt, der spezifisch an das Tumor-assoziierte Antigen oder den Teil davon bindet.
43. Verfahren nach einer der Ziffern 33-36, wobei der Nachweis des Antikörpers mit einem Protein oder Peptid erfolgt, das spezifisch an den Antikörper bindet.
44. Verfahren zur Bestimmung der Regression, des Verlaufs oder des Ausbruchs einer Erkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe bestehend aus:
   (i) der Menge der Nukleinsäure, die für das Tumor-assoziierte Antigen kodiert, oder eines Teils davon,
   (ii) der Menge des Tumor-assoziierten Antigens oder eines Teils davon,
   (iii) der Menge an Antikörpern, die an das Tumor-assoziierte Antigen oder einen Teil davon binden, und
   (iv) der Menge an cytolytischen oder Zytokin-ausschüttenden T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist bestehend aus:
      (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist, und
      (d) einer Nukleinsäure, die zu der Nukleinsäure unter (a), (b) oder (c) komplementär ist.
45. Verfahren nach Ziffer 44, wobei das Verfahren die Bestimmung des oder der Parameter zu einem ersten Zeitpunkt in einer ersten Probe und zu einem zweiten Zeitpunkt in einer weiteren Probe umfasst und durch einen Vergleich der beiden Proben der Verlauf der Erkrankung ermittelt wird.
46. Verfahren nach Ziffer 44 oder 45, wobei die Erkrankung sich durch die Expression oder abnormale Expression mehrerer verschiedener Tumor-assoziierter Antigene auszeichnet und die Überwachung eine Überwachung
   (i) der Menge mehrerer Nukleinsäuren, die für die mehreren verschiedenen Tumor-assoziierten Antigene kodieren, oder von Teilen davon,
   (ii) der Menge der mehreren verschiedenen Tumor-assoziierten Antigene oder von Teilen davon,
   (iii) der Menge mehrerer Antikörper, die an die mehreren verschiedenen Tumor-assoziierten Antigene oder an Teile davon binden, und/oder
   (iv) der Menge mehrerer cytolytischer oder Zytokine-ausschüttender T-Zellen, die für Komplexe zwischen den mehreren verschiedenen Tumor-assoziierten Antigenen oder von Teilen davon und MHC-Molekülen spezifisch sind, umfasst.
47. Verfahren nach einer der Ziffern 44-46, wobei die Überwachung der Menge der Nukleinsäure oder des Teils davon mit einer Polynuldeotid-Sonde erfolgt, die spezifisch mit der Nukleinsäure oder dem Teil davon hybridisiert.
48. Verfahren nach Ziffer 47, wobei die Polynukleotid-Sonde eine Sequenz von 6-50 zusammenhängenden Nukleotiden aus der Nukleinsäure, die für das Tumor-assoziierte Antigen kodiert, umfasst.
49. Verfahren nach einer der Ziffern 44-46, wobei die Überwachung der Menge der Nukleinsäure oder des Teils davon durch selektive Amplifikation der Nukleinsäure oder des Teils davon erfolgt.
50. Verfahren nach einer der Ziffern 44-46, wobei die Überwachung der Menge des Tumor-assoziierten Antigens oder des Teils davon mit einem Antikörper erfolgt, der spezifisch an das Tumor-assoziierte Antigen oder den Teil davon bindet.
51. Verfahren nach einer der Ziffern 44-46, wobei die Überwachung der Menge an Antikörpern mit einem Protein oder Peptid erfolgt, das spezifisch an den Antikörper bindet.
52. Verfahren nach einer der Ziffern 44-46, wobei die Überwachung der Menge an cytolytischen oder Zytokin-aussschüttenden T-Zellen mit einer Zelle erfolgt, die den Komplex zwischen dem Tumor-assoziierten Antigen oder dem Teil davon und einem MHC-Molekül präsentiert.
53. Verfahren nach einer der Ziffern 37-38, 42-43, 47-48 und 50-52, wobei die Polynukleotid-Sonde, der Antikörper, das Protein oder Peptid oder die Zelle nachweisbar markiert sind.
54. Verfahren nach Ziffer 53, wobei der nachweisbare Marker ein radioaktiver Marker oder ein Enzymmarker ist.
55. Verfahren nach einer der Ziffern 33-54, wobei die Probe Körperflüssigkeit und/oder Körpergewebe umfasst.
56. Verfahren zur Behandlung einer Erkrankung, die sich durch die Expression oder abnormale Expression eines Tumor-assoziierten Antigens auszeichnet, umfassend die Verabreichung einer pharmazeutischen Zusammensetzung nach einer der Ziffern 1-32, wobei das Tumor-assoziierte Antigen eine Sequenz aufweist, die von einer Nukleinsäure kodiert wird, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
57. Verfahren zur Behandlung, Diagnose oder Überwachung einer Erkrankung, die sich durch die Expression oder abnormale Expression eines Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist, wobei das Tumor-assoziierte Antigen eine Sequenz aufweist, die von einer Nukleinsäure kodiert wird, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 2S, 29, 33, 37, 41, 45, 49, 53, 57, 61, 65, 69, 73, 77, 81, 8S, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
58. Verfahren nach Ziffer 42, 50 oder 57, wobei der Antikörper ein monoklonaler Antikörper ist.
59. Verfahren nach Ziffer 42, 50 oder 57, wobei der Antikörper ein chimärer oder humanisierter Antikörper ist.
60. Verfahren nach Ziffer 42, 50 oder 57, wobei der Antikörper ein Fragment eines natürlichen Antikörpers ist.
61. Verfahren zur Behandlung eines Patienten mit einer Erkrankung, die sich durch die Expression oder abnormale Expression eines Tumor-assoziierten Antigens auszeichnet, umfassend:
   (i) die Entfernung einer Probe mit immunreaktiver Zellen aus dem Patienten,
   (ii) die Kontaktierung der Probe mit einer Wirtszelle, die das Tumor-assoziierte Antigen oder einen Teil davon exprimiert, unter Bedingungen, die eine Produktion cytolytischer oder Zytokine-ausschüttender T-Zellen gegen das Tumor-assoziierte Antigen oder einen Teil davon begünstigen, und
   (iii) das Einbringen der cytolytischen oder Zytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist bestehend aus:
      (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist, und
      (d) einer Nukleinsäure, die zu der Nukleinsäure unter (a), (b) oder (c) komplementär ist.
62. Verfahren nach Ziffer 61, wobei die Wirtszelle ein HLA-Molekül rekombinant exprimiert, das an das Tumor-assoziierte Antigen oder einen Teil davon bindet.
63. Verfahren nach Ziffer 62, wobei die Wirtszelle ein HLA-Molekül endogen exprimiert, das an das Tumor-assoziierte Antigen oder einen Teil davon bindet.
64. Verfahren zur Behandlung eines Patienten mit einer Erkrankung, die sich durch die Expression oder abnormale Expression eines Tumor-assoziierten Antigens auszeichnet, umfassend:
   (i) die Identifizierung einer Nukleinsäure, die von Zellen exprimiert wird, die mit der Erkrankung assoziiert sind, wobei die Nukleinsäure aus der Gruppe ausgewählt ist bestehend aus:
      (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist, und
      (d) einer Nukleinsäure, die zu der Nuldeinsäure unter (a), (b) oder (c) komplementär ist,
   (ii) die Transfektion einer Wirtszelle mit der Nukleinsäure oder einem Teil davon,
   (iii) die Kultivierung der transfizierten Wirtszelle für eine Expression der Nukleinsäure, und
   (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen.
65. Verfahren nach Ziffer 64, ferner umfassend die Identifizierung eines MHC-Moleküls, das das Tumor-assoziierte Antigen oder einen Teil davon präsentiert, wobei die Wirtszelle das identifizierte MHC-Molelcül exprimiert und das Tumor-assoziierte Antigen oder einen Teil davon präsentiert.
66. Verfahren nach Ziffer 64 oder 65, wobei die Immunreaktion eine B-Zellen-Reaktion oder eine T-Zellen-Reaktion umfasst.
67. Verfahren nach Ziffer 66, wobei die Immunreaktion eine T-Zellen-Reaktion ist, umfassend die Produktion cytolytischer oder Zytokine-ausschüttender T-Zellen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.
68. Verfahren nach einer der Ziffern 61-67, wobei die Wirtszellen nicht-proliferativ sind.
69. Verfahren zur Behandlung einer Erkrankung, die sich durch die Expression oder abnormale Expression eines Tumor-assoziierten Antigens auszeichnet, umfassend:
   (i) die Identifikation von Zellen aus dem Patienten, die abnormale Mengen des Tumor-assoziierten Antigens exprimieren,
   (ii) die Isolierung einer Probe der Zellen,
   (iii) die Kultivierung der Zellen, und
   (iv) das Einbringen der Zellen in den Patienten in einer Menge, die geeignet ist, eine Immunreaktion gegen die Zellen auszulösen, wobei das Tumor-assoziierte Antigen eine Sequenz aufweist, die von einer Nukleinsäure kodiert wird, die aus der Gruppe ausgewählt ist bestehend aus:
      (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
      (b) einer Nukleinsäure, die unter stringenten Bedingungen mit der Nuldeinsäure unter (a) hybridisiert,
      (c) einer Nukleinsäure, die in Bezug auf die Nukleinsäure unter (a) oder (b) degeneriert ist, und
      (d) einer Nukleinsäure, die zu der Nukleinsäure unter (a), (b) oder (c) komplementär ist.
70. Verfahren nach einer der Ziffern 33-69, wobei die Erkrankung Krebs ist.
71. Verfahren zur Hemmung der Entwicklung von Krebs bei einem Patienten, umfassend die Verabreichung einer wirksamen Menge einer pharmazeutischen Zusammensetzung nach einer der Ziffern 1-32.
72. Verfahren nach einer der Ziffern 33-71, wobei das Tumor-assoziierte Antigen eine Aminosäuresequenz umfasst, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.
73. Nukleinsäure, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267,269,271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
74. Nukleinsäure, die für ein Protein oder Polypeptid kodiert, das eine Aminosäuresequenz umfasst, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.
75. Rekombinantes DNA- oder RNA-Molekül, das eine Nukleinsäure nach Ziffer 73 oder 74 umfasst.
76. Rekombinantes DNA-Molekül nach Ziffer 75, wobei das rekombinante DNA-Molekül ein Vektor ist.
77. Rekombinantes DNA-Molekül nach Ziffer 76, wobei der Vektor ein viraler Vektor oder ein Bakteriophage ist.
78. Rekombinantes DNA-Molekül nach einer der Ziffern 75-77, das ferner Expressionskontrollsequenzen umfasst, die die Expression der Nukleinsäure steuern.
79. Rekombinantes DNA-Molekül nach Ziffer 78, wobei die Expressionskontrollsequenzen homo- oder heterolog zu der Nuldeinsäure sind.
80. Wirtszelle, die eine Nukleinsäure nach Ziffer 73 oder 74 oder ein rekombinantes DNA-Molekül nach einer der Ziffern 75-79 umfasst.
81. Wirtszelle nach Ziffer 80, die ferner eine Nukleinsäure umfasst, die für ein HLA-Molekül kodiert.
82. Protein oder Polypeptid, das von einer Nuldeinsäure nach Ziffer 73 kodiert wird.
83. Protein oder Polypeptid, das eine Aminosäuresequenz umfasst, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.
84. Immunogenes Fragment des Proteins oder Polypeptids nach Ziffer 82 oder 83.
85. Fragment des Proteins oder Polypeptids nach Ziffer 82 oder 83, das an einen menschlichen HLA-Rezeptor oder menschlichen Antikörper bindet.
86. Mittel, das spezifisch an ein Protein oder Polypeptid oder an einen Teil davon bindet, wobei das Protein oder Polypeptid von einer Nukleinsäure kodiert wird, die aus der Gruppe ausgewählt ist bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist, und
   (d) einer Nukleinsäure, die zu der Nukleinsäure unter (a), (b) oder (c) komplementär ist.
87. Mittel nach Ziffer 86, wobei das Protein oder Polypeptid eine Aminosäuresequenz umfasst, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126,130, 134, 138, 142, 146, 150,154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.
88. Mittel nach Ziffer 86 oder 87, wobei das Mittel ein Antikörper ist.
89. Mittel nach Ziffer 88, wobei der Antikörper ein monoklonaler, chimärer oder humanisierter Antikörper oder ein Fragment eines Antikörpers ist.
90. Antikörper, der selektiv an einen Komplex aus:
   (i) einem Protein oder Polypeptid oder einem Teil davon und
   (ii) einem MHC-Molekül bindet, an das das Protein oder Polypeptid oder der Teil davon bindet, wobei der Antiköper nicht alleine an (i) oder (ii) bindet und das Protein oder Polypeptid von einer Nukleinsäure kodiert wird, die aus der Gruppe ausgewählt ist bestehend aus:
      (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist, und
      (d) einer Nukleinsäure, die zu der Nukleinsäure unter (a), (b) oder (c) komplementär ist.
91. Antikörper nach Ziffer 90, wobei das Protein oder Polypeptid eine Aminosäuresequenz umfasst, die aus der Gruppe ausgewählt ist, bestehend aus SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.
92. Antikörper nach Ziffer 90 oder 91, wobei der Antikörper ein monoklonaler, chimärer oder humanisierter Antikörper oder ein Fragment eines Antikörpers ist.
93. Konjugat zwischen einem Mittel nach einer der Ziffern 86-89 oder einem Antikörper nach einer der Ziffern 90-92 und einem therapeutischen oder diagnostischen Mittel.
94. Konjugat nach Ziffer 93, wobei das therapeutische oder diagnostische Mittel ein Toxin ist.
95. Kit zum Nachweis der Expression oder abnormalen Expression eines Tumor-assoziierten Antigens, umfassend Mittel zum Nachweis
   (i) der Nukleinsäure, die für das Tumor-assoziierte Antigen kodiert, oder eines Teils davon,
   (ii) des Tumor-assoziierten Antigens oder eines Teils davon,
   (iii) von Antikörpern, die an das Tumor-assoziierte Antigen oder einen Teil davon binden, und/oder
   (iv) von T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist bestehend aus:
      (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist, und
      (d) einer Nukleinsäure, die zu der Nukleinsäure unter (a), (b) oder (c) komplementär ist.
96. Kit nach Ziffer 95, wobei die Mittel zum Nachweis der Nukleinsäure, die für das Tumor-assoziierte Antigen kodiert, oder eines Teils davon Nukleinsäuremoleküle für die selektive Amplifikation der Nukleinsäure sind.
97. Kit nach Ziffer 96, wobei die Nukleinsäuremoleküle für die selektive Amplifikation der Nukleinsäure eine Sequenz von 6-50 zusammenhängenden Nukleotiden aus der Nukleinsäure, die für das Tumor-assoziierte Antigen kodiert, umfassen.
98. Rekombinantes DNA-Molekül, umfassend eine Promotorregion, die von einer Nukleinsäure abgeleitet ist, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls.
99. Pharmazeutische Zusammensetzung, umfassend ein Mittel, das die Expression oder Aktivität der Tumorantigene gemäß SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls hemmt.
100. Antikörper, der an die extrazellulären Bereiche von Proteinen umfassend eine Sequenz gemäß SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls bindet.
101. Pharmazeutische Zusammensetzung nach Ziffer 99, wobei das Mittel eine Antisense-Nukleinsäure ist, die selektiv mit der Nukleinsäure hybridisiert, die für die Tumorantigene kodiert.
102. Pharmazeutische Zusammensetzung nach Ziffer 101, wobei die Antisense-Nukleinsäure eine Sequenz von 6-50 zusammenhängenden Nukleotiden aus den Nukleinsäuren, die für die Tumorantigene kodieren, umfasst.
103. Pharmazeutische Zusammensetzung nach Ziffer 99, wobei das Mittel RNA Interferenz (RNAi) ist.
104. Pharmazeutische Zusammensetzung nach Ziffer 103, wobei die RNAi eine sog. short hairpin Struktur (shRNA) enthält.
105. Pharmazeutische Zusammensetzung nach Ziffer 104, wobei shRNA durch Transkription nach Transfektion mit Expressionsvektoren entstehen.
106. Pharmazeutische Zusammensetzung nach Ziffer 104, wobei shRNA durch Transkription von Retroviren entsteht
107. Pharmazeutische Zusammensetzung nach Ziffer 104, wobei shRNA durch lentivirale Systeme vermittelt wird.
108. Pharmazeutische Zusammensetzung nach Ziffer 99, wobei das Mittel ein kleines chemisches Molekül ist.
109. Pharmazeutische Zusammensetzung nach Ziffer 108, wobei die kleinen chemischen Moleküle an die Tumorantigene binden
110. Pharmazeutische Zusammensetzung nach Ziffer 109, wobei die kleinen chemischen Moleküle an die extrazellulären Bereiche von Proteinen umfassend eine Sequenz gemäß SEQ ID NOs; 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls binden.
111. Verfahren zur Behandlung Diagnose oder Überwachung eines metastasierenden Tumors, der sich durch die Expression oder abnormaler Expression mindestens eines Tumorantigenes auszeichnet, umfassend die Verabreichung eines Antikörpers, der an mindestens eines der Tumorantigene oder einen Teil davon bindet und mit einem therapeutischen oder diagnostischen Mittel gekoppelt ist, wobei das mindestens eine Tumorantigen eine Sequenz aufweist, die von einer Nukleinsäure kodiert wird bestehend aus:
   (a) einer Nukleinsäure, die eine Nukleinsäuresequenz umfasst, die aus der Gruppe ausgewählt ist, bestehend aus SEQ ID NOs: 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141,145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
112. Verfahren nach Ziffer 111, wobei der Antikörper ein monoklonaler Antikörper ist.
113. Verfahren nach Ziffer 111, wobei der Antikörper ein chimärer oder humanisierter Antikörper ist.
114. Verfahren nach Ziffer 111, wobei der Antikörper ein Fragment eines natürlichen Antikörpers ist.

### Detaillierte Beschreibung der Erfindung

Erfindungsgemäß werden Gene beschrieben, die in Tumorzellen selektiv exprimiert oder aberrant exprimiert werden und Tumor-assoziierte Antigene darstellen.

Erfindungsgemäß sind diese Gene oder ihre Derivate bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Base, einem Zucker oder Phosphat eines Nukleotids. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (üi) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplexmolekül eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989, oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5 mM NaH₂PO₄ (pH 7), 0,5% SDS, 2 mM EDTA). SSC ist eine Lösung mit jeweils 0,15 M Natriumchlorid und Natriumcitrat, pH 7. Nach der Hybridisierung wird die Membran, auf die die DNA übertragen wurde, beispielsweise in 2 x SSC bei Raumtemperatur und sodann in 0,1 - 0,5 x SSC / 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumorassoziierten Antigene mit beliebigen Expressionskontrollsequenzen und Promotoren kombiniert werden. Zum anderen aber können erfindungsgemäß die Promotoren der hier dargestellten Tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

In einer bevorzugten Ausführungsform ist ein rekombinantes DNA-Molekül erfindungsgemäß ein Vektor, gegebenenfalls mit einem Promotor, der die Expression einer Nukleinsäure, z.B. einer Nukleinsäure, die für eine erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide, Bacteriophage oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch *in vitro*-Transkription von einer DNA-Matritze hergestellt werden. Sie kann des weiteren vor Applikation durch stabilisierende Sequenzen, Capping und Poly-Adenylierung modifiziert werden. Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E*. *coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht), und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Erfindungsgemäß umfasst sind Kits zur Amplifikation einer Nukleinsäure, die für ein Tumor-assoziierte Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst erfindungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann ein Duplexmolekül mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte erfindungsgemäße Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-SpleißStelle.

In einer Ausführungsform besteht ein erfindungsgemäßes Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein erfindungsgemäßes Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Intemukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist erfindungsgemäß eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer-T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen. Ein Teil oder Fragment eines Tumor-assoziierten Antigens ist vorzugsweise ein Teil des Tumor-assoziierten Antigens, der dem nicht-Transmembrananteil, insbesondere dem extrazellulären Anteil des Antigens entspricht oder davon umfasst wird.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen und/oder für einen Teil oder ein Fragment des Tumor-assoziierten Antigens wie vorstehend definiert kodiert. Vorzugsweise ist ein Teil oder Fragment einer Nukleinsäure, die für ein Tumor-assoziiertes Antigen kodiert, derjenige Teil, der dem offenen Leserahmen, insbesondere wie im Sequenzprotokoll angegeben entspricht.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Erfindung ermöglicht auch die Isolierung von Proteinen, die an hier beschriebene Tumor-assoziierte Antigene binden, einschließlich Antikörper und zelluläre Bindepartner der Tumor-assoziierten Antigene.

Erfindungsgemäß werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Erfindungsgemäß umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Erfindungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Vorzugsweise ist ein erfindungsgemäß verwendeter Antikörper gegen eine der Sequenzen gemäß SEQ ID NOs: 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einen Teil oder ein Derivat davon, insbesondere eine Sequenz gemäß SEQ ID NOs: 281 bis 308 des Sequenzprotokolls gerichtet und/oder kann durch Immunisierung mit diesen Peptiden erhalten werden.

Erfindungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluor und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas*-Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Colon-, Rektal-, Nieren-, Brust-, Prostata-, Gebärmutter-, Ovarial-, Endometrial-, Speiseröhren-, Blut-, Leber-, Pankreas-, Haut-, Gehirn- und Lungenkrebs, Lymphome und Neuroblastome. Beispiele hierfür sind Lungen-, Brust-, Prostata-, Colontumor, Nierenzell-, Zervix-, Colon- und Mammakarzinom oder Metastasen der vorstehenden Krebsarten oder Tumore.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, Helfer-T-Zelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer-T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer-T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-96/33265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen), mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen), mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat. Immunol. 2:962-70, 2001 ; Kessels et al., Nat. Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigen-Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigens in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer-T-Zell-Antworten entstehen können. Eine effektive Immunantwort benötigt diese (Ossendorp et al., Immunol. Lett. 74:75-9, 2000; Ossendorp et al., J. Exp. Med. 187:693-702, 1998). Im allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc. Natl. Acad. Sci. USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J. Immunol. 167:787-96, 2001; Appella et al., Biomed. Pept. Proteins Nucleic Acids 1:177-84, 1995; Wentworth et al., Mol. Immunol. 32:603-12, 1995).

Die erfindungsgemäß beschriebenen pharmazeutischen Zusammensetzungen können auch als Vakzinen für die Immunisierung eingesetzt werden. Die Begriffe "Immunisierung" oder "Vakzinierung" bedeuten erfindungsgemäß eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Krieg et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Thl-Zytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Erfindungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch ex vivo-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen *in vitro* oder *in vivo* einzubringen. Solche Verfahren umfassen die Transfektion von Nukleinsäure-Kalziumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Oligonukleotide) und Zytokine und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

### Abbildungen:

### Abb.1: qPCR Analyse der SEQ ID NO: 1 in Melanomen

Quantitative Expressionsanalyse der SEQ ID NO: 1 in gesundem Hautgewebe, in der Testis und in Melanomen. Logarithmische Darstellung der relativen Expression (-fache Aktivierung).

### Abb.2: konventionelle RT-PCR Analyse der SEQ ID NO: 1 in Melanomen

RT-PCR Expressionsanalyse von SEQ ID NO: 1 in Melanomen (n=14) und Melanomzelllinien (n=4) im Vergleich zur gesunden Haut (n=4) und zur Testis (n=3).

### Abb.3: qPCR Analyse der SEQ ID NO: 5 in gesundem Gewebe und in Tumorenproben

Quantitative Expressionsanalyse der SEQ ID NO: 5 in Normalgeweben (linke Seite) und in verschiedenen Tumoren (Pools bestehend aus jeweils 3-5 Einzelproben, rechte Seite). **A** Logarithmische Darstellung der relativen Expression (fache Aktivierung). **B** Bild nach gelelektrophoretischer Auftrennung der amplifizierten Fragmente.

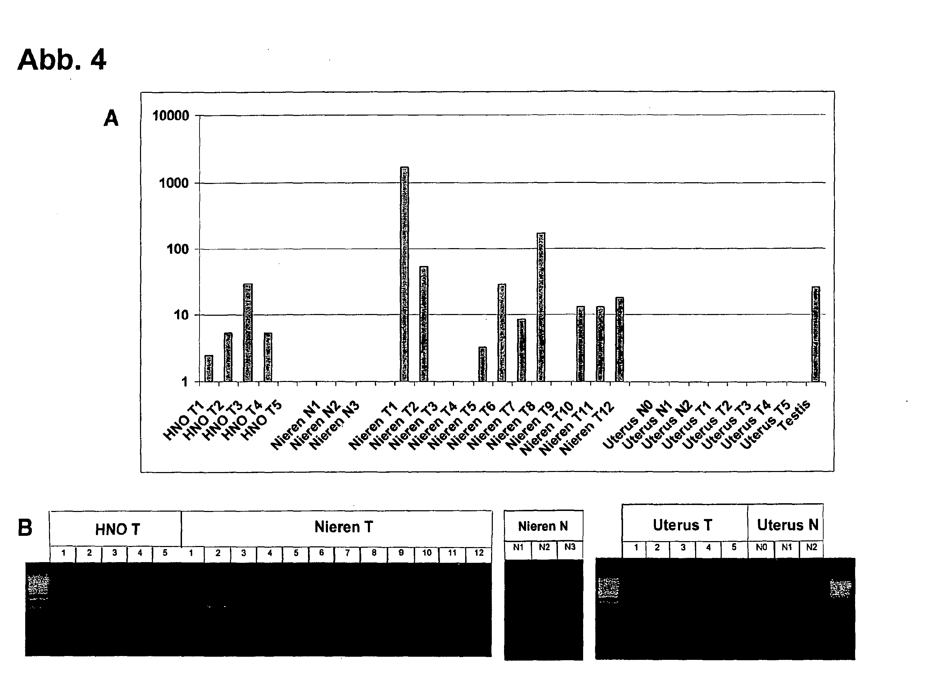

### Abb.4: Detailanalyse der SEQ ID NO: 5-spezifischen Expression

**A** Quantitative Expressionsanalyse der SEQ ID NO: 5 in verschiedenen HNO-, Nieren- und Uterustumoren im Vergleich zur Expression in den zugehörigen Normalgeweben. Logarithmische Darstellung. **B** Bild nach gelelektrophoretischer Auftrennung der amplifizierten Fragmente.

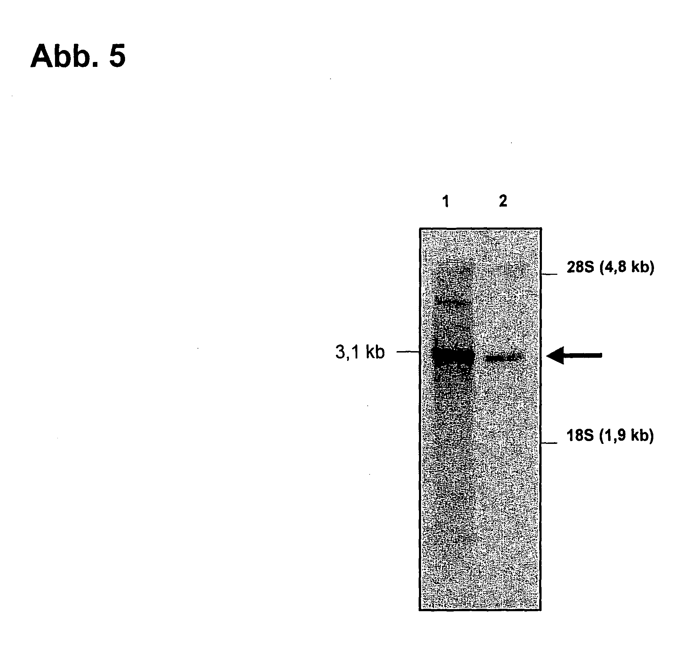

### Abb.5: Northern blot Analyse mit einer SEQ ID NO: 5-spezifischen Sequenz

Hybridisierung einer DIG-markierten DNA-Sonde, die durch PCR-Amplifikation mit den Primern gemäß SEQ ID NO: 7 und 8 hergestellt wurde, mit Testis-spezifischer RNA. Spur 1: 2 µg Testis-spezifische RNA; Spur 2: 1 µg Testis-spezifische RNA

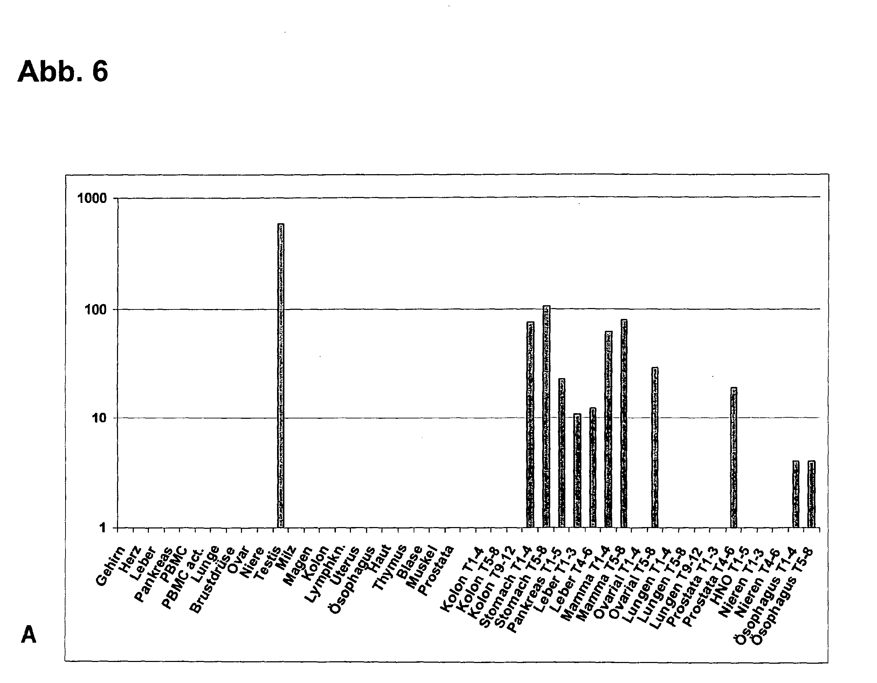

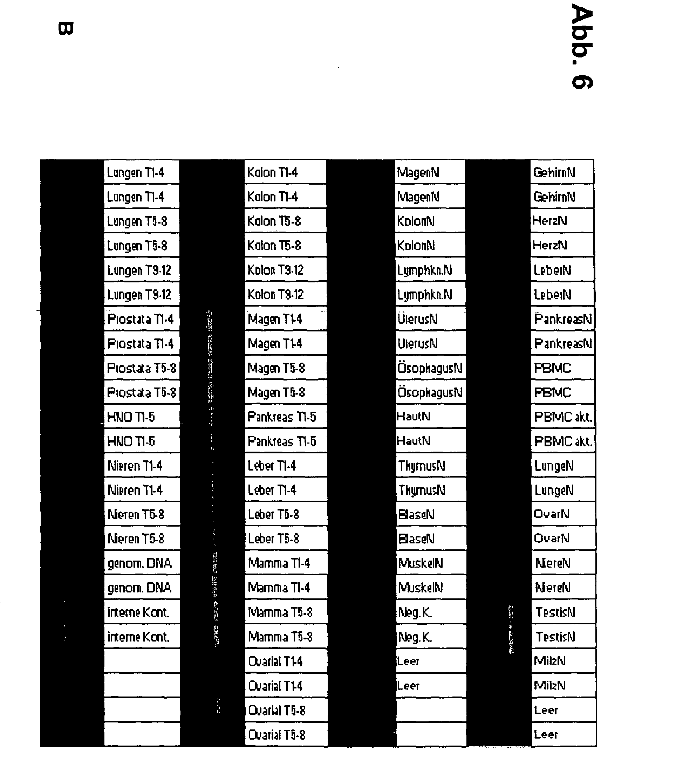

### Abb.6: qPCR Analyse von LOC203413

Quantitative Expressionsanalyse von LOC203413 in gesunden Geweben (links) und in Tumorproben (Pools bestehend aus jeweils 3-5 Einzelproben, rechts). **A** Logarithmische Darstellung der Expression (fache Aktivierung). **B** Resultat nach gelelektrophoretischer Auftrennung.

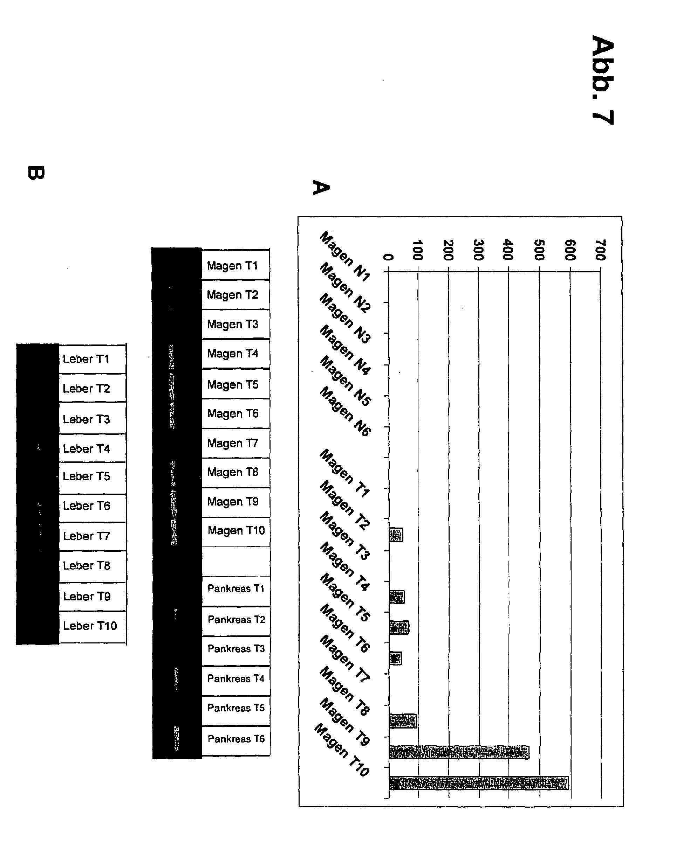

### Abb. 7: Detailanalyse der LOC203413-spezifischen Expression in Magenkarzinomen

Quantitative Expressionsanalyse von LOC203413 in verschiedenen Magentumorproben (n=10) im Vergleich zur Expression im gesunden Magen (n=6). **A** Lineare Darstellung der relativen Expression. **B** Bild nach gelelektrophoretischer Auftrennung der Amplifikate

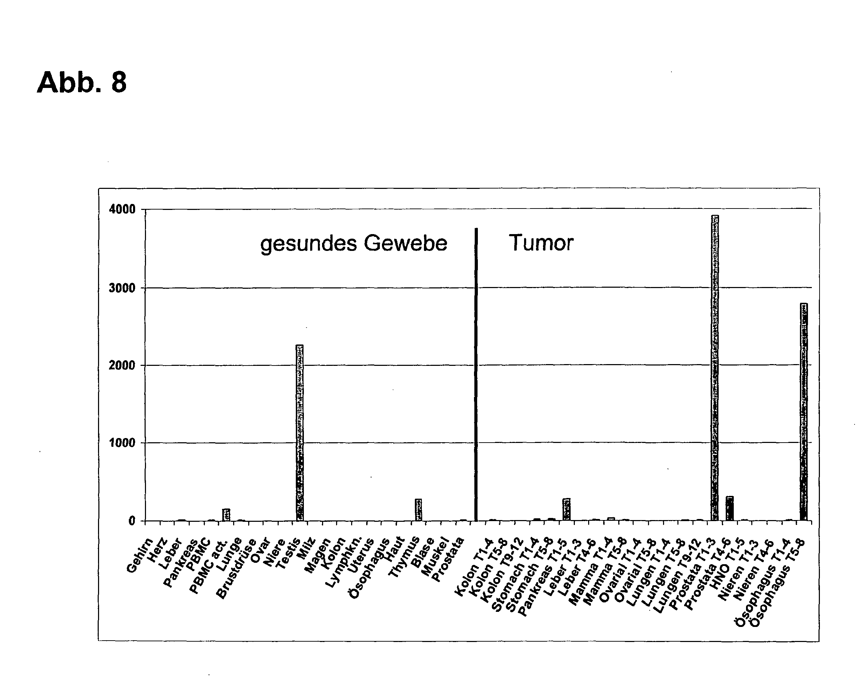

### Abb.8: qPCR Analyse der LOC90625 -spezifischen Expression

Quantitative Expressionsanalyse von LOC90625 in Normalgeweben (links) und Tumorgeweben (Pools bestehend aus jeweils 3-5 Einzelproben, rechts). Lineare Darstellung der relativen Expression (fache Aktivierung).

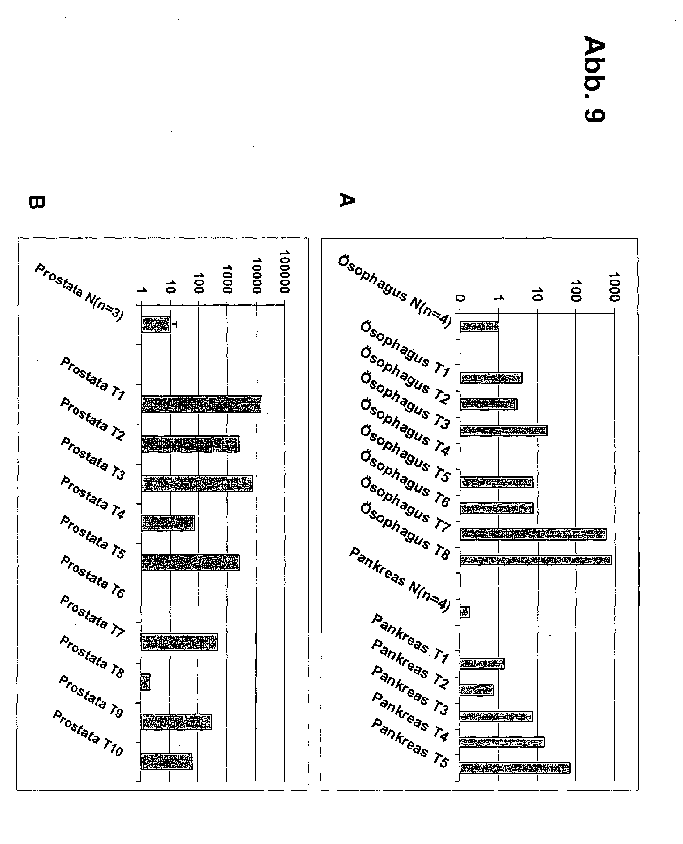

### Abb.9: Detailanalyse der LOC90652-spezifischen Expression in verschiedenen Tumortypen

Quantitative Expressionsanalyse von LOC90625 in Ösophagus- (n=8), Pankreas- (n=5) und Prostatakarzinomproben (n=10) im Vergleich zum jeweiligen gesunden Gewebe (n=3/4); Logarithmische Darstellung der relativen Expression (fache Aktivierung).

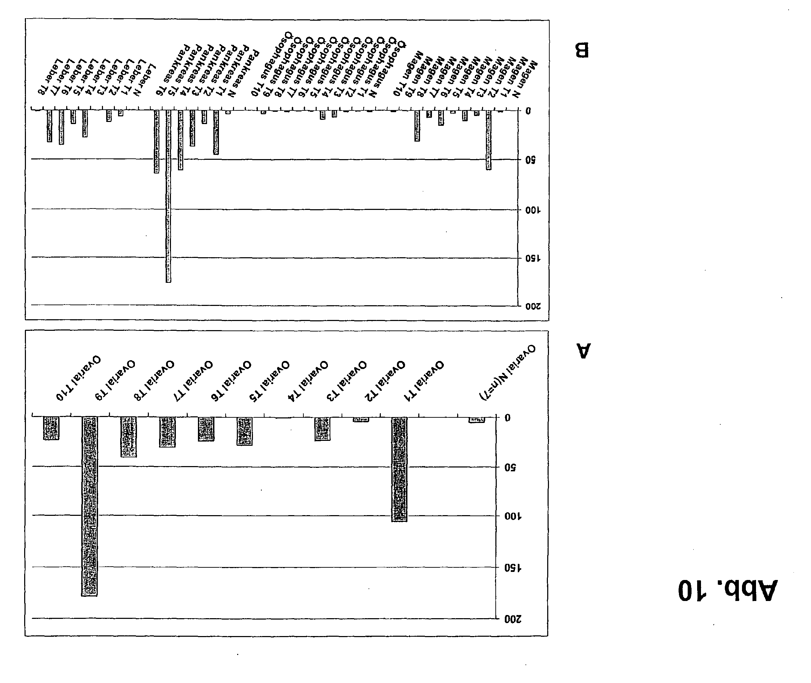

### Abb.10: qRT-PCR Analyse von FAM26A in verschiedenen Tumortypen

Quantitative RT-PCR Expressionsanalyse von FAM26A in **A** Ovarial-, **B** Magen-, Ösophagus-, Pankreas- und Leberkarzinomen im Vergleich zum jeweiligen gesunden Gewebe. Lineare Darstellung der relativen Expression (fache Aktivierung).

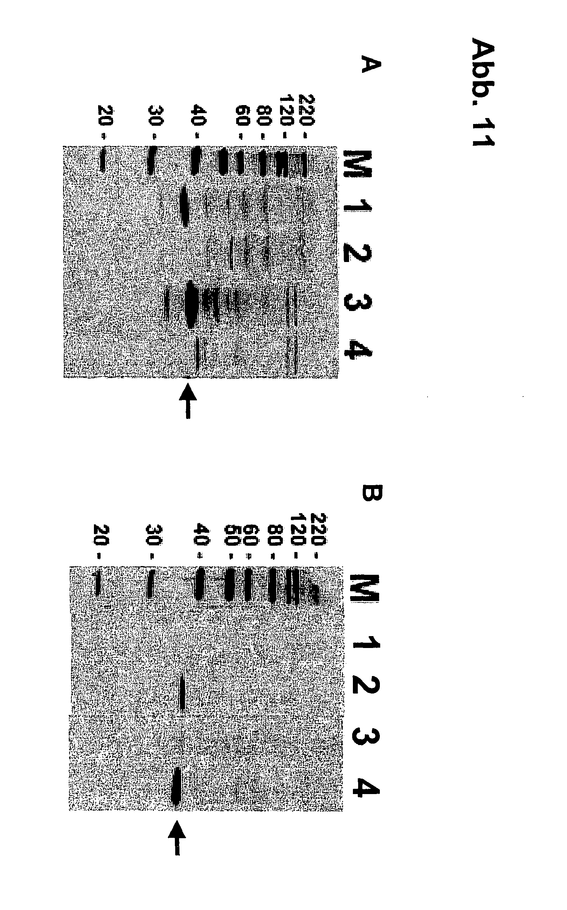

### Abb.11: Charakterisierung FAM26A-spezifischer Antikörper

Westernblot-Analyse der Antiseren, die durch Immunisierung mit einem Peptid der SEQ ID NO: 291 **(A)** bzw. der SEQ ID NO: 292 **(B)** generiert wurden. Analysiert wurden Extrakte von CHO-Zellen nach Transfektion mit jeweils Epitop-spezifischen (A 1, 3; B 2, 4) bzw. jeweils Epitop-unspezifischen (A2, 4; B 1, 3) Plasmiden. Der Pfeil bezeichnet die spezifischen Fragmente.

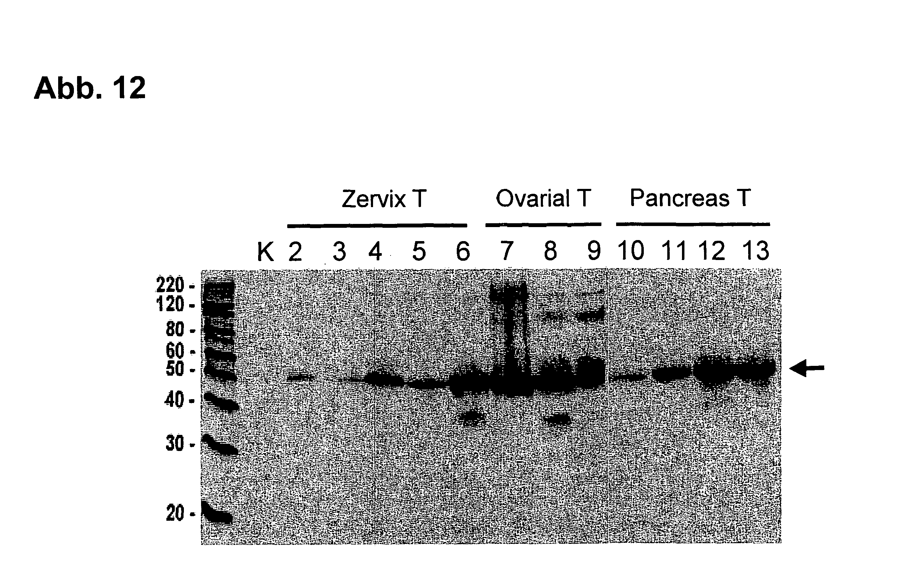

### Abb.12: Analyse des FAM26A-Proteins in Tumoren

Nachweis von FAM26A in Zervix-, Ovarial- und Pankreastumoren mittels FAM26A-spezifischen Antikörpern (SEQ ID NO: 292).

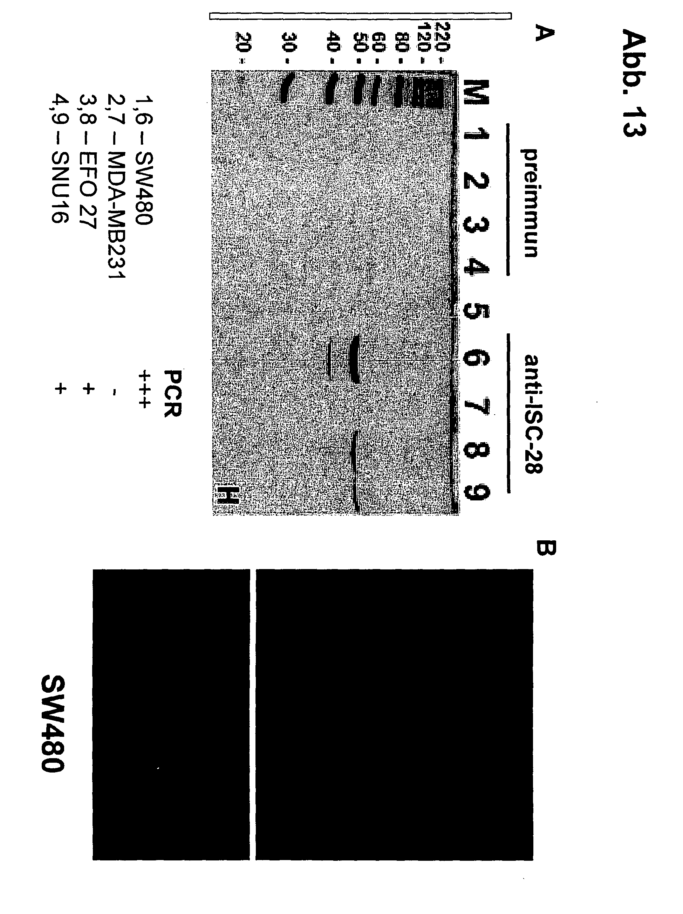

### Abb.13:Analyse des FAM26A-Proteins in Zelllinien

Analyse des FAM26A-Proteins in Zelllinien mit Hilfe von SEQ ID NO: 291-spezifischen Antikörpern. **A** Westernblot Analyse mit Präimmunserum als Spezifitätskontrolle (Spuren 1-5) und FAM26A-spezifischen Antikörpern. **B** Immunfluoreszenz Analyse von SW480 Zellen.

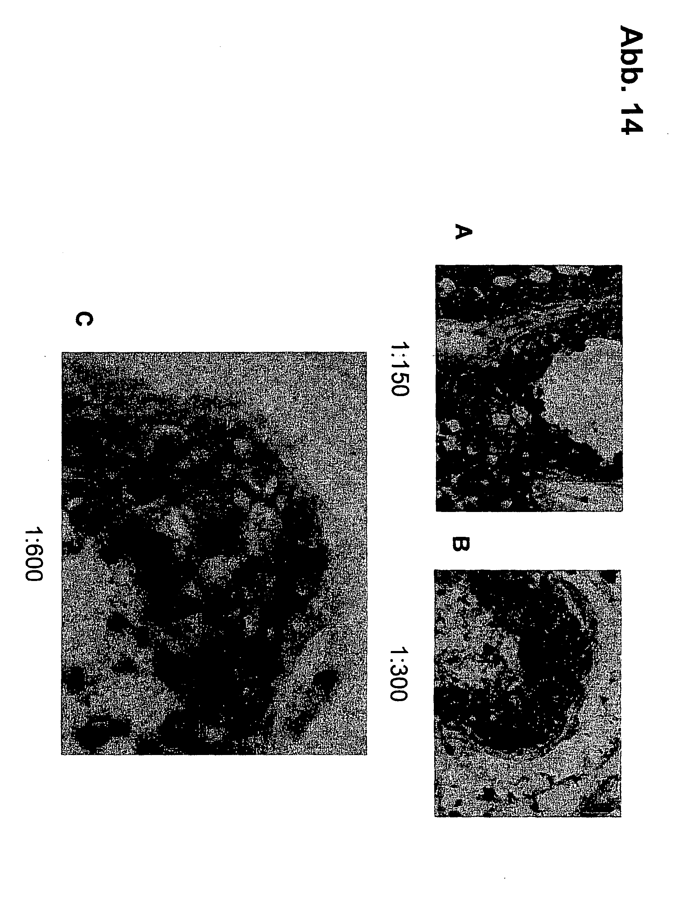

### Abb.14: Immunhistochemischer Nachweis von FAM26A in der Testis

Immunhistochemische Analyse des FAM26A-Proteins in der gesunden Testis mit Hilfe des SEQ ID NO: 292-spezifischen Antiserums in unterschiedlichen Verdünnungen **(A-C)**

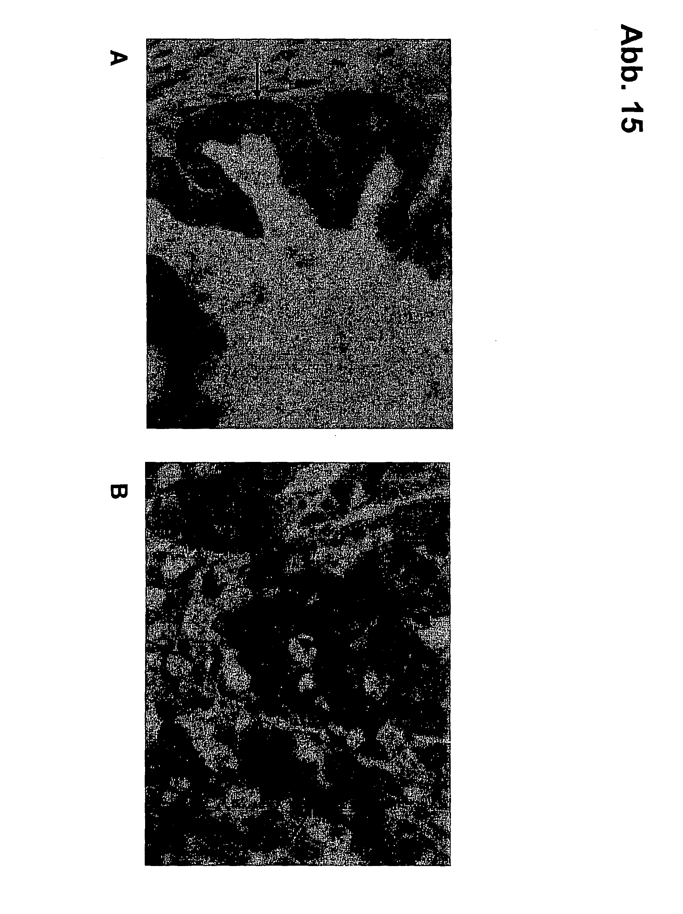

### Abb.15: Immunhistochemische Analyse von FAM26A in Tumoren

Immunhistochemische Analyse des FAM26A-Proteins in Karzinomproben (40-fache Vergrößerung, Verdünnung 1:300) mit Hilfe des SEQ ID NO 292-spezifischen Antiserums. **A** papilläres ovariales Zystadenokarzinom. **B** Plattenepithelkarzinom der Zervix

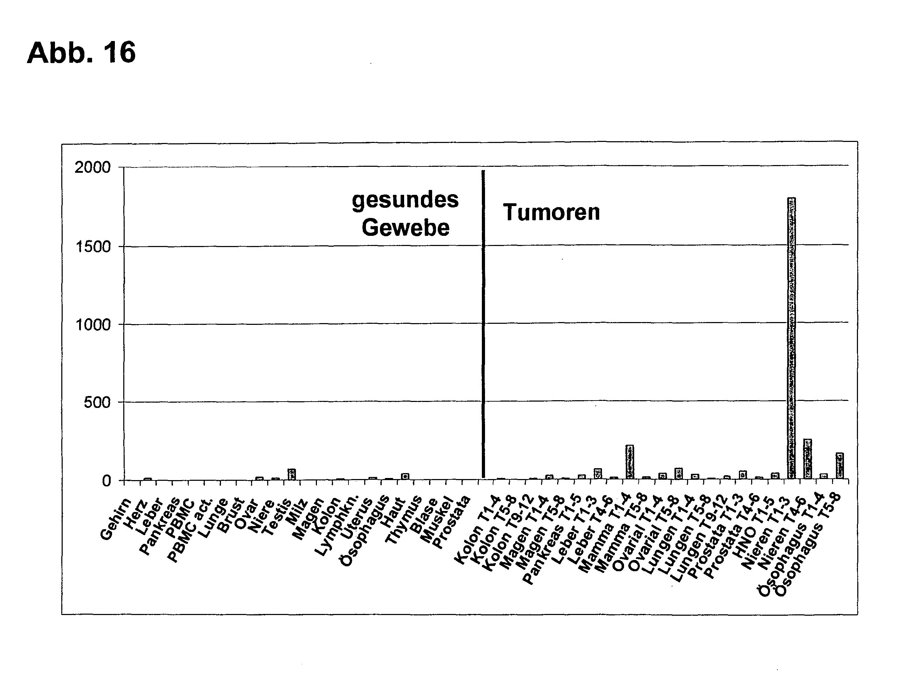

### Abb.16: qRT-PCR Analyse der SEMA5B-spezifischen Expression

Quantitative Expressionsanalyse von SEMASB in Normalgewebe (links) und Tumorproben (Pools bestehend aus jeweils 3-5 Einzelproben, rechts). Lineare Darstellung der relativen Expression (fache Aktivierung).

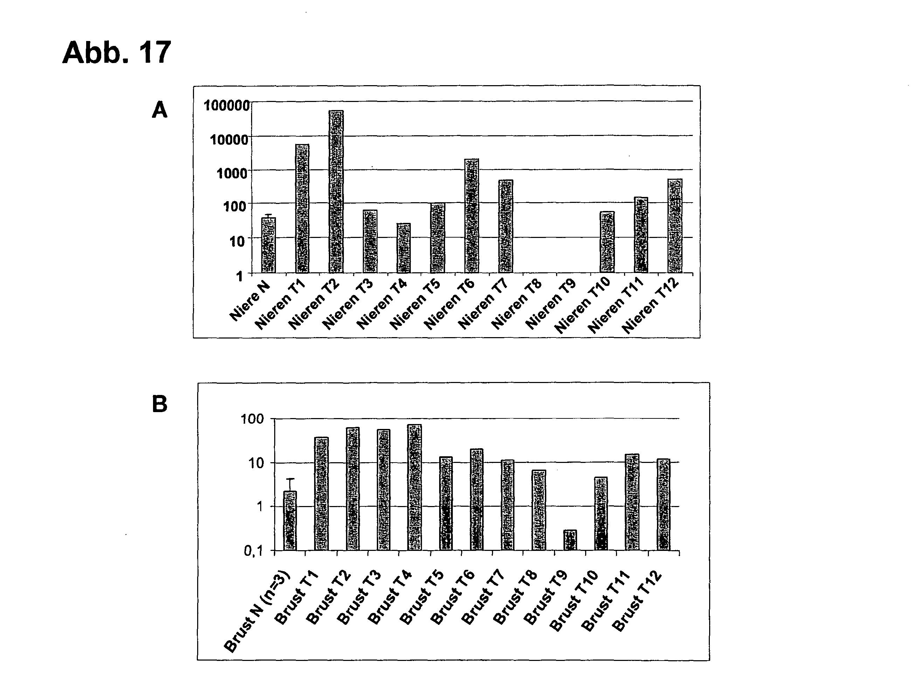

### Abb.17: Detailanalyse der SEM-A5B-spezifischen Expression in Nierenzelkarzinomproben

Quantitative Expressionsanalyse von SEMA5B in **A** Nierenzellkarzinomproben (n=12) im Vergleich zu gesundem Nierengewebe (N=3) und in **B** Mammakarzinomen (N=12) im Vergleich zu gesundem Brustgewebe (N=3); Logarithmische Darstellung der relativen Expression (fache Aktivierung).

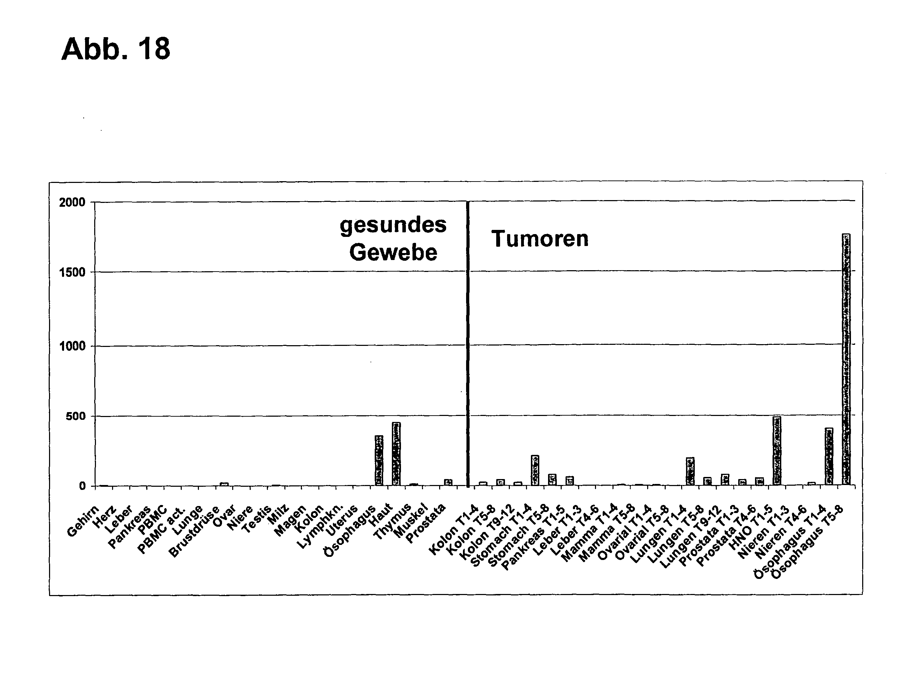

### Abb.18: qRT-PCR Analyse der GJB5-spezifischen Expression

Quantitative Expressionsanalyse von GJB5 in gesunden Gewebeproben (links) und Karzinomen (Pools bestehend aus jeweils 3-5 Einzelproben, rechts). Lineare Darstellung der relativen Expression (fache Aktivierung).

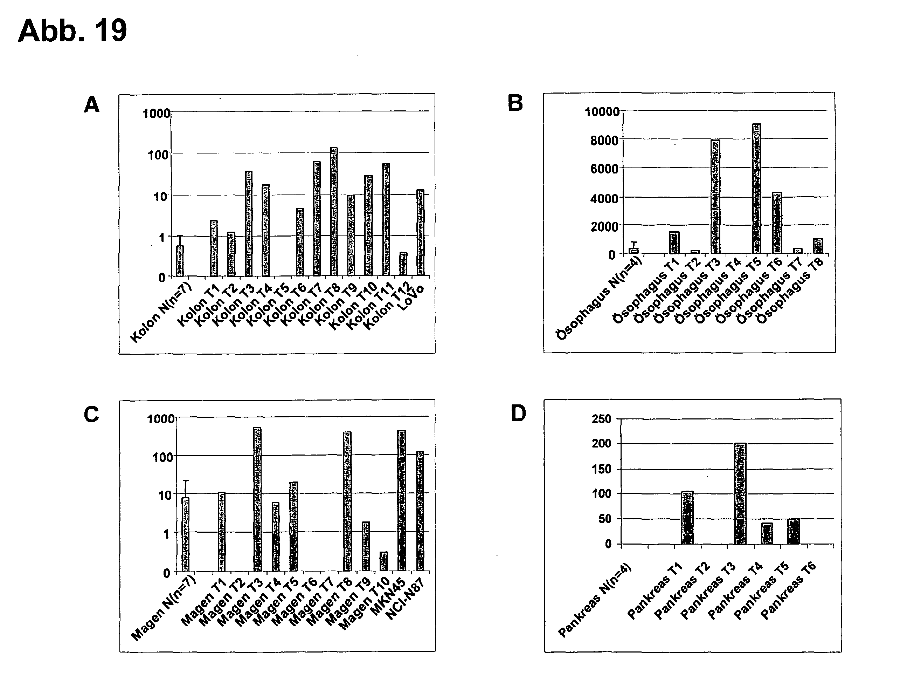

### Abb.19: Detailanalyse der GJB5-spezifischen Expression in verschiedenen Tumortypen

Quantitative Expressionsanalyse von GJB5 in **A** Kolonkarzinomproben (n=12) **B** Ösophagustumoren (n=8), **C** Magenkarzinomen (n=10) und **D** Pankreastumoren (n=5) im Vergleich zu jeweils gesunden Gewebeproben; Logarithmische (A, C) bzw. Lineare (B, D) Darstellung der relativen Expression (fache Aktivierung).

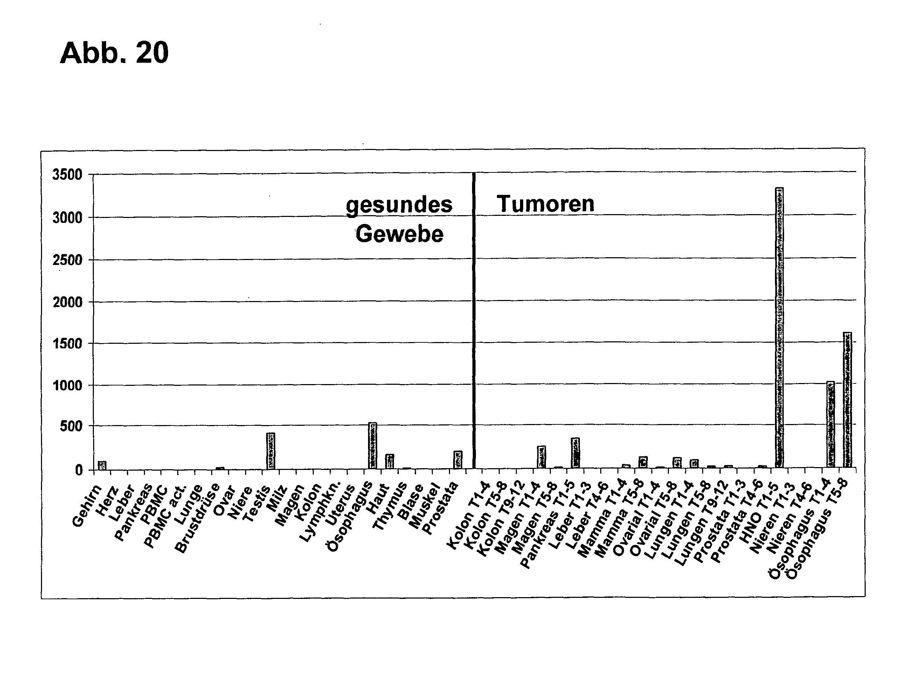

### Abb.20: qRT-PCR Analyse der KLK5-spezifischen Expression

Quantitative Expressionsanalyse von KLK5 in gesunden Gewebeproben (links) und Tumoren (Pools bestehend aus jeweils 3-5 Einzelproben, rechts). Lineare Darstellung der relativen Expression (fache Aktivierung).

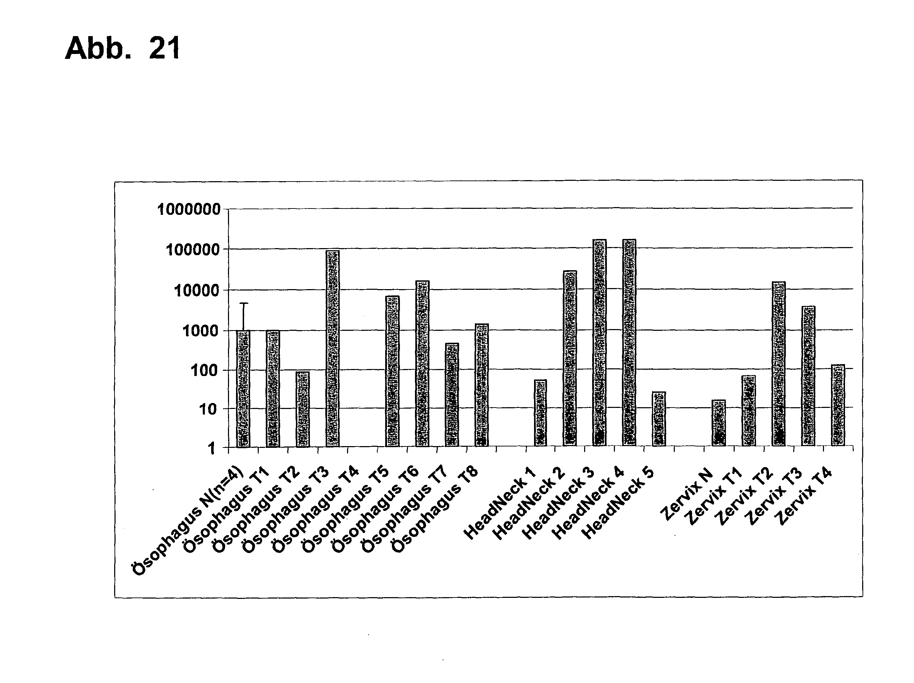

### Abb.21: Detailanalyse der KLK5-spezifischen Expression in verschiedenen Tumortypen

Quantitative Expressionsanalyse von KLK5 in Ösophagustumoren (n=8), in HNO-Karzinomen (n=5) und in Zervixtumoren (n=4) im Vergleich zu den jeweiligen gesunden Gewebeproben; Logarithmische Darstellung der relativen Expression (fache Aktivierung).

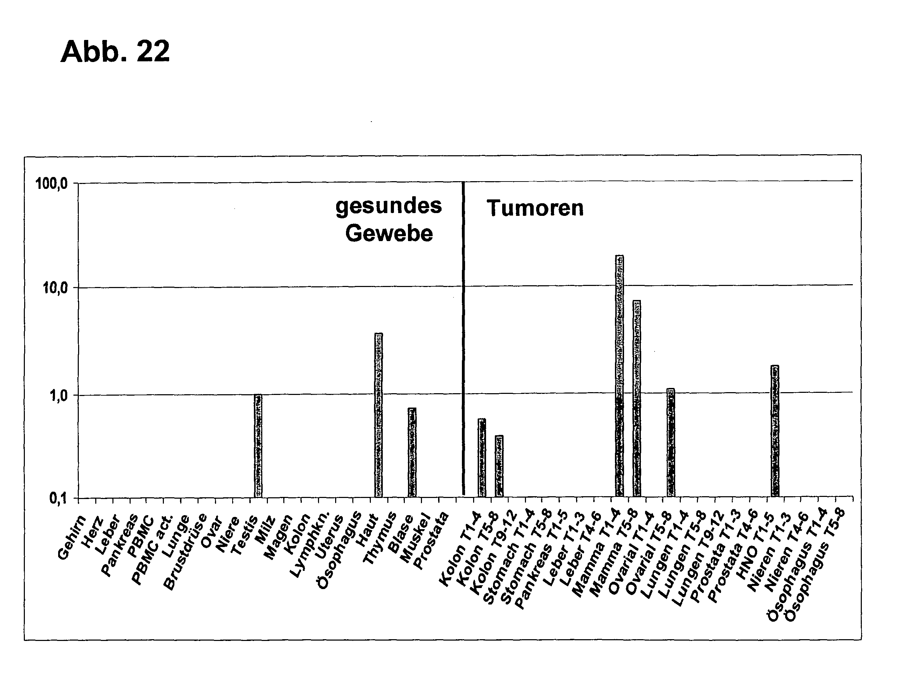

### Abb.22: qRT-PCR Analyse der LOC352765-spezifischen Expression

Quantitative Expressionsanalyse von LOC352765 in gesunden Gewebeproben (links) und Tumoren (Pools bestehend aus jeweils 3-5 Einzelproben, rechts). Logarithmische Darstellung der relativen Expression (fache Aktivierung).

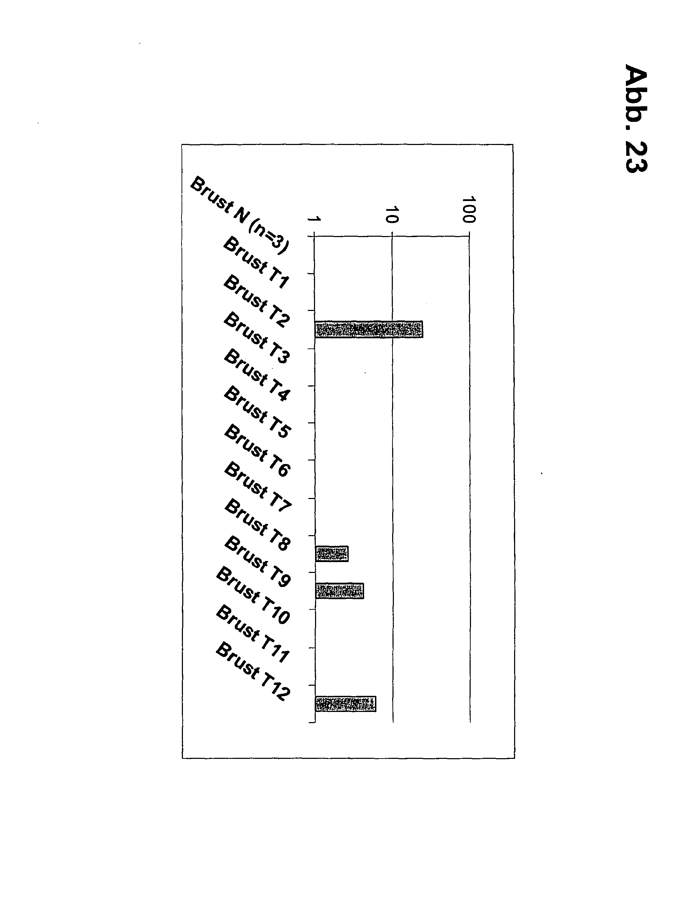

### Abb.23: Detailanalyse der LOC352765-spezifischen Expression in verschiedenen Tumortypen

Quantitative Expressionsanalyse von LOC352765 in Kolonkarzinomen (n=8), in Mammakarzinomen (n=5) und in HNO-Tumoren (n=4) im Vergleich zu jeweiligen gesunden Gewebeproben; Logarithmische Darstellung der relativen Expression (fache Aktivierung).

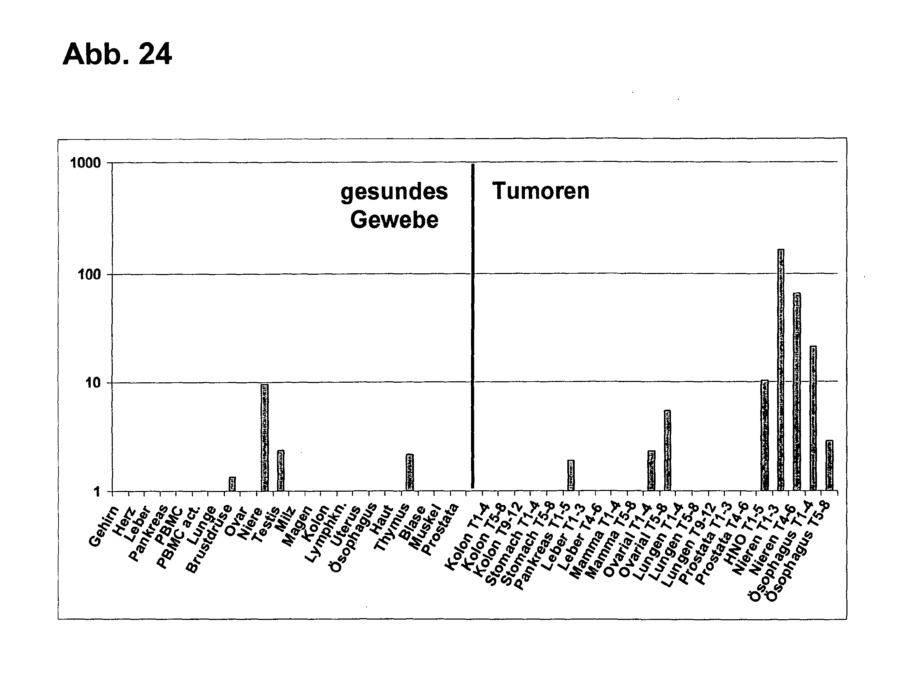

### Abb.24: qRT-PCR Analyse der SVCT1-spezifischen Expression

Quantitative Expressionsanalyse von SVCT1 in gesunden Gewebeproben (links) und Tumoren (Pools bestehend aus jeweils 3-5 Einzelproben, rechts). Logarithmische Darstellung der relativen Expression (fache Aktivierung).

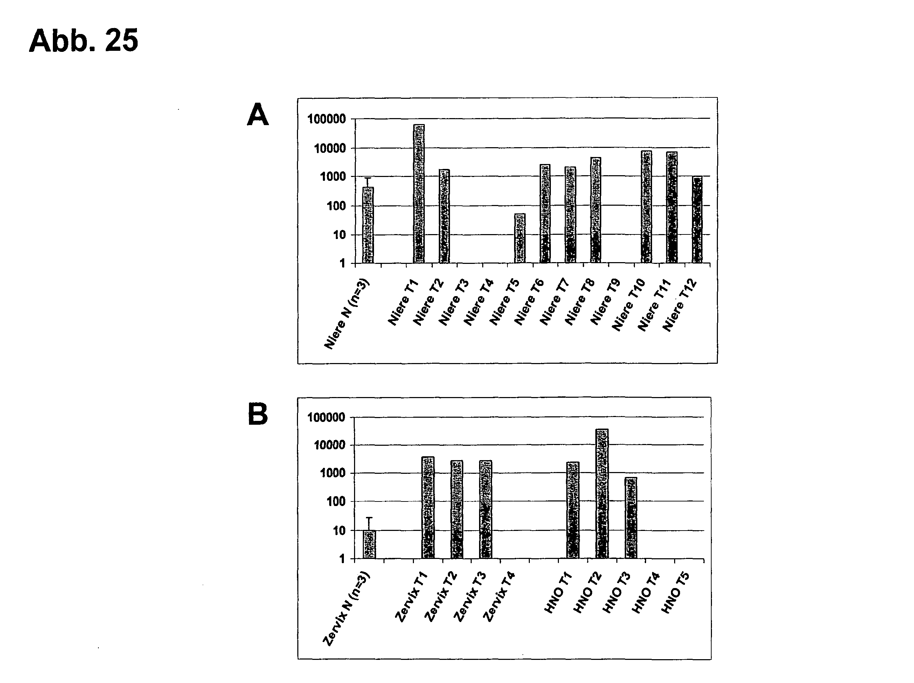

### Abb.25: Detailanalyse der SVCT1-spezifischen Expression in verschiedenen Tumortypen

Quantitative Expressionsanalyse von SVCT1 in **A** Nierenkarzinomen (n=8), **B** Ösophagialtumoren (n=5) und HNO-Tumoren (n=4) im Vergleich zu jeweils gesunden Gewebeproben; Logarithmische Darstellung der relativen Expression (fache Aktivierung).

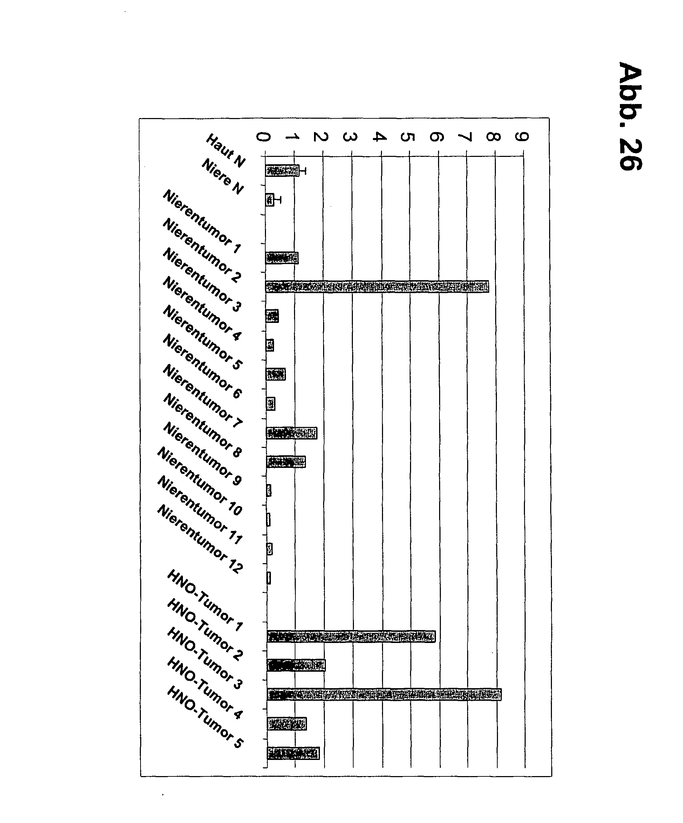

### Abb.26: qRT-PCR Analyse der LOC199953-spezifischen Expression in Nierenzellkarzinomen und in HNO-Tumoren

Quantitative Expressionsanalyse von LOC199953 in Nierenzellkarzinomen (n=12) und HNO-Tumoren (n=5) im Vergleich zu gesunden Nieren- und Haut-spezifischen Gewebeproben; Lineare Darstellung der relativen Expression (fache Aktivierung).

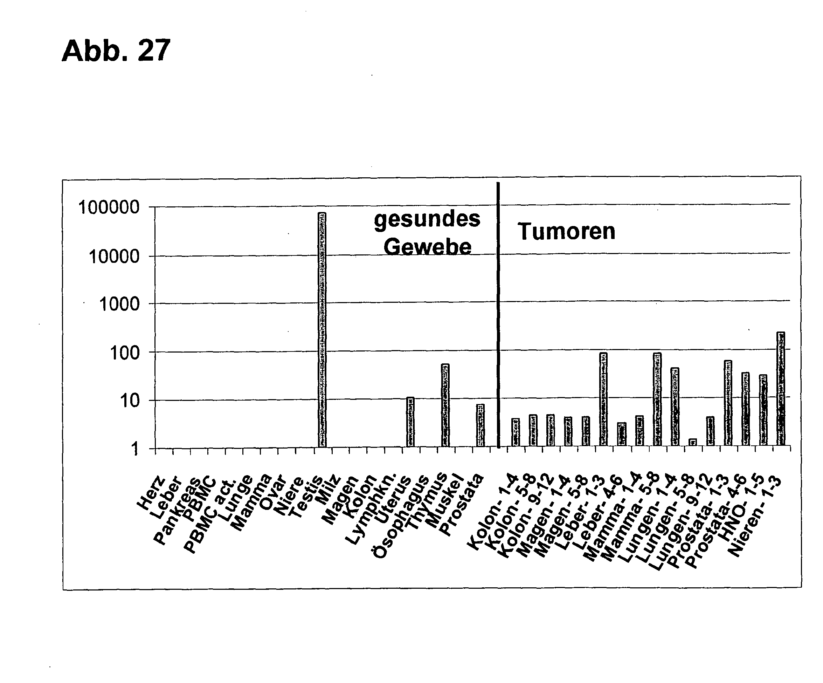

### Abb.27: qRT-PCR Analyse der TMEM31-spezifischen Expression

Quantitative Expressionsanalyse von TMEM31 in gesunden Gewebeproben (links) und Tumoren (Pools bestehend aus jeweils 3-5 Einzelproben, rechts). Logarithmische Darstellung der relativen Expression (fache Aktivierung).

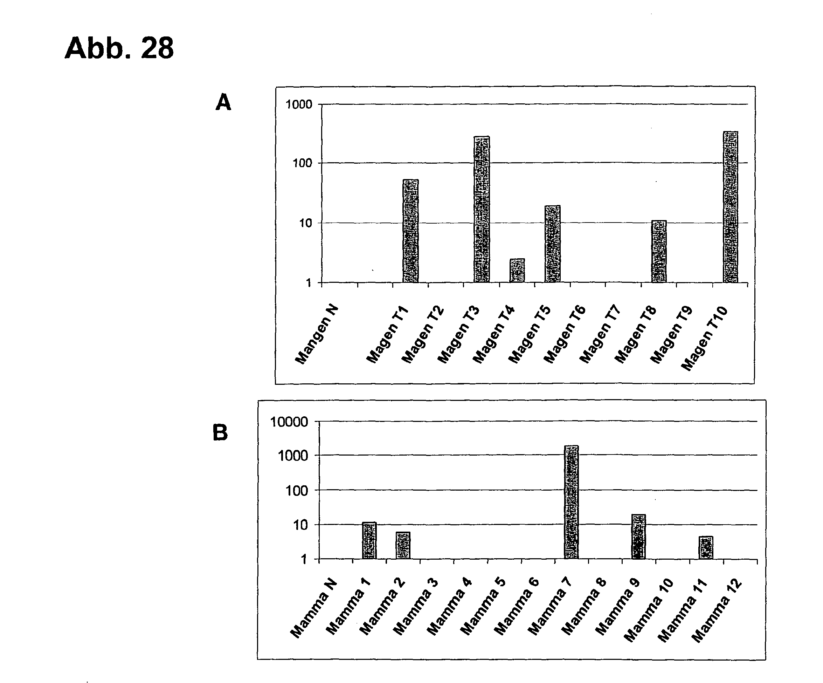

### Abb.28: Detailanalyse der TMEM31-spezifischen Expression in verschiedenen Tumortypen

Quantitative Expressionsanalyse von TMEM31 in **A** Magenkarzinomen (n=10) und **B** Mammakarzinomen (n=12) im Vergleich zu jeweils gesunden Gewebeproben; Logarithmische Darstellung der relativen Expression (fache Aktivierung).

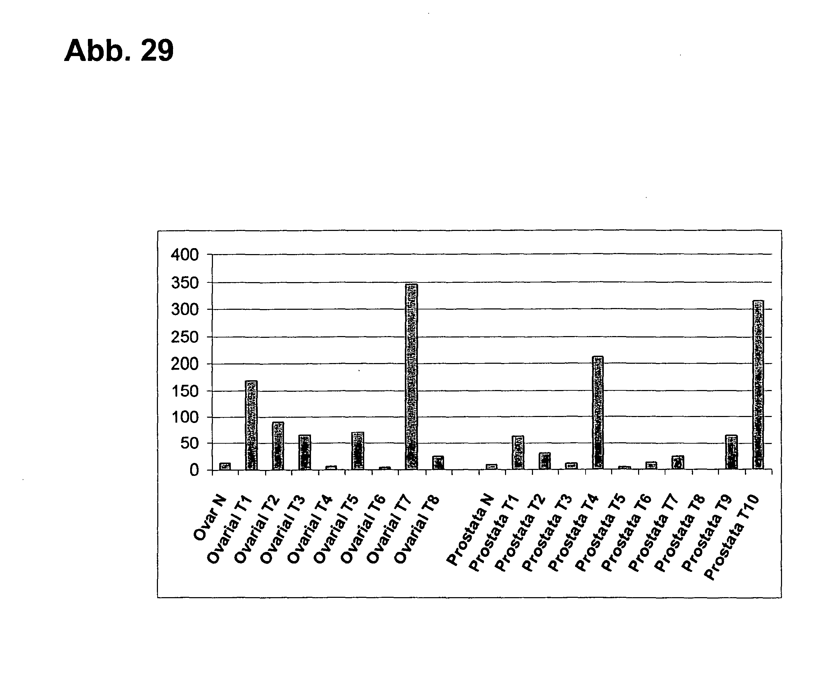

### Abb.29: qRT-PCR Analyse der FLJ25132-spezifischen Expression in Ovarialtumoren und in Prostatakarzinomen

Quantitative Expressionsanalyse von FLJ25132 in Ovarialtumoren (n=8) und in Prostatakarzinomen (n=10) im Vergleich zu jeweils gesunden Gewebeproben; Lineare Darstellung der relativen Expression (fache Aktivierung).

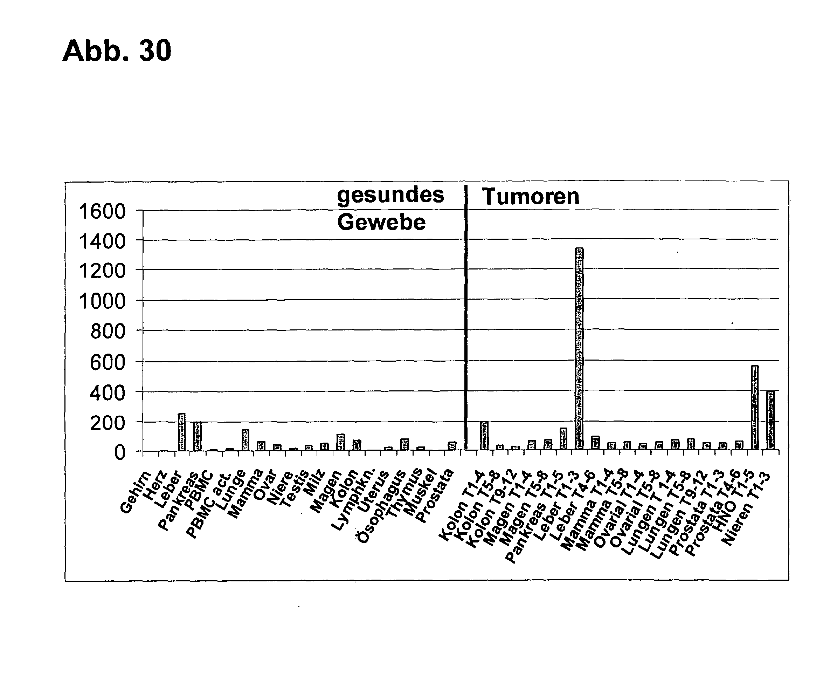

### Abb.30: qRT-PCR Analyse der SEQ ID NO: 57-spezifischen Expression

Quantitative Expressionsanalyse der SEQ ID NO: 57 in gesunden Gewebeproben (links) und in Tumoren (Pools bestehend aus jeweils 3-5 Einzelproben, rechts). Lineare Darstellung der relativen Expression (fache Aktivierung).

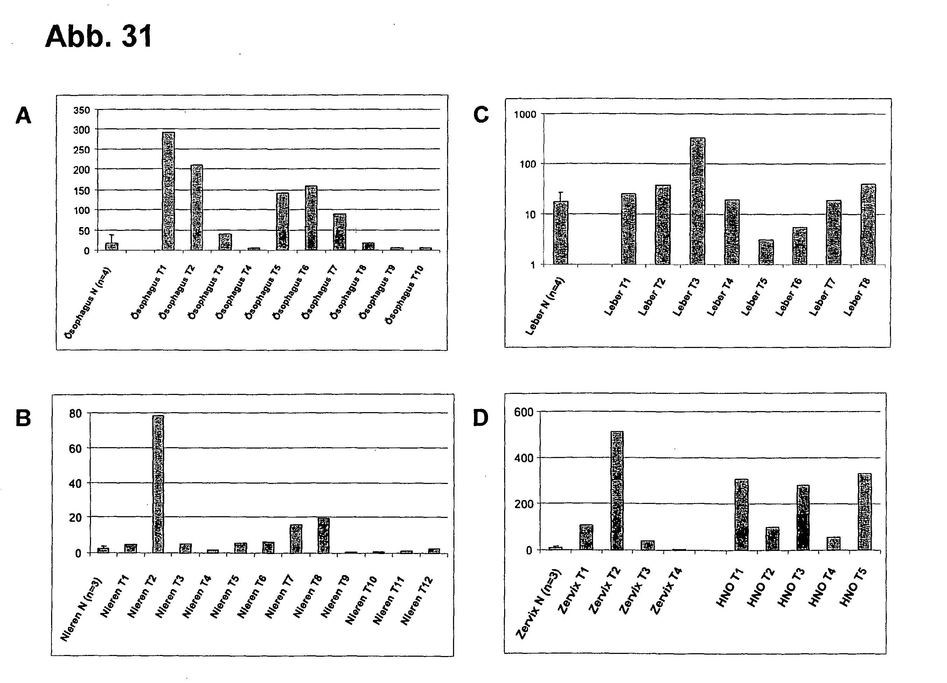

### Abb.31: Detailanalyse der SEQ ID NO: 57-spezifischen Expression in verschiedenen Tumortypen

Quantitative Expressionsanalyse der SEQ ID NO: 57 in **A** Ösophagustumoren (n=8), **B** Leberkarzinomen (n=8), **C** Nierenkarzinomen und **D** Zervix- und HNO-Tumoren im Vergleich zu jeweils gesunden Gewebeproben; Lineare (A, C, D) bzw. Logarithmische (B) Darstellung der relativen Expression (fache Aktivierung).

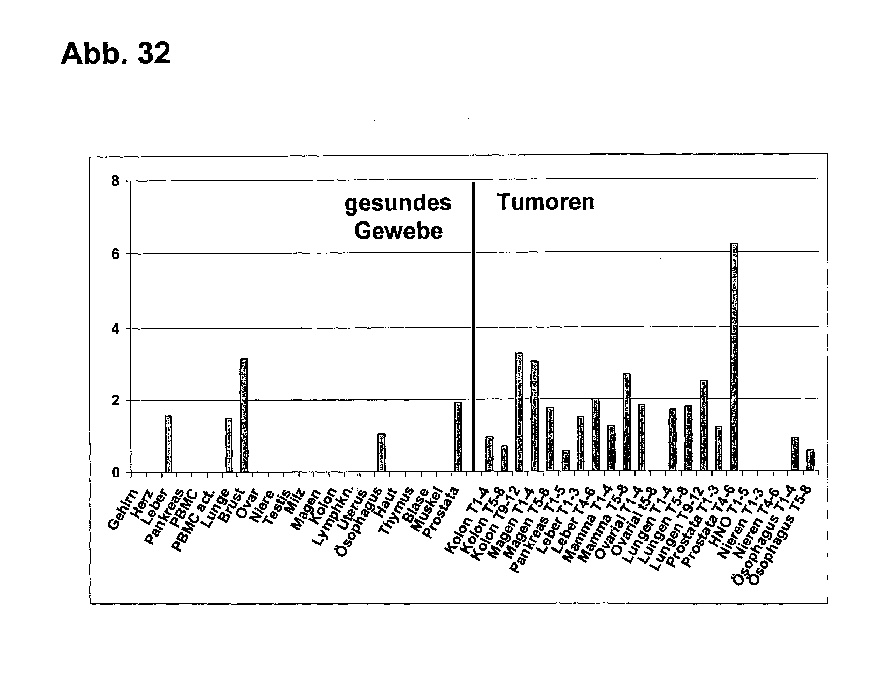

### Abb.32: qRT-PCR Analyse der LOC119395-spezifischen Expression

Quantitative Expressionsanalyse von LOC119395 in gesunden Gewebeproben (links) und in Tumoren (Pools bestehend aus jeweils 3-5 Einzelproben, rechts). Lineare Darstellung der relativen Expression (fache Aktivierung).

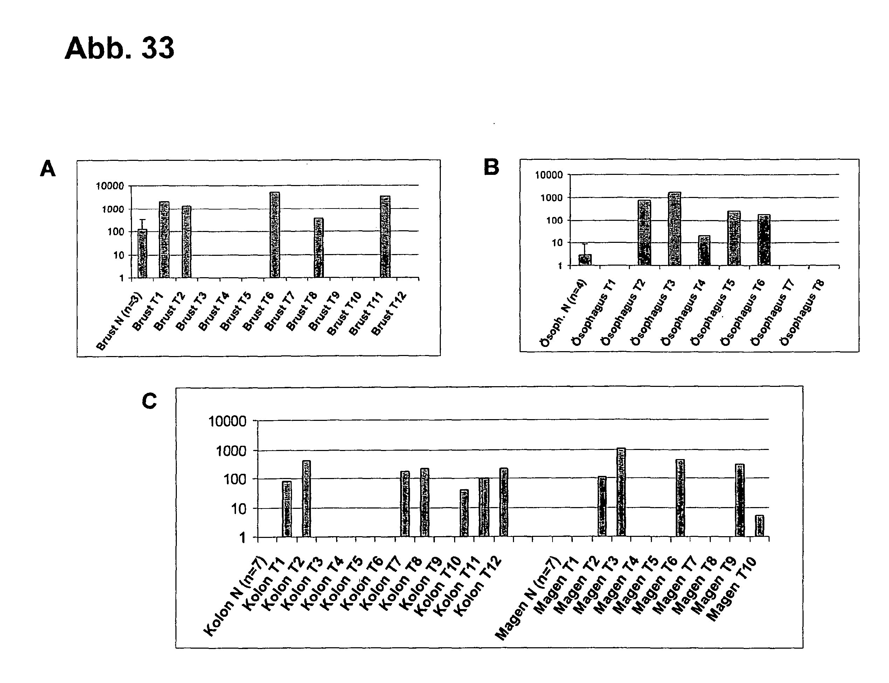

### Abb.33: Detailanalyse der LOC119395-spezirischen Expression in verschiedenen Tumortypen

Quantitative Expressionsanalyse von LOC119395 in **A** Brusttumoren (n=12), **B** Ösophaguskarzinomen (n=8) und C Kolon- und Magenkarzinomen im Vergleich zu jeweils gesunden Gewebeproben; Logarithmische Darstellung der relativen Expression (fache Aktivierung).

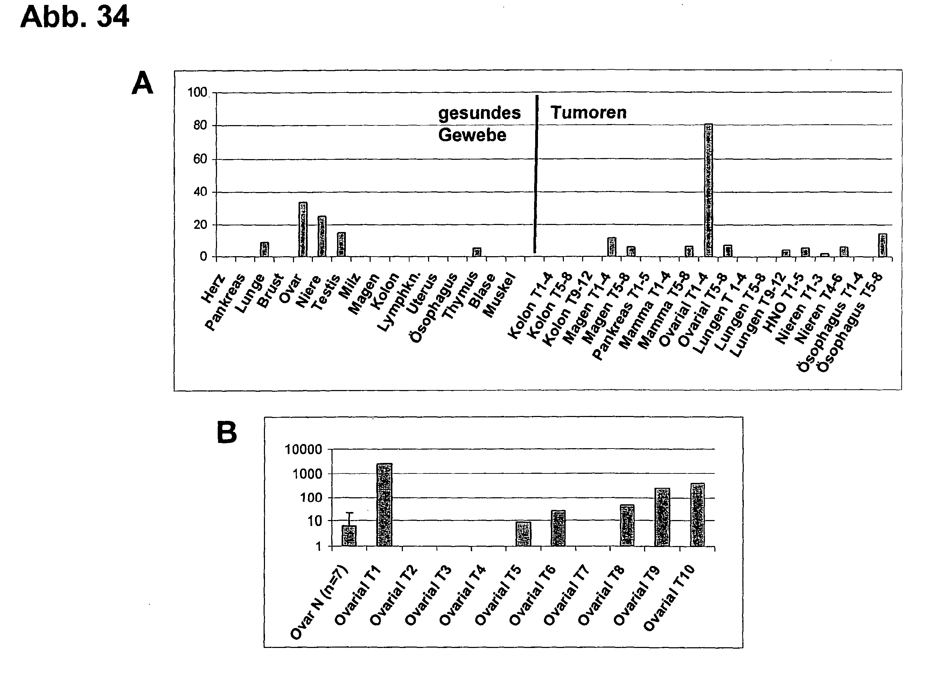

### Abb.34: qRT-PCR Analyse der LOC121838-spezifischen Expression

**A** Quantitative Analyse der LOC121838-spezifischen Expression in gesunden Gewebeproben (links) und in Tumoren (Pools bestehend aus jeweils 3-5 Einzelproben, rechts). Lineare Darstellung der relativen Expression (fache Aktivierung). **B** Detailanalyse von LOC121838-spezifischer RNA in Ovarialgeweben, logarithmische Darstellung.

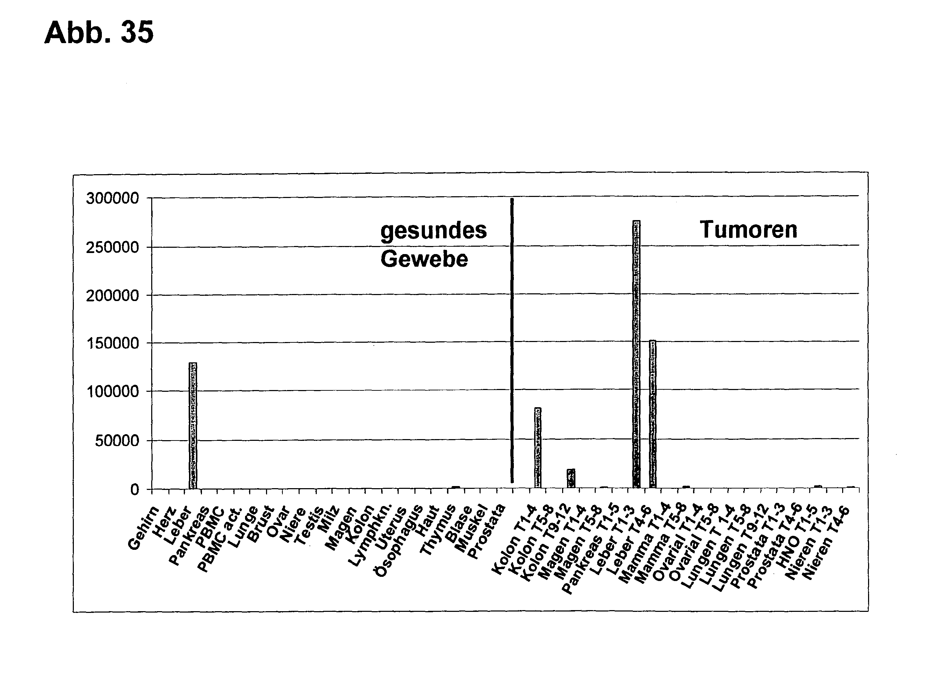

### Abb.35: qRT-PCR Analyse der LOC221103-spezifischen Expression

Quantitative Expressionsanalyse von LOC221103-RNA in gesunden Gewebeproben (links) und in Tumoren (Pools bestehend aus jeweils 3-5 Einzelproben, rechts). Lineare Darstellung der relativen Expression (fache Aktivierung).

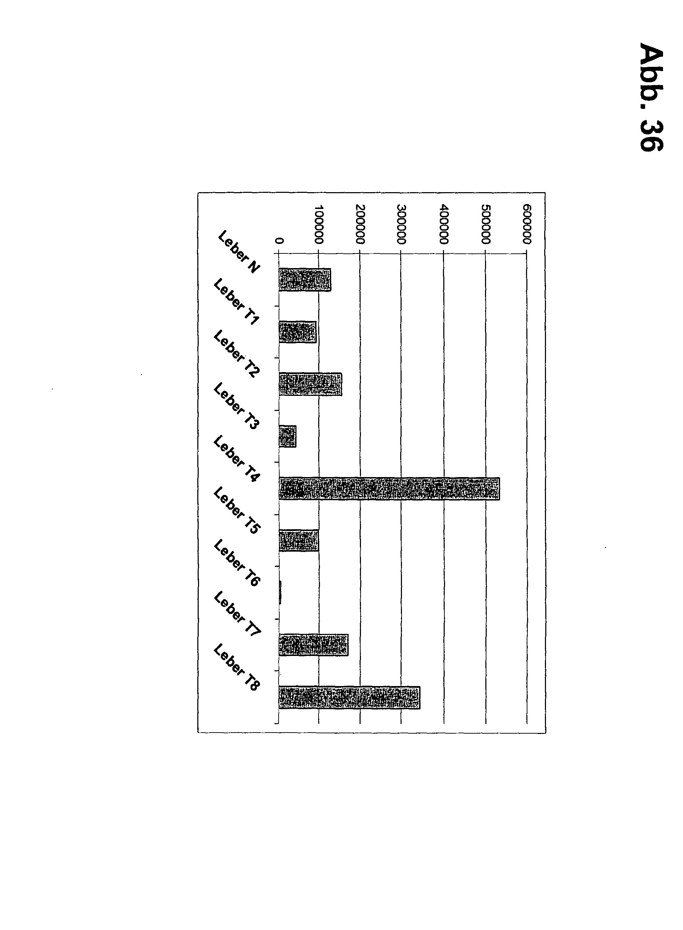

### Abb.36: Detaillierte qRT-PCR Analyse der LOC221103-spezifischen Expression in Leberproben

Quantitative Expressionsanalyse von LOC221103-RNA in Lebertumoren (n=8) und in einer gesunden Leberprobe. Lineare Darstellung der relativen Expression (fache Aktivierung).

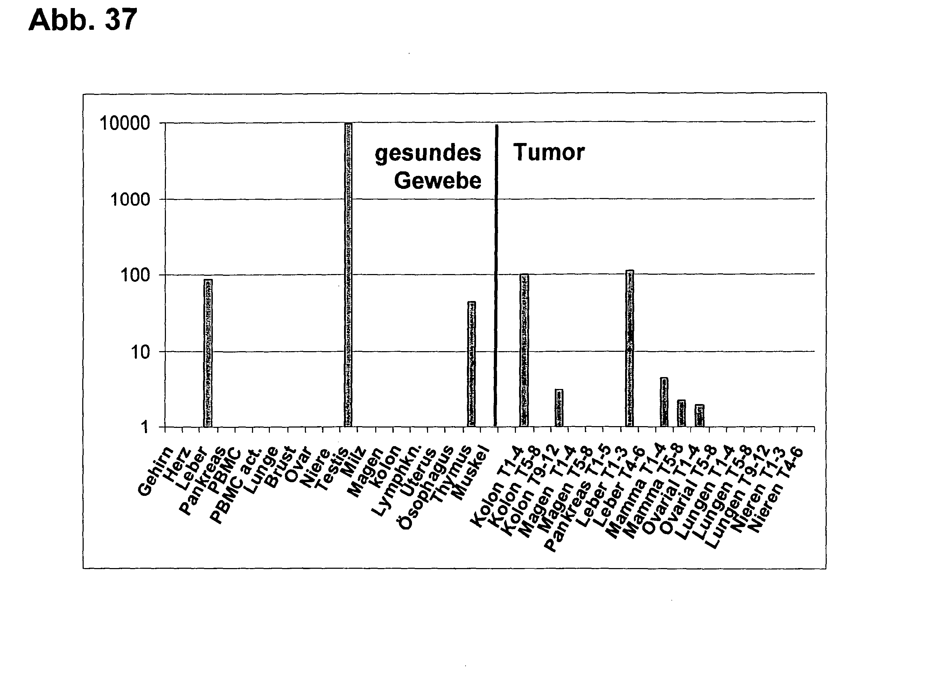

### Abb.37: qRT-PCR Analyse der LOC338579-spezifischen Expression

Quantitative Expressionsanalyse von LOC338579-spezifischer RNA in gesunden Gewebeproben (links) und in Tumoren (Pools bestehend aus jeweils 3-5 Einzelproben, rechts). Logarithmische Darstellung der relativen Expression (fache Aktivierung).

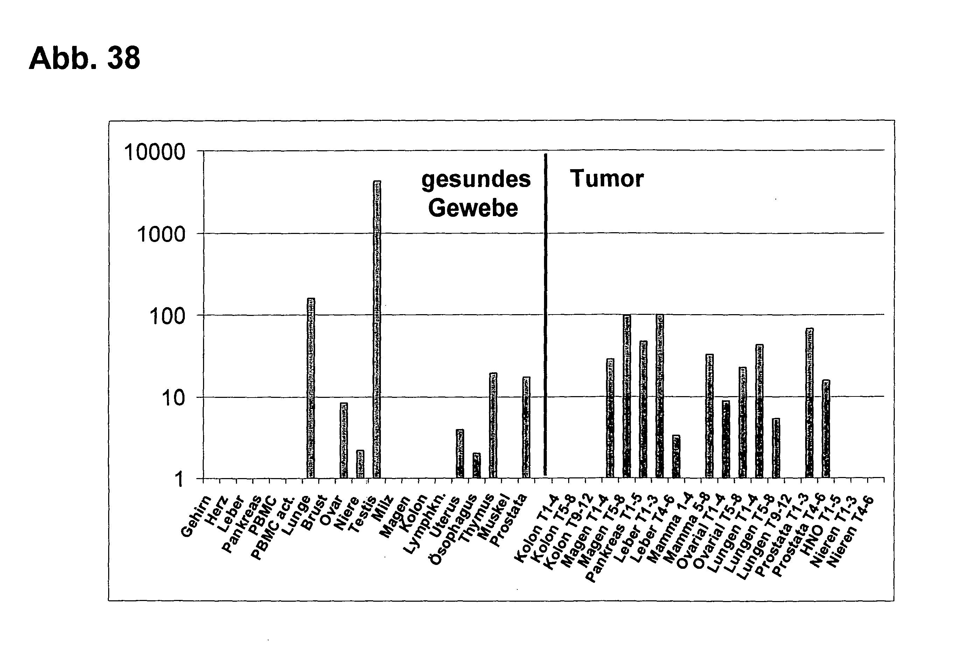

### Abb.38: qRT-PCR Analyse der LOC90342-spezifischen Expression

Quantitative Expressionsanalyse von LOC90342-spezifischer RNA in gesunden Gewebeproben (links) und in Tumoren (Pools bestehend aus jeweils 3-5 Einzelproben, rechts). Logarithmische Darstellung der relativen Expression (fache Aktivierung).

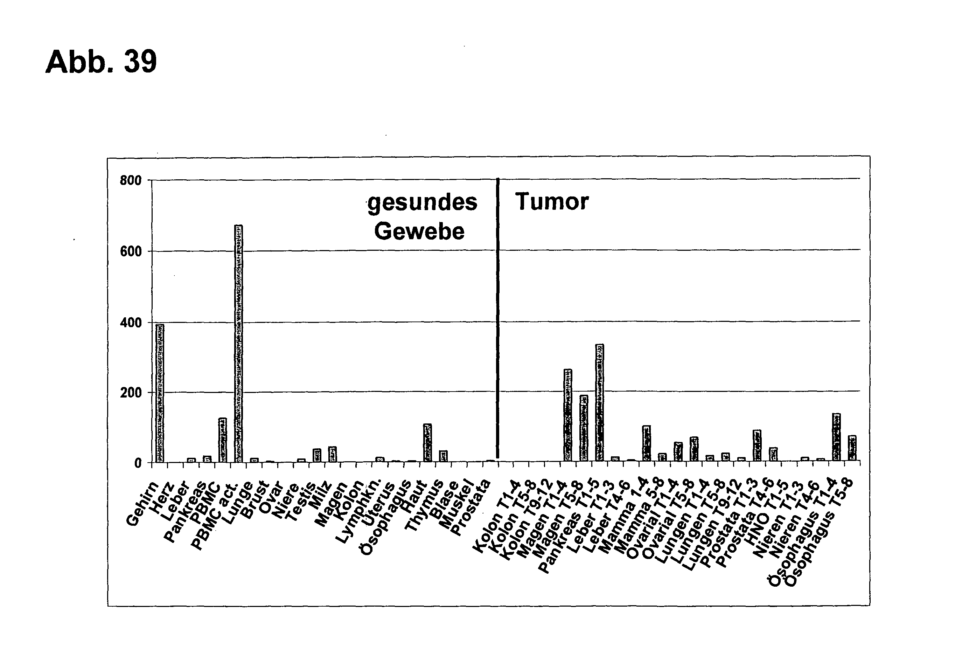

### Abb.39: qRT-PCR Analyse der LRFN1-spezifischen Expression

Quantitative Expressionsanalyse von LRFN1-spezifischer RNA in gesunden Gewebeproben (links) und in Tumoren (Pools bestehend aus jeweils 3-5 Einzelproben, rechts). Logarithmische Darstellung der relativen Expression (fache Aktivierung).

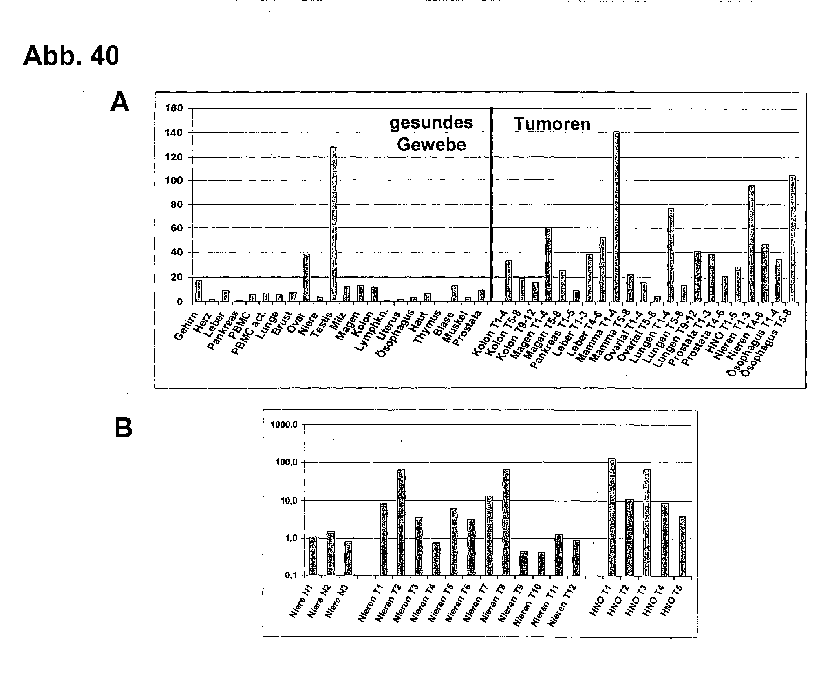

### Abb.40: qRT-PCR Analyse der LOC285916-spezifischen Expression

**A** Quantitative Analyse der LOC285916-spezifischen Expression in gesunden Gewebeproben (links) und in Tumoren (Pools bestehend aus jeweils 3-5 Einzelproben, rechts). Lineare Darstellung der relativen Expression (fache Aktivierung). **B** Detailanalyse von LOC285916-spezifischer RNA in Nierengeweben und in HNO-Tumoren, logarithmische Darstellung.

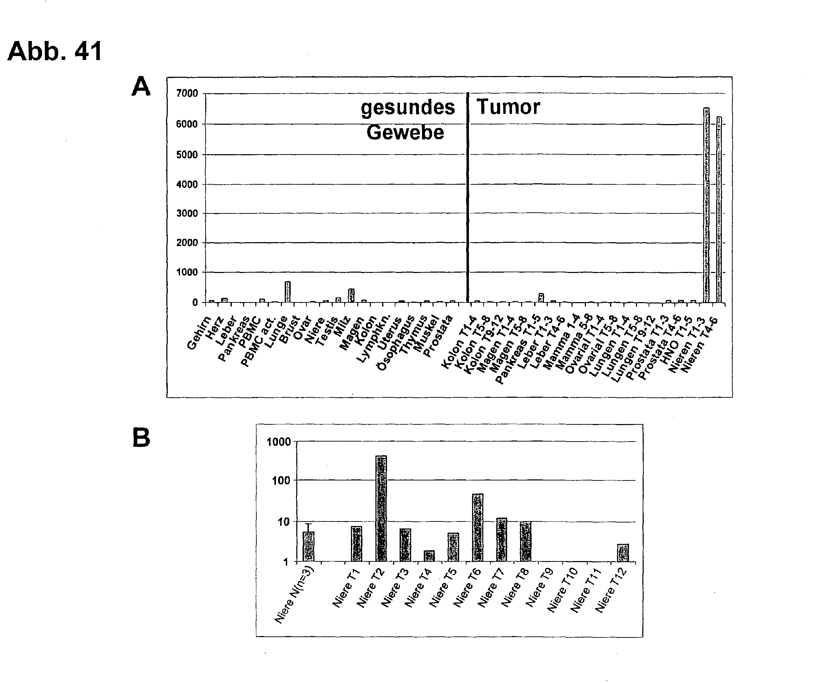

### Abb.41: qRT-PCR Analyse der MGC71744-spezifischen Expression

**A** Quantitative Analyse der MGC71744-spezifischen Expression in gesunden Gewebeproben (links) und in Tumoren (Pools bestehend aus jeweils 3-5 Einzelproben, rechts). Lineare Darstellung der relativen Expression (fache Aktivierung). **B** Detailanalyse von MGC71744-spezifischer RNA in verschiedenen Nierengeweben, logarithmische Darstellung.

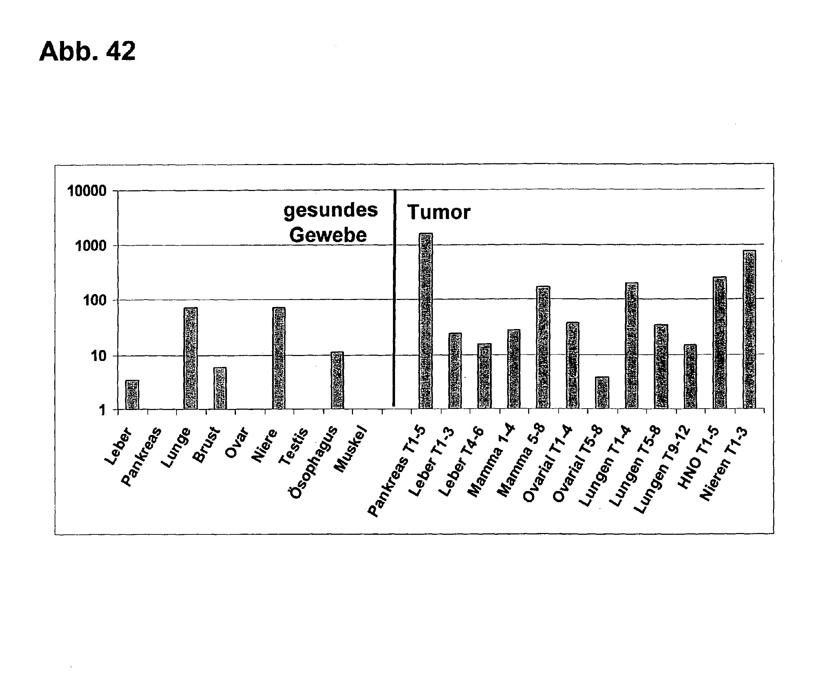

### Abb.42: qRT-PCR Analyse der LOC342982-spezifischen Expression

Quantitative Expressionsanalyse von LOC342982-spezifischer RNA in gesunden Gewebeproben (links) und in Tumoren (Pools bestehend aus jeweils 3-5 Einzelproben, rechts). Logarithmische Darstellung der relativen Expression (fache Aktivierung).

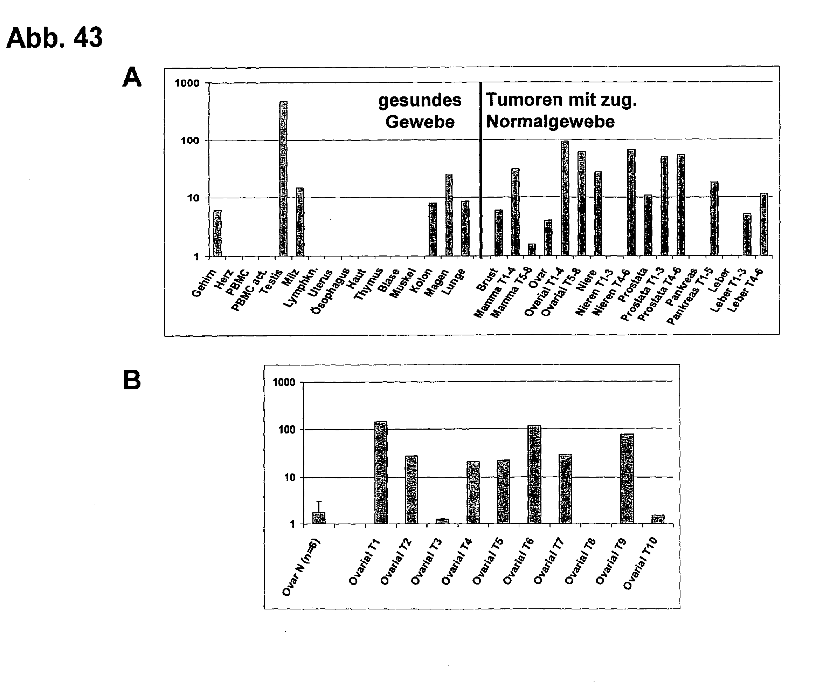

### Abb.43: qRT-PCR Analyse der LOC343169-spezifischen Expression

**A** Quantitative Analyse der LOC343169-spezifischen Expression in gesunden Gewebeproben (links) und in Tumoren (Pools bestehend aus jeweils 3-5 Einzelproben, rechts). Lineare Darstellung der relativen Expression (fache Aktivierung). **B** Detailanalyse von LOC343169-spezifischer RNA in verschiedenen Ovarialgeweben, logarithmische Darstellung.

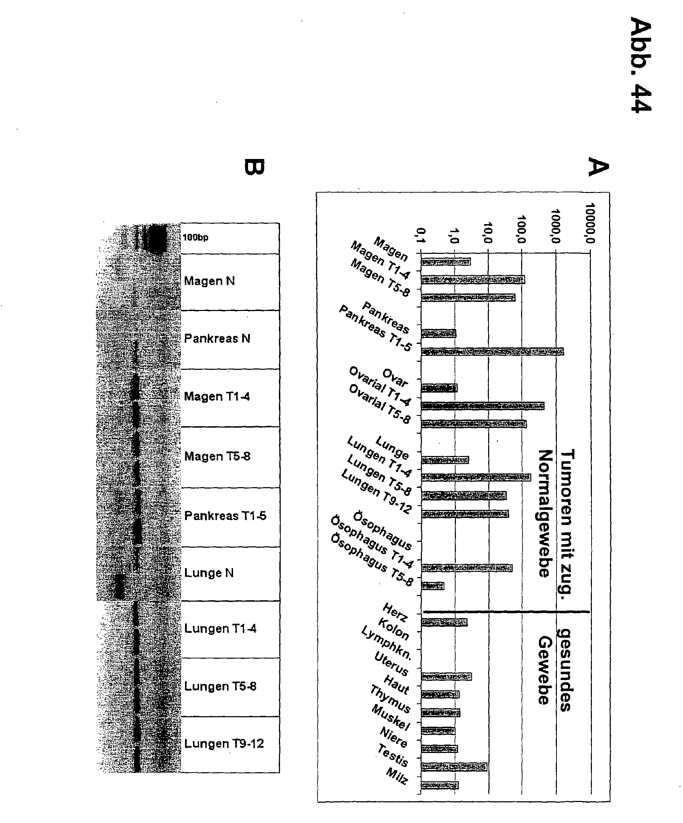

### Abb.44: qRT-PCR Analyse der LOC340204-spezifischen Expression

**A** Quantitative Analyse der LOC340204-spezifischen Expression in gesunden Gewebeproben (links) und in Tumoren (Pools bestehend aus jeweils 3-5 Einzelproben, rechts). Lineare Darstellung der relativen Expression (fache Aktivierung). **B** Gelbild ausgewählter Gewebeproben nach gelelektrophoretischer Auftrennung.

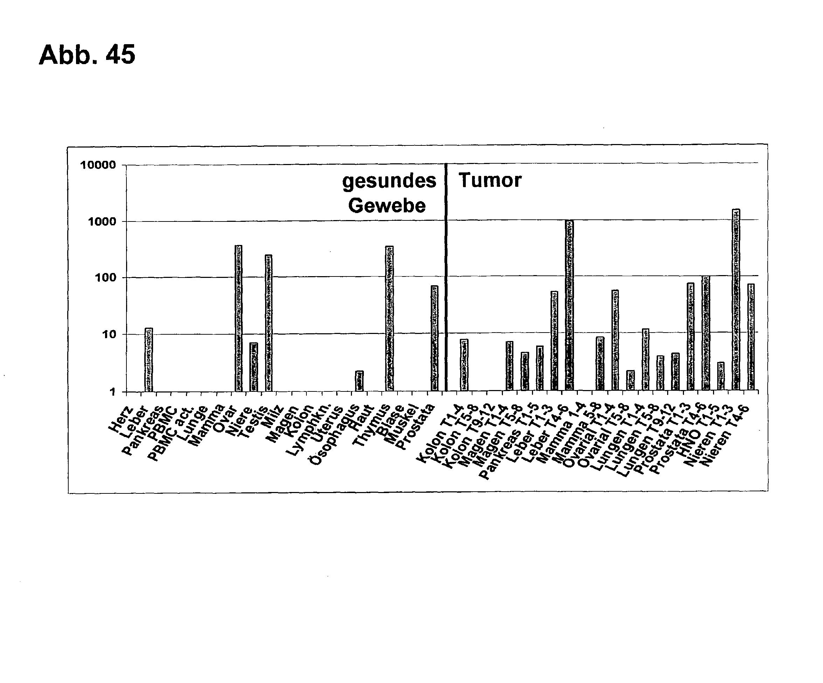

### Abb.45: qRT-PCR Analyse der LOC340067-spezifischen Expression

Quantitative Expressionsanalyse von LOC340067-spezifischer RNA in gesunden Gewebeproben (links) und in Tumoren (Pools bestehend aus jeweils 3-5 Einzelproben, rechts). Logarithmische Darstellung der relativen Expression (fache Aktivierung).

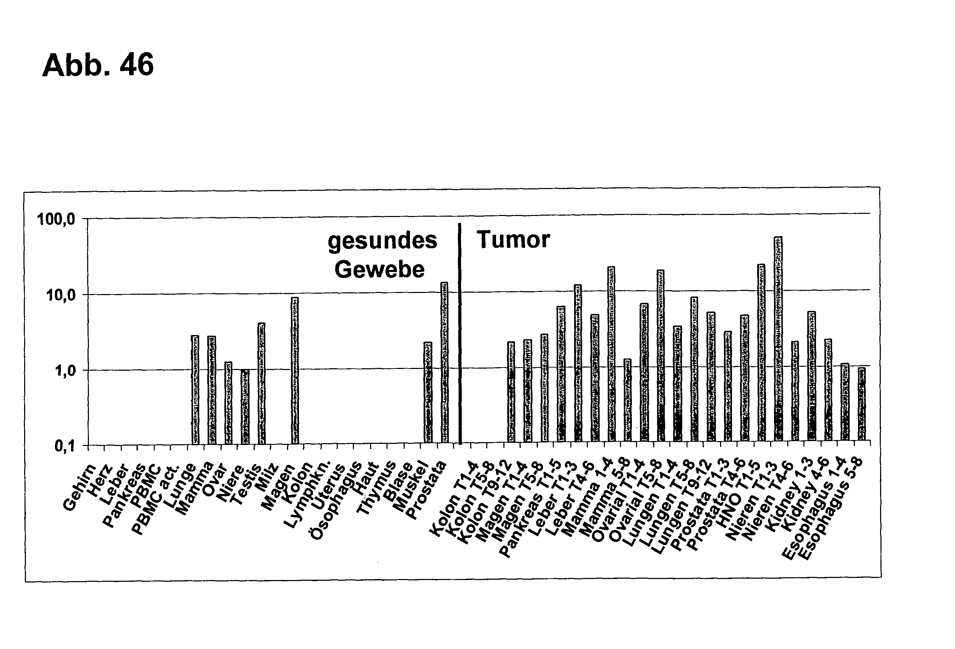

### Abb.46: qRT-PCR Analyse der LOC342780-spezifischen Expression

Quantitative Expressionsanalyse von LOC342780-spezifischer RNA in gesunden Gewebeproben (links) und in Tumoren (Pools bestehend aus jeweils 3-5 Einzelproben, rechts). Logarithmische Darstellung der relativen Expression (fache Aktivierung).

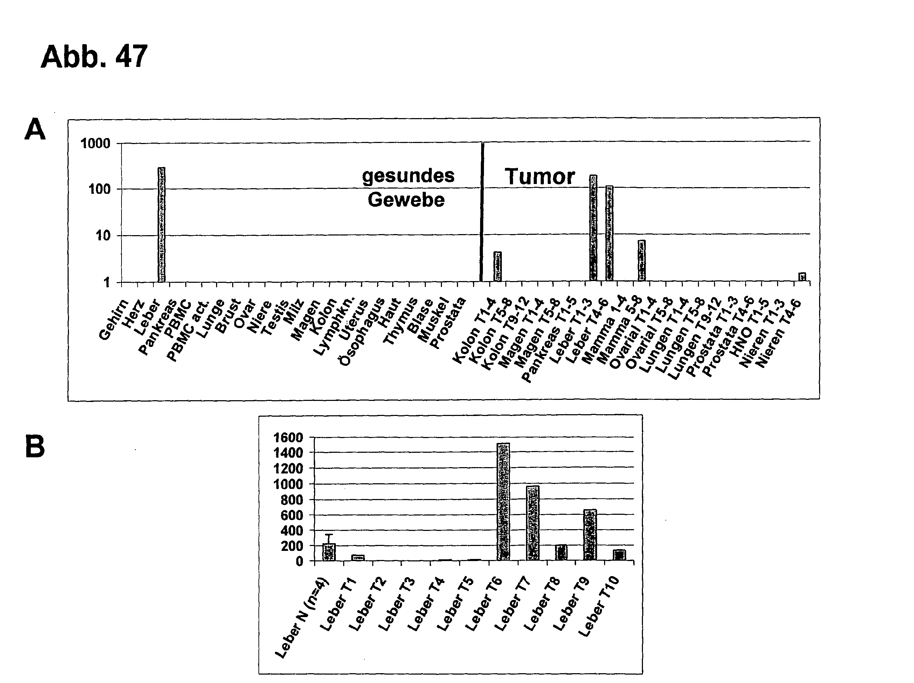

### Abb.47: qRT-PCR Analyse der LOC339511-spezifischen Expression

**A** Quantitative Analyse der LOC339511-spezifischen Expression in gesunden Gewebeproben (links) und in Tumoren (Pools bestehend aus jeweils 3-5 Einzelproben, rechts). Lineare Darstellung der relativen Expression (fache Aktivierung). **B** Detailanalyse von LOC339511-spezifischer RNA in verschiedenen Leber-spezifischen Geweben, lineare Darstellung.

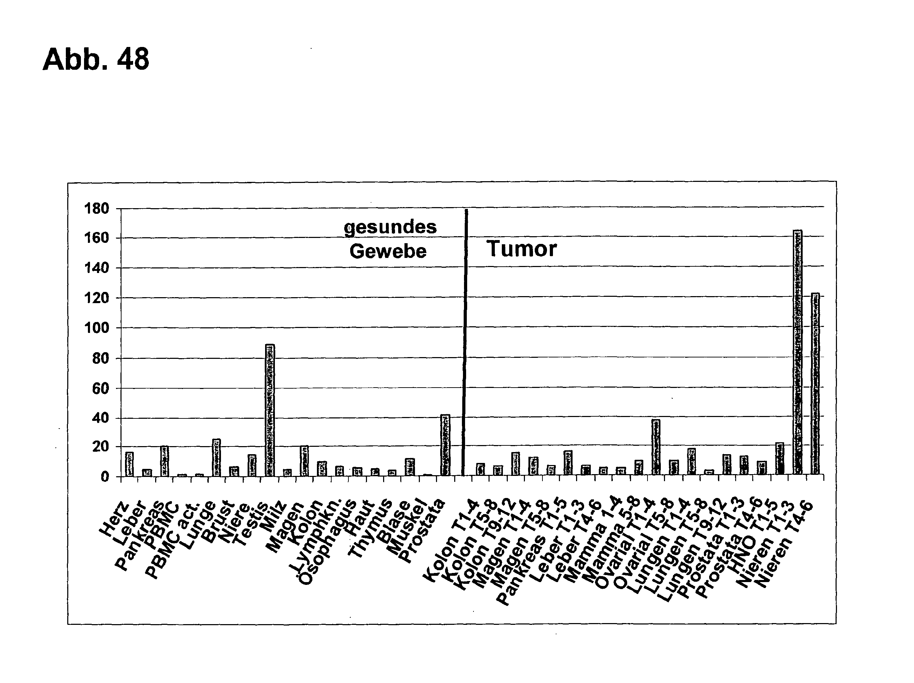

### Abb.48: qRT-PCR Analyse der C14orf37-spezifischen Expression

Quantitative Expressionsanalyse von C14orf37 in gesunden Gewebeproben (links) und in Tumoren (Pools bestehend aus jeweils 3-5 Einzelproben, rechts). Lineare Darstellung der relativen Expression (fache Aktivierung).

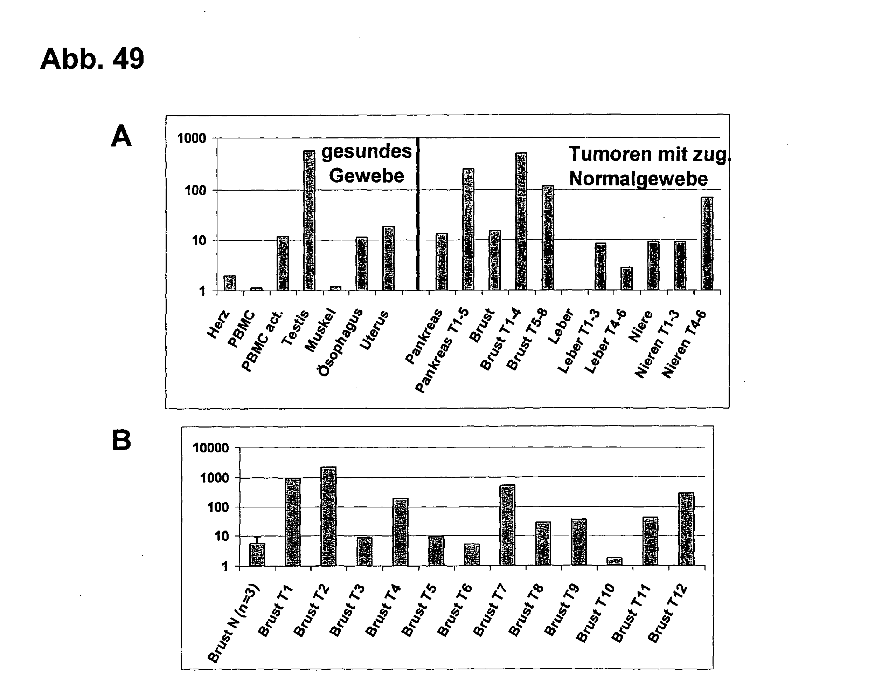

### Abb.49: qRT-PCR Analyse der ATP1A4-spezifischen Expression

**A** Quantitative Expressionsanalyse von ATP1A4 in gesunden Gewebeproben und in Tumoren (Pools bestehend aus jeweils 3-5 Einzelproben). Logarithmische Darstellung der relativen Expression (fache Aktivierung). **B** Detailanalyse von ATP1A4-spezifischer RNA in verschiedenen Brust-spezifischen Geweben, logarithmische Darstellung.

### Beispiele:

### Material und Methoden

Die Begriffe "*in silico*", und "elektronisch" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Verfahren erfolgen in an sich bekannter Weise und sind z.B. in Sambrook et al., Molecular Cloning: A Laboratory Manual, 2. Auflage (1989), Cold Spring Harbor Laboratory Press, Cold Spring Harbor, N.Y. beschrieben. Alle Verfahren, die die Verwendung von Kits und Reagenzien einschließen, sind entsprechend den Angaben der Hersteller durchgeführt.

### Beispiel 1:

### Datamining-basierte Strategie zur Identifizierung von Tumor-assoziierten Antigenen

Erfindungsgemäß wurden öffentliche humane Protein- und Nukleinsäuredatenbanken im Hinblick auf krebsspezifische Antigene untersucht, die auf der Zelloberfläche zugänglich sind. Die Definition der dafür notwendigen Filterkriterien zusammen mit einer Hochdurchsatz-Methodik zur Analyse möglichst aller Proteine bildeten den zentralen Bestandteil dieser Strategie.

Den Ausgangspunkt bildeten die hauptsächlich aus dem humanen Genomprojekt vorhergesagten potenziellen Gene, die in der "RefSeq" Datenbank (Pruitt et al., Trends Genet. Jan;16(1):44-47, 2000) des "National Center for Biotechnology Information" (NCBI) als rein modellhafte Protein- (XP-) bzw. mRNA-Einträge (XM-) abgelegt sind. In einem weiteren Ansatz wurden auch die validierten Proteineinträge (NP-) bzw. die korrespondierenden mRNAs (NM-) derselben Datenbank in gleicher Weise analysiert. Dem Grundprinzip (hypothetisches) Gen zu mRNA zu Protein folgend wurden die Proteine unter Kombination mehrerer Prädiktionsprogramme für Proteinanalyse zunächst auf das Vorhanden sein von Transmembrandomänen hin untersucht. Aus der humanen XP-Fraktion der "RefSeq" Datenbank wurden insgesamt 19.544 Einträge analysiert, wobei 2.025 hypothetische Proteine den Filterkriterien genügten. Die humane NP-Fraktion lieferte insgesamt 19.110 Einträge mit einem Anteil von 4.634 gefilterten Proteinen.

Die korrespondierende mRNA jedes dieser 2.025 bzw. 4.634 Proteine wurde anschließend einer Homologiesuche in der EST-Datenbank (Boguski et al., Nat. Genet. 4(4):332-333, 1993) des NCBI mit Hilfe des "BLAST" Algorithmus (Altschul et al., Nucleic Acids Res.25:3389-3402, 1997) unterzogen. Die Filterkriterien wurden bei dieser Suche stringent eingestellt. Insgesamt 1.270 hypothetische mRNAs erzielten dabei mindestens einen Treffer in der EST-Datenbank, wobei die Anzahl der Treffer in Einzelfällen mehr als 1.000 betrug.

Für jeden Einzelnen dieser validen Treffer wurde anschließend die gewebsspezifische Herkunft der zugrunde liegenden cDNA Bibliothek sowie der Name der Bibliothek ermittelt. Die daraus resultierenden Gewebe wurden in vier verschiedene Gruppen eingeteilt, die von dispensiblen Organen (Gruppe 3) bis hin zu absolut lebensnotwendigen Organen reichten (Gruppe 0). Eine weitere Gruppe 4 bildeten alle Proben, die aus Krebsgewebe gewonnen wurden. Die Verteilung der Treffer auf die fünf Gruppen wurde in einer Tabelle festgehalten, die nach dem besten Verhältnis der Summe der Gruppen 3 und 4 gegenüber der Summe der Gruppen 0-2 sortiert wurde. Dabei erreichten diejenigen mRNAs einen Spitzenplatz, deren EST Treffer ausschließlich Krebsgewebe entstammten, gefolgt von denjenigen, die darüber hinaus noch in Geweben dispensibler Organe der Gruppe 3 zu finden sind.

Da es sich bei den im ersten Ansatz ermittelten Transkripten und den korrespondierenden Proteinen zunächst um hypothetische Konstrukte handelt, wurden noch weitere Filterkriterien hinzugezogen, die die reale Existenz der mRNAs und damit auch der Proteine belegen sollten. Dazu wurde jede mRNA mit dem vorhergesagten Genlokus verglichen. Nur diejenigen Transkripte, die mindestens einen Spleißvorgang aufweisen, d.h. die sich auf mindestens 2 Exons verteilen, wurden für weitergehende Analysen verwendet.

Die sequenzielle Anwendung aller genannten Filter führte zu den erfindungsgemäßen Tumor-assoziierten Antigenen, die aufgrund einer vorhergesagten Transmembrandomäne und der damit verbundenen Topologie als von extrazellulär zugänglich anzusehen sind. Das aus den EST-Daten abgeleitete Expressionsprofil weist in allen Fällen auf eine krebsspezifische Expression hin, die sich höchstens noch auf dispensible Organe erstrecken kann.

### Beispiel 2:

### Validierungsstrategie der durch in silico Analyse identifizierten Tumor-assoziierten Antigene

Zur Nutzung der Targets für immuntherapeutische Zwecke (Antikörpertherapie mittels monoklonaler Antikörper, Vakzinierung, T-Zell Rezeptor-vermittelte therapeutische Ansätze; vgl. EP-B-0 879 282) oder andere zielgerichtete Ansätze (small compounds, siRNA etc.) bei der Krebstherapie sowie für diagnostische Fragestellungen ist die Validierung der erfindungsgemäß identifizierten Targets von zentraler Bedeutung. Die Validierung erfolgt dabei durch Expressionsanalyse sowohl auf RNA als auch auf Proteinebene.

### 1. Untersuchung der RNA Expression

Die erste Validierung der identifizierten Tumorantigene erfolgt mit Hilfe von RNA, die aus verschiedenen Geweben bzw. aus gewebespezifischen Zelllinien gewonnen wird. Weil das differentielle Expressionsmuster aus gesundem Gewebe im Vergleich zu Tumorgewebe eine entscheidende Bedeutung für die spätere therapeutische Anwendung hat, erfolgt die Charakterisierung der Zielgene bevorzugt mit Hilfe dieser Gewebeproben.

Die Isolierung von Gesamt-RNA aus nativen Gewebeproben oder aus Tumorzelllinien erfolgt mit Verfahren, die in der Molekularbiologie Standard sind. Zum Beispiel kann die Isolierung mit Hilfe des RNeasy Maxi Kits (Qiagen, Kat. Nr. 75162) nach Vorschrift durch den Hersteller erfolgen. Dieses Isolierungsverfahren beruht auf der Verwendung von Guanidiniumisothiocyanat als chaotropes Reagenz. Alternativ kann die Isolierung mit saurem Phenol durchgeführt werden (Chomczynski & Sacchi, Anal. Biochem. 162: 156-159, 1987). Nach Aufarbeitung des Gewebes mittel Guamdiniumisothiocyanat wird die RNA mit saurem Phenol extrahiert, anschließend die RNA mit Isopropanol gefällt und in DEPC-behandeltes Wasser aufgenommen.

2-4 µg der so isolierten RNA werden anschließend z.B. mittels Superscript II (Invitrogen) entsprechend dem Protokoll des Herstellers in cDNA umgeschrieben. Das Priming der cDNA Synthese erfolgt dabei mit Hilfe von zufälligen Hexameren (z.B. Roche Diagnostics) nach Standardprotokollen des jeweiligen Herstellers. Zur Qualitätskontrolle werden die cDNAs mit Primern in 30 Zyklen amplifiziert, die spezifisch für das nur gering exprimierte p53 Gen sind. Nur p53 positive cDNA Proben werden für die weiteren Reaktionsschritte verwendet.

Zur detaillierten Analyse der Targets wird auf Basis eines cDNA-Archivs, das aus verschiedenen Normal- und Tumorgeweben sowie aus Tumorzelllinien isoliert wurde, eine Expressionsanalyse mittels PCR bzw. quantitativer PCR (qPCR) durchgeführt. Dazu werden 0,5 µl cDNA aus dem obigen Ansatz mit einer DNA-Polymerase (z.B. 1 U HotStarTaq DNA-Polymerase, Qiagen) analog den Protokollen des jeweiligen Herstellers amplifiziert (Gesamtvolumen des Ansatzes: 25-50 µl). Neben der Polymerase enthält der Amplifikationsansatz 0,3 mM dNTPs, Reaktionsbuffer (Endkonzentration 1 x, abhängig vom Hersteller der DNA-Polymerase) und je 0,3 mM des gen-spezifischen forward und reverse Primers.

Die spezifischen Primer des Zielgens werden, soweit möglich, so ausgewählt, das sie in zwei unterschiedlichen Exons liegen und somit genomische Kontaminationen nicht zu falsch positiven Ergebnissen führen. Bei einer nicht quantitativen Endpunkt-PCR wird die cDNA typischerweise 15 Minuten bei 95°C inkubiert, um die DNA zu denaturieren und um das Hot-Start-Enzyms zu aktivieren. Anschließend wird die DNA in 35 Zyklen amplifiziert (1 min 95°C, 1 min Primer spezifische Hybridisierungstemperatur (ca. 55-65°C), 1 min 72°C zur Elongation der Amplifikate). 10 µl des PCR Ansatzes werden anschließend auf Agarosegelen aufgetragen und im elektrischen Feld aufgetrennt. Durch Färben mit Ethidiumbromid wird die DNA in den Gelen sichtbar gemacht und das Ergebnis der PCR durch ein Foto dokumentiert.

Alternativ zur konventionellen PCR kann die Expressionsanalyse eines Zielgens auch durch quantitative real time PCR erfolgen. Zu dieser Analyse sind inzwischen verschiedene Analysesysteme erhältlich, die bekanntesten sind das ABI PRISM Sequence detection system (TaqMan, Applied Biosystems), der iCycler (Biorad) sowie der Light cycler (Roche Diagnostics). Wie oben beschrieben wird ein spezifischer PCR Ansatz einem Lauf in den real time Geräten unterzogen. Durch Zusatz eines DNA interkalierenden Farbstoffes (z.B Ethidiumbromid, CybrGreen) wird die neu synthetisierte DNA durch spezifische Lichtanregung (nach Angaben der Farbstoffhersteller) sichtbar gemacht. Durch eine Vielzahl von Messpunkten während der Amplifikation kann der gesamte Prozess verfolgt und eine quantitative Bestimmung der Nukleinsäurekonzentration des Zielgens durchgeführt werden. Die Normalisierung des PCR Ansatzes erfolgt durch Messung eines "housekeeping Gens" (z.B. 18S RNA, β-Actin). Alternative Strategien über Fluoreszenz- markierte DNA-Sonden erlauben ebenfalls die quantitative Bestimmung des Zielgens aus einer spezifischen Gewebeprobe (siehe TaqMan Applikationen der Fa. Applied Biosystems).

### 2. Klonierung

Die Klonierung des gesamten Zielgens, die für die weitere Charakterisierung des Tumorantigens notwendig ist, erfolgt nach gängigen molekularbiologischen Verfahren (z.B. in "Current Protocols in Molecular Biology", John Wiley & Sons Ltd., Wiley InterScience). Zur Klonierung bzw. Sequenzanalyse des Zielgens wird dieses zunächst mit einer DNA-Polymerase mit "proof reading Funktion" (z.B. pfu, Roche Diagnostics) amplifiziert. Das Amplifikat wird anschließend mit Standardverfahren in einen Klonierungsvektor ligiert. Positive Klone werden durch Sequenzanalyse identifiziert und anschließend mit Hilfe von Prädiktionsprogrammen und bekannten Algorithmen charakterisiert.

### 3. Prädiktion des Proteins

Viele erfindungsgemäß gefundene Gene (insbesondere aus der XM Domäne der RefSeq) sind Gen-Neuentdeckungen, für die das Volllänge Gen kloniert, das offene Leseraster ermittelt und die Proteinsequenz abgeleitet und analysiert werden muss.
Für die Volllängeklonierung der Sequenz haben wir gängige Protokolle zur "Rapid amplification of cDNA ends", sowie Screening von cDNA Expressionsbanken mit genspezifischen Sonden verwendet *(*Sambrook et al., Molecular Cloning: A Laboratory Manual, 2. Auflage (1989), Cold Spring Harbor Laboratory Press, Cold Spring Harbor, N.Y*.*).
Nach Zusammensetzung der so gefundenen Fragmente wurden potentielle Offene Leseraster (ORF) durch gängige Prädiktionsprogramme prädiziert. Da durch die Position des PolyA-Schwanzes und Polyadenylierungs-Motiven die Orientierung des potentiellen Genproduktes vorgegeben wird, verbleiben von möglichen 6 Leserastern nur noch die 3 der jeweiligen Orientierung. Oft ergibt sich aus diesen nur ein hinreichend großes offenes Leseraster, das für ein Protein kodieren kann, während die anderen Leseraster zu viele Stop-Codons aufweisen und für kein realistisches Proteine kodieren würden. Bei alternativen offenen Leserastem unterstützt die Berücksichtigung der Kozak-Kriterien für optimale Transkriptions-Initierung sowie die Analyse der sich potentiell ergebenden abgeleiteten Proteinsequenzen die Identifizierung des authentischen ORF. Dies wird weiter verifiziert durch Generierung von Immunseren gegen abgeleitete Proteine der potentiellen ORFs und ihre Analyse auf Erkennung eines realen Proteins in Geweben und Zelllinien.

### 4. Gewinnung von Antikörpern

Die Charakterisierung der erfindungsgemäß identifizierten Tumor-assoziierten Antigene erfolgt beispielsweise durch die Verwendung von Antikörpern. Ferner umfasst die Erfindung die diagnostische oder therapeutische Verwendung von Antikörpern. Dabei können Antikörper Proteine in nativem und/oder denaturierten Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein als auch mit extrazellulären Teilsequenzen immunisiert werden.

### Immunisierung und Gewinnung von polyklonalen Antikörpern

Eine Spezies (z.B. Kaninchen, Mäuse) wird durch eine erste Injektion des gewünschten Zielproteins immunisiert. Durch eine zweite oder dritte Immunisierung innerhalb eines definierten Zeitraums (ca. 2-4 Wochen nach der letzten Immunisierung) lässt sich die Immunantwort des Tieres gegen das Immunogen verstärken. Wiederum nach verschiedenen definierten Zeitabständen (1. Blutung nach 4 Wochen, anschließend alle 2-3 Wochen bis zu 5 Entnahmen) wird den Tieren Blut entnommen und Immunserum gewonnen. Die so entnommenen Immunseren enthalten polyklonalen Antikörper, mit denen das Zielprotein im Western blot, durch die Durchflusszytometrie, Immunfluoreszenz oder Immunhistochemie nachgewiesen und charakterisiert werden kann.

Die Immunisierung der Tiere erfolgt in der Regel über eines von vier gut etablierten Verfahren, wobei auch andere Verfahren existieren. Immunisiert werden kann dabei mit für das Zielprotein spezifischen Peptiden, dem gesamten Protein, mit extrazellulären Teilsequenzen eines Proteins, das experimentell oder über Prädiktionsprogramme identifiziert werden kann. Da die Prädiktionsprogramme nicht immer fehlerfrei arbeiten wird u.U. auch mit zwei Domänen gearbeitet, die voneinander durch eine Transmembrandomäne getrennt sind. Eine der beiden Domänen muss dann extrazellulär sein, was dann experimentell belegt werden kann (siehe nachstehend).
(1) Im ersten Fall werden Peptide (Länge: 8-12 Aminosäuren) über *in vitro* Verfahren synthetisiert (durch einen kommerziellen Service möglich) und diese Peptide zur Immunisierung verwendet. In der Regel erfolgen 3 Immunisierungen (z.B. mit einer Konzentration von 5-100 µg/Immunisierung). Die Durchführung der Immunisierung kann auch als Service von Dienstleistem erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei *in vitro,* in Bakterien (z.B. *E. coli*), in Hefe (z.B. *S. pombe*), in Insektenzellen oder in Säugetierzellen synthetisiert. Dabei ist auch die Synthese des Zielproteins mit Hilfe von viralen Expressionssystemen möglich (z.B. Baculovirus, Vacciniavirus, Adenovirus). Nach Synthese in einem der Systeme wird das Zielprotein aufgereinigt. Die Aufreinigung erfolgt dabei in der Regel über chromatographische Verfahren. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; GST-Fusionsproteine). Eine Vielzahl von Protokollen befinden sich z.B. in den "Current Protocols in Molecular Biology", John Wiley & Sons Ltd., Wiley InterScience. Nach Reinigung des Zielproteins erfolgt eine Immunisierung wie vorstehend beschrieben.
(3) Falls eine Zelllinie zur Verfügung steht, die das gewünschte Protein endogen synthetisiert, kann auch diese Zelllinie direkt zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA-Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend wird DNA (z.B. 1-10 µg pro Injektion) als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielprotein (Jung et al., Mol. Cells 12: 41-49, 2001; Kasinrerk et al., Hybrid Hybridomics 21: 287-293, 2002).

### Gewinnung monoklonaler Antikörper

Monoklonale Antikörper werden traditionell mit Hilfe der Hybridoma Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447). Als ein neues Verfahren wird auch die so genannte "SLAM" Technologie eingesetzt. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Eilzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

### 5. Validierung der Targets mit proteinchemischen Verfahren unter Verwendung von Antikörpern

Mit den Antikörpern, die wie vorstehend beschrieben herstellbar sind, lassen sich eine Reihe von wichtigen Aussagen zu dem Targetprotein treffen. Im Einzelnen sind die nachstehenden Analysen zur Validierung des Zielproteins sinnvoll:

### Spezifität des Antikörpers

Um zu zeigen, dass ein Antikörper spezifisch nur an das gewünschte Zielprotein bindet, eignen sich am besten auf Zellkultur-basierende Tests mit anschließendem Western blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter Kontrolle eines starken eukaryontischen Promotors steht (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgen-spezifische RT-PCR). Zur Kontrolle werden im Experiment im Idealfall homologe Gene mit transfiziert, um im folgenden Western blot die Spezifität des analysierten Antikörpers nachweisen zu können.

Im anschließenden Western blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid Gelelektrophorese, SDS-PAGE). Anschließend werden die Proteine durch eines von mehreren Blotting Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

### Lokalisation des Zielproteins

Zur Bestätigung der im in silico Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western blot) oder aber mit Plasmid-DNA transfiziert worden sind. Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz die Demonstration, ob ein immunogenes Epitop innerhalb oder außerhalb der Zelle lokalisiert ist. Die Prädiktion der ausgewählten Proteine kann so experimentell untermauert werden.

Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zu Immunfluoreszenz kann durch Verwendung von z.B. Propidiumiodid oder Trypanblau zwischen toten und lebenden Zellen unterschieden werden und damit falsch positive Ergebnisse vermieden werden.

Ein weiterer wichtiger Nachweis erfolgt durch die Immunhistochemie (IHC) an spezifischen Gewebeproben. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder in "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativlcontrollen können dabei auch Zelllinien dienen, bei denen die Präsenz des Zielgens durch RT-PCR Analysen bekannt ist. Eine Hintergrundkontrolle ist immer mitzuführen.

Formalin-fixierte (ein anderes Fixierungsverfahren mit z.B. Methanol ist auch möglich) und in Paraffin eingebettete Gewebestücke mit einer Dicke von 4µm werden auf einem Glasträger aufgebracht und z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der Alkalischen Phosphatase (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Analyse von Proteinmodifikationen

Sekundäre Proteinmodifikationen wie zum Beispiel N- und O-Glykosylierungen oder Myristilierungen können die Zugänglichkeit von immunogenen Epitopen behindern oder sogar ganz verhindern und damit die Wirksamkeit von Antikörpertherapien in Frage stellen. Zudem konnte vielfach nachgewiesen werden, dass sich Art und Menge der sekundären Modifikationen in Normal- und Tumorgewebe unterscheiden (z.B. Durand & Seta, 2000; Clin. Chem. 46: 795-805; Hakomori, 1996; Cancer Res. 56: 5309-18). Die Analyse dieser Modifikationen ist daher essentiell für den Therapieerfolg eines Antikörpers. Potentielle Bindestellen lassen sich durch spezifische Algorithmen prädizieren.

Die Analyse von Proteinmodifikationen erfolgt in der Regel im Western blot (siehe vorstehend). Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N- Glykosylasen inkubiert (nach Angaben des jeweiligen Herstellers, z.B. PNgase, Endoglykosidase F, Endoglykosidase H, Roche Diagnostics). Anschließend erfolgt ein Western blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden.

### Funktionsanalyse des Zielgens

Die Funktion des Targetmoleküls kann entscheidend für seinen therapeutischen Nutzen sein, so dass funktionelle Analysen ein wichtiger Baustein bei der Charakterisierung von therapeutisch nutzbaren Molekülen sind. Die Funktionsanalyse kann entweder in Zellen in Zellkulturexperimenten oder aber in vivo mit Hilfe von Tiermodellen erfolgen. Dabei wird das Gen des Zielmoleküls entweder durch Mutation ausgeschaltet ("knockout") oder aber die Zielsequenz in die Zelle bzw. den Organismus eingefügt ("knockin"). Man kann so funktionelle Veränderungen im zellulären Kontext einerseits durch den Funktionsverlust des zu analysierenden Genes ("loss of function") analysieren. Im zweiten Fall lassen sich Veränderungen analysieren, die durch die Ergänzung des analysierten Genes verursacht werden ("gain of function").

### a. Funktionsanalyse in Zellen

Transfektion. Zur Analyse des "gain of function" muss das Gen des Zielmoleküls in die Zelle transferiert werden. Dazu werden Zellen mit einer DNA transfiziert, die die Synthese des Zielmoleküls erlauben. In der Regel steht das Gen des Zielmoleküls dabei unter Kontrolle eines starken eukaryontischen Promotors (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das Gen kann dabei entweder ohne genomische Integration transient oder aber mit genomischer Integration nach Selektion mit z.B. Neomycin stabil synthetisiert werden.

RNA interference (siRNA). Eine Expressionsinhibition des Zielgens, die unter Umständen einen vollständigen Funktionsverlust des Zielmoleküls in Zellen induziert, kann durch die "RNA interference" (siRNA) Technologie in Zellen erzeugt werden (Hannon, GJ. 2002. RNA interference. Nature 418: 244-51; Czauderna et al. 2003. Nucl. Acid Res. 31: 670-82). Dazu werden Zellen mit kurzen, ca. 20-25 Nukleotide langen, doppelsträngigen RNA Molekülen transfiziert, die für das Zielmolekül spezifisch sind. Ein enzymatischer Prozess führt anschließend zum Abbau der spezifischen RNA des Zielgens und damit zu einer Funktionsinhibition des Zielproteins und ermöglicht damit die Analyse des Zielgens.

Zelllinien, die mittels Transfektion oder siRNA modifiziert wurden, können anschließend auf unterschiedliche Art und Weise analysiert werden. Nachstehend sind die geläufigsten Beispiele aufgeführt.

### 1. Proliferation und Zellzyklusverhalten

Eine Vielzahl von Verfahren sind zur Analyse der Zellproliferation etabliert und werden von verschiedenen Unternehmen kommerziell angeboten (z.B. Roche Diagnostics, Invitrogen, Details zu den Testverfahren sind in den zahlreichen Applikationsprotokollen beschrieben). Die Zellzahl in Zellkulturexperimenten lässt sich durch einfaches Auszählen oder durch kolometrische Tests ermitteln, die die metabolische Aktivität der Zellen messen (z.B. wst-1, Roche Diagnostics). Metabolische Testverfahren messen indirekt über enzymatische Marker die Zellzahl in einem Experiment. Direkt kann die Zellproliferation durch Analyse der DNA Syntheserate z.B. durch Zugabe von Bromdesoxyuridin (BrdU) gemessen werden, der Nachweis des integrierten BrdU erfolgt über spezifische Antikörper kolometrisch.

### 2. Apoptose und Zytotoxizität

Eine große Anzahl von Testsystemen zum Nachweis von zellulärer Apoptose und von Zytotoxizität sind verfügbar. Ein entscheidendes Charakteristikum ist die spezifische, enzymabhängige Fragmentierung der genomischen DNA, die irreversibel ist und sicher zum Tod der Zelle führt. Verfahren zum Nachweis dieser spezifischen DNA Fragmente sind kommerziell erhältlich. Als zusätzliches Verfahren steht der "TUNEL assay" zur Verfügung, der DNA Einzelstrangbrüche auch in Gewebeschnitten nachweisen kann. Zytotoxizität wird vor allem über eine veränderte Zellpermeabilität nachgewiesen, die als Marker für den Vitalitätszustand von Zellen dient. Dazu werden entweder im Zellkulturüberstand Marker analysiert, die normalerweise intrazellulär zu finden sind. Alternativ kann auch die Aufnahmefähigkeit von Farbmarkern analysiert werden, die von intakten Zellen nicht aufgenommen werden. Die bekanntesten Beispiele für Farbmarker sind Trypanblau und Propidiumiodid, ein üblicher intrazellulärer Marker ist die Laktatdehydrogenase, die im Überstand enzymatisch nachgewiesen werden kann. Unterschiedliche Testsysteme stehen von verschiedenen kommerziellen Anbietern (z.B. Roche Diagnostics, Invitrogene) zur Verfügung.

### 3. Migrationsassay

Die Fähigkeit von Zellen zur Migration wird in einem spezifischen Migrationstest vorzugsweise mit Hilfe einer Boyden Kammer (Coming Costar) analysiert (Cinamon G., Alon R. J. Immunol. Methods. 2003 Feb; 273(1-2):53-62; Stockton et al. 2001. Mol. Biol. Cell. 12: 1937-56). Dazu werden Zellen auf einem Filter mit spezifischer Porengröße kultiviert. Zellen, die migrieren können, sind in der Lage, durch diesen Filter in ein weiteres darunter liegendes Kulturgefäß zu wandern. Eine anschließende mikroskopische Analyse erlaubt dann die Bestimmung eines möglicherweise veränderten Migrationsverhaltens, dass durch den "gain of function" bzw. "loss of function" des Zielmoleküls induziert wurde.

### b. Funktionsanalyse in Tiermodellen

Alternativ zu Zellkulturexperimenten bieten sich zur Analyse der Zielgenfunktion aufwendige in vivo Experimente in Tiermodellen an. Diese Modelle haben im Vergleich zu den zellbasierenden Verfahren den Vorteil, dass sie Fehlentwicklungen bzw. Krankheiten nachweisen können, die erst im Kontext des gesamten Organismus nachweisbar sind. Eine Vielzahl von Modellen für humane Erkrankungen sind inzwischen verfügbar (Abate-Shen & Shen. 2002. Trends in Genetics S1-5; Matsusue et. al. 2003. J. Clin. Invest. 111:737-47). Verschiedene Tiermodelle wie zum Beispiel Hefe, Nematoden oder Zebrafische sind inzwischen intensiv charakterisiert worden. Bevorzugte Modelle sind aber im Vergleich zu anderen Spezies mammale Tiermodelle wie zum Beispiel die Maus (Mus musculus), weil sie die biologischen Prozesse im humanen Kontext am besten abbilden können. Für Mäuse sind in den letzten Jahren sowohl transgene Verfahren etabliert worden, die neue Gene in das Mausgenom integrieren ("gain of function"; Jegstrup I. et al. 2003. Lab Anim. 2003 Jan.;37(1):1-9). Alternativ werden durch andere methodische Ansätze Gene im Mausgenom ausgeschaltet und so ein Funktionsverlust eines gewünschten Gens induziert (knockout Modelle, "loss of function"; Zambrowicz BP & Sands AT. 2003. Nat. Rev. Drug Discov. 2003 Jan;2(1):38-51; Niwa H. 2001. Cell Struct. Funct. 2001 Jun;26(3):137-48.); technische Details sind vielfältig publiziert.

Nach Generierung der Mausmodelle können Veränderungen, die durch das Transgen bzw. durch den Funktionsverlust eines Gens induziert wurden, im Kontext des Gesamtorganismus analysiert werden (Balling R, 2001. Ann. Rev. Genomics Hum. Genet. 2:463-92). So sind zum Beispiel Verhaltenstests genauso wie biochemische Untersuchen etablierter Blutparameter möglich. Histologische Analysen, Immunhistochemie oder die Elektronenmikroskopie ermöglichen die Charakterisierung von Veränderungen auf zellulärer Ebene. Das spezifische Expressionsmuster eines Genes kann durch eine in situ Hybridisierung nachgewiesen werden (Peters T. et. al. 2003. Hum. Mol. Genet 12: 2109-20).

### Beispiel 3: Identifizierung der SEQ ID NO: 1/2 als diagnostisches und therapeutisches Krebs-Target

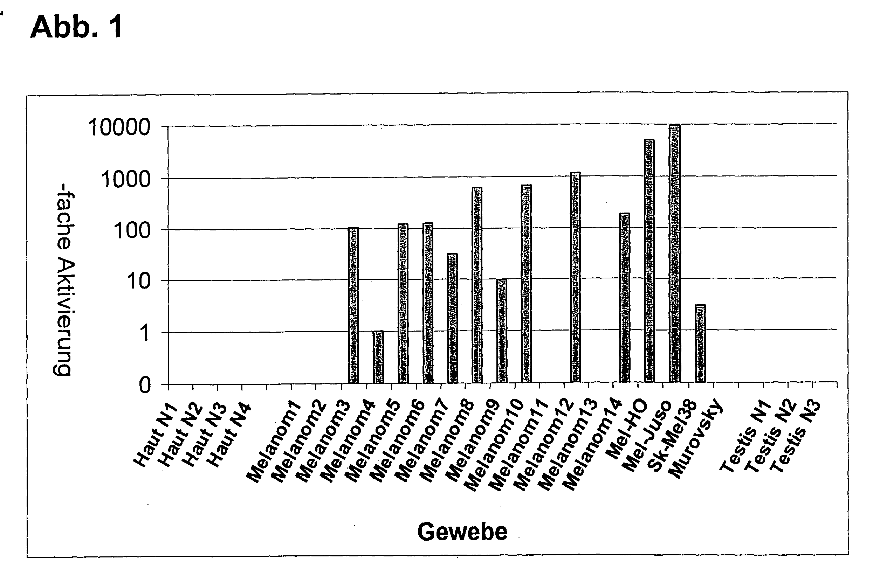

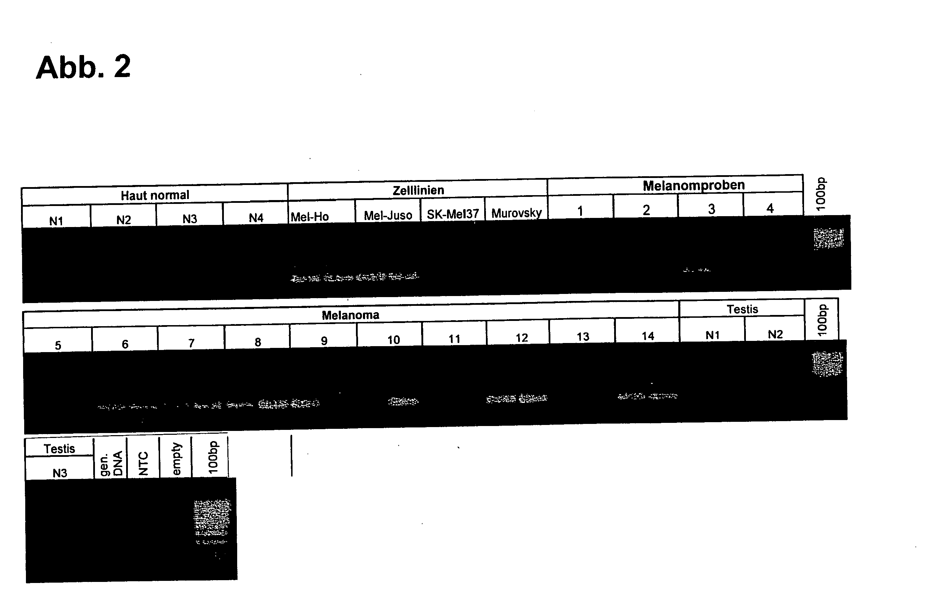

Die SEQ ID NO: 1 (Nukleinsäuresequenz) wird von einem neuen Gen auf Chromosom 6 (6q26-27) kodiert und repräsentiert die abgeleitete Proteinsequenz (SEQ ID NO: 2). Ein alternatives offenes Leseraster dieses Genlocus ist SEQ ID NO: 267 die für die abgeleitete Proteinsequenz SEQ ID NO: 268 kodiert. Beide Proteinsequenzen zeigen keine Homologien zu bereits bekannten Proteinen.
Erfindungsgemäß wurde nach der Etablierung einer spezifischen quantitativen RT-PCR (Primerpaar SEQ ID NO: 3 und 4) die Menge der genspezifischen Transkripte in gesundem Gewebe und in Karzinomproben untersucht. Das Transkript ließ sich in keiner der analysierten Normalgewebe nachweisen. Überraschend detektierten wir ganz spezifisch substantielle Mengen dieses Transkripts in fast allen untersuchten Melanomproben, obwohl das Gen in normaler Haut als Ursprungsgewebe nicht exprimiert ist (Abb. 1). Durch eine konventionelle RT-PCR wurde die Selektivität dieses Markers für Melanome bestätigt (Abb. 2). Überraschend amplifizierten wir dabei zwei Fragmente, die genspezifische Varianten (wahrscheinlich SEQ ID NO: 1 und SEQ ID NO: 267) widerspiegeln.
Wir zeigen damit, dass dieses Gen ein absolut spezifischer Marker für Melanomzellen ist und durch sein fehlen in jedem untersuchten Normalgewebe für zielgerichtete Therapie- und Diagnostik Ansätze als Biomarker geeignet ist.
Insbesondere extrazelluläre Anteile von SEQ ID NO: 2 bzw. 268 können erfindungsgemäß als Zielstruktur von monoklonalen Antikörpern genutzt werden. Dies betrifft u.a. folgende Epitope: Aminosäuren 1-50, bezogen auf die SEQ ID NO: 2; Aminosäuren 1-12, bezogen auf die SEQ ID NO: 268, Aminosäuren 70-88 bezogen auf die SEQ ID NO: 2, Aminosäuren 33-129 bezogen auf die SEQ ID NO: 268, sowie SEQ ID NO: 281.
Erfindungsgemäß sind therapeutisch auch andere zielorientierte Ansätze wie Vakzine und Therapien mit "small compounds" denkbar, die nur dieses Gen als Zielstruktur haben und somit keine gesunden Zellen betreffen. Auch diagnostisch kann dieses Gen aufgrund seiner Selektivität für Tumorzellen genutzt werden.

### Beispiel 4: Identifizierung der SEQ ID NO: 5/6 als diagnostisches und therapeutisches Krebs-Target

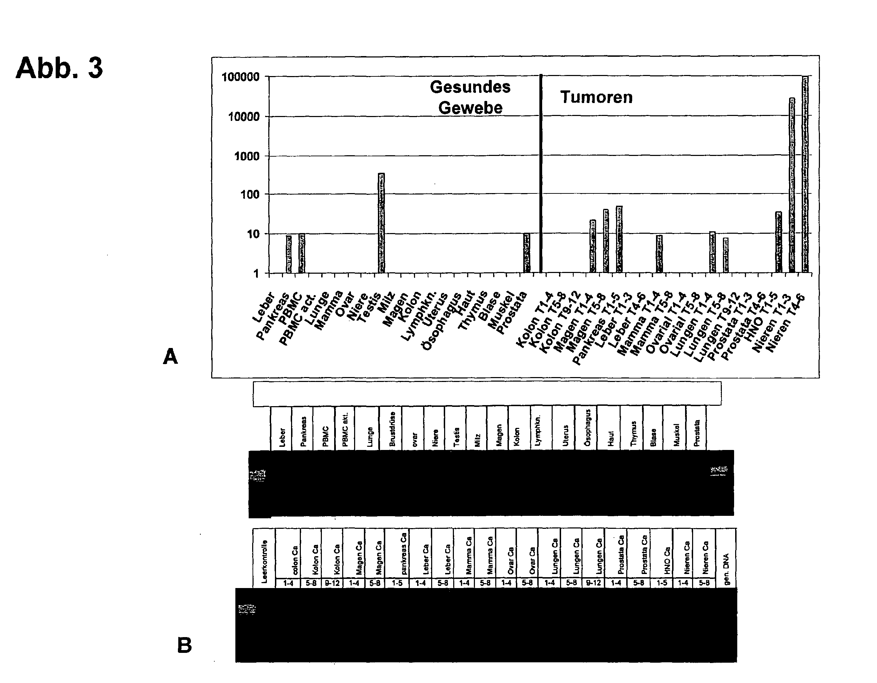

Die SEQ ID NO: 5 (Nukleinsäuresequenz) wird von einem neuen Gen auf Chromosom 11 (11q12.1) kodiert und repräsentiert die abgeleitete Proteinsequenz (SEQ ID NO: 6). Ein alternatives offenes Leseraster dieses Genlokus ist SEQ ID NO: 269 die für die abgeleitete Proteinsequenz SEQ ID NO: 270 kodiert. Beide Proteinsequenzen zeigen keine Homologien zu bereits bekannten Proteinen.
Erfindungsgemäß wurde nach der Etablierung einer genspezifischen quantitativen RT-PCR (Primerpaar SEQ ID NO: 7 und 8) die Menge der genspezifischen Transkript in gesundem Gewebe und in Karzinomproben (jeweils Pool von Proben) untersucht. Spezifische RNA detektierten wir mit Ausnahme von Testis gar nicht oder aber nur in geringen Mengen in den von uns untersuchten gesunden Geweben (Abb. 3; A quantitative RT-PCR; B Gelbild). Der Lokus exprimiert demnach mit hoher Wahrscheinlichkeit ein keimzellspezifisches Genprodukt. Allerdings ist das Gen in vielen Tumorproben aktiviert, spezifische RNA war in substantiellen Mengen nachweisbar (Abb.3). Die höchste Prävalenz und Expressionshöhe fanden wir in Nierenzelltumoren. Aber auch in Magen-, Pankreas-, HNO- und Lungentumoren waren spezifische Transkripte nachweisbar (Abb. 4; A quantitative RT-PCR; B Gelbild). Auch wiederholte Untersuchungen der korrespondierenden Normalgewebe waren gen-spezifische Transkripte nicht detektierbar. Um die Expression von diesem Genlokus zusätzlich zu belegen wurde zusätzlich ein Northern blot durchgeführt. Dazu wurde eine Sonde in einer spezifischen PCR der Primer SEQ ID NO: 7 und 8 unter Einbau von Digoxygenin-dUTP (Roche Diagnostics) nach Angaben des Herstellers hergestellt. Die Sonde wurde anschließend mit 2µg (Abb. 5, Spur 1) bzw. 1 µg (Abb. 5, Spur 2) Gesamt-RNA aus Testisgewebe hybridisiert und das Digoxygenin der Sonde anschließend in einer spezifischen Farbreaktion nachgewiesen. Ein ca. 3,1 kB großes, Gen-spezifisches Fragment konnte in dem Experiment nachgewiesen werden (Abb. 5), und bestätigte somit zusätzlich die Expression dieses Lokus.
Der Genlokus ist somit ein typischer Vertreter der Klasse der sog. Cancer/Testis-Antigene, die in Normalgeweben fast ausschließlich in den Keimzellen der Testis exprimiert sind. In Tumoren jedoch werden Cancer/Testis-Antigene häufig eingeschaltet, obwohl sie in den zugrunde liegenden somatischen Normalgewebszellen nicht exprimiert werden. Mehrere Mitglieder dieser funktionell und strukturell heterogenen Klasse werden aufgrund ihrer attraktiven selektiven Gewebsverteilung bereits für spezifische immuntherapeutischen Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).
Zur Herstellung von Antikörpern können die Peptide gemäß SEQ ID NO: 282 und 283 genutzt werden. Insbesondere die extrazellulären Domänen der SEQ ID NO: 6 bzw. SEQ ID NO: 270 können erfindungsgemäß als Zielstruktur von monoklonalen Antikörpern genutzt werden.

### Beispiel 5: Identifizierung von LOC203413 als diagnostisches und therapeutisches Krebs-Target

Das Gen bzw. Protein des Genlokus LOC203413 (Nukleinsäuresequenz: SEQ ID NO: 9; Aminosäuresequenz: SEQ ID NO: 10) ist ein bisher nicht charakterisiertes Gen auf dem X-Chromosom (Xq24). Es hat außer einer Transmembrandomäne keine weiteren funktionellen Motive und keine Homologien zu bereits bekannten Proteinen.
Erfindungsgemäß wurde nach der Etablierung einer LOC203413-spezifischen quantitativen RT-PCR (Primerpaar SEQ ID NO: 11 und 12) die Menge des Transkripts in gesundem Gewebe und in Karzinomproben (Pool von Proben, die Anzahl ist in der Abb. angegeben) untersucht (Abb. 6; A: quantitative Auswertung, B: Bild nach gelelektrophoretischer Auftrennung). LOC203413-spezifische RNA lässt sich mit Ausnahme von Testis in keinem der von uns untersuchten gesunden Gewebe nachweisen. LOC203413 ist demnach mit hoher Wahrscheinlichkeit ein keimzellspezifisches Genprodukt. Wie Abb. 6 zeigt, waren LOC203413-spezifische Transkripte in Magen-, Pankreas-, Ösophagus-, Mamma-, Ovarial- und Prostatakarzinomen nachweisbar, insbesondere in Magen- und Mammakarzinomen war eine hohe Expression zu beobachten. Um die Analyse zu vertiefen wurden zusätzlich gesunde Magenproben sowie Magenkarzinomproben in einer quantitativen RT-PCR charakterisiert (Abb. 7A). In 70% der Karzinome war LOC203413 exprimiert, wogegen in keiner der gesunden Magenproben eine signifikante Expression nachweisbar war. Auch die Zelllinie MKN45, deren Ursprung ein Magenkarzinom ist, exprimiert LOC203413. Zusätzlich konnte eine spezifische Expression in 2/3 der untersuchten Pankreastumoren und in 40% der Leberkarzinome nachgewiesen werden (Abb. 7B). LOC203413 ist somit ein typischer Vertreter der Klasse der sog. Cancer/Testis-Antigene, die in Normalgeweben ausschließlich in den Keimzellen der Testis exprimiert sind. In Tumoren jedoch werden Cancer/Testis-Antigene häufig eingeschaltet, obwohl sie in den zugrunde liegenden somatischen Normalgewebszellen nicht exprimiert werden. Mehrere Mitglieder dieser funktionell und strukturell heterogenen Klasse werden aufgrund ihrer attraktiven selektiven Gewebsverteilung bereits für spezifische immuntherapeutischen Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).
Insbesondere die extrazelluläre Domäne von LOC203413 kann erfindungsgemäß als Zielstruktur von monoklonalen Antikörpern genutzt werden. So sind die Aminosäuren 22-113 (SEQ ID NO: 284) als Epitope interessant. In der Sequenz sind bezogen auf die SEQ ID NO: 10 an den Aminosäureposition 34 und 83 konservierte N-Glycosylierungsmotive lokalisiert, die sich unter Umständen besonders für die Herstellung von tumorspezifischen Antikörpern eignen. Zur Herstellung von LOC203413-spezifischen Antikörpern wurden die unter SEQ ID NO: 285 und 286 aufgeführten Peptide verwendet.
Erfindungsgemäß sind therapeutisch auch andere zielorientierte Ansätze wie Vakzine und Therapien mit "small compounds" denkbar, die nur dieses Gen als Zielstruktur haben und somit keine gesunden Zellen betreffen. Auch diagnostisch kann dieses Gen aufgrund seiner Selektivität für Tumorzellen genutzt werden.

### Beispiel 6: Identifizierung von LOC90625 als diagnostisches und therapeutisches Krebs-Target

Das Gen LOC90625 (Nukleinsäuresequenz: SEQ ID NO: 13) ist ein bisher nicht charakterisiertes Gen auf Chromosom 21 (21q22.3). Es kodiert für ein Protein (Aminosäuresequenz: SEQ ID NO: 14) mit einer Transmembrandomäne aber ansonsten keinerlei Homologien zu bereits bekannten Proteinen.
Erfindungsgemäß wurde nach der Etablierung einer LOC90625-spezifischen quantitativen RT-PCR (Primerpaar SEQ ID NO: 15 und 16) die Menge an genspezifischen Transkripten in gesundem Gewebe und in Karzinomproben (Pool von Proben, die Anzahl ist in der Abb. angegeben) untersucht (Abb. 8). LOC90625 ist in gesundem Gewebe sehr selektiv exprimiert, spezifische Transkripte sind vor allem in der Testis nachweisbar. Gering oder gar nicht nachweisbar war die LOC90625-spezifische Expression in allen anderen analysierten gesunden Geweben (Abb. 8). Überraschenderweise detektierten wir LOC90625-spezifische Überexpression in einigen Tumortypen. Insbesondere in Prostata-, Ösophagus- und Pankreaskarzinomen war LOC90625 im Vergleich zu den jeweiligen gesunden Gewebeproben stark überexprimiert (Abb. 8 und 9A)
LOC90625 ist ein selektiv exprimiertes Antigen, das offensichtlich in proliferierenden Geweben verstärkt exprimiert wird. Es ist somit eine selektive Überexpression in Tumoren zu beobachten, die therapeutisch nutzbar ist.
Insbesondere die extrazelluläre Domäne von LOC90625 kann erfindungsgemäß als Zielstruktur von monoklonalen Antikörpern genutzt werden. Dies können z.B. 1-19 (SEQ ID NO: 287 oder aber die Aminosäuren 40-160 (SEQ ID NO: 288) sein. Zur Herstellung von LOC203413-spezifischen Antikörpern wurden die Peptide gemäß SEQ ID NO: 289 und 290 verwendet.

### Beispiel 7: Identifizierung des Proteins FAM26A als diagnostisches und therapeutisches Krebs-Target

Das Gen FAM26A (SEQ ID NO: 17; NM_182494), das auf Chromosom 10 (10q24) lokalisiert ist, kodiert das Genprodukt der SEQ ID NO: 18 (NP_872300). FAM26A besitzt mehrere Transmembran-Domänen, an Aminosäureposition 142 ist ein Motiv für eine N-Glykosylierung lokalisiert. Die abgeleitete Proteinsequenz zeigt eine entfernte Homologie zu der PMP/Claudin Familie.
Erfindungsgemäß wurde nach der Etablierung einer FAM26A-spezifischen quantitativen RT-PCR (Primerpaar SEQ ID NO: 19 und 20) die Menge der genspezifischen Transkripte in gesundem Geweben und in Tumorproben untersucht (Abb. 10). Überraschend konnten wir die Überexpression von FAM26A in verschiedenen Tumoren nachweisen. Insbesondere in Ovarial-, Magen-, Ösophagus-, Pankreas- und Lebertumoren war FAM26A im Vergleich zum dazugehörenden gesunden Gewebe deutlich stärker exprimiert. Die selektiv hohe Expression von FAM26A in verschiedenen Tumorgeweben kann erfindungsgemäß für molekulare diagnostische Verfahren wie z.B. RT-PCR zum Nachweis von Tumorzellen in Gewebebiopsien genutzt werden.
Um die Expressionsdaten weiter zu verifizieren wurden FAM26A-spezifische Antikörper durch Immunisierung von Tieren hergestellt. Zur Herstellung von polyklonalen Antikörpern wurden die unter SEQ ID NO: 291 und 292 aufgeführten Peptide verwendet. Die Spezifität der Antikörper wurde durch Western blot-Analyse nachgewiesen (Abb. 11A: SEQ ID NO: 291; B: SEQ ID NO: 292). Dazu wurden COS-Zellen mit einem FAM26-Fragmentkodierenden Plasmidkonstrukt transfiziert. Der Western blot zeigte mit beiden Antikörpern ein spezifisches Signal, das in den jeweiligen Kontrollen nicht nachweisbar war (Abb. 11). Wir wiesen mit einem SEQ ID NO: 292-spezifischen Antikörper FAM26A auch in verschiedenen Zervix-, Ovarial- und Pankreastumoren nach (Abb. 12), wie auch mit einem SEQ ID NO: 291-spezifischen Antikörper in den Zelllinien SW480, EFO 27 und SNU 16, die jeweils RT-PCR positiv waren (Abb. 13A). Dabei fanden wir neben einer ca. 50 kDa großen spezifischen Bande auch eine schwächere bei ca. 40 kDa. Letztere entspricht etwa der erwarteten Größe. Das Hauptfragment bei 50 kDa repräsentiert ein posttranslational modifiziertes Protein. Das endogene FAM26A Protein wurde außerdem in SW480-Zellen mittels Immunfluoreszenz unter Verwendung eines SEQ ID NO: 292-spezifischen Antikörpers nachgewiesen. Die Analyse zeigt eine Lokalisation in der Plasmamembran (Abb. 13B). Um die Lokalisation von FAM26A in einem Gewebeverband zu analysieren, wurden gesunde Testisproben immunhistologisch charakterisiert. In der Testis konnte das FAM26A-Protein spezifisch in der Membran von Spermatozyten nachgewiesen werden, eine Membranlokalisation von FAM26A erscheint aufgrund der Ergebnisse wahrscheinlich (Abb. 14). Dies konnte auch in Tumorproben bestätigt werden (Abb. 15).
Insbesondere die extrazellulären Domänen von FAM26A können erfindungsgemäß als Zielstrukturen von monoklonalen Antikörpern genutzt werden. Dies sind bezogen auf die SEQ ID NO: 17 die Aminosäuren 38-48 (SEQ ID NO: 293) sowie die Aminosäuren 129-181 (SEQ ID NO: 294). Alternativ können auch die C-terminalen Aminosäuren 199-334 (SEQ ID NO: 295) bevorzugte Epitope für die Herstellung von Antikörpern für diagnostische oder therapeutische Zwecke sein. Zusätzlich kann das N-Glykosylierungsmotiv an Position 142 ein interessanter Angriffspunkt für therapeutische Antikörper sein.

### Beispiel 8: Identifizierung von SEMA5B als diagnostisches und therapeutisches Krebs-Target

Das Gen Semaphorin 5B (SEMA5B; SEQ ID NO: 21), das das Protein der SEQ ID NO: 22 kodiert, ist auf Chromosom 3 (3q21.1) lokalisiert. SEMA5B ist ein Typ-I-Transmembranprotein und gehört zur Familie der Semaphorine.
Erfindungsgemäß wurde nach der Etablierung einer SEMA5B-spezifischen quantitativen RT-PCR (Primerpaar SEQ ID NO: 23 und 24) die Menge an genspezifischen Transkripten in gesundem Gewebe und in Karzinomproben (Pool von Proben, die Anzahl ist in der Abb. angegeben) untersucht (Abb. 16). Wir fanden SEMA5B in gesundem Gewebe sehr selektiv auf Testis und Haut beschränkt. Gering oder gar nicht nachweisbar war die SEMA5B-spezifische Expression in allen anderen analysierten gesunden Geweben (Abb. 16). Überraschend fanden wir dagegen in einigen Tumortypen, insbesondere in Nierenkarzinomen und Brusttumoren, eine SEMA5B-spezifische Überexpression (Abb. 17A und B) im Vergleich zu den jeweiligen gesunden Geweben.
Die selektive Überexpression in Tumoren ist therapeutisch nutzbar.
Insbesondere die extrazelluläre Domäne von SEMA5B (As 20-1035; SEQ ID NO: 296) kann erfindungsgemäß als Zielstruktur von Antikörpern genutzt werden. SEMA5B ist ein Typ-I-Transmembrandomänenprotein (TM As 1035-1057), dessen C-Terminus im Inneren der Zelle lokalisiert ist (As 1058-1151). Zur Herstellung SEMA5B-spezifischer Antikörper wurden die Peptide gemäß SEQ ID NO: 297 und 298 verwendet.

### Beispiel 9: Identifizierung von GJB5 als diagnostisches und therapeutisches Krebs-Target

Das Protein GBJ5 (Nukleinsäuresequenz: SEQ ID NO: 25; Aminosäuresequenz: SEQ ID NO: 26) ist ein Mitglied der Connexin-Familie. Das Gen besteht aus zwei Exons und liegt auf Chromosom 1 (1p35.1). Die abgeleitete Aminosäuresequenz kodiert für ein Protein mit 273 Aminosäuren. Connexine haben eine wichtige Funktion bei Zell-Zell-Kontakten über sogenannte "Gap Junctions", die dem Austausch von kleinen cytoplasmatischen Molekülen, Ionen und Sekundärtransmittern dienen und somit die Kommunikation zwischen individuellen Zellen ermöglichen. Gap Junctions bestehen aus mehreren Connexinuntereinheiten, die einen Membrankanal ausbilden. Bisher wurden 11 verschiedene Mitglieder der Connexine beschrieben, die alle in einem Genkluster auf Chromosom 1 lokalisiert sind (Richard, G.; Nature Genet. 20: 366-369, 1998). GBJ5 hat vier Transmembrandomänen, der N- und C-Terminus des Proteins sind im Innern der Zelle lokalisiert.
Erfindungsgemäß wurde nach Etablierung einer GBJ5-spezifischen quantitativen RT-PCR (Primerpaar SEQ ID NO: 27, 28) die Menge der genspezifischen Transkripte in gesundem Gewebe und in Karzinomproben untersucht (Pool von Proben, die Anzahl ist in der Abb. angegeben). Unsere Untersuchungen zeigen eine differentielle Verteilung der Expression in Normalgeweben. GBJ5 Transkripte fanden wir fast ausschließlich im Ösophagus und in der Haut exprimiert, in allen anderen analysierten Geweben ist eine Transkription sehr schwach oder nicht nachweisbar (Abb. 18). In Ösophagus-, Kolon-, Magen-, und Pankreaskarzinomen konnte eine sehr starke tumorspezifische Überexpression beobachtet werden (Abb.18). Dies konnte durch die Analyse von Einzelproben der vier Karzinome bestätigt werden (Abb.19 A-D). Zusätzlich lässt sich das GBJ5-spezifische Transkript in den etablierten Zelllinien LoVo, MKN45 und NCI-N87 deutlich nachweisen (Abb.19 A-D).
Insbesondere die extrazellulären Domänen von GBJ5 können erfindungsgemäß als Zielstruktur von therapeutischen Antikörpern genutzt werden. Bezogen auf die SEQ ID NO: 26 sind die Aminosäuren 41-75 (SEQ ID NO: 299) sowie der Bereich zwischen den Aminosäuren 150 und 187 (SEQ ID NO: 300) extrazellulär lokalisiert. Zur Herstellung von GJB5-spezifischen Antikörpern wurden die Peptide gemäß SEQ ID NO: 301 und 302 verwendet.

### Beispiel 10: Identifizierung von KLK5 als diagnostisches und therapeutisches Krebs-Target

Das Gen KLK5 (SEQ ID NO: 29) und sein Translationsprodukt (SEQ ID NO: 30) ist ein Mitglied der Kallikrein Familie, einer Gruppe von Serinproteasen mit unterschiedlichsten physiologischen Funktionen. Das Gen liegt auf Chromosom 19 (19q13.3-13.4) und kodiert für eine Serinprotease. KLK5 wird als Proform synthetisiert und im Stratum Corneum durch Proteolyse aktiviert (Brattsand, M et al; J. Biol. Chem. 274: 1999). Die aktive Protease (As 67-293) wird sekretiert und ist im Prozess der Hautabschilferung beteiligt. Das Propeptid (As 30-67) verbleibt über die Transmembrandomäne (As 1-29) an der Zelloberfläche gebunden (Ekholm, E et al; Jour Investigative Dermatol, 114; 2000).
Erfindungsgemäß wurde nach Etablierung einer KLK5-spezifischen quantitativen RT-PCR (Primerpaar SEQ ID NO: 31, 32) die Verteilung von KLK5-spezifischen Transkripten in gesundem Gewebe und in Karzinomproben untersucht (Abb. 20). In den meisten Normalgeweben ist KLK5 sehr gering bis gar nicht exprimiert, eine moderate Expression von KLK5 fanden wir lediglich in Testis, Ösophagus, Haut und Prostata. Eine signifikante Überexpression von KLK5 im Vergleich zu den entsprechenden normalen Herkunftsgeweben detektierten wir in Ösophaguskarzinomen, Zervix- sowie in HNO-Tumoren (Abb. 20, 21). Eine deutliche schwächere, aber nachweisbare KLK5-spezifische Expression konnte zudem in einigen Tumoren anderer Gewebe nachgewiesen werden (z.B. in Magen- und Pankreaskarzinomen).
Insbesondere die extrazelluläre Domäne von KLK5 kann erfindungsgemäß als Zielstruktur von therapeutischen Antikörpern genutzt werden (SEQ ID NO: 303). Besonders geeignet ist hierfür der Bereich des Propeptids (Aminosäure 30 bis 67). Zur Herstellung von KLK5-spezifischen Antikörpern wurden das unter SEQ ID NO: 304 aufgeführte Peptid verwendet.

### Beispiel 11: Identifizierung von LOC352765 als diagnostisches und therapeutisches Krebs-Target

Der Genlokus LOC352765 ist auf Chromosom 9 (9q34.12) lokalisiert. Das Gen (SEQ ID NO: 33) kodiert das Genprodukt der SEQ ID NO: 34. Das LOC352765-Protein besitzt eine Transmembrandomäne am N-Terminus. Das hypothetische Protein zeigt keine Homologien zu bereits bekannten Proteinen.
Erfindungsgemäß wurde nach der Etablierung einer LOC352765-spezifischen quantitativen RT-PCR (Primerpaar SEQ ID NO: 35 und 36) die Menge an genspezifischen Transkripten in gesundem Gewebe und in Karzinomproben (Pool von Proben) untersucht (Abb. 22). LOC352765 ist in gesundem Gewebe sehr selektiv exprimiert, spezifische Transkripte fanden wir lediglich in der Testis, der Haut und der Blase nachweisbar. Dagegen konnte in einigen Tumortypen eine LOC352765-spezifische Überexpression nachgewiesen werden. Insbesondere in Brusttumoren lag die Expression über der des am stärksten exprimierenden Normalgewebes. Auch in Kolon- und Ovarialkarzinomen und in HNO-Tumoren fanden wir LOC352765 deutlich überexprimiert (Abb. 22, 23).
LOC352765 ist aufgrund seiner selektiven Überexpression in Tumoren therapeutisch nutzbar.
Insbesondere die extrazelluläre Domäne von LOC352765 (Aminosäuren 44-211, SEQ ID NO:34) kann erfindungsgemäß als Zielstruktur von Antikörpern und anderen zielgerichteten Therapieformen genutzt werden. Zur Herstellung von spezifischen Antikörpern wurde die Peptide gemäß SEQ ID NO: 305 und 306 verwendet.

### Beispiel 12: Identifizierung von SVCT1 als diagnostisches und therapeutisches Krebs-Target

Das Gen SVCT1 (SEQ ID NO: 37) ist auf Chromosom 7 (7q33) lokalisiert und kodiert für das Genprodukt der SEQ ID NO: 38. Das SVCT1 Protein hat vier Transmembrandomänen und zeigt keine Homologien zu bereits bekannten Proteinen.
Erfindugsgemäß wurde nach der Etablierung einer SVCT1-spezifischen quantitativen RT-PCR (Primerpaar SEQ ID NO: 39 und 40) die Menge an genspezifischen Transkripten in gesundem Gewebe und in Karzinomproben (Pool von Proben) untersucht (Abb. 24). SVCT1 ist in gesundem Gewebe selektiv auf Niere, Testis, Thymus und Brustdrüse beschränkt. Überraschend konnte dagegen in einigen Tumortypen eine SVCT1-spezifische Überexpression nachgewiesen werden. Insbesondere in Nieren-, Ösophagus- und Pankreaskarzinomen und in HNO-Tumoren ist SVCT1 stark überexprimiert (Abb. 24, 25) und zwar nicht nur im Vergleich zum entsprechenden gesunden Herkunftsgewebe, sondern auch im Bezug auf das am stärksten exprimierende Normalgewebe überhaupt.
SVCT1 ist aufgrund seiner selektiven Überexpression in Tumoren therapeutisch nutzbar. Insbesondere die extrazellulären Domänen von SVCT1 können erfindungsgemäß als Zielstruktur von Antikörpern oder für andere zielgerichtete Therapieformen genutzt werden. Zur Herstellung spezifischer Antikörper wurden die Peptide gemäß SEQ ID NO: 307 und 308 verwendet.

### Beispiel 13: Identifizierung von LOC199953 als diagnostisches und therapeutisches Krebs-Target

Das Gen bzw. Protein des Genlolcus LOC199953 (Nukleinsäuresequenz: SEQ ID NO: 41; Aminosäuresequenz: SEQ ID NO: 42) ist auf Chromosom 1 (1q36.22) lokalisiert. Das Protein besitzt mehrere Transmembrandomänen. Alternative offene Leserahmen dieses Genlokus stellen die SEQ ID NO: 271 mit ihrem Genprodukt SEQ ID NO: 272 und die SEQ ID NO: 273 mit dem dazugehörigen Genprodukt SEQ ID NO: 274 dar. Darüber hinaus zeigt das hypothetische Protein keine weiteren Homologien zu bereits bekannten Proteindomänen. Erfindungsgemäß wurde nach der Etablierung einer LOC199953-spezifischen quantitativen RT-PCR (Primerpaar SEQ ID NO: 43 und 44) die Menge der genspezifischen Transkripte in gesundem Gewebe und in Karzinomproben untersucht. LOC199953 ist in gesunden Geweben selektiv exprimiert und in einigen Tumoren überexprimiert. Insbesondere konnte in HNO-und Nierenkarzinomen (Abb. 26) in ca. 50% der Tumorproben eine Überexpression im Vergleich zu Normalgeweben identifiziert werden.
Die extrazellulären Domänen von LOC199953 können erfindungsgemäß als Zielstruktur von Antikörpern genutzt werden.

### Beispiel 14: Identifizierung von TMEM31 als diagnostisches und therapeutisches Krebs-Target

Das Gen TMEM31 (SEQ ID NO: 45) des Genlokus LOC203562 ist auf Chromosom X (Xq22.2) lokalisiert. Das Gen kodiert für das Protein der SEQ ID NO: 46. Das Protein hat zwei Transmembrandomänen und zeigt sonst keine Homologien zu bereits bekannten Proteinen.
Erfindungsgemäß wurde nach der Etablierung einer TMEM31-spezifischen quantitativen RT-PCR (Primerpaar SEQ ID NO: 47 und 48) die Menge der genspezifischen Transkripte in gesundem Gewebe und in Karzinomproben untersucht. TMEM31 ist in gesunden Geweben sehr selektiv vor allem auf Testis beschränkt (Abb. 27). Überraschenderweise fanden wir auch Expression in einigen Tumortypen, während in den korrespondierenden Normalgeweben keine Expression feststellbar war. Dies sind insbesondere Nieren-, Kolon-, Magen-, Brust-, Leber-, Lungen- und HNO-Karzinome (Abb. 27, 28).
TMEM31 ist somit ein typischer Vertreter der Klasse der sog. Cancer/Testis-Antigene, die in Normalgeweben ausschließlich in den Keimzellen der Testis exprimiert sind. In Tumoren jedoch werden Cancer/Testis-Antigene häufig eingeschaltet, obwohl sie in den zugrunde liegenden somatischen Normalgewebszellen nicht exprimiert werden. Mehrere Mitglieder dieser funktionell und strukturell heterogenen Klasse werden aufgrund ihrer attraktiven selektiven Gewebsverteilung bereits für spezifische immuntherapeutischen Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).
Die extrazellulären Domänen von TMEM31 können erfindungsgemäß als Zielstruktur von Antikörpern genutzt werden.

### Beispiel 15: Identifizierung von FLJ25132 als diagnostisches und therapeutisches Krebs-Target

Das Gen/Protein FLJ25132 (Nukleinsäuresequenz: SEQ ID NO: 49; Aminosäuresequenz: SEQ ID NO: 50) ist auf Chromosom 17 (17q25.3) lokalisiert. FLJ25132 besitzt eine Transmembrandomäne, sonst zeigen sich keine Homologien zu bereits bekannten Proteinen. Erfindungsgemäß wurde nach der Etablierung einer FLJ25132-spezifischen quantitativen RT-PCR (Primerpaar SEQ ID NO: 51 und 52) die Menge der genspezifischen Transkripte in gesundem Gewebe und in Karzinomproben untersucht. FLJ25132 ist in den von uns untersuchten Karzinomproben im Vergleich zum gesunden Gewebe teilweise überexprimiert (Abb. 29). Insbesondere in Ovarial- und in Prostatakarzinomen konnte eine deutliche Überexpression von FLJ25132 nachgewiesen werden.
Die extrazellulären Domänen von FLJ25132 können erfindungsgemäß als Zielstruktur von Antikörpern genutzt werden.

### Beispiel 16: Identifizierung von LOC143724, LOC284263, LOC283435 und LOC349260 als diagnostisches und therapeutisches Krebs-Targets

Die Genloci (mit den entsprechend kodierten Genen und Genprodukten) LOC143724, LOC284263, LOC283435 und LOC349260 sind aufgrund ihres ähnlichen Profils zusammengefasst.
Das im Genlokus LOC143724 enthaltene Gen mit der SEQ ID NO: 53 auf Chromosom 11 (11q13.1) kodiert als Genprodukt die SEQ ID NO: 54. Ein alternatives offenes Leseraster dieses Genlokus ist durch die SEQ ID NO: 275 mit ihrem Genprodukt SEQ ID NO: 276 repräsentiert und stellt entweder ein eigenständiges Transkript oder eine Spleissvariante von SEQ ID NO: 53 dar. Für die genspezifische Amplifikation des Gens wurden die Primer gemäß SEQ ID NO: 55 und 56 verwendet.
Das im Genlokus LOC284263 enthaltene Gen mit der SEQ ID NO: 89 auf Chromosom 18 (18q21.1) kodiert das Genprodukt mit der SEQ ID NO: 90. Für die genspezifische Amplifikation des Gens wurden die Primer gemäß SEQ ID NO: 91 und 92 verwendet.
Das im Genlokus LOC283435 enthaltene Gen mit der SEQ ID NO: 117 auf Chromosom 12 (12q24.32) kodiert das Genprodukt mit der SEQ ID NO: 118. Für die genspezifische Amplifikation des Gens wurden die Primer gemäß SEQ ID NO: 119 und 120 verwendet. Das im Genlokus LOC349260 enthaltene Gen mit der SEQ ID NO: 121 auf Chromosom 9 (9q11.2) kodiert das Genprodukt mit der SEQ ID NO: 122. Für die genspezifische Amplifikation des Gens wurden die Primer gemäß SEQ ID NO: 123 und 124 verwendet. Alle Proteine besitzen Transmembrandomänen und zeigen zusätzlich keine Homologien zu bereits bekannten Proteinen.
Erfindungsgemäß wurde nach der Etablierung von spezifischen quantitativen RT-PCR-Analysen die Menge der genspezifischen Transkripte in gesundem Gewebe und in Karzinomproben untersucht. Alle vier Gene lassen sich mit Ausnahme von Testis in keinem der von uns untersuchten gesunden Gewebe nachweisen. Die Gene sind demnach mit hoher Wahrscheinlichkeit keimzellspezifisch. Überraschenderweise findet man aber signifikante Expression in verschiedenen Tumorproben.
Die vier Gene sind somit typische Vertreter der Klasse der sog. Cancer/Testis-Antigene, die in Normalgeweben ausschließlich in den Keimzellen der Testis exprimiert sind. In Tumoren jedoch werden Cancer/Testis-Antigene häufig eingeschaltet, obwohl sie in den zugrunde liegenden somatischen Normalgewebszellen nicht exprimiert werden. Mehrere Mitglieder dieser funktionell und strukturell heterogenen Klasse werden aufgrund ihrer attraktiven selektiven Gewebsverteilung bereits für spezifische immuntherapeutischen Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).
Die extrazellulären Domänen der vier Gene können erfindungsgemäß als Zielstrukturen von Antikörpern genutzt werden.

### Beispiel 17: Identifizierung der Sequenz gemäß SEQ ID NO: 57 als diagnostisches und therapeutisches Krebs-Target

Die Sequenz gemäß SEQ ID NO: 57 ist von einem Gen auf Chromosom 1 (1p21.3) abgeleitet und kodiert die Proteinsequenz gemäß SEQ ID NO: 58. Ein alternatives Transkript des Genlokus ist durch die SEQ ID NO: 277 mit seinem Genprodukt SEQ ID NO: 278 repräsentiert. Das Transmembranprotein zeigt keine Homologien zu bereits bekannten Proteinen.
Erfindungsgemäß wurde nach der Etablierung einer spezifischen quantitativen RT-PCR (Primerpaar SEQ ID NO: 59 und 60) die Menge der genspezifischen Transkripte in gesundem Gewebe und in Karzinomproben untersucht. Die SEQ ID NO: 57 ist in den von uns untersuchten gesunden Geweben selektiv exprimiert (Abb. 30). Spezifische Transkripte waren in fast allen analysierten Tumortypen nachweisbar und insbesondere in Leber-, HNO- und Nierentumoren überexprimiert. Dies konnte bei der Analyse einzelner Tumorproben im Vergleich zu gesunden Gewebeproben bestätigt werden (Abb. 31).
Die extrazellulären Domänen der Sequenz gemäß SEQ ID NO: 58 können erfindungsgemäß als Zielstruktur von Antikörpern genutzt werden, insbesondere die Aminosäuren 20-38 und 90-133 sind extrazellulär lokalisiert.

### Beispiel 18: Identifizierung von LOC119395 als diagnostisches und therapeutisches Krebs-Target

Das im Genlokus LOC119395 auf Chromosom 17 (17q25.3) enthaltene Gen mit der SEQ ID NO: 61 kodiert ein Genprodukt mit der SEQ ID NO: 62. Das Transmembranprotein zeigt keine Homologien zu bereits bekannten Proteinen.
Erfindungsgemäß wurde nach der Etablierung einer LOC119395-spezifischen quantitativen RT-PCR (Primerpaar SEQ ID NO: 63 und 64) die Menge der genspezifischen Transkripte in gesundem Gewebe und in Karzinomproben untersucht (Abb. 32). LOC119395 ist in den von uns untersuchten gesunden Geweben sehr selektiv exprimiert und nur in wenigen Geweben nachweisbar (Abb. 32). Dagegen waren LOC119395-spezifische Transkripte in fast allen analysierten Tumortypen nachweisbar. Insbesondere in Magen-, Ovarial- und Prostatakarzinomen war eine zum Teil deutliche tumorselektive Überexpression von LOC119395 zu beobachten. Dies konnte bei der Analyse einzelner Tumorproben im Vergleich zu gesunden Gewebeproben bestätigt werden (Abb. 33). Im Vergleich zum jeweiligen gesunden Gewebe war eine Überexpression von LOC119395 in Mammakarzinomen und Ösophagustumoren nachzuweisen. Eine tumorselektive Expression konnte in Kolon- und in Magenkarzinomen identifiziert werden (Abb. 33).
Die extrazelluläre Domäne von LOC119395 (Aminosäuren 44-129) kann erfindungsgemäß als Zielstruktur von Antikörpern genutzt werden.

### Beispiel 19: Identifizierung von LOC121838 als diagnostisches und therapeutisches Krebs-Target

Das im Genlokus LOC121838 auf Chromosom 13 (13q14.11) lokalisierte Gen mit dem Transkript der SEQ ID NO: 65 kodiert das Protein mit der SEQ ID NO: 66. Das Transmembranprotein zeigt keine Homologien zu bereits bekannten Proteinen. Erfindungsgemäß wurde nach der Etablierung einer LOC121838-spezifischen quantitativen RT-PCR (Primerpaar SEQ ID NO: 67 und 68) die Menge der genspezifischen Transkripte in gesundem Gewebe und in Karzinomproben untersucht (Abb. 34A). LOC121838 ist in den von uns untersuchten gesunden Geweben sehr selektiv exprimiert und nur in wenigen Geweben nachweisbar (Abb. 34A und B). Dagegen waren LOC121838-spezifische Transkripte in vielen analysierten Tumortypen nachweisbar. Insbesondere in Ovarial- und Ösophaguskarzinomen fanden wir eine deutliche tumorselektive Überexpression von LOC121838.
Die extrazellulären Domänen von LOC121838 können erfindungsgemäß als Zielstruktur von Antikörpern genutzt werden.

### Beispiel 20: Identifizierung von LOC221103 als diagnostisches und therapeutisches Krebs-Target

Das im Genlokus LOC221103 auf Chromosom 11 (11q12.3) lokalisierte Gen mit dem Transkript der SEQ ID NO: 69 kodiert das Protein mit der SEQ ID NO: 70. Das Transmembranprotein zeigt keine Homologien zu bereits bekannten Proteinen. Erfindungsgemäß wurde nach der Etablierung einer LOC221103-spezifischen quantitativen RT-PCR (Primerpaar SEQ ID NO: 71 und 72) die Menge der genspezifischen Transkripte in gesundem Gewebe und in Karzinomproben untersucht. LOC221103 ist in den von uns untersuchten gesunden Geweben lediglich in der Leber exprimiert und ansonsten nicht nachweisbar (Abb. 35). Überraschenderweise sind LOC221103-spezifische Transkripte in Leberkarzinomen überexprimiert (Abb. 36).
Die extrazellulären Domänen von LOC221103 können erfindungsgemäß als Zielstruktur von Antikörpern genutzt werden.

### Beispiel 21: Identifizierung von LOC338579 als diagnostisches und therapeutisches Krebs-Target

Das im Genlokus LOC338579 auf Chromosom 10 (10q11.21) lokalisierte Gen mit dem Transkript der SEQ ID NO: 73 kodiert das Protein mit der SEQ ID NO: 74. Das Transmembranprotein zeigt keine Homologien zu bereits bekannten Proteinen.
Erfindungsgemäß wurde nach der Etablierung einer LOC338579-spezifischen quantitativen RT-PCR (Primerpaar SEQ ID NO: 75 und 76) die Menge der genspezifischen Transkripte in gesundem Gewebe und in Karzinomproben untersucht. Expression fanden wir in gesunden Geweben ausschließlich in Testis und schwächer in der Leber und im Thymus. Überraschenderweise fanden wir eine Überexpression von LOC338579 in Kolon- und Leberkarzinomen im Vergleich zum gesunden Gewebe (Abb. 37).
Die extrazellulären Domänen von LOC338579 können erfindungsgemäß als Zielstruktur von Antikörpern genutzt werden.

### Beispiel 22: Identifizierung von LOC90342 als diagnostisches und therapeutisches Krebs-Target

Das im Genlokus LOC90342 auf Chromosom 2 (2q11.2) lokalisierte Gen mit dem Transkript der SEQ ID NO: 77 kodiert das Protein mit der SEQ ID NO: 78. Das Transmembranprotein enhält ein in Proteinkinase C und in verschiedenen Phospholipasen konserviertes calciumbindendes Motiv (Ca1B).
Erfindungsgemäß wurde nach der Etablierung einer LOC90342-spezifischen quantitativen RT-PCR (Primerpaar SEQ ID NO: 79 und 80) die Menge der genspezifischen Transkripte in gesundem Gewebe und in Karzinomproben untersucht (Abb. 38). LOC90342 fanden wir nur in einer kleinen Anzahl gesunder Geweben, von denen die meisten wenig toxizitätsrelevant sind (Abb. 38). Dagegen fanden wir LOC90342-spezifische Transkripte in einer Vielzahl der analysierten Tumortypen. Insbesondere in Magen-, Leber-, Pankreas-, Prostata-, Ovarial- und Lungenkarzinomen war eine zum Teil deutlich tumorselektive Überexpression von LOC90342 zu beobachten.
Das Membranprotein besitzt eine einzige Transmembrandomäne (As 707-726). Die extrazelluläre Domäne von LOC90342 kann erfindungsgemäß als Zielstruktur von therapeutischen Antikörpern genutzt werden.

### Beispiel 23: Identifizierung von LRFN1 als diagnostisches und therapeutisches Krebs-Target

LRFN1 (SEQ ID NO: 81) ist ein Gen, das auf Chromosom 19 (19q13.2) lokalisiert ist. Das Gen kodiert für das Protein der SEQ ID NO: 82 Das Protein enthält eine Transmembrandomäne und zeigt Homologien zur Myb-DNA-Bindungsdomäne und zu einer Immunglobulindomäne vom C2-Typ.
Erfindungsgemäß wurde nach der Etablierung einer LRFN1-spezifischen quantitativen RT-PCR (Primerpaar SEQ ID NO: 83 und 84) die Menge der genspezifischen Transkripte in gesundem Gewebe und in Karzinomproben untersucht. LRFN1 ist in den meisten untersuchten Normalgeweben bis auf aktivierte PBMC und Hirn sehr schwach exprimiert. (Abb. 39). Dagegen fanden wir LRFN1-spezifische Transkripte in einigen der analysierten Tumortypen verstärkt nachweisbar. Insbesondere in Magen-, Pankreas-, Ösophagus- und Brustkarzinomen fanden wir eine deutliche tumorselektive Überexpression von LRFN1 im Vergleich zu den dazugehörigen Normalgeweben.

Das Protein enthält eine Transmembrandomäne (As 448-470). Die extrazellulären Domänen von LFRN1 können erfindungsgemäß als Zielstruktur von therapeutischen Antikörpern genutzt werden.

### Beispiel 24: Identifizierung von LOC285916 als diagnostisches und therapeutisches Krebs-Target

Das im (Genlokus LOC285916 auf Chromosom 7 (7p22.3) lokalisierte Gen mit dem Transkript der SEQ ID NO: 85 kodiert das Protein mit der SEQ ID NO: 86. Das Transmembranprotein zeigt keine Homologien zu bereits bekannten Proteinen. Erfindungsgemäß wurde nach der Etablierung einer LOC285916-spezifischen quantitativen RT-PCR (Primerpaar SEQ ID NO: 87 und 88) die Menge der genspezifischen Transkripte in gesundem Gewebe und in Karzinomproben untersucht. LOC285916 ist in den von uns untersuchten gesunden Geweben selektiv in Testis exprimiert, in allen anderen untersuchten Geweben konnten wir keine oder nur geringe Expression nachweisen (Abb. 40A). Überraschenderweise fanden wir LOC285916-spezifische Transkripte in allen getesteten Tumortypen. Insbesondere in Brust-, Ösophagus-, Nieren-, HNO- und Lungenkarzinomen war eine deutliche tumorspezifische Überexpression nachweisbar (Abb. 40A und B).
Die extrazellulären Domänen von LOC285916 (Aminosäuren 42 bis 93) können erfindungsgemäß als Zielstruktur von Antikörpern genutzt werden.

### Beispiel 25: Identifizierung von MGC71744 als diagnostisches und therapeutisches Krebs-Target

Das Gen MGC71744 mit der SEQ ID NO: 93 auf Chromosom 17 (17p13.2) kodiert das Protein mit der SEQ ID NO: 94. Das Transmembranprotein zeigt keine Homologien zu bereits bekannten Proteinen.
Erfindungsgemäß wurde nach der Etablierung einer MGC71744-spezifischen quantitativen RT-PCR (Primerpaar SEQ ID NO: 95 und 96) die Menge an genspezifischen Transkripten in gesundem Gewebe und in Karzinomproben (Pool von Proben) untersucht (Abb. 41). MGC71744 ist in gesundem Gewebe kaum exprimiert. Geringe Mengen spezifischer Transkripte fanden wir lediglich in der Lunge und in der Milz. Gering oder gar nicht nachweisbar war die MGC71744-spezifische Expression in allen anderen analysierten gesunden Geweben (Abb. 41A). Überraschend fanden wir dagegen in einigen Tumortypen, insbesondere in Nierenkarzinomen, eine MGC71744-spezifische Überexpression (Abb. 41 A & B) im Vergleich zu gesundem Gewebe.
Insbesondere die extrazelluläre Domäne von MGC71744 (N-Terminus, As 67-85) kann erfindungsgemäß als Zielstruktur von Antikörpern genutzt werden.

### Beispiel 26: Identifizierung von LOC342982 als diagnostisches und therapeutisches Krebs-Target

Das im Genlokus LOC342982 auf Chromosom 19 (19p13.13) lokalisierte Gen mit dem Transkript der SEQ ID NO: 97 kodiert das Protein mit der SEQ ID NO: 98. Das Transmembranprotein zeigt Homologien zur Kohlenhydratbindungsdomäne des C-Typ der Lektine.
Erfindungsgemäß wurde nach der Etablierung einer LOC342982-spezifischen quantitativen RT-PCR (Primerpaar SEQ ID NO: 99 und 100) die Menge der genspezifischen Transkripte in gesundem Gewebe und in Karzinomproben (Pool von Proben) untersucht. LOC342982-spezifische RNA ist selektiv exprimiert, in vielen analysierten Normalgeweben war nur eine geringe oder keine Expression nachweisbar (Abb. 42). Hingegen zeigten fast alle der getesteten Tumorklassen eine zum Teil tumorspezifische Überexpression. Hauptsächlich Pankreas-, Nieren-, Lungen- und Brustkarzinome zeigen eine sehr starke Expression der LOC342982-spezifischen RNA (Abb. 42).
Insbesondere die extrazelluläre Domäne von LOC342982 (Aminosäuren 178-339) kann erfindungsgemäß als Zielstruktur von monoklonalen Antikörpern genutzt werden.

### Beispiel 27: Identifizierung von LOC343169/OR6F1 als diagnostisches und therapeutisches Krebs-Target

Das im Genlokus LOC343169 auf Chromosom 1 (1q44) lokalisierte Gen OR6F1 mit dem Transkript der SEQ ID NO: 101 kodiert das Protein mit der SEQ ID NO: 102. OR6F1 hat mehrere Transmembrandomänen und gehört zur Familie der olfaktorischen Rezeptoren und somit zur großen Familie der G-Protein-gekoppelten Rezeptoren.
Erfindungsgemäß wurde nach der Etablierung einer LOC343169/OR6F1-spezifischen quantitativen RT-PCR (Primerpaar SEQ ID NO: 103 und 104) die Menge an genspezifischen Transkripten in gesundem Gewebe und in Karzinomproben (Pool von Proben) untersucht (Abb. 43A). LOC343169/OR6F1 ist in gesundem Gewebe sehr selektiv exprimiert, spezifische Transkripte sind vor allem in Testis und Milz nachweisbar. Gering oder gar nicht nachweisbar war die LOC343169/OR6F1-spezifische Expression in allen anderen analysierten gesunden Geweben (Abb. 43A). Überraschend konnte dagegen in einigen Tumortypen eine LOC343169/OR6F1-spezifische Überexpression nachgewiesen werden. Insbesondere in Brust-, Ovarial-, Nieren-, Prostata-, Pankreas- und Leberkarzinomen zeigt sich eine tumorspezifische Überexpression von LOC343169/OR6F1 (Abb. 43A). Durch Analyse von Einzelproben konnte die Überexression in Ovarialkarzinomen bestätigt werden. LOC343169/OR6F1 ist ein selektiv exprimiertes Antigen, das offensichtlich in proliferierenden Geweben verstärkt exprimiert wird. Es ist somit eine selektive Überexpression in Tumoren zu beobachten, die therapeutisch nutzbar ist. insbesondere die extrazellulären Domänen können erfindungsgemäß als Zielstrukturen von monoklonalen Antikörpern genutzt werden.

### Beispiel 28: Identifizierung von LOC340204 als diagnostisches und therapeutisches Krebs-Target

Das im Genlokus LOC340204 auf Chromosom 6 (6p21.31) lokalisierte Gen mit dem Transkript der SEQ ID NO: 105 kodiert das Protein mit der SEQ ID NO: 106. Das Protein besitzt eine Transmembrandomäne. Darüber zeigt das Protein eine starke Homologie zu einer "Colipase" Domäne. Der Colipase wird eine Funktion als Kofaktor für die pankreatische Lipase zugeschrieben. Ein alternatives Transkript des Genlokus ist durch SEQ ID NO: 279 mit ihrem Genprodukt SEQ ID NO: 280 repräsentiert und könnte sowohl ein eigenständiges Transkript als auch eine Spleissvariante der SEQ ID NO: 105 darstellen.
Erfindungsgemäß wurde nach der Etablierung einer LOC340204-spezifischen quantitativen RT-PCR (Primerpaar SEQ ID NO: 107 und 108) die Menge der genspezifischen Transkripte in gesundem Gewebe und in Karzinomproben untersucht. LOC340204 ist in gesunden Geweben selektiv exprimiert und in einigen Tumoren stark überexprimiert. Insbesondere in Magen-, Pankreas-, Ovarial-, Lungen- und Ösophaguskarzinomen (Abb. 44) konnte eine deutliche Überexpression in Tumorproben im Vergleich zu verschiedenen Normalgeweben nachgewiesen werden.
Die extrazellulären Domänen von LOC340204 können erfindungsgemäß als Zielstrulctur von monoklonalen Antikörpern genutzt werden.

### Beispiel 29: Identifizierung von LOC340067 als diagnostisches und therapeutisches Krebs-Target

Das im Genlokus LOC340067 auf Chromosom 5 (5q22.3) lokalisierte Gen mit dem Transkript der SEQ ID NO: 109 kodiert das Protein mit der SEQ ID NO: 110. Das Transmembranprotein zeigt keine Homologien zu anderen Proteindomänen. Erfindungsgemäß wurde nach der Etablierung einer für die LOC340067-spezifischen quantitativen RT-PCR (Primerpaar SEQ ID NO: 111 und 112) die Menge der genspezifischen Transkripte in gesundem Gewebe und in Karzinomproben untersucht. LOC340067 ist in gesunden Geweben selektiv exprimiert und in einigen Tumoren stark überexprimiert (Abb. 45). Insbesondere in Pankreas-, Mamma-, Leber-, Ovarial-, Lungen- und Nierenkarzinomen konnte eine deutliche Überexpression in Tumorproben im Vergleich zu verschiedenen gesunden Geweben nachgewiesen werden.
Die extrazelluläre Domäne von LOC340067 kann erfindungsgemäß als Zielstruktur von monoklonalen Antikörpern genutzt werden.

### Beispiel 30: Identifizierung von LOC342780 als diagnostisches und therapeutisches Krebs-Target

Das im Genlokus LOC342780 auf Chromosom 18 (18q21.32) lokalisierte Gen mit dem Transkript der SEQ ID NO: 309 kodiert das Protein mit der SEQ ID NO: 310. Das Transmembranprotein enthält eine Acyltransferase-Domäne, die in vielen bisher nicht weiter charakterisierten Proteinen aus C. elegans vorkommt.
Erfindungsgemäß wurde nach der Etablierung einer LOC342780-spezifischen quantitativen RT-PCR (Primerpaar SEQ ID NO: 311 und 312) die Menge an genspezifischen Transkripten in gesundem Gewebe und in Karzinomproben (Pool von Proben, die Anzahl ist in der Abb. angegeben) untersucht. LOC342780 ist in gesundem Gewebe sehr selektiv exprimiert, spezifische Transkripte sind vor allem in Prostata, Magen, Testis, Lunge und der Brustdrüse, nachweisbar (Abb. 46). Überraschend konnte dagegen in allen analysierten Tumorarten eine LOC342780-spezifische Expression nachgewiesen werden. Insbesondere in Brust-, Ovarial-, Nieren- und Leberkarzinomen zeigt sich eine tumorspezifische Überexpression von LOC342780 (Abb. 46).
LOC342780 ist ein selektiv exprimiertes Antigen das offensichtlich in proliferierenden Geweben verstärkt exprimiert wird. Es ist somit eine selektive Überexpression in Tumoren zu beobachten, die therapeutisch nutzbar ist. Die extrazellulär lokalisierten Aminosäuren 76-89, 316-345, 399-493 sowie 650-665 (bezogen auf SEQ ID NO: 310) können erfindungsgemäß als Zielstrukturen von monoklonalen Antikörpern genutzt werden.

### Beispiel 31: Identifizierung von LOC339511 als diagnostisches und therapeutisches Krebs-Target

Die Sequenz gemäß SEQ ID NO: 113 ist von einem Gen abgeleitet, das auf Chromosom 1 (1q23.1) lokalisiert ist. Das Gen kodiert das Protein der SEQ ID NO: 114. Das Transmembranprotein zeigt Homologien zur Gruppe der olfaktorischen 7-Transmembranrezeptoren.
Erfindungsgemäß wurde nach der Etablierung einer für LOC339511-spezifischen quantitativen RT-PCR (Primerpaar SEQ ID NO: 115 und 116) die Menge der genspezifischen Transkripte in gesundem Gewebe und in Karzinomproben untersucht. LOC339511 ist in gesunden Geweben selektiv in der Leber exprimiert (Abb. 47A). In den Karzinomproben konnten LOC339511-spezifische Transkripte in Lebertumoren identifiziert werden, außerdem war eine schwache Expression in Kolon-, Mamma- und Lungenkarzinomen nachweisbar. Beim Vergleich der leberspezifischen Expression in Tumor und in gesundem Gewebe konnte in einigen Tumorproben eine erhöhte Expression nachgewiesen werden (Abb. 47B).
Die extrazellulären Domänen der SEQ ID NO: 113 können erfindungsgemäß als Zielstrukturen von monoklonalen Antikörpern genutzt werden. Insbesondere die extrazellulär lokalisierten Aminosäurereste 1-23, 82-100, 167-175 und 226-236 eignen sich daher besonders zur Herstellung von monoklonalen Antikörpern.

### Beispiel 32: Identifizierung von C14orf37 als diagnostisches und therapeutisches Krebs-Target

C14orf37 (SEQ ID NO: 125) ist ein auf Chromosom 14 (14q22.3) lokalisiertes Gen, das das Genprodukt mit der SEQ ID NO: 126 kodiert. Das Transmembranprotein zeigt keine Homologien zu bereits bekannten Proteinen.
Erfindungsgemäß wurde nach der Etablierung einer für die C14orf37-spezifischen quantitativen RT-PCR (Primerpaar SEQ ID NO: 127 und 128) die Menge der genspezifischen Transkripte in gesundem Gewebe und in Karzinomproben untersucht. C14orf37 ist in verschiedenen gesunden Geweben und am stärksten in der Testis exprimiert (Abb. 48). Insbesondere in Nierenkarzinomen konnte eine deutliche Überexpression im Vergleich zu verschiedenen gesunden Geweben nachgewiesen werden.
Die extrazelluläre Domäne der SEQ ID NO: 126 kann erfindungsgemäß als Zielstruktur von monoklonalen Antikörpern genutzt werden.

### Beispiel 33: Identifizierung von ATP1A4 als diagnostisches und therapeutisches Krebs-Target

Das Gen ATP1A4 (SEQ ID NO: 129) ist auf Chromosom 1 (1q21-23) lokalisiert. Das Gen kodiert für ein Protein mit der SEQ ID NO: 130. ATP1A4 ist ein integrales Transmembranprotein mit acht Transmembrandomänen, das in der Plasmamembran lokalisiert ist. ATP1A4 ist Teil eines Proteinkomplexes, wobei der katalytische Teil der Natrium/Kalium ATPase N-terminal gelegen ist (Woo et al. J. 2000. Biol Chem. 275, 20693-99). ATP1A4 zeigt starke Homologien zu zahlreichen anderen Vertretern der Kation-ATPase Familie.
Erfindungsgemäß wurde nach der Etablierung einer ATP1A4-spezifischen quantitativen RT-PCR (Primerpaar SEQ ID NO: 131 und 132) die Menge der genspezifischen Transkripte in gesundem Gewebe und in Karzinomproben untersucht. ATP1A4 ist in gesunden Geweben selektiv vor allem in der Testis exprimiert (Abb. 49). In einigen Tumorproben konnte eine im Vergleich zum jeweiligen gesunden Gewebe starke Überexpression von ATP1A4 nachgewiesen werden. Insbesondere in Pankreas-, Brust-, Leber- und Nierenkarzinomen (Abb. 49) konnte eine deutliche Überexpression in Tumorproben im Vergleich zu gesunden Geweben nachgewiesen werden, insgesamt sehr hoch war die Expression in Pankreas- und Brustkarzinomen.
Die extrazellulären Domänen von ATP1A4 können erfindungsgemäß als Zielstruktur von monoklonalen Antikörpern genutzt werden. Die folgenden Aminosäurereste in Bezug auf die SEQ ID NO: 130 sind extrazellulär lokalisiert: Aminosäurereste 129-137, 321-329, 816-857, und 977-990.

### Beispiel 34: Identifizierung der SEQ ID NO: 133 bis 264 als diagnostische und therapeutische Krebs-Targets

Bei den Sequenzen gemäß SEQ ID NO: 133-266 handelt es sich um 33 Gene (Nukleinsäuresequenz, Aminosäuresequenz), zusammen mit den jeweiligen PCR-Primern für spezifische RT-PCR-Reaktionen. Alle Proteine verfügen über eine oder mehrere Transmembrandomänen, über Homologien zu Proteindomänen ist wenig bekannt. Erfindungsgemäß wurden für diese Gene in spezifischen quantitativen RT-PCR-Reaktionen die Menge der jeweiligen genspezifischen Transkripte in gesundem Gewebe und in Karzinomproben untersucht. Für alle Gene konnte in Tumorproben eine im Vergleich zum jeweiligen gesunden Gewebe zum Teil starke Überexpression nachgewiesen werden.

Alle Gene dieser Gruppe sind therapeutisch und diagnostisch nutzbar. Die extrazellulären Domänen können dabei erfindungsgemäß als Zielstruktur von Antikörpern genutzt werden.

## Patentansprüche

1. Pharmazeutische Zusammensetzung umfassend einen oder mehrer Bestandteile, die aus der Gruppe ausgewählt sind, bestehend aus:
(i) einem Tumor-assoziierten Antigen oder einem Teil davon,
(ii) einer Nukleinsäure, die für ein Tumor-assoziiertes Antigen oder einen Teil davon kodiert,
(iii) einem Antikörper, der an ein Tumor-assoziiertes Antigen oder einen Teil davon bindet,
(iv) einer Antisense-Nukleinsäure, die spezifisch mit einer Nukleinsäure, die für ein Tumor-assoziierte Antigen kodiert, hybridisiert,
(v) einer Wirtszelle, die ein Tumor-assoziiertes Antigen oder einen Teil davon exprimiert, und
(vi) isolierten Komplexen zwischen einem Tumor-assoziierten Antigen oder einem Teil davon und einem HLA-Molekül,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

2. Pharmazeutische Zusammensetzung nach Anspruch 1, wobei die Wirtszelle das Tumor-assoziierte Antigen oder den Teil davon sekretiert, auf der Oberfläche exprimiert oder ein HLA-Molekül exprimiert, das an das Tumor-assoziierte Antigen oder den Teil davon bindet.

3. Pharmazeutische Zusammensetzung nach Anspruch 1 oder 2 zur Behandlung einer Erkrankung, die sich durch die Expression oder abnormale Expression eines Tumor-assoziierten Antigens auszeichnet.

4. Verfahren zur Diagnose einer Erkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, oder eines Teils davon, und/oder
(ii) den Nachweis des Tumor-assoziierten Antigens oder eines Teils davon, und/oder
(iii) den Nachweis eines Antikörpers gegen das Tumor-assoziierte Antigen oder eines Teils davon und/oder
(iv) den Nachweis von cytotoxischen oder Helfer-T-Lymphozyten, die für das Tumor-assoziierte Antigen oder einen Teil davon spezifisch sind,
in einer aus einem Patienten isolierten biologischen Probe,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

5. Verfahren zur Bestimmung der Regression, des Verlaufs oder des Ausbruchs einer Erkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, oder eines Teils davon,
(ii) der Menge des Tumor-assoziierten Antigens oder eines Teils davon,
(iii) der Menge an Antikörpern, die an das Tumor-assoziierte Antigen oder einen Teil davon binden, und
(iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

6. Antikörper, der spezifisch an ein Protein oder Polypeptid oder an einen Teil davon bindet, wobei das Protein oder Polypeptid von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

7. Pharmazeutische Zusammensetzung nach Anspruch 1 oder Antikörper nach Anspruch 6, wobei der Antikörper mit einem therapeutischen oder diagnostischen Mittel gekoppelt ist.

8. Antikörper nach Anspruch 7 zur Verwendung in einem Verfahren zur Behandlung, Diagnose oder Überwachung einer Erkrankung, die sich durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, l, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

9. Antikörper nach Anspruch 7 zur Verwendung in einem Verfahren zur Behandlung Diagnose oder Überwachung eines metastasierenden Tumors, der sich durch die Expression oder abnormaler Expression eines Tumorantigens auszeichnet, wobei das Verfahren die Verabreichung des Antikörpers umfasst, der Antikörper an das Tumorantigen oder einen Teil davon bindet und das Tumorantigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

10. Pharmazeutische Zusammensetzung nach Anspruch 1 oder 7 oder Antikörper nach einem der Ansprüche 6 bis 9, wobei der Antikörper ein monoklonaler, chimärer oder humanisierter Antikörper oder ein Fragment eines Antikörpers ist.

11. Cytolytische oder Cytokine-ausschüttende T-Zellen zur Verwendung in einem Verfahren zur Behandlung eines Patienten mit einer Erkrankung, die sich durch die Expression oder abnormale Expression eines Tumor-assoziierten Antigens auszeichnet, umfassend:
(i) die Entfernung einer Probe mit immunreaktiven Zellen aus dem Patienten,
(ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
(iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

12. Cytolytische oder Cytokine-ausschüttende T-Zellen nach Anspruch 11, wobei die Wirtszelle ein HLA-Molekül rekombinant oder endogen exprimiert, das an das Tumor-assoziierte Antigen oder einen Teil davon bindet.

13. Pharmazeutische Zusammensetzung nach Anspruch 3, Verfahren nach Anspruch 4 oder 5, Antikörper nach einem der Ansprüche 8 bis 10, oder cytolytische oder Cytokine-ausschüttende T-Zellen nach Anspruch 11 oder 12, wobei die Erkrankung Krebs ist.

14. Pharmazeutische Zusammensetzung nach Anspruch 3, Verfahren nach Anspruch 4 oder 5, Antikörper nach einem der Ansprüche 8 bis 10, oder cytolytische oder Cytokine-ausschüttende T-Zellen nach Anspruch 11 oder 12, wobei die Erkrankung ein Colon-, Rektal-, Nieren-, Brust-, Prostata-, Gebärmutter-, Ovarial-, Endometrial-, Speiseröhren-, Blut-, Leber-, Pankreas-, Haut-, Gehirn- oder Lungenkrebs, ein Lymphom oder Neuroblastom, ein Lungen-, Brust-, Prostata-, Colontumor, Nierenzell-, Zervix-, Colon- oder Mammakarzinom oder Metastasen der vorstehenden Krebsarten oder Tumore ist.

15. Pharmazeutische Zusammensetzung nach einem der Ansprüche 1 bis 3, 7, 10, 13 und 14, Verfahren nach einem der Ansprüche 4, 5, 13 und 14, Antikörper nach einem der Ansprüche 6 bis 10, 13 und 14, oder cytolytische oder Cytokine-ausschüttende T-Zellen nach einem der Ansprüche 11 bis 14, wobei das Protein oder Polypeptid oder das Tumor-assoziierte Antigen eine Aminosäuresequenz umfasst, die aus der Gruppe ausgewählt ist, bestehend aus SEQ ID NOs: 90, 2, 6, 10, 14, 18, 22, 26, 30, 34, 38, 42, 46, 50, 54, 58, 62, 66, 70, 74, 78, 82, 86, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.

16. Nukleinsäure, die für ein Protein oder Polypeptid kodiert, das eine Aminosäuresequenz umfasst, die aus der Gruppe ausgewählt ist, bestehend aus SEQ ID NOs: 90, 2, 6, 10, 14, 18, 22, 26, 30, 34, 38, 42, 46, 50, 54, 58, 62, 66, 70, 74, 78, 82, 86, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon.

17. Nukleinsäure nach Anspruch 16, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, 1, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

18. Wirtszelle, die eine Nukleinsäure nach Anspruch 16 oder 17 umfasst.

19. Protein oder Polypeptid, das von einer Nukleinsäure nach Anspruch 16 oder 17 kodiert wird, oder ein immunogenes Fragment des Proteins oder Polypeptids.

20. Protein oder Polypeptid, das eine Aminosäuresequenz umfasst, die aus der Gruppe ausgewählt ist, bestehend aus SEQ ID NOs: 90, 2, 6, 10, 14, 18, 22, 26, 30, 34, 38, 42, 46, 50, 54, 58, 62, 66, 70, 74, 78, 82, 86, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls, einem Teil oder Derivat davon, oder ein immunogenes Fragment des Proteins oder Polypeptids.

21. Kit zum Nachweis der Expression oder abnormalen Expression eines Tumor-assoziierten Antigens, umfassend Mittel zum Nachweis
(i) der Nukleinsäure, die für das Tumor-assoziierte Antigen kodiert, oder eines Teils davon,
(ii) des Tumor-assoziierten Antigens oder eines Teils davon,
(iii) von Antikörpern, die an das Tumor-assoziierte Antigen oder einen Teil davon binden, und/oder
(iv) von T-Zellen, die für einen Komplex zwischen dem Tumor-assoziierten Antigen oder einem Teil davon und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe ausgewählt ist, bestehend aus SEQ ID NOs: 89, l, 5, 9, 13, 17, 21, 25, 29, 33, 37, 41, 45, 49, 53, 57, 61, 65, 69, 73, 77, 81, 85, 93, 97, 101, 105, 109, 113, 117, 121, 125, 129, 133, 137, 141, 145, 149, 153, 157, 161, 165, 169, 173, 175, 179, 183, 187, 191, 195, 199, 203, 207, 211, 215, 219, 223, 227, 231, 235, 239, 243, 247, 251, 255, 259, 263, 267, 269, 271, 273, 275, 277, 279, 309 des Sequenzprotokolls, einem Teil oder Derivat davon,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

22. Pharmazeutische Zusammensetzung, die RNA Interferenz (RNAi) umfasst, die die Expression oder Aktivität der Tumorantigene gemäß SEQ ID NOs: 90, 2, 6, 10, 14, 18, 22, 26, 30, 34, 38, 42, 46, 50, 54, 58, 62, 66, 70, 74, 78, 82, 86, 94, 98, 102, 106, 110, 114, 118, 122, 126, 130, 134, 138, 142, 146, 150, 154, 158, 162, 166, 170, 174, 176, 180, 184, 188, 192, 196, 200, 204, 208, 212, 216, 220, 224, 228, 232, 236, 240, 244, 248, 252, 256, 260, 264, 268, 270, 272, 274, 276, 278, 280 bis 308, 310 des Sequenzprotokolls hemmt.

23. Pharmazeutische Zusammensetzung nach Anspruch 22, wobei die RNAi eine short hairpin Struktur (shRNA) enthält.
